# EUROPEAN PATENT SPECIFICATION

(11) **EP 3 692 152 B1**
(45) Date of publication and mention of the grant of the patent: **03.09.2025**
(21) Application number: 18863974.4
(22) Date of filing: 04.10.2018
(51) Int. Cl.: C12N 15/09, C12N 15/113, C12Q 1/68

(54) **METHODS AND COMPOSITIONS FOR ALTERING FUNCTION AND STRUCTURE OF CHROMATIN LOOPS AND/OR DOMAINS**
VERFAHREN UND ZUSAMMENSETZUNGEN ZUR VERÄNDERUNG DER FUNKTION UND STRUKTUR VON CHROMATINSCHLEIFEN UND/ODER -DOMÄNEN
PROCÉDÉS ET COMPOSITIONS PERMETTANT DE MODIFIER LA FONCTION ET LA STRUCTURE DE BOUCLES ET/OU DE DOMAINES DE CHROMATINE

(30) Priority: 04.10.2017 US 201762568306 P
(43) Date of publication of application: 12.08.2020
(73) Proprietor: The Broad Institute, Inc., Cambridge, MA 02142 (US); Baylor College of Medicine, Houston, TX 77030 (US)
(72) Inventor: RAO, Suhas S.P., Houston, Texas 77030 (US); AIDEN, Erez Lieberman, Houston, Texas 77030 (US); HUANG, Su-Chen, Houston, Texas 77030 (US); LANDER, Eric S., Cambridge, Massachusetts 02142 (US)
(74) Representative: Impact Intellectual Property LLP
(86) International application number: PCT/US2018/054476
(87) International publication number: WO 2019/071054

(56) References cited:
- WO-A1-2016/089920
- WO-A1-2017/031370
- WO-A1-2017/031370
- US-A1- 2014 287 932
- ELPHÈGE P. NORA ET AL: "Targeted Degradation of CTCF Decouples Local Insulation of Chromosome Domains from Genomic Compartmentalization", CELL, vol. 169, no. 5, 1 May 2017 (2017-05-01), Amsterdam NL, pages 930 - 944.e22, XP055588991, ISSN: 0092-8674, DOI: 10.1016/j.cell.2017.05.004
- GHIRLANDO RODOLFO ET AL: "CTCF: making the right connections", 15 April 2016 (2016-04-15), pages 1 - 11, XP055821900, Retrieved from the Internet <URL:https://www.ncbi.nlm.nih.gov/pmc/articles/PMC4840295/pdf/881.pdf> [retrieved on 20210707], DOI: 10.1101/gad.277863
- SHIN HA YOUN ET AL: "CRISPR/Cas9 targeting events cause complex deletions and insertions at 17 sites in the mouse genome", NATURE COMMUNICATIONS, vol. 8, no. 1, 1 August 2017 (2017-08-01), XP055821911, Retrieved from the Internet <URL:https://www.ncbi.nlm.nih.gov/pmc/articles/PMC5460021/pdf/ncomms15464.pdf> DOI: 10.1038/ncomms15464
- HANSSEN LARS L. P. ET AL: "Tissue-specific CTCF-cohesin-mediated chromatin architecture delimits enhancer interactions and function in vivo", NATURE CELL BIOLOGY, vol. 19, no. 8, 1 August 2017 (2017-08-01), GB, pages 952 - 961, XP055821908, ISSN: 1465-7392, Retrieved from the Internet <URL:https://www.ncbi.nlm.nih.gov/pmc/articles/PMC5540176/pdf/emss-73118.pdf> DOI: 10.1038/ncb3573
- SKIBBENS ROBERT V: "Correction: Of Rings and Rods: Regulating Cohesin Entrapment of DNA to Generate Intra- and Intermolecular Tethers", PLOS GENETICS, 1 December 2016 (2016-12-01), United States, pages e1006478 - e1006478, XP055821989, Retrieved from the Internet <URL:https://www.ncbi.nlm.nih.gov/pmc/articles/PMC5082857/pdf/pgen.1006337.pdf> [retrieved on 20210707], DOI: 10.1371/journal.pgen.1006478
- RAO SUHAS S P ET AL: "Cohesin Loss Eliminates All Loop Domains", vol. 171, no. 2, 5 October 2017 (2017-10-05), pages 305 - 320, e24, XP085207525, ISSN: 0092-8674, Retrieved from the Internet <URL:https://www.sciencedirect.com/science/article/pii/S0092867417311200?via%3Dihub> [retrieved on 20171005], DOI: 10.1016/J.CELL.2017.09.026
- NORA ET AL.: "Targeted Degradation of CTCF Decouples Local Insulation of Chromosome Domains from Genomic Compartmentalization", CELL, vol. 169, 18 May 2017 (2017-05-18), pages 930 - 944, XP055588991
- ZUIN ET AL.: "Cohesln and CTCF differentially affect chromatin architecture and gene expression in human cells", PROC NATL ACAD SCI USA, vol. 111, 13 December 2013 (2013-12-13), pages 996 - 1001, XP055352674, DOI: doi:10.1073/pnas.1317788111
- RAO ET AL.: "Cohesin Loss Eliminates All Loop Domains", CELL, vol. 171, 5 October 2017 (2017-10-05), pages 305 - 320,e24, XP085207525
- CERMAKOVA ET AL.: "Next-Generation Drugs and Probes for Chromatin Biology: From Targeted Protein Degradation to Phase Separation", MOLECULES, vol. 23, no. 8, 6 August 2018 (2018-08-06), pages 1 - 26, XP055589000, DOI: 10.3390/molecules23081958
- RAO ET AL.: "A 3D map of the human genome at kilobase resolution reveals principles of chromatin looping", CELL, vol. 159, no. 7, 11 December 2014 (2014-12-11), pages 1665 - 1600, XP055432114, DOI: 10.1016/j.cell.2014.11.021
- SANBORN ET AL.: "Chromatin extrusion explains key features of loop and domain formation in wild-type and engineered genomes", PROC NATL ACAD SCI USA, vol. 112, no. 47, 23 October 2015 (2015-10-23), pages E6456 - E6465, XP055365407

## Description

### STATEMENT REGARDING FEDERALLY SPONSORED RESEARCH

This invention was made with government support under Grant Nos. PHY-1427654 granted by the National Science Foundation, OD008540, HG006193, HL130010 and HG009375 granted by the National Institutes of Health. The government has certain rights in the invention.

### REFERENCE TO AN ELECTRONIC SEQUENCE LISTING

### TECHNICAL FIELD

The present invention is in the field of genetic engineering and medicine. The present invention provides methods and tools for altering chromatin four-dimensional (4D) structure in a cell, in particular chromatin loop formation and structure over time. The present invention allows the altering the transcriptional activity of chromatin domains or genomic loci, including such domains and loci associated with a disease, such as cancer or a genetic disease, through use of such methods and tools. Also described herein but not specifically claimed aremethods of treatment comprising altering chromatin 3D structure or gene expression within a chromatin domain. The present invention further provides methods of modulating chromatin loop formation to thereby interfere with higher-order chromatin structure, and ultimately control gene expression.

### BACKGROUND

It has been suggested that the three-dimensional structure of nucleic acids in a cell may be involved in complex biological regulation, for example compartmentalizing the nucleus and bringing widely separated functional elements into close spatial proximity. Understanding how nucleic acids interact, and perhaps more importantly how this interaction, or lack thereof, regulates cellular processes, presents a new frontier of exploration. For example, understanding chromosomal folding and the patterns therein can provide insight into the complex relationships between chromatin structure, gene activity, and the functional state of the cell. Adding ribonucleic acids (RNAs) into the mix adds a further complexity.

Typically, deoxyribonucleic acid (DNA) is viewed as a linear molecule, with little attention paid to the three-dimensional organization. However, chromosomes are not rigid, and while the linear distance between two genomic loci need may be vast, when folded, the special distance may be small. For example, while regions of chromosomal DNA may be separated by many megabases, they can also can be immediately adjacent in 3-dimensional space. Much the same way a protein can fold to bring sequence elements together to form an active site, from the standpoint of gene regulation, long-range interactions between genomic loci may for the same sort of active centers. For example, gene enhancers, silencers, and insulator elements might function across vast genomic distances.

The existence of long-range interactions complicates efforts to understand the pathways that regulate cellular processes, because the interacting regulatory elements could lie at a great genomic distance from a target gene, even on another chromosome. In the case of oncogenes and other disease-associated genes, identification of long-range genetic regulators would be of great use in identifying the genomic variants responsible for the disease state and the process by which the disease state is brought about.

The roughly two meters of DNA in the human genome is intricately packaged to form the chromatin and chromosomes in each cell nucleus. In addition to its structural role, this organization has critical regulatory functions. In particular, the formation of loops in the human genome plays an essential role in regulating genes. Applicants herein demonstrate the ability to create reliable maps of these loops, using an in situ Hi-C method for three-dimensional genome sequencing, and to control the formation of such loops, thereby altering gene expression. Hi-C characterizes the three-dimensional configuration of the genome by determining the frequency of physical contact between all pairs of loci, genome-wide.

In order to control the regulatory function of chromatin folding, it would be required to provide methods for altering chromatin three dimensional (3D) structure in a cell, to remove or otherwise modify existing chromatin loop structures, or to introduce new chromatin loop structures where their presence is required or beneficial, for instance, in the context of treatment of disease conditions, such as cancer or genetic disease. However, to date, no such methods exist. The present invention aims to provide essential methods and tools for altering chromatin three dimensional (3D) structure.

In order to associate the dynamics of chromatin loop structure to cellular processes in health and disease, the chromatin three dimensional (3D) structure from a large number of cells in different stages of development, from diseased and healthy subjects, and from a wide variety of cellular lineages and biological species need to be analysed and their genomes sequenced. Such studies are hampered by costs. There is therefore a need for further improvements in methods for *de novo* assembly of whole genomes and genomic fragments. The present invention aims to provide such improved methods.

Further, while existing methods for assessing chromatin three dimensional (3D) structure are very suitable for indicating that two loci are spatially co-localized in the nucleus, it may be expected that there are multiple loci spatially co-localized in a living cell. Yet, methods that can indicate simultaneous co-localization of more than 2, such as up to 10 or more different loci are not available. The present invention aims to provide such methods.

Many studies have shown that the insulator protein CTCF and the ring-shaped cohesin complex colocalize on chromatin (Wendt et al., 2008) and lie at the anchors of loops (Rao et al., 2014; Splinter et al., 2006) and the boundaries of contact domains (also called "topologically constrained domains", "topologically associated domains", or "physical domains") (Dixon et al., 2012; Lieberman-Aiden et al., 2009; Nora et al., 2012; Rao et al., 2014). This suggests that these proteins help regulate genome folding (Merkenschlager and Nora, 2016). Consistent with this, deletion of CTCF sites interferes with loop and contact domain formation (Guo et al., 2015; Sanborn et al., 2015; de Wit et al., 2015). However, initial, low-resolution experiments examining genome-wide depletion of CTCF and cohesin observed only limited effects, reporting that compartments and contact domains still appear to be present (Seitan et al., 2013; Sofueva et al., 2013; Zuin et al., 2014). These results have made it difficult to ascertain the role of CTCF and cohesin in regulating genome architecture.
Nora et al., 2017 (Targeted degradation of CTCF decouples local insulation of chromosome domains from genomic compartmentalization. Cell. Vol. 169, no. 5, pages 930-9441 May 2017) describe the influence of dose-dependent CTCF on looping between CTCF target sites and insulation of topologically associating domains (TADs).

Thus new methods are needed to examine the effects of cohesin loss on nuclear architecture, epigenetic state, and transcription.

### SUMMARY

In one aspect, the present invention provides an *in vitro* or *ex vivo* method of reversibly eliminating chromatin loops in a cell comprising:
contacting the cell with an agent capable of reducing expression, function or activity of one or more members of the cohesin complex,
wherein the cells comprise an inducible degradation system,
further wherein the one or more members of the cohesin complex proteins are tagged with an inducible degradation molecule and the agent induces reversible degradation of the tagged protein.

In certain embodiments, the one or more members of the cohesin complex are selected from the group consisting of Rad21, SA1/2, Smc3 and Smc1.

In certain embodiments, the cells comprise an inducible degradation system, wherein the CTCF protein or one or more members of the cohesin complex proteins are tagged with an inducible degradation molecule and the agent induces reversible degradation of the tagged protein. In certain embodiments, the degradation system is an inducible degron system wherein the target protein is fused to an auxin-inducible degron and the agent is auxin.

In certain embodiments, the agent is a small molecule or a genetic modifying agent. In certain embodiments, the agent comprises a degrader molecule. In certain embodiments, the degrader molecule is a PROTAC molecule. In certain embodiments, the genetic modifying agent comprises a Cas13 system or RNAi.

In another aspect (described but not specifically claimed herein), a method of modulating one or more superenhancers that co-localize and form links within and across chromosomes in a cell comprising contacting the cell with one or more agents capable of targeting the one or more superenhancers is provided. In certain embodiments, cohesin dependent loops are eliminated in the cell according to any embodiment herein.

In certain embodiments, the agent is a small molecule or a genetic modifying agent. In certain embodiments, the small molecule is targeted to the one or more superenhancers in a sequence dependent manner. In certain embodiments, the small molecule is targeted to a superenhancer with a pyrrole-imidazole polyamide. In certain embodiments, the small molecule is selected from the group consisting of a histone deacetylase (HDAC) inhibitor, a bromodomain containing protein inhibitor and 1,6-hexanediol. In certain embodiments, the HDAC inhibitor is selected from the group consisting of vorinostat, givinostat, panobinostat, belinostat, entinostat, CG-1521, romidepsin, ITF-A, ITF-B, valproic acid, OSU-HDAC-44, HC-toxin, magnesium valproate, plitidepsin, tasquinimod, sodium butyrate, mocetinostat, carbamazepine, SB939, CHR-2845, CHR-3996, JNJ-26481585, sodium phenylbutyrate, pivanex, abexinostat, resminostat, dacinostat, droxinostat, RGFP966, and trichostatin A (TSA). In certain embodiments, the bromodomain containing protein inhibitor is selected from the group consisting of AZD5153, JQ1, PFI-1, CPI-203, CPI-0610, RVX-208, OTX015, I-BET151, I-BET762, I-BET-726, dBET1, ARV-771, ARV-825, BETd-260/ZBC260 and MZ1. In certain embodiments, the genetic modifying agent comprises a CRISPR system, a zinc finger nuclease system or a TALE system. In certain embodiments, the genetic modifying agent comprises a functional domain. In certain embodiments, the functional domain comprises a histone acetyltransferase (HAT) or HDAC.

In another aspect (described but not specifically claimed herein), a method for determining chromatin loops independent of cohesin or CTCF comprising: contacting chromatin with a cohesin or CTCF reducing or degrading agent or causing cohesin-dependent loop domains to diminish or be eliminated, and measuring remaining chromatin loops to thereby ascertain cohesin-independent chromatin loops is provided.

In another aspect (described but not specifically claimed herein), a method for genome and expression analysis comprising dividing a population of cells into a first portion of cells and a second portion of cells; determining cohesin-independent chromatin loops in the first portion of cells; measuring gene expression of the second portion of cells; and correlating the cohesin-independent chromatin loops and gene expression measurements is provided.

In certain embodiments, the determining cohesin-independent chromatin loops comprises: contacting chromatin with a cohesin- reducing or degrading agent or causing cohesin-dependent loop domains to diminish or be eliminated; and measuring remaining chromatin loops to thereby ascertain cohesin-independent chromatin loops. In certain embodiments, the cohesin- reducing or degrading agent or causing cohesin-dependent loop domains to diminish or be eliminated comprises treating with auxin. In certain embodiments, measuring chromatin loops comprises a process that combines DNA-DNA proximity ligation and high throughput screening or in situ Hi-C. In certain embodiments, gene expression is measured using RNA-Seq or L1000.

In another aspect (described but not specifically claimed herein), a method for genome and expression analysis comprising: dividing a population of cells into a first portion of cells and a second portion of cells; creating a map showing frequency of physical contact between pairs of loci across the genome with the first portion of cells; measuring gene expression of the second portion of cells; and correlating the map and gene expression measurements is provided. In certain embodiments, the method further comprises treating the population of cells ahead of the dividing step. In certain embodiments, the treating comprises reducing or degrading CTCF or one or more members of the cohesin complex or causing loop domains to diminish or be eliminated. In certain embodiments, CTCF or one or more members of the cohesin complex are tagged with an inducible degron system and treating is with auxin. In certain embodiments, creating the map comprises a process that combines DNA-DNA proximity ligation and high throughput screening or in situ Hi-C. In certain embodiments, gene expression is measured using RNA-Seq or L1000.

In another aspect, (described but not specifically claimed herein) a method for measuring superenhancers that co-localize and form links within and across chromosomes, comprising contacting chromatin with a cohesin- reducing or degrading agent or causing cohesin-dependent loop domains to diminish or be eliminated, measuring remaining superenhancers that co-localize and form links within and across chromosomes is provided.

In certain embodiments, the method of any of the preceding embodiments further comprises measuring the rate of cohesin independent loop formation after contacting or treating with an agent capable of reducing expression, function or activity of CTCF or one or more members of the cohesin complex.

In certain embodiments, the method of any of the preceding embodiments further comprises withdrawing the cohesin- reducing or degrading agent or ceasing causing cohesin-dependent loop domains to diminish or be eliminated, and measuring rate of loop reforming after withdrawal.

In certain embodiments, the method further comprises performing gene expression and a process that combines DNA-DNA proximity ligation and high throughput screening or in situ Hi-C, thereby reforming loops and observing gene expression change.

In certain embodiments, the method further comprises introducing a small molecule or protein into a population of cells; and measuring the rate of cohesin independent loop forming after contacting or treating with the cohesin- reducing or degrading agent.

In certain embodiments, the method further comprises contacting or treating a population of cells with an agent to reduce or degrade cohesin; introducing a small molecule or protein into the population of cells; withdrawing the cohesin- reducing or degrading agent or ceasing causing cohesin-dependent loop domains to diminish or be eliminated; and measuring the rate of loop reforming after withdrawal, thereby assessing the effect of a small molecule or protein on the rate of loop formation.

In certain embodiments, the method further comprises screening a library of small molecules or proteins to identify candidates that inhibit or promote loop formation. In certain embodiments, the protein comprises a genetic modifying agent. In certain embodiments, the small molecule is selected from the group consisting of flavopiridol, thymidine, hydroxyurea, oligomycin, JQ1, and 1-6 hexanediol.

In another aspect (described but not specifically claimed herein), a method of identifying loops associated with gene expression comprising: temporarily eliminating cohesion in a population of cells; determining loop formation and gene expression at one or more time points after cohesion recovery; and associating loop formation and gene expression over time. In certain embodiments, the population of cells comprises tumor cells is provided. In certain embodiments, loops affecting expression of genes associated with a disease are identified.

In another aspect (described but not specifically claimed herein a method of blocking the extrusion complex and loop formation at a specific genomic locus comprising recruiting two or more enzymatically inactive CRISPR enzymes to at least one loop anchor of a pair of convergent loop anchors is provided. In certain embodiments, the CRISPR enzyme is dCas9. In certain embodiments, at least 3, preferably 7 dCas9s are recruited to the loop anchor. In certain embodiments, the pair of convergent loop anchors is a pair of convergent CTCF binding sites.

In another aspect (described but not specifically claimed herein), a method for identifying exogenous proteins that can complement loss of a target protein required for chromatin loop formation comprising: contacting chromatin with a reducing or degrading agent for the target protein; introducing an exogenous protein; and measuring chromatin loops to ascertain whether the exogenous protein complements the loss of the target protein is provided. In certain embodiments, the target protein is CTCF or a member of the cohesin complex. In certain embodiments, the agent is a small molecule or a genetic modifying agent. In certain embodiments, the small molecule induces degradation via an inducible degron fused to the target protein. In certain embodiments, the small molecule is auxin and the target protein is fused to an auxin-inducible degron. In certain embodiments, the exogenous protein is introduced transiently on an expression plasmid or is stably introduced by way of an integrated gene. In certain embodiments, measuring chromatin loops comprises a process that combines DNA-DNA proximity ligation and high throughput screening or in situ Hi-C. In certain embodiments, the method further comprises screening a library of exogenous proteins to identify candidates that can complement loss of the target protein in target protein-dependent chromatin loop formation. In certain embodiments, the library of exogenous proteins comprises a plurality of point and/or deletion mutants of the target protein. In certain embodiments, the exogenous protein comprises a mutant of the target protein associated with a disease. In certain embodiments, the disease is cancer.

Described but not specifically claimed herein is a method for determining cohesin-independent chromatin loops comprising: contacting chromatin with a cohesin- reducing or degrading agent or causing cohesin-dependent loop domains to diminish or be eliminated, and measuring the remaining chromatin loops to thereby ascertain cohesin-independent chromatin loops.

Described but not specifically claimed herein is a method for genome and expression analysis comprising dividing a population of cells into a first portion of cells and a second portion of cells; determining cohesin-independent chromatin loops in the first portion of cells; and measuring gene expression of the second portion of cells; and correlating the cohesin-independent chromatin loops and gene expression measurements.

Described but not specifically claimed herein is a method of identifying loops associated with gene expression comprising temporarily eliminating cohesion in a population of cells; determining loop formation and gene expression at one or more time points after cohesion recovery; and associating loop formation and gene expression over time.

The population of cells in the methods can comprise tumor cells.

The loops affecting expression of genes associated with a disease can be identified using methods herein.

Determining cohesin-independent chromatin loops can comprise: contacting chromatin with a cohesin- reducing or degrading agent or causing cohesin-dependent loop domains to diminish or be eliminated, measuring remaining chromatin loops to thereby ascertain cohesin-independent chromatin loops.

A cohesin- reducing or degrading agent or causing cohesin-dependent loop domains to diminish or be eliminated can comprise treating with auxin.

Any of the methods can have creating the map comprising a process that combines DNA-DNA proximity ligation and high throughput screening or in situ Hi-C.

Gene expression can be measured using RNA-Seq, Perturb-Seq, or L1000.

Described but not specifically claimed herein is a method for genome and expression analysis comprising dividing a population of cells into a first portion of cells and a second portion of cells; creating a map showing frequency of physical contact between pairs of loci across the genome with the first portion of cells measuring gene expression of the second portion of cells; and correlating the map and gene expression measurements.

The methods can include treating the population of cells ahead of the dividing step.

The treating can comprise reducing or degrading cohesin or causing loop domains to diminish or be eliminated. The treating can be with auxin.

Creating the map comprises a process that combines DNA-DNA proximity ligation and high throughput screening or in situ Hi-C. Gene expression can be measured using RNA-Seq, Perturb-Seq, or L1000.

Described but not specifically claimed herein is a method for measuring superenhancers that co-localize and form links within and across chromosomes, comprising contacting chromatin with a cohesin- reducing or degrading agent or causing cohesin-dependent loop domains to diminish or be eliminated, measuring remaining superenhancers that co-localize and form links within and across chromosomes.

The methods can further comprise measuring rate of cohesin independent loop forming after contacting or treating. The methods can yet further comprise withdrawing cohesin- reducing or degrading agent or ceasing causing cohesin-dependent loop domains to diminish or be eliminated, and measuring rate of loop reforming after withdrawal. The methods can also further comprising performing gene expression and a process that combines DNA-DNA proximity ligation and high throughput screening or in situ Hi-C, thereby reforming loops and observing gene expression change.

In one embodiment, the present invention provides a method to engineer chromatin loops and contact domains in a target region of chromatin DNA inside the nucleus of a cell, said method comprising the step of interfering with the function of CTCF and/or cohesin during the extrusion process wherein chromatin DNA is extruded by each of the two subunits of a CTCF and/or cohesin-comprising extrusion complex in opposite direction with respect to the genome and halted by a forward and reverse CTCF or cohesin binding motif in convergent orientation on opposite strands of the extruded chromatin DNA.

In one embodiment of the method of the invention, the interfering results in the removal of one or more existing chromatin loops or contact domains, the introduction of one or more new chromatin loops or contact domains, or the modification of one or more existing loops or contact domains.

In one embodiment of the method of the invention, the removal of one or more existing chromatin loops or contact domains comprises the targeted removal or modification of one or more existing forward and/or reverse CTCF or cohesin binding motifs in or proximate to said target region.

In one embodiment of the method of the invention, the introduction of one or more new chromatin loops or contact domains comprises the targeted introduction of one or more new forward and/or reverse CTCF or cohesin binding motifs in or proximate to said target region.

In one embodiment of the method of the invention, the modification of one or more existing loops or contact domains comprises the targeted introduction of one or more new forward and/or reverse CTCF or cohesin binding motifs.

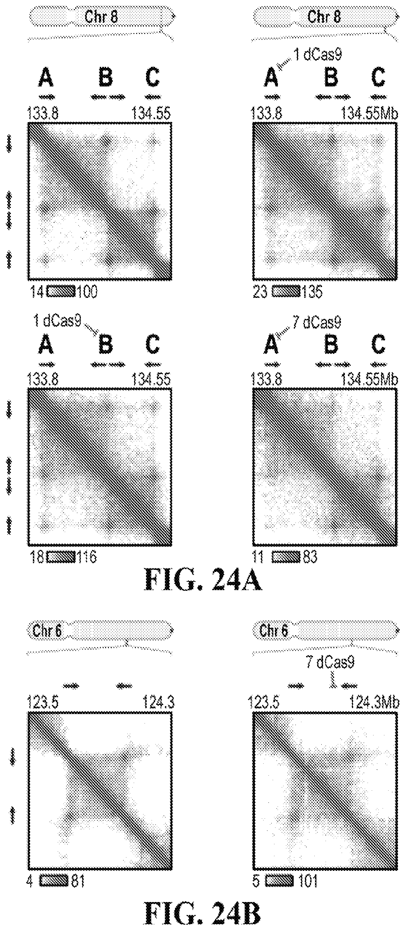

In one embodiment of the method of the invention, the modification of one or more existing loops or contact domains comprises the targeted introduction of one or more extrusion-blocking proteins or protein-binding sites in or proximate to said target region to thereby prevent or attenuate the extrusion of at least one chromatin strand through the extrusion complex whereby a smaller loop is formed or a loop is blocked from forming, preferably said introduction being in a location between the forward and reverse CTCF or cohesin binding motifs at an existing loop or contact domain boundary, more preferably in a location within 150,000 base pairs, 125,000 base pairs, 100,000 base pairs, 90,000 base pairs, 80,000 base pairs, 70,000 base pairs, 60,000 base paris, 50,000 base pairs, 40,000 base pairs, 30,000 base pairs, 20,000 base pairs, 10,000 base pairs, 9,000 base pairs, 8,000 base paris, 7,000 base pairs, 6,000 base pairs, 5,000 base pairs, 4,000 base pairs, 3,000 base pairs, 2,000 base pairs, 1000 base pairs, 900 base pairs, 800 base pairs, 700 base pairs, 600 base pairs, 500 base pairs, 400 base pairs, 300 base pairs, 200 base pairs, 100 base pairs, 50 base pairs, 25 base pairs, 10 base paris, or 5 base pairs of an existing forward CTCF or cohesin binding motif. See Fig. 24A.

In one embodiment of the invention, extrusion-blocking proteins or protein binding sites may be introduced upstream or downstream of an existing CTCF or cohesin binding motif in order to introduce a new loop anchor to which a new chromatin loop may form. In certain example embodiments, the distance from an existing CTCF or cohesin motif may be within 1,000 - 150,000 base pairs of an existing CTCF or cohesin domain, or any sub-range therebetween. The target sites for introduction of an extrusion-blocking protein or protein binding site will depend on the distance from an existing CTCT or cohesin domain. For example, if the extrusion-blocking protein is a dCa9 the corresponding gRNA will be based on the genomic distance located at the desired distance from the existing CTCF or cohesin domain.

In one embodiment of the method of the invention, the removal of one or more contact domains comprises the targeted removal or modification of one or more, preferably all, CTCF or cohesin binding motifs located at the contact domain boundary.

In one embodiment of the method of the invention, the introduction of one or more new contact domains comprises the targeted introduction of one or more new forward and/or reverse CTCF or cohesin binding motifs in or proximate to said target region to thereby create two consecutive CTCF or cohesin binding motifs that do not loop to one another.

In one embodiment of the method of the invention, the targeted removal or modification comprises the mutation or inversion of said one or more CTCF or cohesin binding motifs, preferably wherein said targeted removal or modification comprises the mutation of at least a single base pair in said one or more CTCF binding motifs.

In one embodiment of the method of the invention, the targeted introduction comprises the introduction of one or more CTCF or cohesin binding motifs, preferably in convergent orientation on opposite strands of the chromatin DNA.

In one embodiment of the method of the invention, the targeted removal, modification or introduction comprises genome editing.

In one embodiment of the method of the invention, the targeted removal, modification or introduction comprises the use of a CRISPR/Cas system, an inactivate CRISPR/Cas system, a Cas protein, a zinc finger protein (ZFP), a zinc finger nuclease (ZFN), a transcription activator-like effector (TALE), a transcription activator-like effector nuclease (TALEN), or a meganuclease.

In one embodiment of the method of the invention, the CTCF or cohesin binding motif is the CTCF motif.

In one embodiment of the method of the invention, the domain is an exclusion domain, and wherein said exclusion domain is introduced by inserting, a CTCF or cohesin binding motif downstream or upstream from an adjacent CTCF or cohesin binding motifs in convergent orientation. In one embodiment of the method of the invention, the domain is an exclusion domain and wherein said exclusion domain is deleted by deleting a CTCF or cohesion binding motif downstream or upstream from an adjacent CTCF or cohesion binding motif, or inverting a CTCF or cohesion motif downstream or upstream of an adjacent CTCF such that the inverted CTCF or cohesion motif is not in a convergent orientation with the adjacent CTCF motif or cohesin motif.

In one embodiment of the method of the invention, in addition to the step of interfering with the function of CTCF and/or cohesin, said method comprises the step of performing *in situ* Hi-C on said cell prior to or following said step of interfering with the function of CTCF and/or cohesin, optionally combined with HYbrid Capture on the *in situ* Hi-C library generated.

In one embodiment of the method of the invention, the method is for altering chromatin three dimensional (3D) structure in a cell.

In one embodiment of the method of the invention, the method comprises delivering to a cell one or more sequence-specific DNA targeting agents directed to said target region or proximate thereto, preferably wherein said one or more sequence-specific DNA targeting agents are selected from the group consisting of a CRISPR/Cas system, a Cas protein, a catalytically inactive CRISPR-Cas system or Cas protein, a zinc finger protein (ZFP), a zinc finger nuclease (ZFN), a transcription activator-like effector (TALE), a transcription activator-like effector nuclease (TALEN), and a meganuclease. In certain example embodiment the one or more sequence-specific DNA targeting agents are delivered to the nucleus of the cell.

In one embodiment of the method of the invention, the target region comprises genes the expression of which is to be modified, preferably wherein said proximity to the target region is less than 2,000, 1,000, 900, 800, 700, 600, 500, 400, 300, 200, or 100 base pairs.

In one embodiment of the method of the invention, the target region is located in or overlaps with an existing chromatin loop or contact domain, or wherein said target region is to be formed into or is to be made part of a new chromatin loop or contact domain.

In one embodiment of the method of the invention, the delivering of the one or more sequence-specific DNA targeting agents to the nucleus of a cell comprises delivering one or more vectors encoding the one or more sequence-specific DNA targeting agents.

In one embodiment of the method of the invention, the delivering of the one or more sequence-specific DNA targeting agents comprises delivering a cell-permeable reagent, preferably a pyrrole-imidazole polyamide.

In one embodiment of the method of the invention, the one or more sequence-specific DNA targeting agents bind to and mask one or more existing CTCF or cohesin binding motifs such that an existing loop or contact domain is masked and a chromatin loop is attenuated or removed. In other example embodiments, the one or more sequence-specific DNA targeting agents bind to and mask one or more existing CTCF or cohesion binding motifs such that an extrusion complex is not arrested at the existing CTCF or cohesin binding motif thereby allowing the extrusion complex to arrest at a subsequent existing CTCF or cohesin binding motif. In certain example embodiments, the arresting at a subsequent existing CTCF results in formation of a new loop or contact domain and/or formation of a new chromatin loop anchored at the subsequent CTCF or cohesion binding motif.

In one embodiment of the method of the invention, the one or more sequence-specific DNA targeting agents comprise a DNA methyltransferase domain, wherein methylation of one or more existing CTCF or cohesin binding motifs masks the existing CTCF or cohesin binding motif preventing CTCF or cohesin from binding to the masked CTCF or cohesin binding motif, thereby preventing a loop or contact domain from forming at the masked CTCF or cohesin binding motif, preventing a chromatin loop anchored at the masked CTCF or cohesin motif from forming, or whereby an extrusion complex is not arrested at the existing CTCF or cohesin binding motif. In other example embodiments, the one or more sequence-specific DNA targeting agents comprise DNA demethyltransferase, wherein demethylation of one or more existing CTCF or cohesin binding motifs unmasks the existing CTCF or cohesin binding motif thereby allowing a loop or contact domain to form at the unmasked CTCF or cohesin binding motif, a loop anchored at the unmasked CTCF or cohesin binding motif to form, or an extrusion complex

In one embodiment of the method of the invention, the extrusion complex comprises one or more members selected from the group consisting of CTCF, SA1/2, Smc3, Smc1, cohesin and Rad21.

In one embodiment of the method of the invention, one or more members of the extrusion complex, or a part thereof, are fused to a sequence-specific DNA targeting agent as defined hereinabove, wherein biding of the sequence-specific DNA targeting agent to a target region results in formation of a a new chromatin loop anchor and/or new chromatin loop structure.

In one embodiment of the method of the invention, two or more multimerizable sequence-specific DNA targeting agents are targeted to two or more target regions in order to bring them into physical proximity.

In one embodiment of the method of the invention, the multimerizable sequence-specific DNA targeting agents comprise a catalytically inactive CRISPR-Cas system, a zinc finger protein (ZFP), or a transcription activator-like effector (TALE) fused to a dimerization domain.

In one embodiment of the method of the invention, the dimerization domain is inducible upon addition of a ligand.

In one embodiment of the method of the invention, the one or more sequence-specific DNA targeting agents comprises a site-specific nuclease.

In one embodiment of the method of the invention, the site-specific nuclease comprises a CRISPR-Cas system, a zinc finger nuclease (ZFN), or a transcription activator-like effector nuclease (TALEN).

In one embodiment of the method of the invention, the site-specific nuclease comprises a nickase.

In one embodiment of the method of the invention, the one or more agents comprise one or more recombination templates.

In one embodiment of the method of the invention, the one or more site-specific nucleases inserts one or more new CTCF or cohesin binding motifs or inverts an existing CTCF or cohesin binding motif upon binding to the one or more target regions, whereby a new pair of convergent CTCF or cohesin binding motifs is formed.

In one embodiment of the method of the invention, the site-specific nuclease inserts one or more convergent pairs of CTCF or cohesin binding motifs, whereby each convergent CTCF or cohesin binding motif pair generates a new chromatin loop structure.

In one embodiment of the method of the invention, the site-specific nuclease deletes one or more CTCF or cohesin binding motifs.

In one embodiment of the method of the invention, the site-specific nuclease inserts, deletes or substitutes one or more nucleotides in a loop binding motif.

In one embodiment of the method of the invention, the site-specific nuclease inserts an array of CTCF or cohesin binding motifs in a target chromosome, preferably wherein the array comprises between 10-100 copies of a CTCF or cohesin binding motif, so as to alter chromatin 3D structure at chromosome scale.

In one embodiment of the method of the invention, the array is a DXZ4 element.

In one embodiment of the method of the invention, the chromatin loop or contact domain is associated with an actively transcribed gene. In one embodiment of the method of the invention, modification or deletion of the chromatin loop anchor or chromatin loop structure results in preventing the mRNA splicing machinery associated with said actively transcribed gene from interacting with a transcription initiation complex, so as to alter mRNA splicing. In another example embodiment, modification or deletion of the chromatin loop anchor or chromatin loop structure results in allowing a mRNA splicing machinery associated with said actively transcribed gene to interact with a transcription initiation complex, so as to alter mRNA splicing. In certain other example embodiments, introduction of a new chromatin loop anchor or chromain loop structure results in allowing a mRNA splicing machinery to associate diwth an initiation complex of an actively transcribed genes, so as to alter mRNA splicing.

In one embodiment of the method of the invention, a different promoter/transcription start site is utilized, and/or whereby a different mRNA isoform is produced.

In one embodiment of the method of the invention, an enhancer element, silencer element or insulator element is insulated from or brought into contact with said chromatin loop or contact domain or with the promoter of said gene.

In one embodiment of the method of the invention, the method for altering chromatin domain activity comprises delivering to a cell or population of cells one or more sequence-specific DNA targeting agents directed to one or more target regions of chromatin DNA comprising an existing chromatin domain, wherein binding of the one or more DNA targeting agents to one or more target regions alters the transcriptional activity of a chromatin domain.

In one embodiment of such a method of the invention, the sequence-specific DNA targeting agent targets a DNA contact site opposite a promoter site in the chromatin domain.

In one embodiment of the method of the invention, the DNA contact site is at a CTCF or cohesin binding motif.

In one embodiment of the method of the invention, the sequence-specific DNA targeting agents comprise a transcription factor domain and a DNA targeting domain, whereby the transcription factor domain is brought into contact with a contact domain, or a proximity sufficient to allow for interaction with the chromatin domain.

In one embodiment of the method of the invention, the transcription factor domain is selected from the group consisting of an activator protein, a repressor protein, an elongation factor, and a histone modifying enzyme.

In one embodiment of the method of the invention, the histone modifying enzyme is selected from the group consisting of a DNA methyltransferase, a histone methyltransferase, a histone demethylase, histone deacetylase and a histone acetyltransferase.

In one embodiment of the method of the invention, the DNA targeting domain comprises a CRISPR-Cas system, a zinc finger protein (ZFP), or a transcription activator-like effector (TALE).

The method of any one of the preceding claims, wherein the one or more vectors are for delivery *in vivo.*

In one embodiment of the method of the invention, the the one or more sequence-specific DNA targeting agents are under the inducible control of a vector promoter.

In one embodiment of the method of the invention, the vector promoter is a tissue-specific promoter or a ubiquitous expression promoter.

In one embodiment of the method of the invention, the vector is a viral vector.

In one embodiment of the method of the invention, the viral vector is selected from the group consisting of lentiviral, adenoviral, adeno-associated viral, and herpes simplex virus vectors.

In one embodiment of the method of the invention, the CRISPR-Cas system is self-inactivating, whereby the self-inactivation of the CRISPR-Cas system limits duration of its activity and/or expression in targeted cells.

In one embodiment of the method of the invention, the target region is associated with a disease.

In one embodiment of the method of the invention, the disease associated with aberrant chromatin folding.

In one embodiment of the method of the invention, the disease is cancer, a genetic disease, or infectious disease.

In one embodiment of the method of the invention, the target region comprises an oncogene or tumor suppressor gene.

In one embodiment of the method of the invention, a target region associated with aberrant expression of an oncogene is targeted, whereby expression of the oncogene is repressed.

In one embodiment of the method of the invention, a target region associated with aberrant expression of a tumor suppressor is targeted, whereby expression of the tumor suppressor is activated.

In one embodiment of the method of the invention, the genetic disease selected from the disorders identified in Tables A B or C herein below.

In one embodiment of the method of the invention, the genetic disease is a disorder associated with genomic imprinting.

In one embodiment of the method of the invention, the imprinted gene is unsilenced.

In one embodiment of the method of the invention, the gene is silenced by establishing imprinting.

In one embodiment of the method of the invention, the target region comprises a virus integration site of an infectious virus, preferably wherein the virus is a retrovirus, an adenovirus, an adeno-associated virus (AAV), a lentivirus or a herpesvirus.

In one embodiment of the method of the invention, the target region is associated with improved yields, disease resistance, drought resistance or salt tolerance in plants or animals.

In one embodiment of the method of the invention, the cells or population of cells are part of a mammal.

In one embodiment of the method of the invention, the cells or population of cells are part of a plant.

Described but not specifically claimed herein is a method of treatment comprising altering chromatin 3D structure or gene expression within a chromatin domain according to any of the preceding methods in a subject in need thereof suffering from a disease associated with aberrant chromatin 3D structure or aberrant gene expression within a chromatin domain.

Described but not specifically claimed herein is a a method of treatment comprising altering chromatin 3D structure around an inserted therapeutic gene according to any of the preceding methods in a subject in need thereof, in order to ensure proper regulation of the inserted therapeutic gene and the surrounding endogenous genes.

Described but not specifically claimed herein is a method of treatment of the invention, the one or more vectors are delivered to the subject, wherein the one or more sequence-specific DNA targeting agents introduced by the one or more vectors corrects the aberrant loop chromatin 3D structure or aberrant gene expression within a chromatin domain.

In one embodiment of the method of treatment, one or more vectors are delivered to the subject suffering from a genetic defect such that the one or more sequence-specific DNA targeting agents introduced by the one or more vectors silences expression of one or more defective genes or rescues expression of one or more silenced functional genes.

In one embodiment of the method of treatment, one or more vectors are delivered to a subject suffering from a cancer such that the one or more sequence-specific DNA targeting agents introduced by the one or more vectors silences expression of one or more oncogenes or induces expression of one or more tumor suppressors.

In any and all embodiments of the methods the invention as described above, in addition to the step of interfering with the function of CTCF and/or cohesin, said method may comprise the step of performing *in situ* Hi-C on said cell prior to or following said step of interfering with the function of CTCF and/or cohesin, optionally combined with HYbrid Capture on the *in situ* Hi-C library generated, wherein said *in situ* HiC method identifies target chromatin loop modification sites or monitors the result of chromatin loop or contact domain modification in a target region, said method comprising performing prior to or following said step of interfering with the function of CTCF and/or cohesin the steps of generating a 3D contact map of the genome of said cell; identifying a target modification site from the 3D contact map, wherein the target modification site comprises either an existing loop or domain or a target nucleic acid sequence for introducing a new chromatin loop or domain, or identifying modified sites from the 3D contact map, wherein a modified site comprises a modified loop or domain.

In one embodiment of such combined methods of the invention, the method further comprises the steps of: generating a set of vectors wherein each vector encodes one or more chromatin loop perturbations, wherein expression of the one or more vectors results in removal of one or more existing chromatin loops or domains, introduction of one or more new chromatin loops or domains, or modification of one or more existing loops or domains at one of the identified target modification sites; delivering each vector in the set of vectors to a different cell or cell population to determine an impact of the introduced chromatin loop perturbations on cell function; and identifying one or more vectors that introduce the one or more chromatin perturbations with a minimal negative impact on cell function.

In a further embodiment of this method of the invention, cell function is assessed by changes in gene expression and/or changes in cell phenotype.

In another aspect (described but not specifically claimed herein), an agent for use as a medicament or for use in the treatment of a disorder in a human or animal subject in need thereof is provided, wherein said agent comprises one or more sequence-specific DNA targeting agents selected from the group consisting of a CRISPR-Cas system, a zinc finger protein (ZFP), a zinc finger nuclease (ZFN), a transcription activator-like effector (TALE), a transcription activator-like effector nuclease (TALEN), a catalytically inactive CRISPR-Cas system, and a self-inactivating CRISPR/Cas system, wherein binding of the sequence-specific DNA targeting agents to the one or more genomic loci removes one or more existing chromatin loop or domain structures, introduces one or more new chromatin loop or domain structures, or modifies one or more existing chromatin loop or domain structures in a cell of said subject.

In one embodiment of said aspect the agent introduces, masks, mutates or inverts one or more existing forward and/or reverse CTCF or cohesin binding motifs or prevents the extrusion of at least one chromatin strand through a CTCF and/or cohesin-comprising extrusion complex in said cell.

In one embodiment of said aspect the agent comprises a DNA-targeting element comprising a nucleotide sequence that hybridizes to one or more CTCF or cohesin binding motifs or to a DNA target region in said chromatin DNA proximate to a location where one or more CTCF or cohesin binding motifs are to be introduced into the genome.

In one embodiment of said aspect the agent comprises a DNA-targeting element comprising a zinc finger motif that binds to one or more CTCF or cohesin binding motifs or to a DNA target region in said chromatin DNA proximate to a location where one or more CTCF or cohesin binding motifs are to be introduced into the genome.

In one embodiment of said aspect the agent is encoded by a vector for delivering said agent to the nucleus of said cell.

In one embodiment of said aspect the vector is a viral vector.

In one embodiment of said aspect the viral vector is selected from the group consisting of lentiviral, adenoviral, adeno-associated viral, and herpes simplex virus vectors.

It is expressly foreseen that embodiments of the method of treatment as disclosed herein are also an embodiment of the agent for medical use as disclosed, including purposes, structures and diseases.

Further embodiments of this invention include a method to engineer chromatin loops and contact domains in a target region of chromatinized DNA inside the nucleus of a cell, said method comprising the step of modifying, adding, or removing a CTCF or cohesin binding motif. Preferable, in such an embodiment, only a single loop anchor or domain boundary is engineered.

Further embodiments of this invention include a method to engineer chromatin loops and contact domains in a target region of chromatin DNA inside the nucleus of a cell, said method comprising the step of interfering with the function of CTCF and/or cohesin.

Still further embodiments of this invention include a method to engineer chromatin loops and contact domains in a target region of chromatin DNA inside the nucleus of a cell, said method comprising the step of interfering with the function of CTCF and/or cohesin. Preferable, in such an embodiment, only a single loop anchor or domain boundary is engineered. Preferably, in such a method said interfering comprises interfering with a CTCF or cohesin binding motif. Preferably, interfering with a CTCF or cohesin binding motif comprises removing nucleotides, adding nucleotides, methylating nucleotides, and/or changing the orientation of all or part of the motif.

Alternatively, or in addition thereto, in embodiments of the methods described above, said interfering comprises adding a new CTCF or cohesin binding motif.

Alternatively, or in addition thereto, in embodiments of the methods described above, said said interfering comprises modifying the native CTCF or cohesin proteins.

Alternatively, or in addition thereto, in embodiments of the methods described above, said interfering comprises introducing modified CTCF or cohesin proteins.

Alternatively, or in addition thereto, in embodiments of the methods described above, said said interfering comprises introducing a protein which interferes with the normal function of CTCF. Preferably said protein is catalytically deactivated CRISPR/Cas protein, such as a catalytically deactivated Cas9 (dCas9). In certain example embodiments the dCas9 targets a CTCF or cohesin binding motif or a region proximate to a CTCF or cohesin motif using one or more guide RNAs. In one example embodiment, one or more gRNAs are used to tile a target region proximate to and/or including an existing CTCF or cohesin motif to cause binding of multiple dCas9s in the target region. In certain example embodiments, the gRNAs target a region within 10 to 5,000 base pairs of an existing CTCF or cohesin motif.

Still further embodiments of this invention include a non-naturally occurring or engineered composition comprising the agents described herein. In one preferred embodiment, wherein the agent is a nucleic acid molecule, said molecule is cloned into an expression vector.

Still further embodiments of this invention include a kit comprising the agents described herein, or the expression vector as described herein, and further comprising instructions for performing a method of the invention as described herein.

Still further embodiments of this invention include a composition as described herein comprising agent as described herein or the expression vector comprising the agent; and optionally one or more pharmaceutically acceptable excipients. In a preferred embodiment, said composition is for use in therapy.

Still further embodiments of this invention include an in vitro method of modifying chromatin loops or contact domains as described herein in a target region (or a genomic locus of interest, which terms are interchangeable), comprising contacting the genomic locus with an agent or composition of the invention as described herein.

Still further embodiments of this invention include the use of an agent or composition of the invention as described herein or the expression vector as described to modify chromatin loops or contact domains as described herein in a mammalian cell.

### BRIEF DESCRIPTION OF THE DRAWINGS

An understanding of the features and advantages of the present invention will be obtained by reference to the following detailed description that sets forth illustrative embodiments, in which the principles of the invention may be utilized, and the accompanying drawings of which:

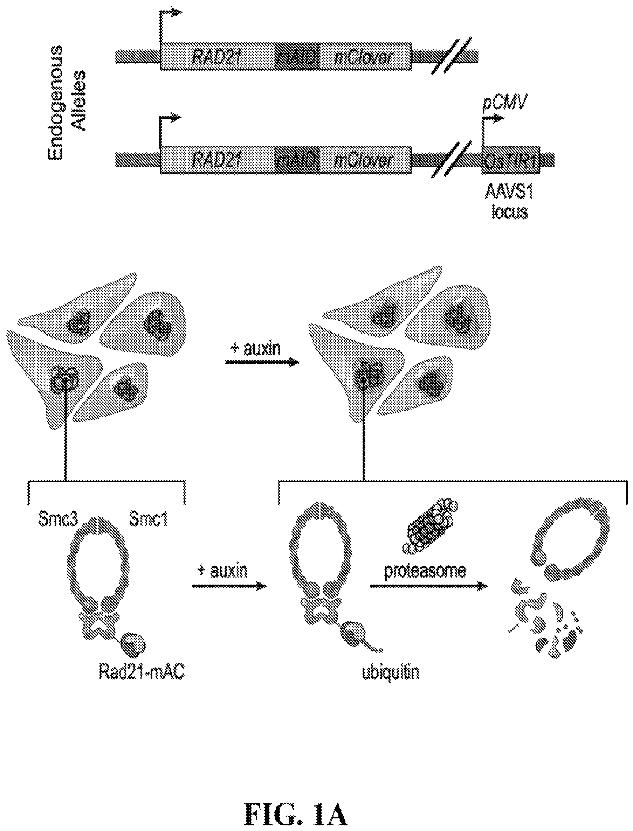

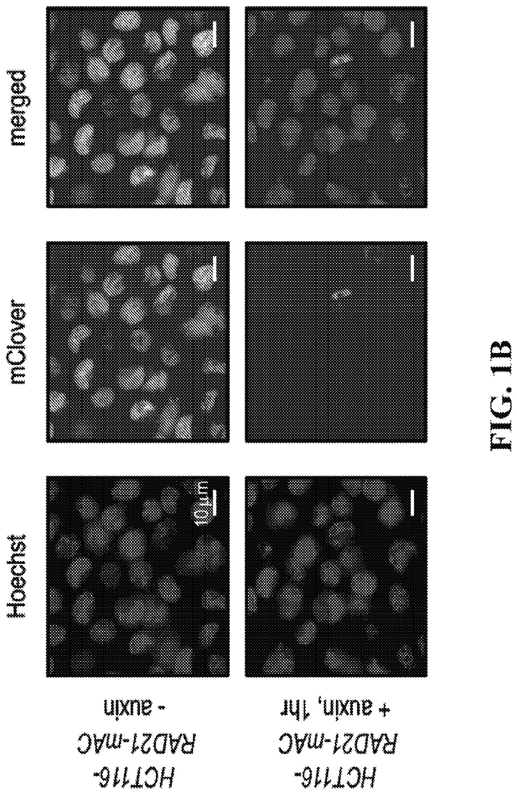

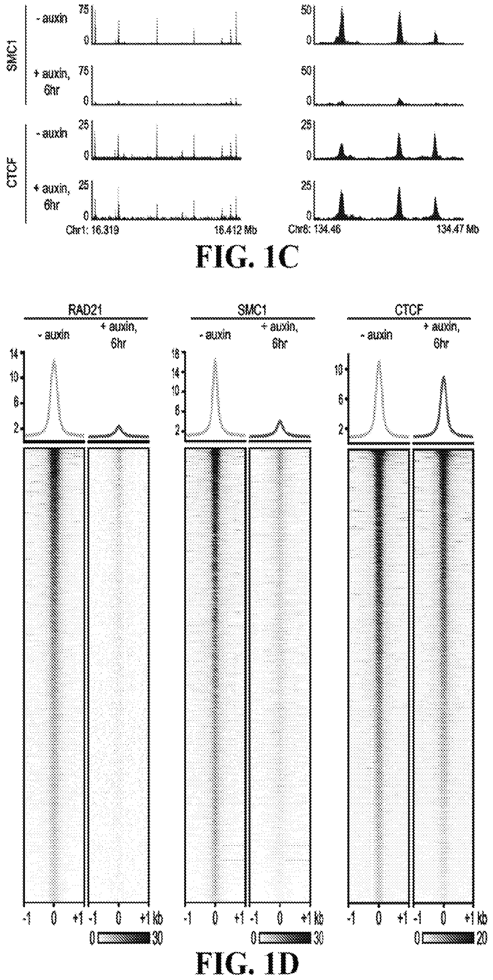

**FIG. 1** **- Tagging of endogenous *RAD21* with an auxin-inducible degron allows for rapid, near complete cohesin loss. (A)** In HCT-116-RAD21-mAC cells, both RAD21 alleles are tagged with auxin-inducible degrons and an mClover reporter, and the OsTIR1 gene is integrated at the AAVS1 locus. Auxin treatment leads to proteasomal degradation of RAD21. **(B)** Live cell imaging after Hoechst 33342 staining to label nuclei. Nuclear mClover fluorescence corresponding to tagged RAD21 was lost after 1 hour of auxin treatment. (See Fig. S1.) **(C)** SMC1 and CTCF ChIP-Seq signal with and without auxin treatment. **(D)** RAD21, SMC1 and CTCF ChIP-Seq signal (left, middle, right) across all peaks called for each of the proteins in untreated RAD21-mAC cells. (Top) Average enrichments for each protein. After RAD21 degradation, the cohesin complex no longer binds to chromatin. CTCF binding is unaffected.

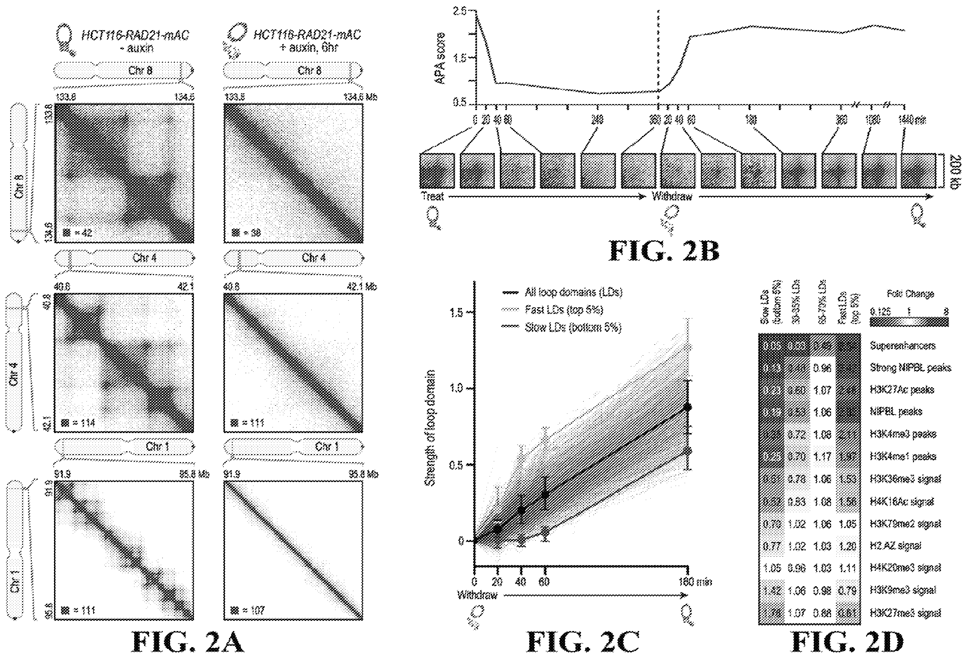

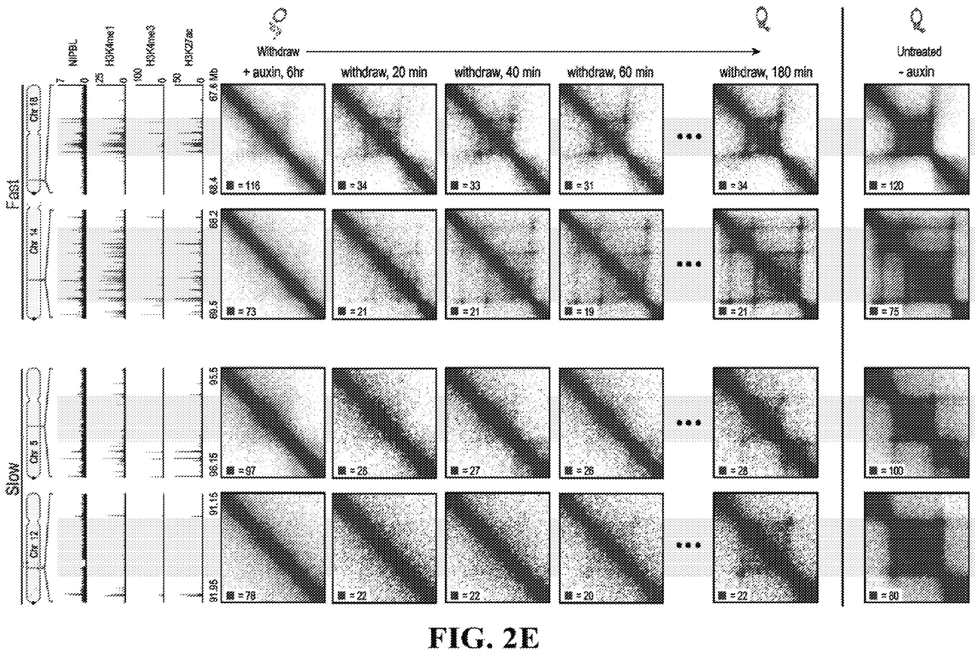

**FIG. 2** - **Cohesin degradation eliminates loop domains. (A)** Contact matrices show that loop domains in untreated RAD21-mAC cells (top) disappear after auxin treatment (bottom). Three representative loci are shown (at 10kb resolution): chr8:133.8-134.6Mb (left), chr4:40.8-42.1Mb (middle) and chr1:91.9-95.8Mb (right). **(B)** Aggregate peak analysis (APA) was used to measure the aggregate strength of the links associated with all loop domains in low-resolution Hi-C maps generated across a time course of auxin treatment and withdrawal. (Top) APA scores; values greater than 1 indicate the presence of loops. (Bottom) APA plots; loop strength is indicated by the extent of focal enrichment at the center of the plot (See Fig S2B). **(C)** Individual loop reformation curves for each of 1,988 loop domains (blue lines); the number of contacts in the untreated map corresponds to a value of 1, and the number of contacts in the auxin-treated map corresponds to 0. Applicants highlight the media (black), the 5th percentile (red) and the 95th percentile (green) in terms of speed of recovery, see Methods. Error bars indicate 25th and 75th percentile within each subset. **(D)** Enrichment of epigenetic features within a loop domain vs. speed of recovery. Enrichment is with respect to all intervals spanned by loop domains. **(E)** Regions containing fast loop domains (1st row: chr18:67.6-68.4Mb; 2nd row: chr14:68.2-69.5Mb) and slow loop domains (3rd row: chr5:95.5-96.15Mb; 4th row: chr12:91.15-91.95Mb) are shown, along with ChIP-Seq tracks (from auxin-treated cells) for NIPBL, H3K4me1, H3K4me3, and H3K27Ac. For fast loop domains, reformation is apparent by 20-40 minutes after auxin withdrawal, whereas for slow loop domains, reformation is not seen until 3 hours after auxin withdrawal. An interactive version of this figure is available at: bit.ly/2wl14TE

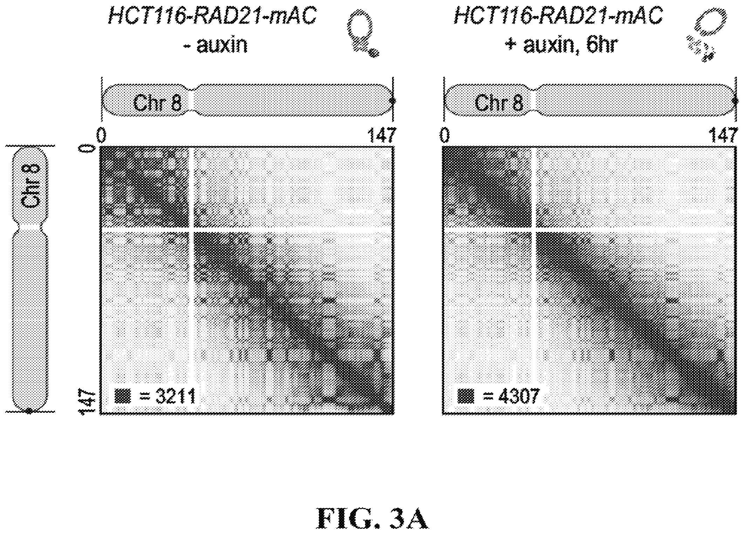

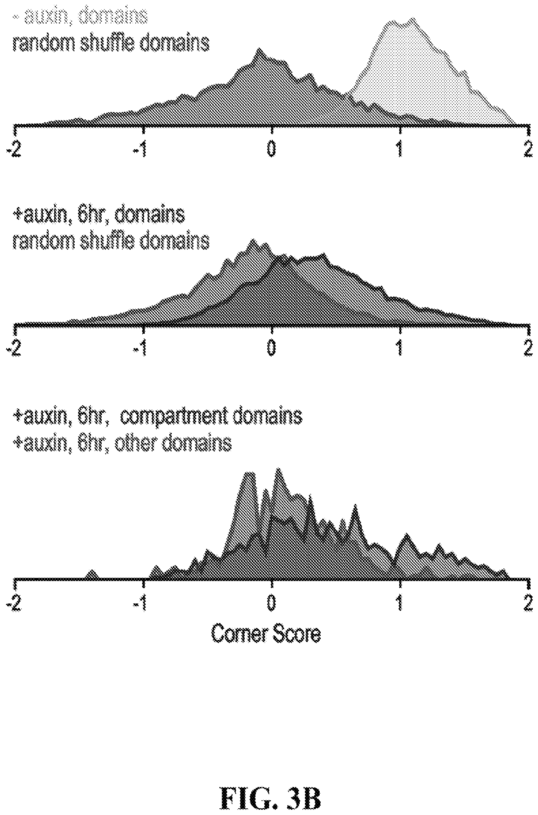

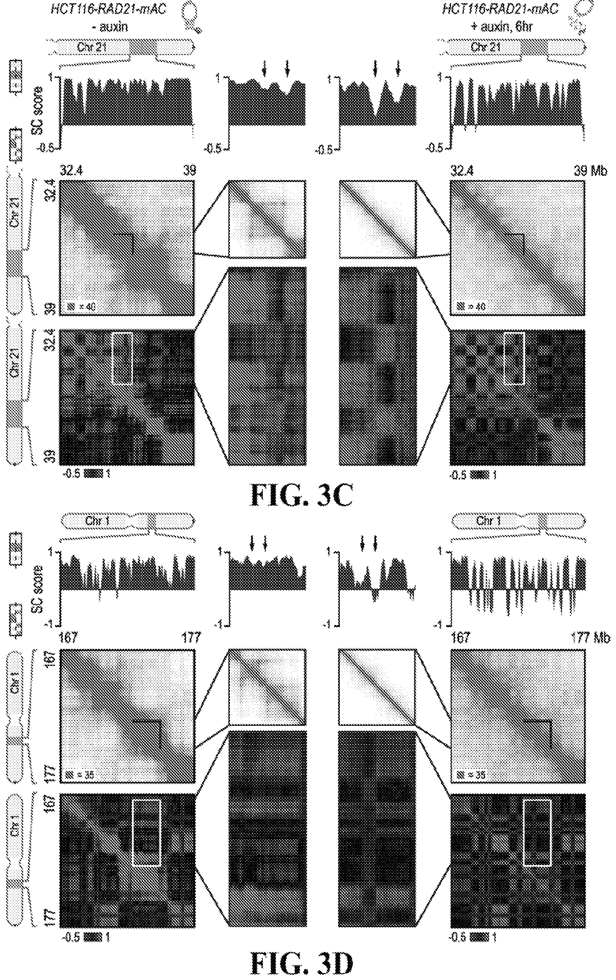

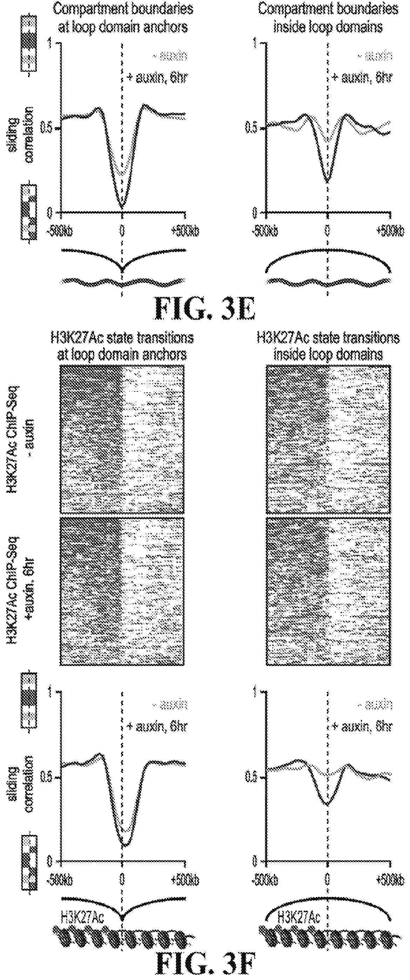

**FIG. 3** - **Genome compartmentalization is strengthened after cohesin degradation. (A)** Contact matrices of chromosome 8 at 500kb resolution. The plaid pattern in the Hi-C map, indicating compartmentalization, is preserved after auxin treatment. **(B)** Strength of contact domains called in untreated cells versus random intervals measured using the corner score (see Methods) in untreated (top) and treated cells (middle). Contact domain strength is reduced, but does not disappear. The remaining signal comes from compartment domains (bottom). The signal in treated maps from contact domains where both boundaries are contained completely inside a compartment interval ('other domains') is not enriched vs. random pixels. **(C,D)** Examples (C: chr21:32.4-39Mb and D: chr1:167-177Mb) showing that the loss of cohesin-associated loops after auxin treatment results in increased fine-scale compartmentalization. Top: Sliding correlation scores; valleys imply strong differences in long-range contact pattern observed at a locus as compared to neighboring loci, indicating a change in compartment (see Methods). Middle: Observed contact matrices. Bottom: Pearson's correlation maps for the local region shown (see Methods). Deeper valleys in the sliding correlation score and increased plaid patterning in the observed and Pearson's correlation maps indicate stronger fine-scale compartment interactions after auxin treatment. Blowouts: loss of a loop domain results in strengthening of a compartment boundary spanned by the loop. Blown-out regions are indicated on zoomed out maps for both the observed (black upper triangle) and Pearson's correlation maps (white rectangle). Observed and Pearson's correlation maps are both shown at 25kb resolution for the zoomed out matrices and 10kb and 25kb resolution respectively for the blown-out matrices. **(E)** Sliding correlation scores before and after auxin treatment for compartment boundaries which either coincide with loop domain anchors (left) or are located in the interior of a loop domain (right). **(F)** Sliding correlation scores before and after auxin treatment for H3K27ac boundaries in untreated cells which either coincide with loop domain anchors (left) or are located in the interior of a loop domain (right). H3K27Ac modification patterns are unchanged after auxin treatment (top and middle). Interactive figure: bit.ly/2vhBT7u

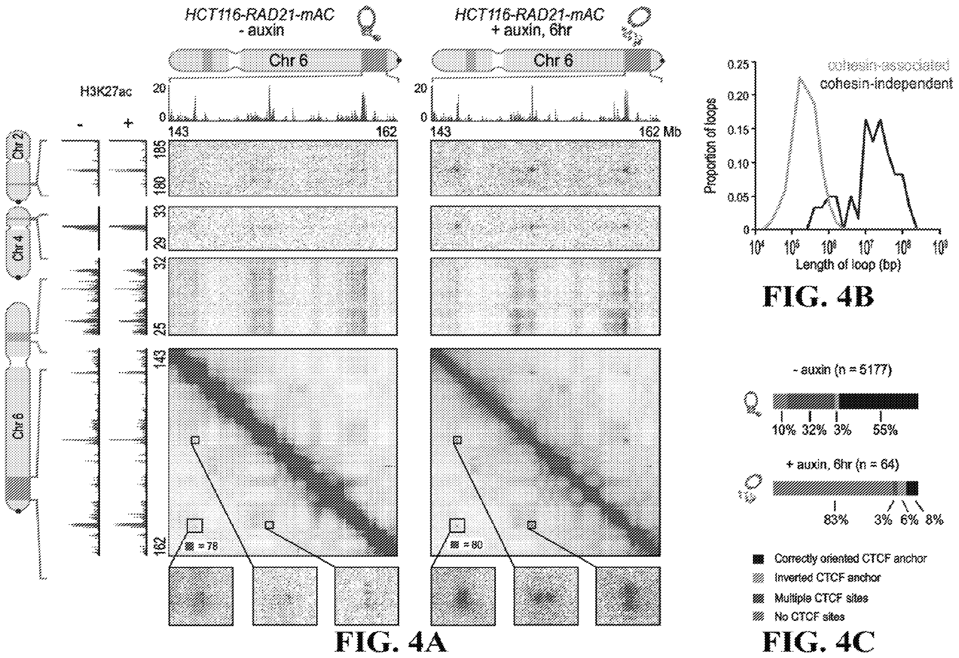

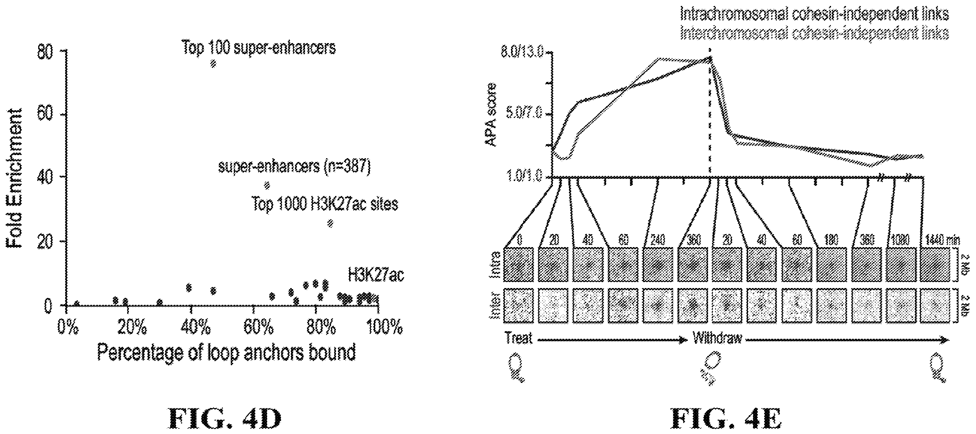

**FIG. 4** - **Cohesin loss causes superenhancers to co-localize, forming hundreds of links within and across chromosomes. (A)** A network of intra- and interchromosomal cohesin-independent links between superenhancers on chr6, chr4, and chr2. H3K27 acetylation does not change with auxin treatment, but cohesin-independent links are significantly strengthened upon treatment. Intrachromosomal matrices are shown at 25kb (on-diagonal) and 50kb (off-diagonal) resolutions; interchromosomal matrices are shown at 100kb resolution. Maximum color intensities are 28 reads for the offdiagonal intrachromosomal matrices and 20 reads for the interchromosomal matrices. **(B)** Length distribution of cohesin-associated loops (green) versus cohesin-independent loops (blue). **(C)** CTCF binding patterns at cohesin-associated (top) versus cohesin-independent loop anchors (bottom). **(D)** Percent of cohesin-independent loop anchors bound versus fold enrichment for 36 DNA-binding proteins and histone modifications. **(E)** APA for intrachromosomal (blue) and interchromosomal (red) cohesin-independent links across a time course of auxin treatment and withdrawal (top: APA scores; bottom: APA plots). Interactive figure: bit.ly/2vhEFts

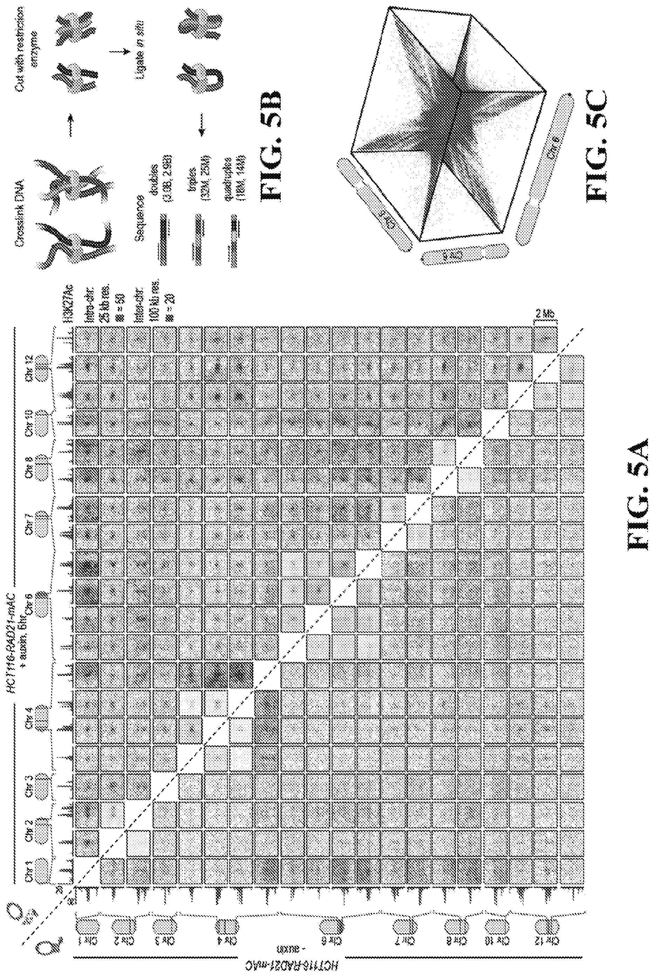

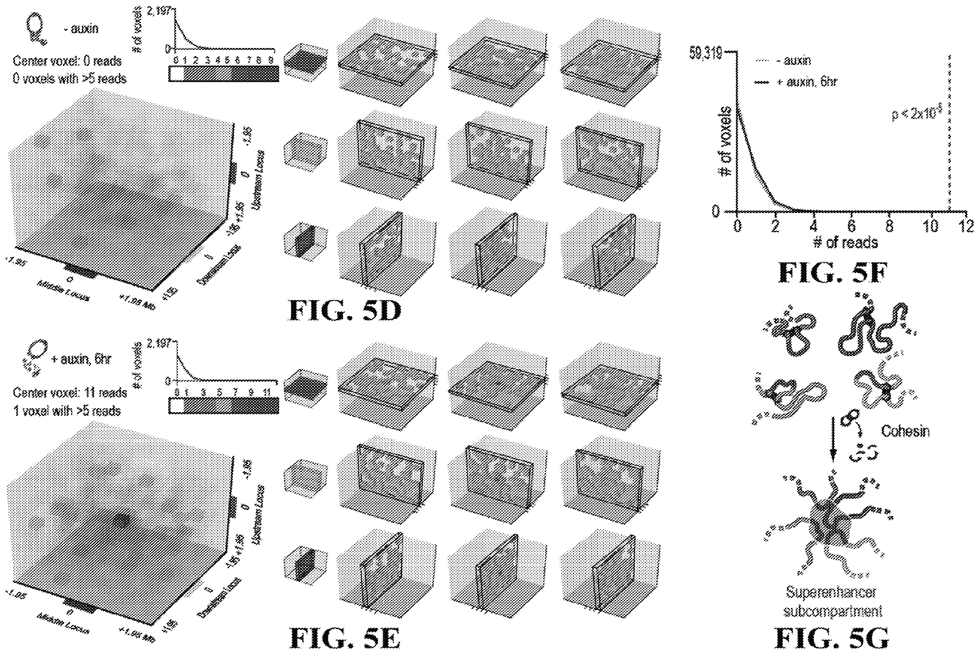

**FIG. 5** **- In the absence of cohesin, a clique spanning more than 20 superenhancers forms pairwise links and higher-order hubs. (A)** The interactions between 20 cohesin-independent loop anchors spread across 9 chromosomes are shown before (lower triangle) and after (upper triangle) auxin treatment. Each matrix shows a 2 Mb by 2 Mb matrix centered on the respective anchors. Intrachromosomal interactions are shown at 25kb resolution; interchromosomal interactions are shown at 100kb resolution. The anchors are strongly enriched for H3K27 acetylation both before and after auxin treatment. (ChIP-Seq data is shown at 25kb resolution.) Cohesin loss causes the anchors to form a clique, with focal interactions seen between nearly all pairs of loop anchors, regardless of whether they lie on the same chromosome. **(B)** In addition to pairwise contacts, in situ Hi-C generates concatemers spanning three or more fragments. There are millions of triples (chimeric reads which align to three loci) and quadruples (chimeric reads which align to four loci) in both our untreated and auxin-treated in situ Hi-C data sets for RAD21-mAC cells. The numbers in parentheses indicate the number of n-mer contacts observed in the untreated (left) and auxin-treated (right) data. **(C)** 3D tensor showing collisions between three loci on chromosome 6 at 1Mb resolution (see Methods). **(D)** (Left) 3D aggregate peak analysis (APA) using the untreated in situ Hi-C data for all 131 intrachromosomal trios of cohesin-independent loop anchors, chosen so that each anchor in a trio lies on the same chromosome as the other two anchors, but no two anchors in a trio lie within 10Mb of one another. To create a 3D APA cube, Applicants excise a 3.9 x 3.9 x 3.9Mb subtensor centered on each trio, and superimpose the results. The cube is shown at 300kb resolution (i.e., each voxel corresponds to all collisions between three loci, each 300kb in length). The subtensors are oriented such that the locus closest to the p-terminus of a chromosome is always located on the z-axis, the one closest to the q-terminus is located on the y-axis, and the locus in between is located on the x-axis. The number of collisions in a voxel is indicated by its color; the histogram above the color scale shows the number of voxels of each color. No voxel contains more than 5 collisions, and the center voxel - reflecting all collisions between three cohesin-independent loop anchors - contains no collisions at all. (Right) Top Row: The central cross-section in z is shown, flanked by the two adjacent cross-sections. Middle Row: The central cross-section in y, flanked by the adjacent cross sections. Bottom Row: The central cross section in x, flanked by the adjacent cross sections. There is no enrichment at the center of the 3D APA cube. **(E)** The preceding analysis is repeated using the auxin-treated data. Now, the center voxel contains 11 collisions, whereas no other voxel contains more than 5 collisions. These findings indicate that, in the absence of cohesin, cohesin-independent loop anchors tend to co-localize to form hubs containing three or more anchors. **(F)** Histogram of number of voxels vs. number of collisions for the two 3D-APA cubes shown in 5D and 5E, as well as for 52 control 3D-APA cubes obtained by shifting one or more of the loci in each of the above trios by 3.9Mb. With the exception of the central voxel in the auxin-treated 3D APA cube, which contains 11 collisions, no voxel contains more than 8 collisions. This indicates that the observation of 11 collisions purely by chance is exceedingly unlikely. **(G)** Under normal circumstances, loop extrusion facilitates short-range contacts between superenhancers and neighboring loci. Upon cohesin loss, superenhancers begin to co-localize, even when located on different chromosomes, and thereby form a subcompartment. Interactive figure: bit.ly/2x9penF

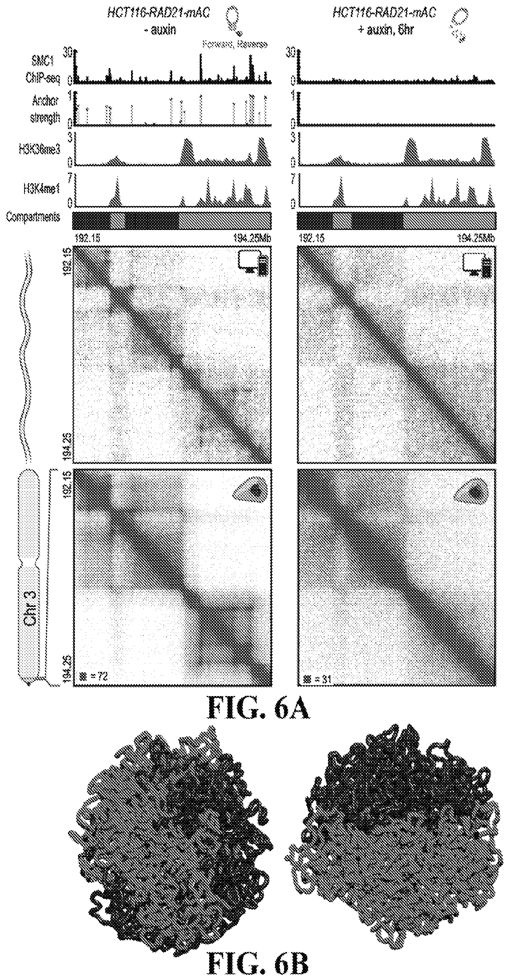

**FIG. 6** **- Molecular dynamics simulations combining extrusion and compartmentalization can recapitulate Hi-C experimental results (A)** Applicants use loop extrusion and compartmentalization to simulate a 2.1 Mb region on chromosome 3 in RAD21-mAC cells before (left) and after (right) auxin treatment. CTCF and SMC1 ChIP-Seq signals are normalized and converted into binding probabilities for the simulated extrusion complex (first and second rows). Each peak is assigned a forward (green) or reverse (red) orientation based on the corresponding CTCF motif. ChIP-Seq data for 9 histone modifications were used to classify loci into two compartments (red and blue, fifth row). Histone modification data for H3K36me3 and H3K4me1 is shown, illustrating the correspondence between the classification tracks and the underlying ChIP-Seq signals (third and fourth rows). The simulations yield an ensemble of polymer configurations. Applicants show contact maps from the simulated ensemble (top) and from the corresponding Hi-C experiments (bottom). **(B)** Examples of globules from simulations of compartmentalization with extrusion (left) and without (right). The globule without extrusion shows stronger segregation of compartment types. Interactive figure: bit.ly/2vsfSDC

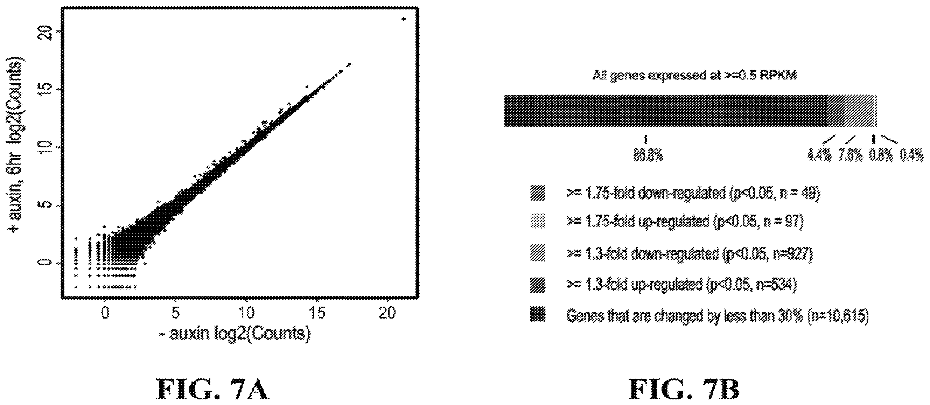

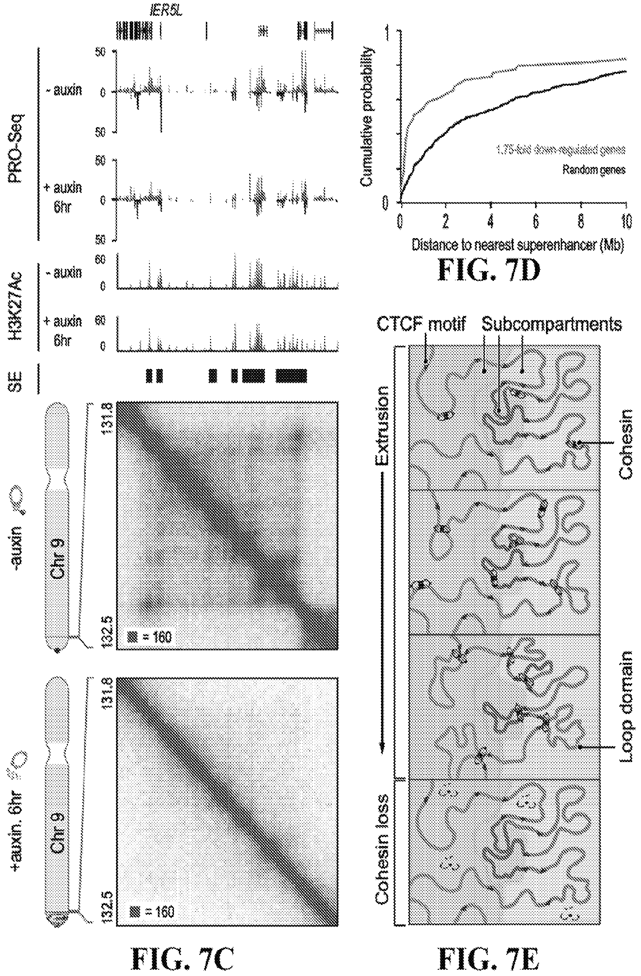

**FIG. 7** **- Cohesin degradation results in strong down-regulation of genes near superenhancers but does not result in widespread ectopic gene activation. (A)** Scatter plot of gene-wide PRO-Seq counts in RAD21-mAC cells before (x-axis) and after (y-axis) treatment. **(B)** Genes that are expressed in untreated cells rarely undergo substantial changes in expression level after cohesin loss. **(C)** An example of a strongly down-regulated gene near a superenhancer. In untreated cells, a series of cohesin-associated loops form between the IER5L promoter and nearby superenhancers. Upon auxin treatment, these loops are lost and IER5L expression is 2.6-fold down-regulated. **(D)** Cumulative probability distributions of distances to the nearest superenhancer for 1.75-fold down-regulated genes after auxin treatment (red) versus random genes (black). **(E)** A model of how extrusion and compartmentalization combine to shape the spatial organization of the genome inside the nucleus. Intervals of chromatin with similar patterns of histone modification co-localize in nuclear subcompartments. Loop extrusion facilitates short-range contacts between nearby loci as the two subunits of the cohesin-based extrusion complex translocate in opposite directions on chromatin. The extrusion subunits halt at CTCF motifs facing inward, thus forming a loop domain between a pair of motifs in the convergent orientation. Loop domains represent dynamic structures that are maintained by cohesin; only a subset of them may be present at any given time. When the loop anchor motifs span multiple compartment intervals, the dynamics of loop extrusion interfere with compartmentalization by facilitating contacts between loci in different compartments. Loss of cohesin leads to the disappearance of loop domains and to a closer correspondence between genome compartmentalization patterns and histone modification patterns. Interactive figure: bit.ly/2uiu514

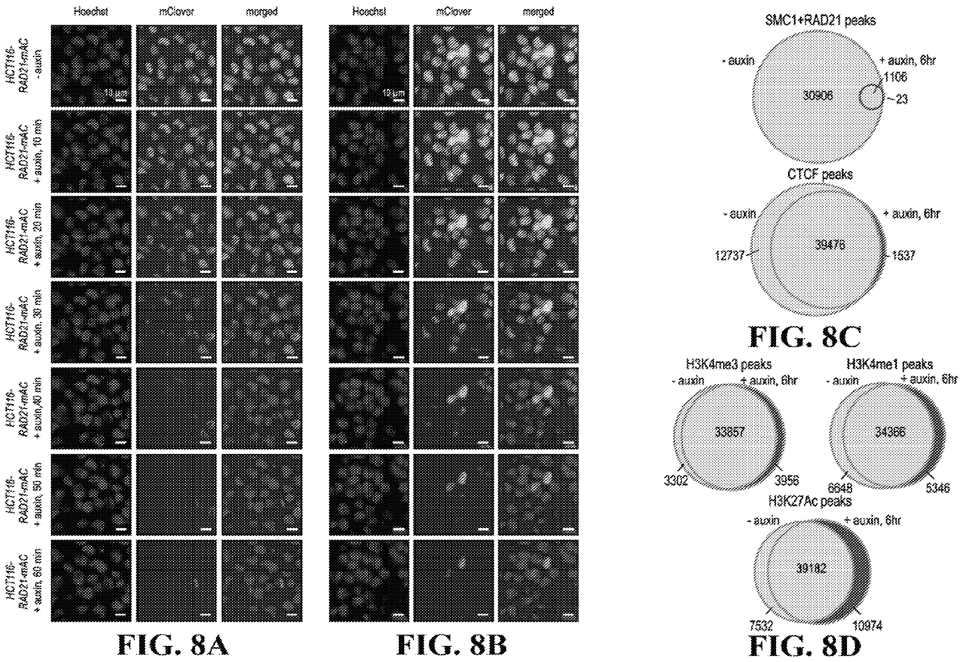

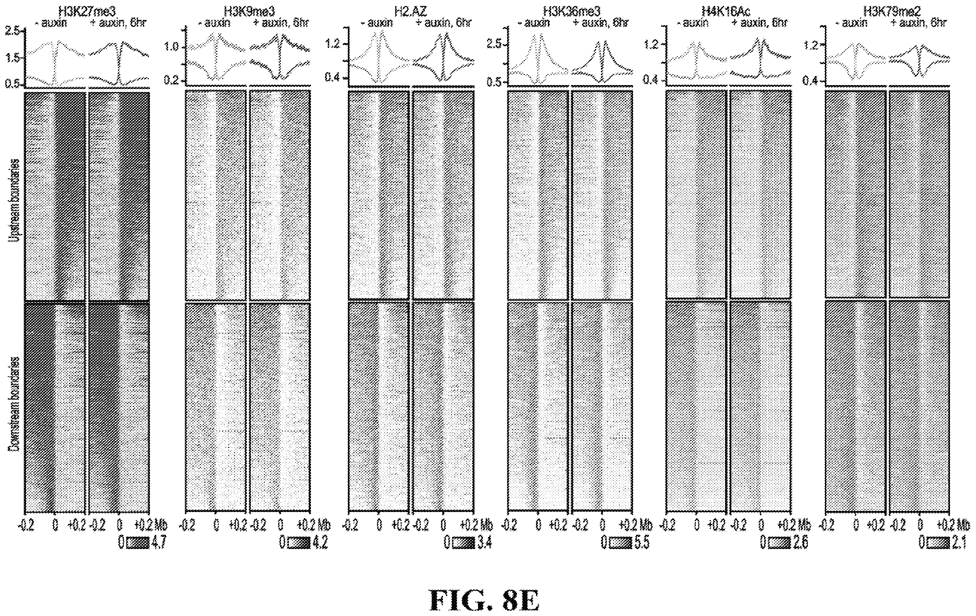

**FIG. 8** - **Analysis of cohesin levels, CTCF binding, and histone modifications after auxin treatment,** Related to Figure 1 **(A)** Live cell imaging of HCT-116-RAD21-mAC cells after Hoechst 33342 staining to label nuclei. After addition of auxin, nuclear mClover signal corresponding to tagged RAD21 protein rapidly disappears and is nearly completely lost by 1 hour. **(B)** Another field, treated as above. **(C)** Overlap of cohesin peaks (top) called by ChIP-Seq (merged SMC1 and RAD21 ChIP-Seq calls) before and after auxin treatment (green and blue, respectively). 97% of cohesin peaks are lost after auxin treatment indicating complete degradation of cohesin. Overlap CTCF peaks (bottom) called by ChIP-Seq. While slightly fewer peaks are called in our experiment after auxin treatment, 96% of CTCF peaks called after auxin treatment are also called before auxin treatment, indicating that CTCF binding is largely unaffected by loss of cohesin. **(D)** Overlap of H3K4me3 (top left), H3K4me1 (top right), and H3K27Ac (bottom) ChIP-Seq peaks called before and after auxin treatment. The high degree of overlap (90%, 85%, 81% respectively) indicate that the positions of active promoters and enhancers are largely unaffected by loss of cohesin. **(E)** ChIP-Seq signal in untreated RAD21-mAC cells (left) and auxin treated RAD21-mAC cells (right) for a number of broad-source histone modifications (from left to right: H3K27me3, H3K9me3, H2.AZ, H3K36me3, H4K16Ac, H3K79me2) at the boundaries of broad enriched domains called in the ChIP-Seq experiments performed in untreated RAD21-mAC cells. Upstream boundaries of broad enriched domains are shown on top and downstream boundaries are shown on the bottom; for each boundary, a window of 400kb centered on the boundary is shown. Average enrichments across the boundary are shown above each heatmap for each mark. No spreading of histone modifications is seen after cohesin loss and enrichments of histone modifications over broad domains are unaffected.

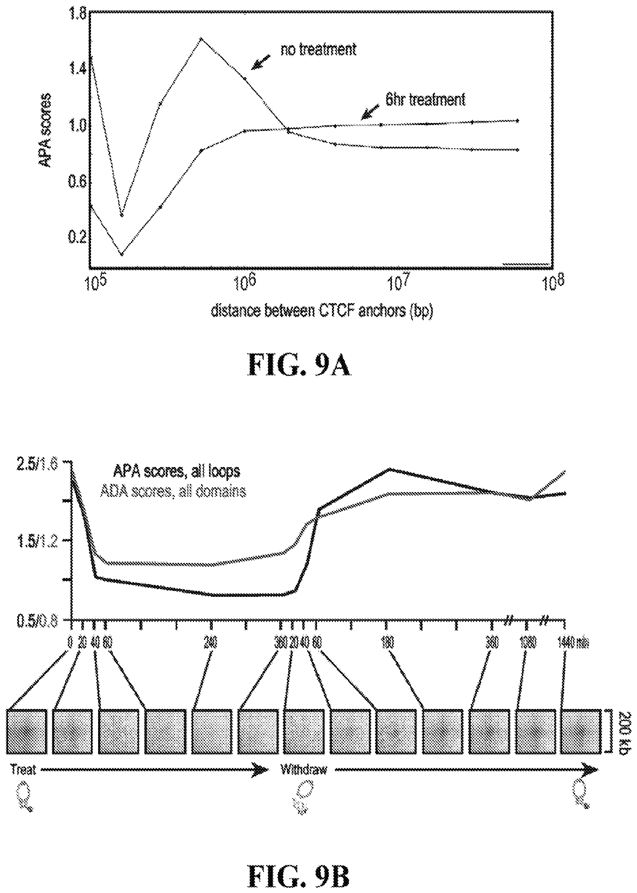

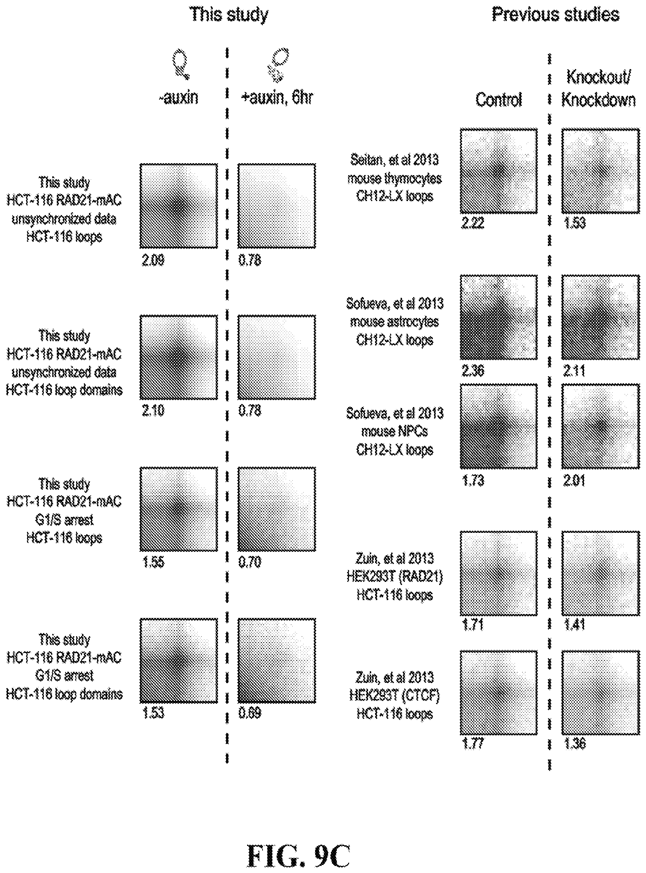

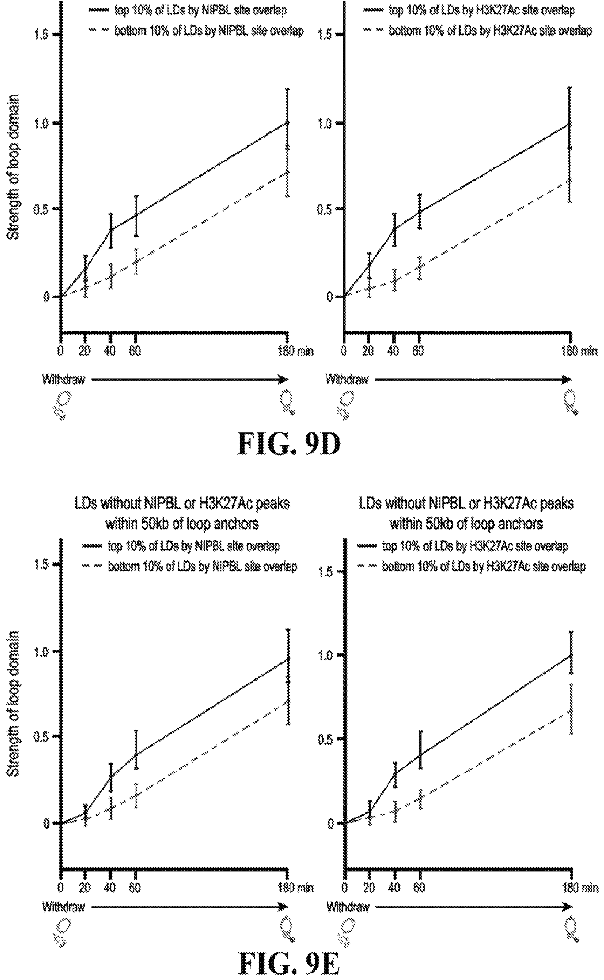

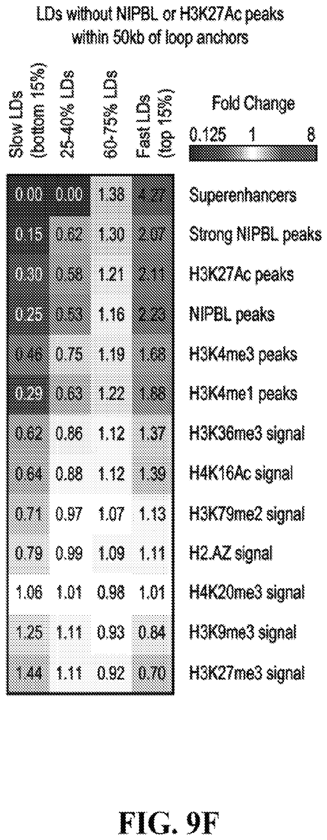

**FIG. 9** - **Cohesin degradation eliminates loop domains and the vast majority of loops,** Related to Figure 2 **(A)** APA scores vs. distance for pairs of convergently oriented CTCF/cohesin-associated loop anchors separated by a given distances. In untreated maps, positive APA scores can be seen for convergently oriented pairs of CTCF/cohesin-associated loop anchors up to distances less than a few megabases, but rapidly drops off at longer distances. In treated maps, positive APA scores are not seen at any distance for convergently oriented pairs of CTCF/cohesin-associated loop anchors. **(B)** APA was used to measure the aggregate strength of the links associated with all loops in low-resolution Hi-C contact maps generated across a time course of auxin treatment and withdrawal. APA scores are shown on top; values greater than 1 indicate the presence of loops. APA plots for each time point are shown on the bottom; the strength of looping is indicated by the extent of focal enrichment at the center of the plot. Loops are rapidly lost as cohesin is degraded, and quickly restored when auxin is withdrawn. ADA was used to measure the aggregate gradient across domain boundaries for all domains annotated in untreated cells in low-resolution Hi-C contact maps generated across a time course of auxin treatment and withdrawal. Domain signal is rapidly lost after auxin treatment, but does not completely disappear (reach 1) consistent with the presence of compartment domains after cohesin degradation. **(C)** Green: APA matrices using loops and loop domains identified in this study in Hi-C maps generated in this study. APA matrices for all loops identified in this study using HiCCUPS with default parameters >300kb long (first and third rows) and for all loop domains >300kb long (second and fourth rows) in Hi-C maps for untreated cells (left) versus in Hi-C maps for treated cells (right). The APA score after auxin treatment shows complete loss of loop signal (APA score <=1), and no focal enrichment is visible. Orange: APA matrices using appropriate loop lists in Hi-C maps from previous studies of cohesin/CTCF depletion. First row: Applicants re-analyzed the Hi-C data from (Seitan et al., 2013) and performed APA on their maps from mouse thymocytes before cohesin deletion (left) and after (right) using a loop list Applicants generated using HiCCUPS in CH12-LX mouse lymphoblasts (Rao et al., 2014). A positive APA score (1.533, indicating ~1.5 fold enrichment of the peak pixel over the pixels to its lower left) is seen even after cohesin deletion suggesting incomplete deletion of RAD21. Second and third rows: Applicants re-analyzed the Hi-C data from (Sofueva et al., 2013) and performed APA on their maps from mouse astrocytes before cohesin deletion (top left) and after (top right) as well as on their maps from mouse NPCs before cohesin deletion (bottom left) and after (bottom right) using a loop list Applicants generated using HiCCUPS in CH12-LX mouse lymphoblasts (Rao et al., 2014). A positive APA score (2.106 and 2.013 respectively, indicating ~2 fold enrichment of the peak pixel over the pixels to its lower left) is seen even after cohesin deletion suggesting incomplete deletion of RAD21. Fourth and fifth rows: Applicants re-analyzed the Hi-C data from (Zuin et al., 2014) and performed APA on their maps from HEK293T cells before cohesin depletion (top left) and after (top right) as well as on their maps from HEK293T cells with a control siRNA (bottom left) and with an siRNA targeting CTCF (bottom right) using the loop list generated with HiCCUPS in untreated HCT-116 RAD21-mAC cells in this study. A positive APA score (1.413 and 1.356 respectively, indicating ~1.4 fold enrichment of the peak pixel over the pixels to its lower left) is seen even after cohesin or CTCF depletion suggesting incomplete depletion. For all the APA matrices in this panel, the color scale for both matrices ranges from the mean of the 6x6 box in the upper right corner of the matrix (white) to five times the mean of the 6x6 box in the upper right corner of the matrix (red). **(D)** Median recovery curves for top 10% of loop domains and bottom 10% of loop domains in terms of density of NIPBL binding sites (left) or density of H3K27Ac peaks (right). Error bars indicate 25th and 75th percentile for each set of loops. The plots are scaled so that the number of contacts in the untreated map corresponds to a value of 1, and the number of contacts in the auxin-treated map corresponds to 0. Loop domains with higher density of NIPBL binding or H3K27Ac binding recover faster upon withdrawal of auxin. **(E)** Median recovery curves for top 10% of loop domains and bottom 10% of loop domains in terms of density of NIPBL binding sites (left) or density of H3K27Ac peaks (right) after restricting to loop domains >=200kb in size that do not have a NIPBL binding site or a H3K27Ac peak within 50kb of either anchor. Error bars indicate 25th and 75th percentile for each set of loops. The plots are scaled so that the number of contacts in the untreated map corresponds to a value of 1, and the number of contacts in the auxin-treated map corresponds to 0. Higher densities of NIPBL binding and H3K27Ac peaks in the interior of a loop domain (i.e. far from the anchors) is still associated with faster loop domain recovery upon withdrawal of auxin. **(F)** Enrichment of epigenetic features within a loop domain as a function of the loop domain's speed of recovery (see Methods), showing the slowest 15%, the 25th-40th percentile, the 60th-75th percentile, and the fastest 15% of loop domains >=200kb in size that do not have a NIPBL binding site or a H3K27Ac peak within 50kb of either anchor. Enrichment is with respect to the average value of the feature across all the intervals spanned by loop domains (see Methods). Superenhancers and strong NIPBL peaks are particularly enriched in fast loop domains vs. slow loop domains despite the restriction that the loop domains analyzed must not show any evidence of H3K27Ac or NIPBL binding in the vicinity of the loop anchors.

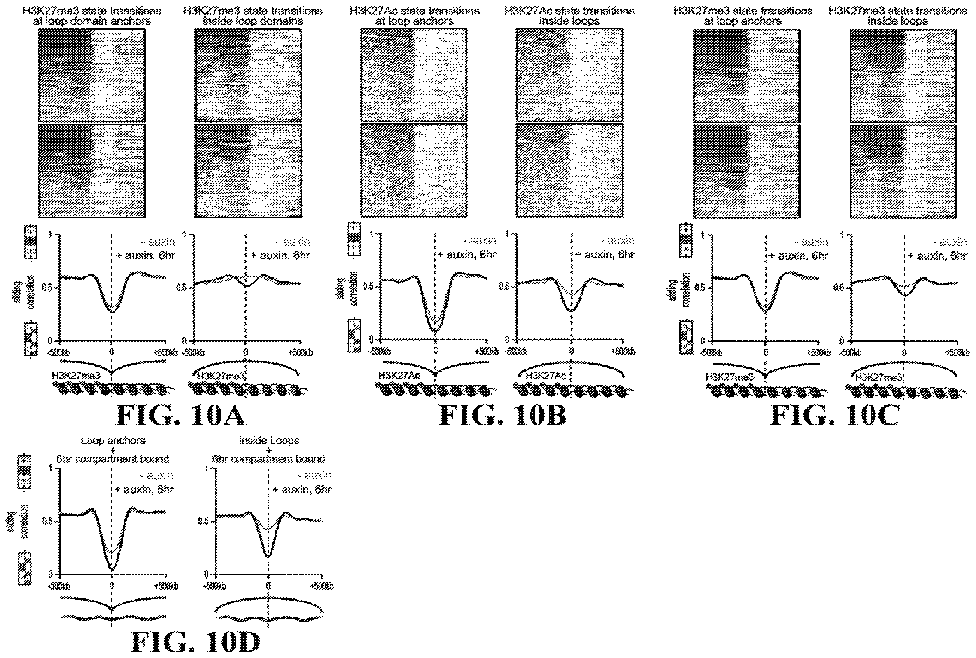

**FIG. 10** - **Cohesin degradation results in genome compartmentalization that better matches histone modification patterns,** Related to Figure 3 **(A)** Sliding correlation scores before and after auxin treatment for H3K27me3 domain boundaries in untreated cells which either coincide with loop domain anchors (left) or are located in the interior of a loop domain (right). H3K27me3 histone modification patterns do not change after loss of cohesin (top and middle). For H3K27me3 boundaries that lie in the interior of a loop domain in untreated cells, the difference in long-range contact pattern on opposite sides of the boundary increases greatly after cohesin treatment. This indicates that loop domains facilitate mixing of chromatin with different histone modifications. **(B)** Sliding correlation scores before and after auxin treatment for H3K27Ac domain boundaries in untreated cells which either coincide with loop anchors (left) or are located in the interior of a loop (right). For H3K27Ac boundaries that lie in the interior of a loop in untreated cells, the difference in long-range contact pattern on opposite sides of the boundary increases greatly after cohesin treatment. This indicates that loops facilitate mixing of chromatin with different histone modifications. **(C)** Sliding correlation scores before and after auxin treatment for H3K27me3 domain boundaries in untreated cells which either coincide with loop anchors (left) or are located in the interior of a loop (right). For H3K27me3 boundaries that lie in the interior of a loop in untreated cells, the difference in long-range contact pattern on opposite sides of the boundary increases greatly after cohesin treatment. This indicates that loops facilitate mixing of chromatin with different histone modifications. **(D)** Sliding correlation scores before and after auxin treatment for compartment boundaries which either coincide with loop anchors (left) or are located in the interior of a loop (right). For compartment boundaries that lie in the interior of a loop in untreated cells, the difference in long-range contact pattern on opposite sides of the boundary increases greatly after cohesin treatment.

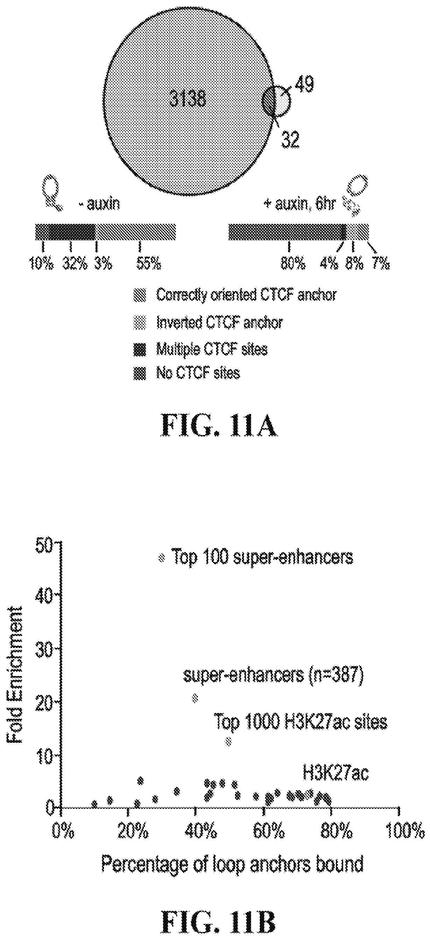

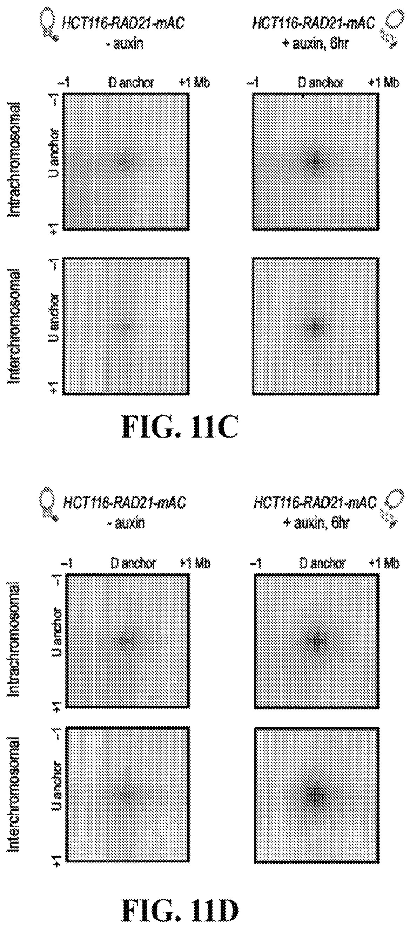

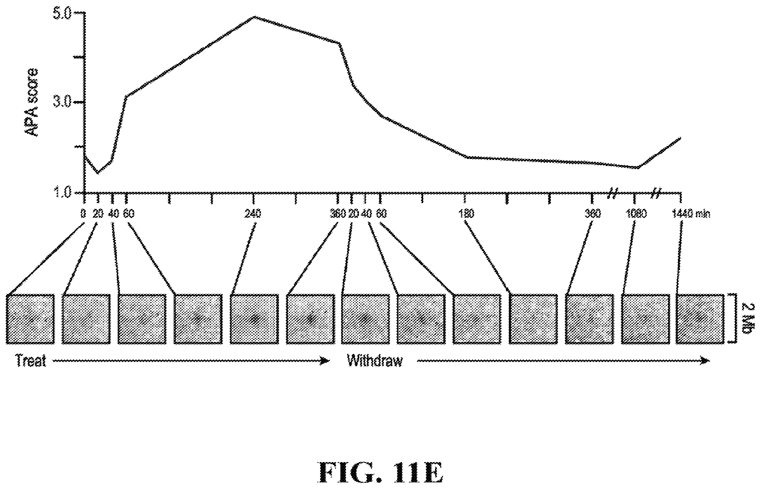

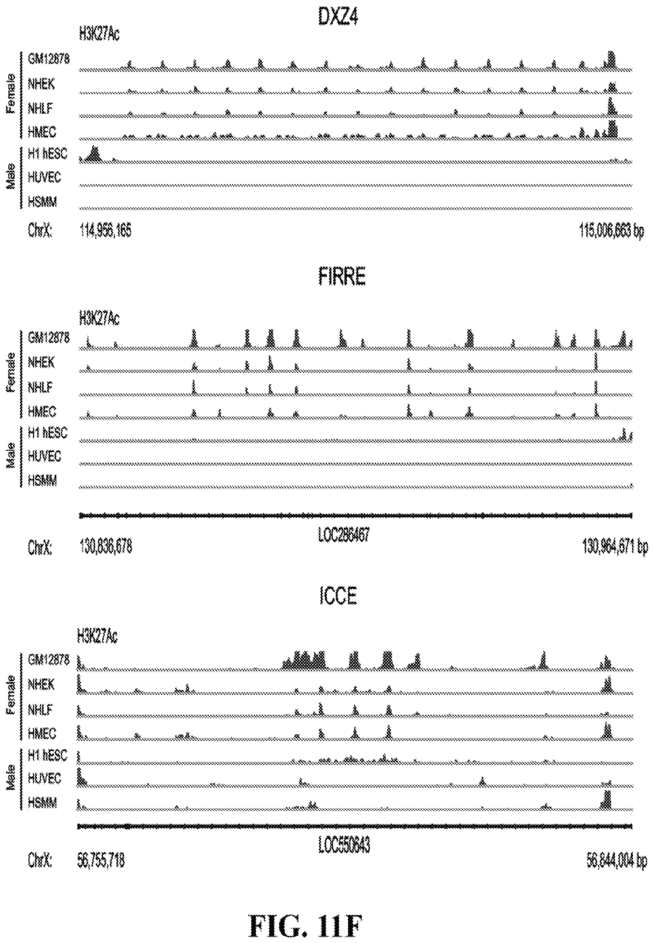

**FIG. 11****- Co-localization of superenhancers after cohesin loss,** Related to Figure 4 **(A)** Venn diagram of loops called in untreated cells with default HiCCUPS parameters with loops called in treated cells with default parameters. The vast majority of loops are lost (>97%) but a small number of "cohesin-independent" loops remain. Loops annotated in treated cells do not frequently bind CTCF and show no CTCF orientation bias. **(B)** Percent of cohesin-independent loop anchors bound versus fold enrichment for 36 DNA-binding proteins and histone modifications. Same analysis as Fig. 4F but using a loop anchor list generated by running HiCCUPS at 50 and 100kb resolution without any manual curation. Superenhancers are still highly enriched at cohesin-independent loop anchors, validating that the result does not stem from hand curation bias. **(C)** APA for intrachromosomal (top) and interchromosomal (bottom) cohesin-independent links in our untreated maps (left) and our treated maps (right) using an automatedly generated list by pairing all superenhancers overlapping cohesin-independent link anchors returned by low-resolution HiCCUPS (same list as Fig. S5B and S5C). Cohesin-independent links are significantly strengthened as cohesin is degraded (Intrachromosomal APA scores: 1.69 (untreated) vs. 2.75 (treated); interchromosomal APA scores: 2.29 (untreated) vs. 3.64 (treated)). **(D)** APA for intrachromosomal (top) and interchromosomal (bottom) cohesin-independent links in our untreated maps (left) and our treated maps (right) using our manually curated HiCCUPS lists of 61 intra- and 203 interchromosomal links. Cohesin-independent links are significantly strengthened as cohesin is degraded (Intrachromosomal APA scores: 2.32 (untreated) vs. 4.02 (treated); interchromosomal APA scores: 3.02 (untreated) vs. 7.02 (treated)). **(E)** APA for intrachromosomal (blue) cohesin-independent links across a time course of auxin treatment and withdrawal using an automatedly generated list by pairing all superenhancers overlapping cohesin-independent link anchors returned by low-resolution HiCCUPS. APA scores are shown on top and APA plots for each time point are shown on the bottom. Cohesin-independent links are rapidly strengthened as cohesin is degraded and weaken as cohesin is restored. **(F)** DXZ4 (top), FIRRE (middle) and ICCE (bottom), the three most prominent superloop anchors on the inactive X chromosome (Rao et al., 2014; Darrow et al., 2016) are enriched for H3K27Ac in female cell lines (GM12878, NHEK, NHLF, HMEC) but not male cell lines (H1-hESC, HUVEC, HSMM). All H3K27Ac tracks shown were generated by ENCODE (ENCODE Consortium, 2012) and are shown with a common maximum enrichment of 50.

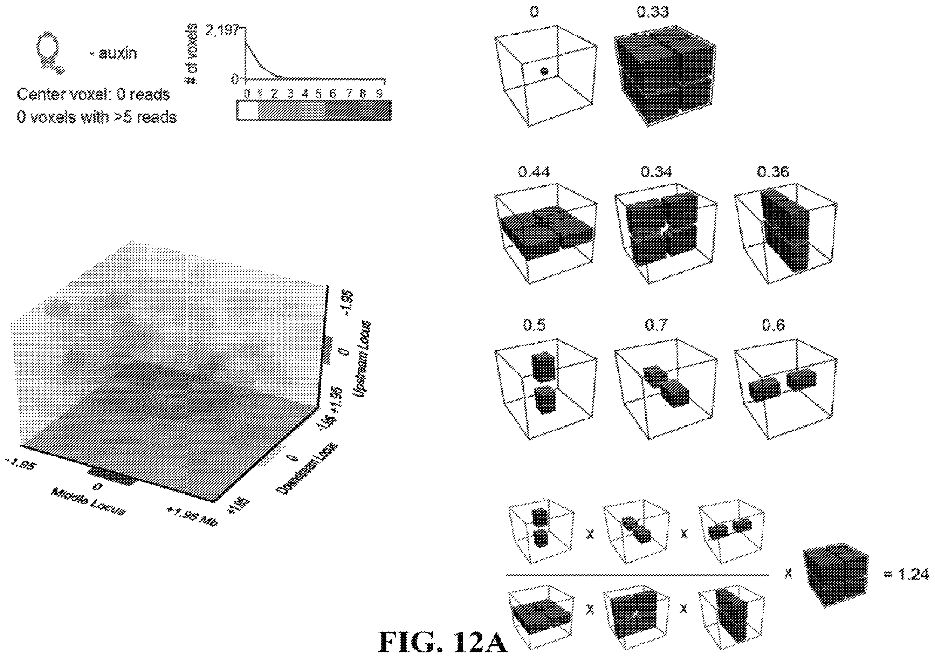

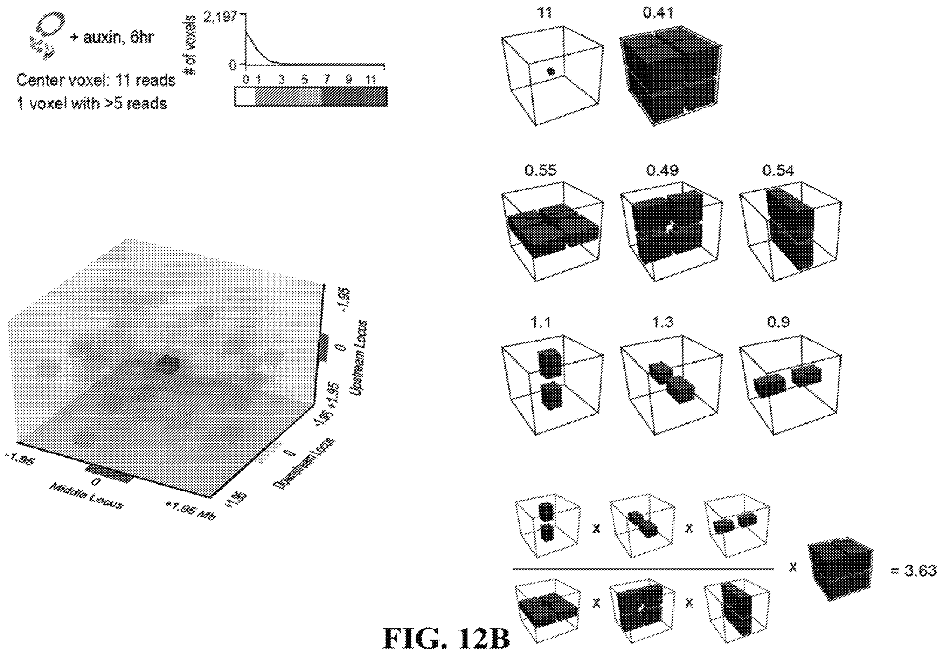

**FIG. 12** **- Higher-order contacts between cohesin-independent loop anchors are enriched after auxin treatment relative to all appropriate controls,** Related to Figure 5 **(A,B)** The 3D APA subtensor for 131 intrachromosomal trios of cohesin-independent loop anchors where no two anchors lie within 10 Mb of each other (left) and the average frequency of contact in various local neighborhoods surrounding the center voxel for the untreated *in situ* Hi-C data **(A)** and the auxin treated *in situ* Hi-C data **(B).** (See Methods for descriptions of the local neighborhood controls.) While there are no contacts in the center voxel in the 3D APA subtensor for the untreated data, the center voxel in the 3D APA subtensor for the treated data (11 contacts) is strongly enriched with respect to every model, including an expected model that accounts for pairwise-enrichments in contact frequency (bottom model, see Methods).

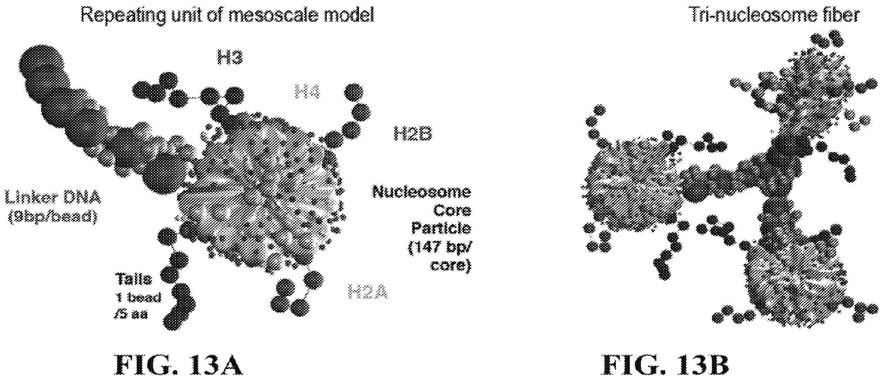

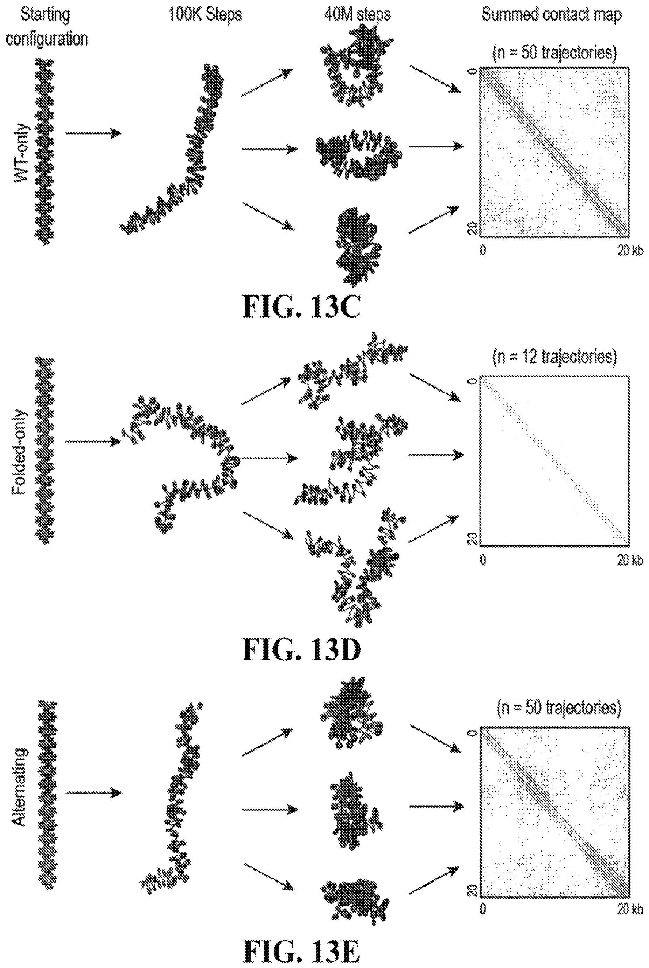

**FIG. 13** - **Mesoscale modeling of oligonucleosome fibers with varying histone modifications,** Related to Figure 6 **(A,B)** Applicants utilized the mesoscale model from Bascom and Schlick, Biophys. J. 2017 to simulate fibers of 100 nucleosomes with NRL = 200bp and no linker histone by Monte Carlo sampling. **(C,D,E)** Using our mesoscale model, ensembles of trajectories were collected and analyzed for three oligonucleosome systems, each simulated for 40 million steps or more. The three systems represent wildtype fibers **(C),** fibers with all folded tails **(D;** as an additional control), and 'alternating' fibers where the pattern of {25 nucleosomes of folded tails/25 nucleosomes with wildtype tails} was repeated twice **(E).** For each system, Applicants show schematic representations of the fiber composition (first column), configurations near the beginning of the MC run (second column), and representative converged structures (third column). Corresponding contact maps are also shown, as described in Grigoryev et al. PNAS 2016, with ensemble size indicated for each system (fourth column). The contact maps are normalized so that the matrix elements are from 0 to 1. Note that while the wildtype fibers adopt a compact hairpin-like/hierarchical looping structure (Bascom and Schlick, Biophys. J. 2017) and the folded-tail control systems unfold due to loss of stabilizing tail/tail intemucleosome interactions (Collepardo-Guevara et al. JACS 2015), the alternating constructs lead to clearly segregated interactions of the two types of nucleosomes. The contact map of the alternating construct shows this emerging checkerboard pattern: the wildtype fibers are clustered together compactly, while the folded-tail region cluster near each other but adopt more open fiber states.

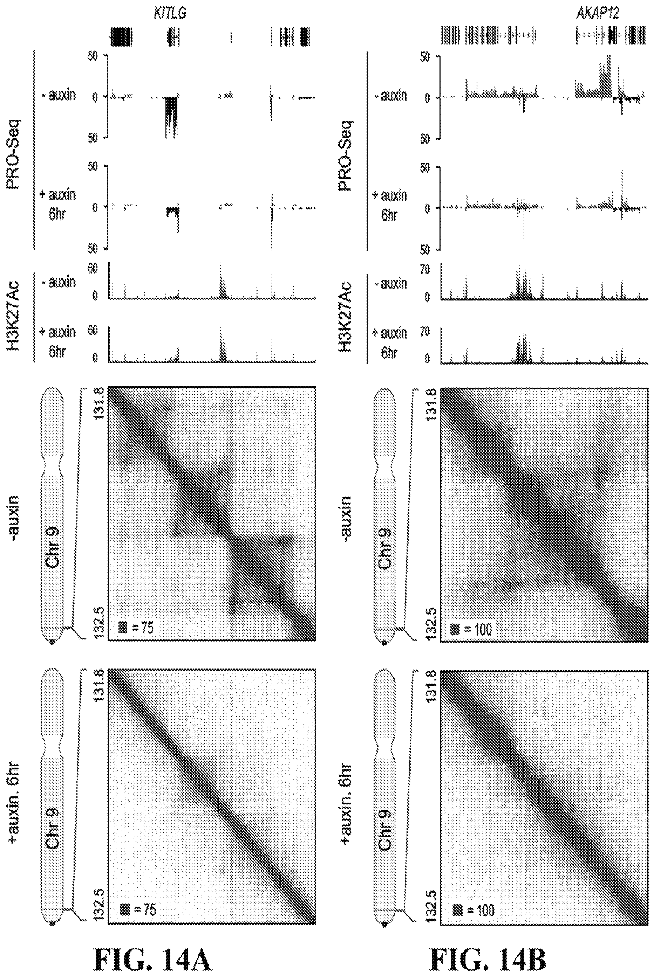

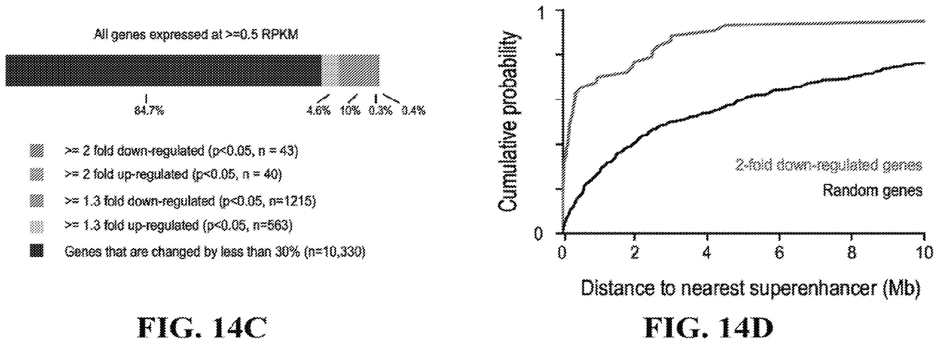

**FIG. 14** - **Examples of downregulation of genes nearby superenhancers after cohesin loss,** Related to Figure 7 **(A)** An example of a strongly down-regulated gene near a superenhancer. In untreated cells, KITLG is contained within a loop domain with a strong superenhancer. Upon auxin treatment, the spanning loops are lost and KITLG expression is 2.73-fold down-regulated. The superenhancer near KITLG forms strong links to other superenhancers intrachromosomally and interchromosomally after auxin treatment. **(B)** An example of a strongly down-regulated gene near a superenhancer. In untreated cells, AKAP12 is contained inside a loop domain with a strong superenhancer. Upon auxin treatment, the spanning loop is lost and AKAP12 expression is 3.3-fold down-regulated. The superenhancer near AKAP12 forms strong links to other superenhancers intrachromosomally and interchromosomally after auxin treatment. **(C)** Genes that are expressed in untreated cells rarely undergo substantial changes in expression level after cohesin loss even when compared to untagged HCT-116 CMV-OsTIR1 cells. Cumulative probability distributions of distances to the nearest superenhancer for 2-fold down-regulated genes between untreated HCT-116 CMV-OsTIR1 cells and treated RAD21-mAC cells (red) versus random genes (black).

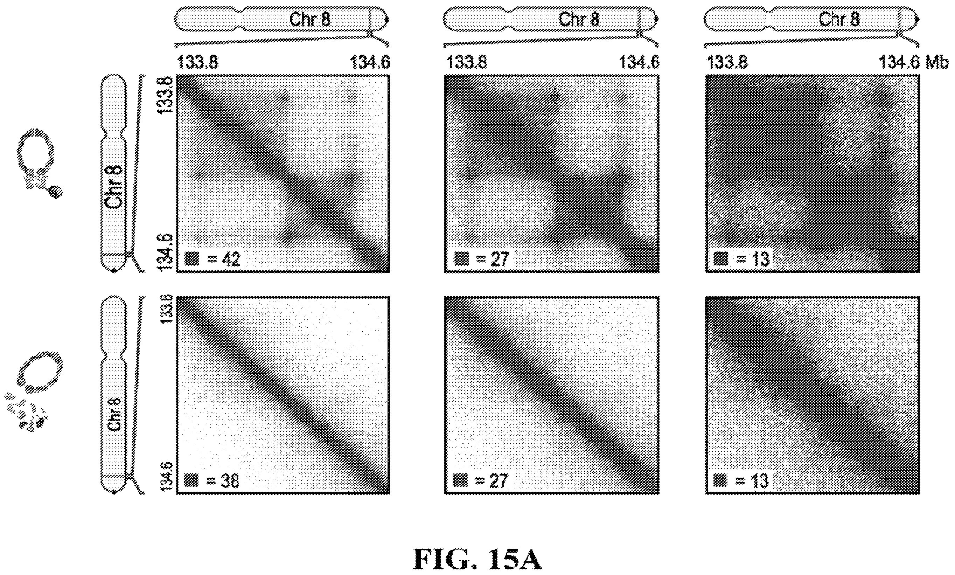

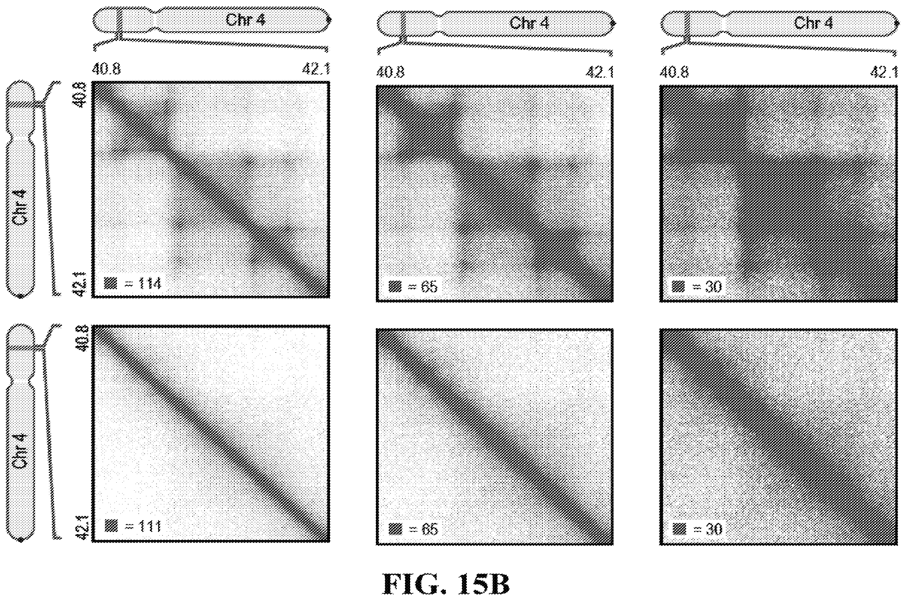

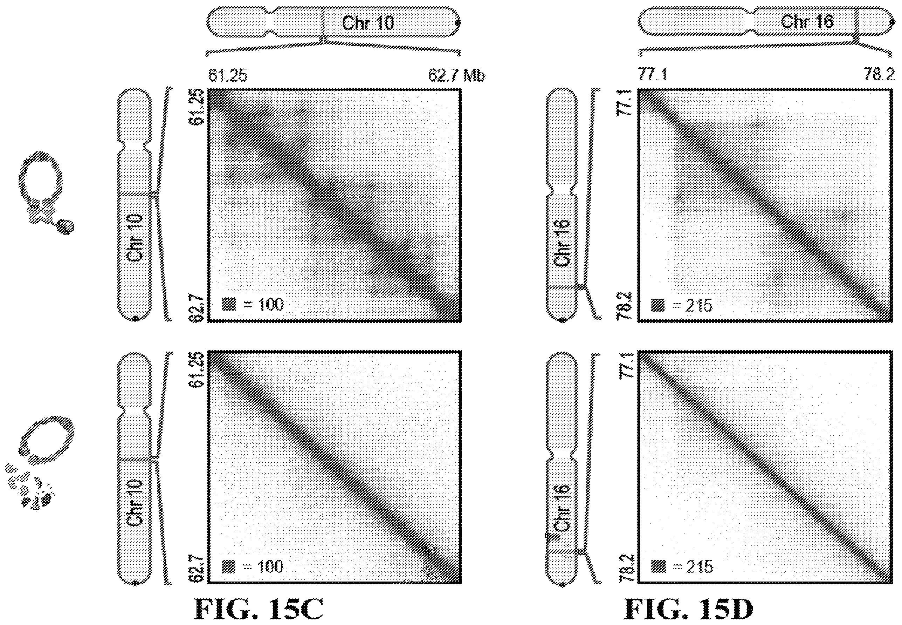

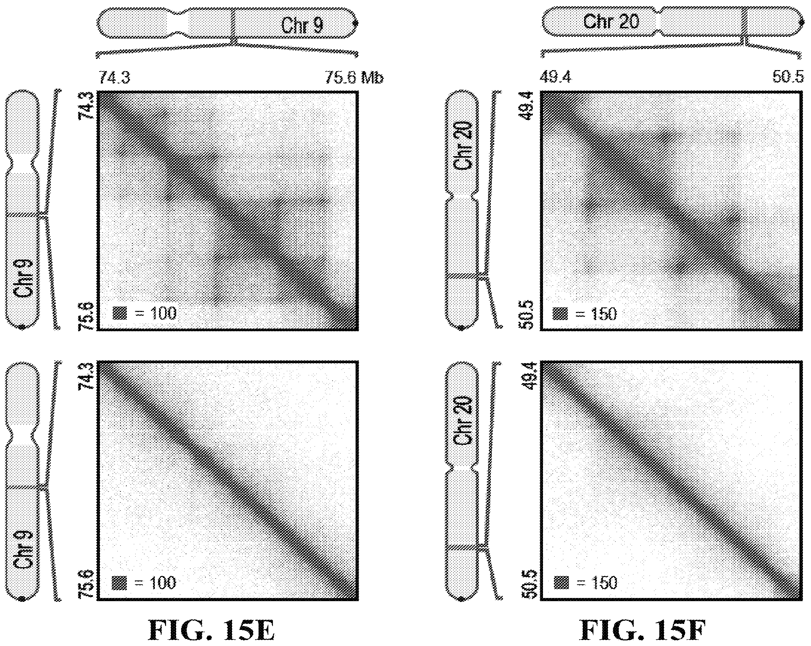

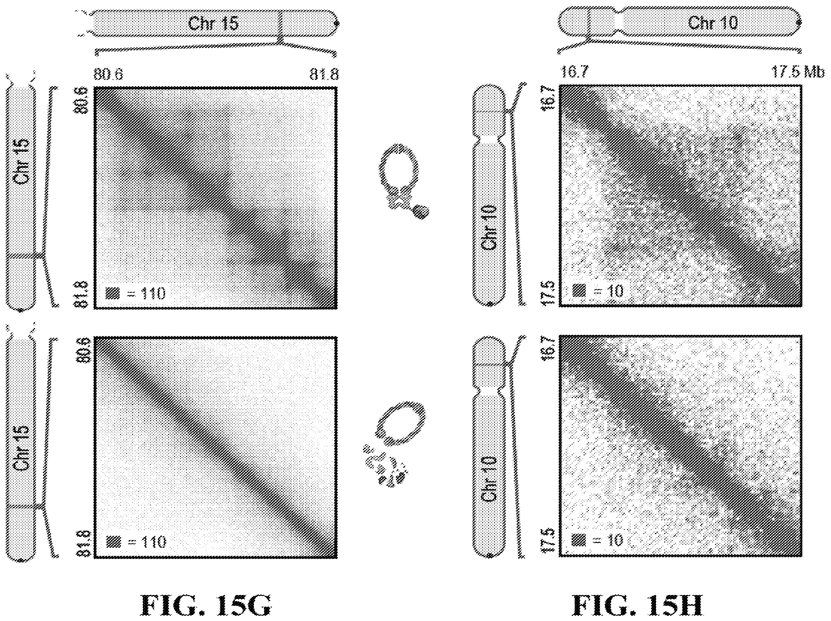

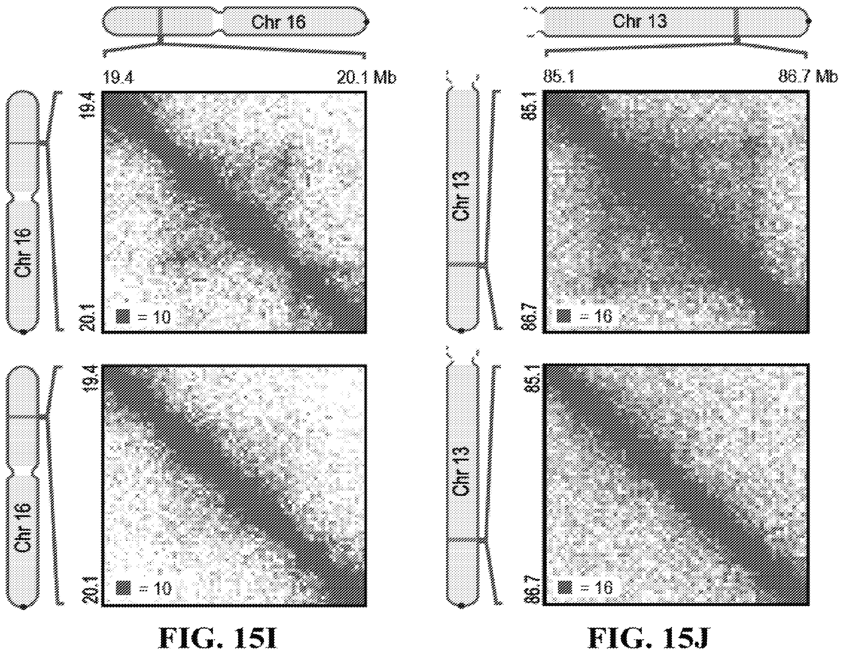

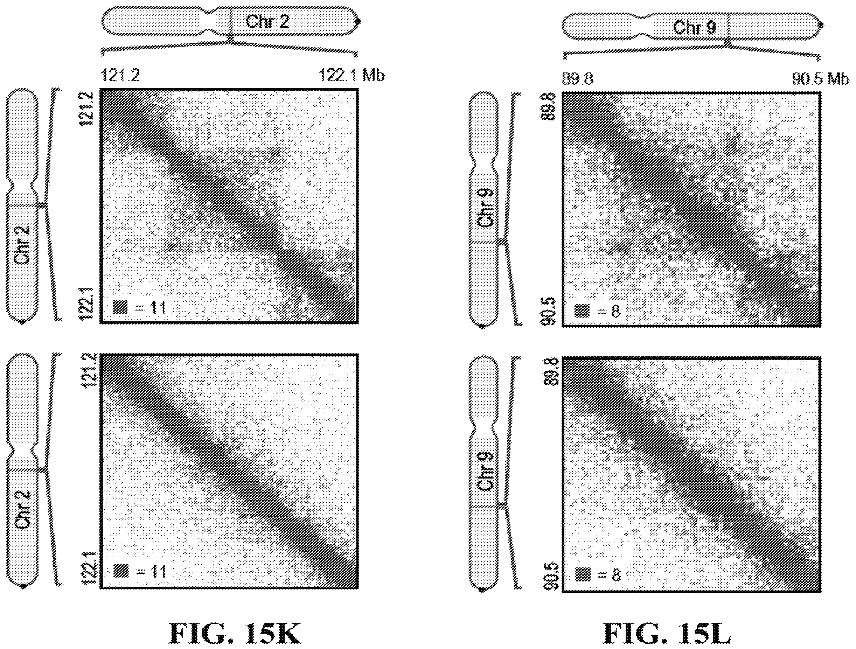

**FIG. 15-20** - Additional Hi-C contact matrices relevant to main findings, Related to Figures 2, 3, 4, 5, and 6.
**FIG. 15** - **Elimination of loop domains after cohesin loss. (A)** An example region from Figure 2A shown at different color scales: contact maps from untreated cells on top and maps from auxin treated cells on the bottom. The lack of visible loop domain structure is not a result of color scale choice; there is no residual loop domain structure. **(B)** Another example region from Figure 2A shown at different color scales; again there is no residual loop domain structure. (C-G) Additional examples of complete elimination of loop domains after auxin treatment and degradation of cohesin. (C: chr10: 61.25-62.7 Mb; D: chr16: 77.1-78.2 Mb; E: chr9: 74.3-75.6 Mb; F: chr20: 49.4-50.5 Mb; G: chr15:80.6-81.8 Mb). **(A)** To assure that the disappearance of loop domains after cohesin degradation did not arise as a result of cell cycle abnormalities, Applicants performed Hi-C on cells that were synchronized and arrested at the G1/S boundary before and during auxin treatment. Here, Applicants show an example of a loop domain (chr10: 16.7-17.5 Mb) that is present in our maps from G1/S-arrested cells and lost after auxin treatment. (I-L) Additional examples of loop domains present in G1/S-arrested cells and lost after auxin treatment. (I: chr16: 19.4-20.1 Mb; J: chr13: 85.1-86.7 Mb; K: chr2: 121.2-122.1 Mb; L: chr9: 89.8-90.5 Mb).

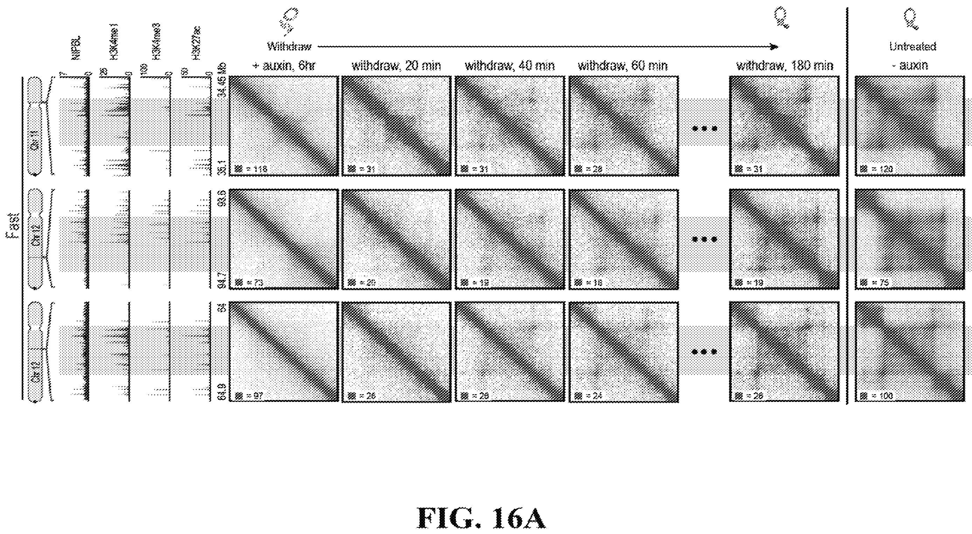

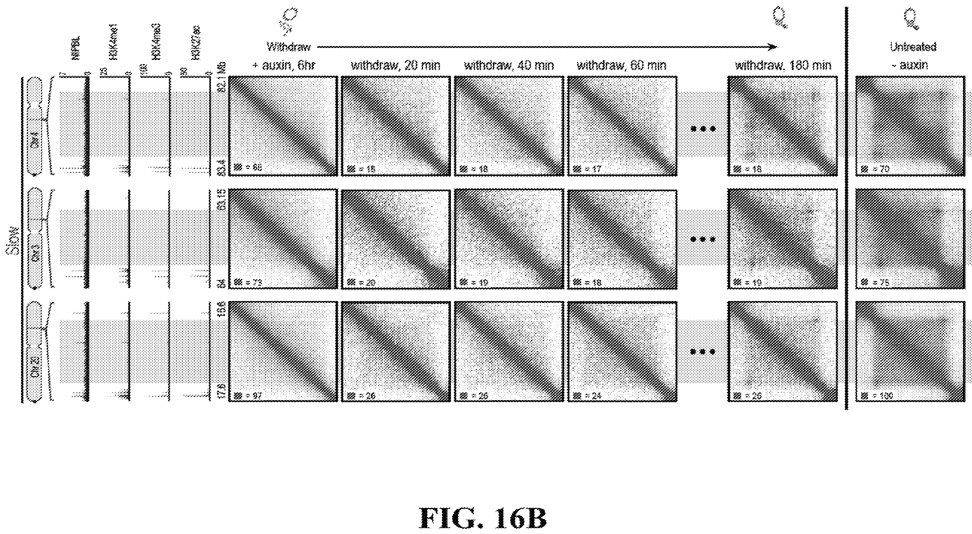

**FIG. 16** - **Variation in loop domain recovery across the genome. (A)** Three examples of regions containing fast loop domains (1st row: chr11:34.45-35.1Mb; 2nd row: chr12:93.6-94.7Mb; 3rd row: chr12:64-64.9Mb) are shown, along with ChIP-Seq tracks (from auxin-treated cells) for NIPBL, H3K4me1, H3K4me3, and H3K27Ac. For fast loop domains, reformation is apparent by 20-40 minutes after auxin withdrawal, and enrichment for NIPBL, H3K4me1, H3K4me3, and H3K27Ac is observed. **(B)** Three examples of regions containing slow loop domains (1st row: chr4:82.1-83.4Mb; 2nd row: chr3:63.15-64Mb; 3rd row: chr20:16.6-17.6Mb) are shown. For slow loop domains, reformation is not seen until 3 hours after auxin withdrawal and no enrichment for NIPBL, H3K4me1, H3K4me3, or H3K27Ac is observed.

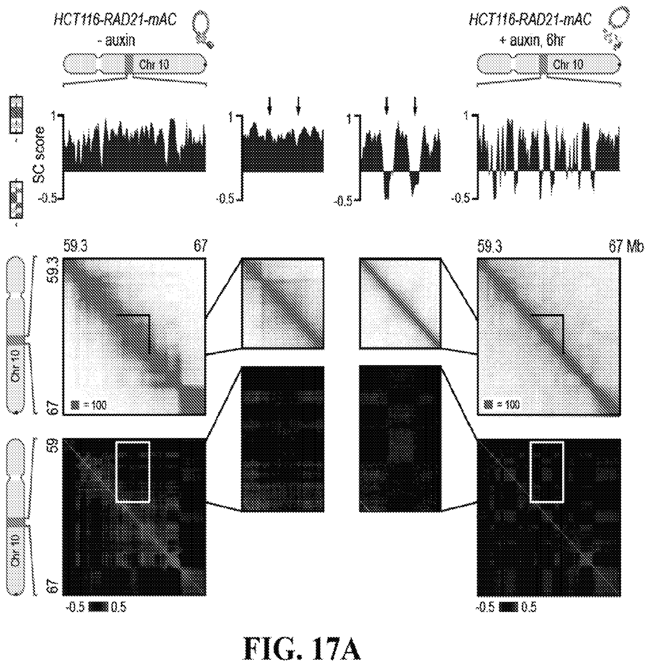

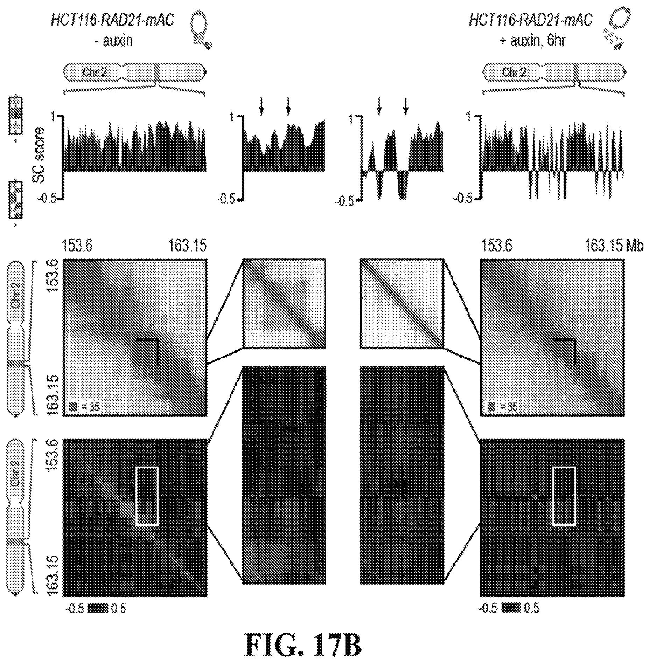

**FIG. 17** **- Comparison of compartment patterns before and after cohesin loss. (A,B)** Examples (A: chr10:59.3-67Mb and B: chr2:153.6-163.15Mb) showing that the loss of cohesin-associated loops after auxin treatment results in increased fine-scale compartmentalization. Top: Sliding correlation scores; valleys imply strong differences in long-range contact pattern observed at a locus as compared to neighboring loci, indicating a change in compartment (see Methods). Middle: Observed contact matrices. Bottom: Pearson's correlation maps for the local region shown (see Methods). Deeper valleys in the sliding correlation score and increased plaid patterning in the observed and Pearson's correlation maps indicate strengthened fine-scale compartment interactions after auxin treatment. Blowouts: loss of a loop domain results in strengthening of a compartment boundary spanned by the loop. Blown-out regions are indicated on zoomed out maps for both the observed (black upper triangle) and Pearson's correlation maps (white rectangle). Observed and Pearson's correlation maps are both shown at 25kb resolution for the zoomed out matrices and 10kb and 25kb resolution respectively for the blown-out matrices.

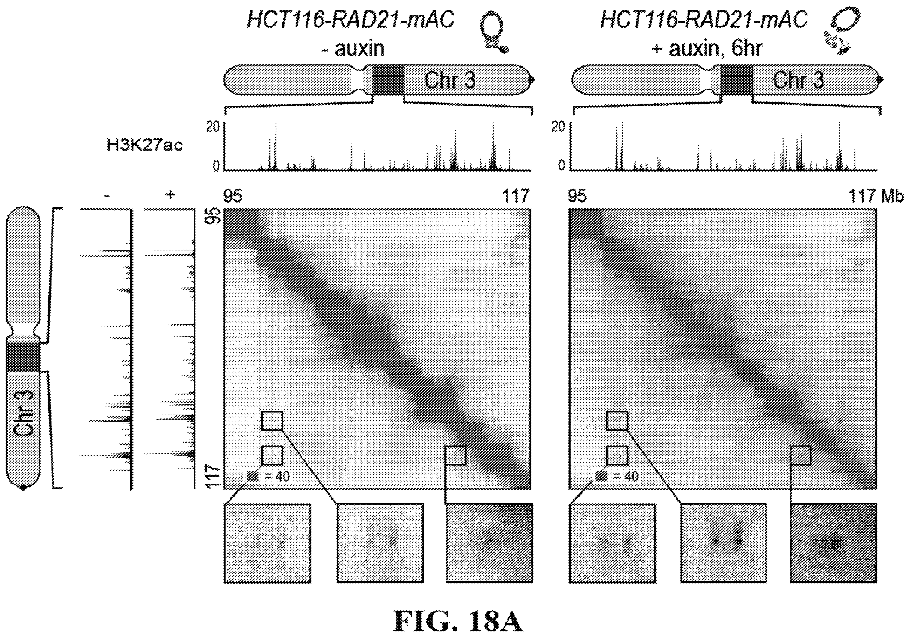

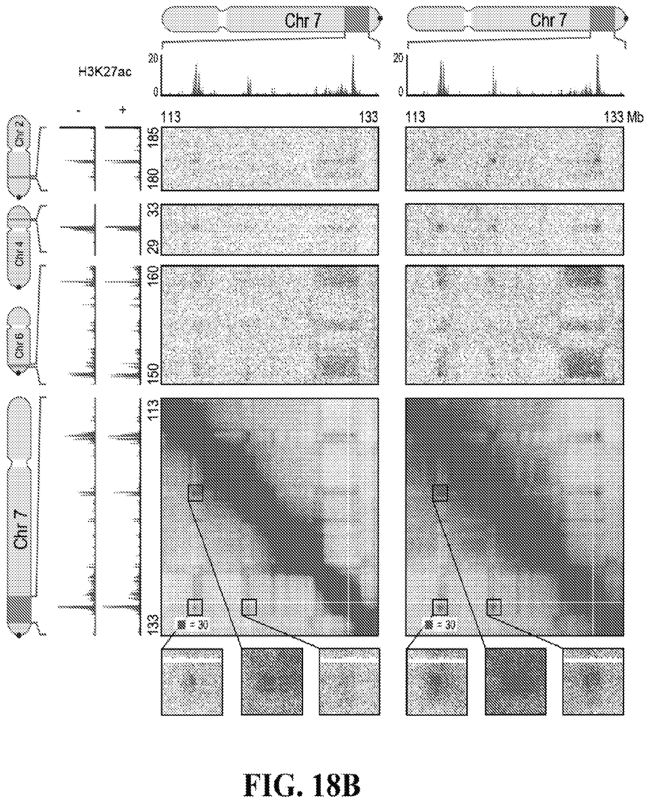

**FIG. 18** **- Examples of cohesin-independent links. (A)** Examples of a network of intrachromosomal. cohesin-independent links between superenhancers on chr3. H3K27 acetylation does not change with auxin treatment, but cohesin-independent links are significantly strengthened upon treatment. **(B)** Examples of a network of intra- and interchromosomal cohesin-independent links between superenhancers on chr7, chr6, chr4, and chr2. H3K27 acetylation does not change with auxin treatment, but cohesin-independent links are significantly strengthened upon treatment.

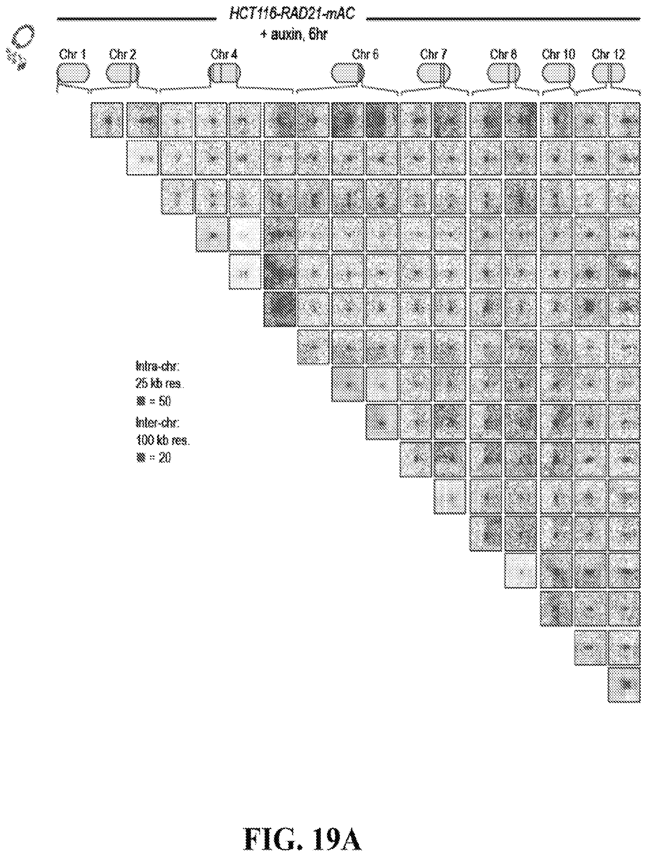

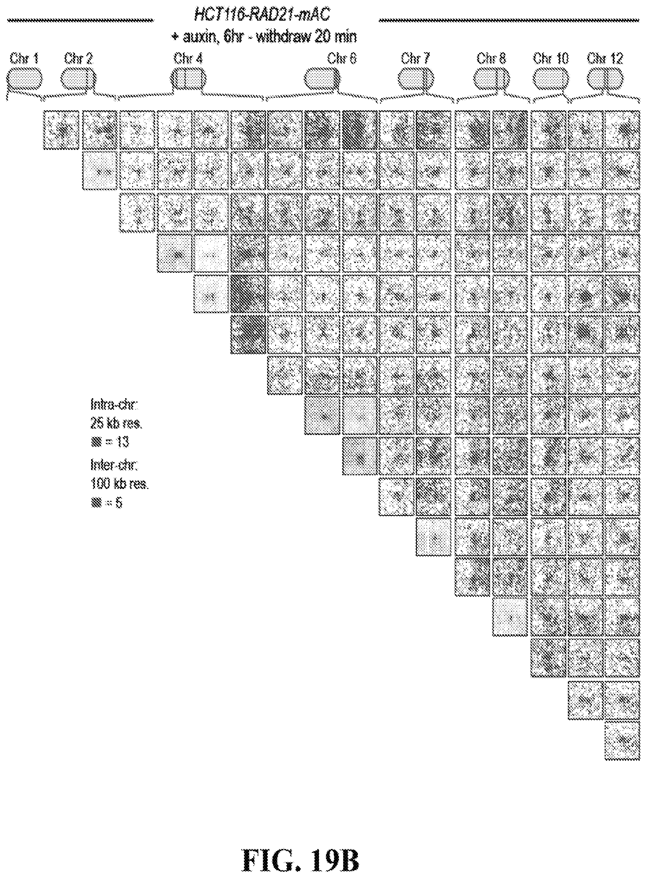

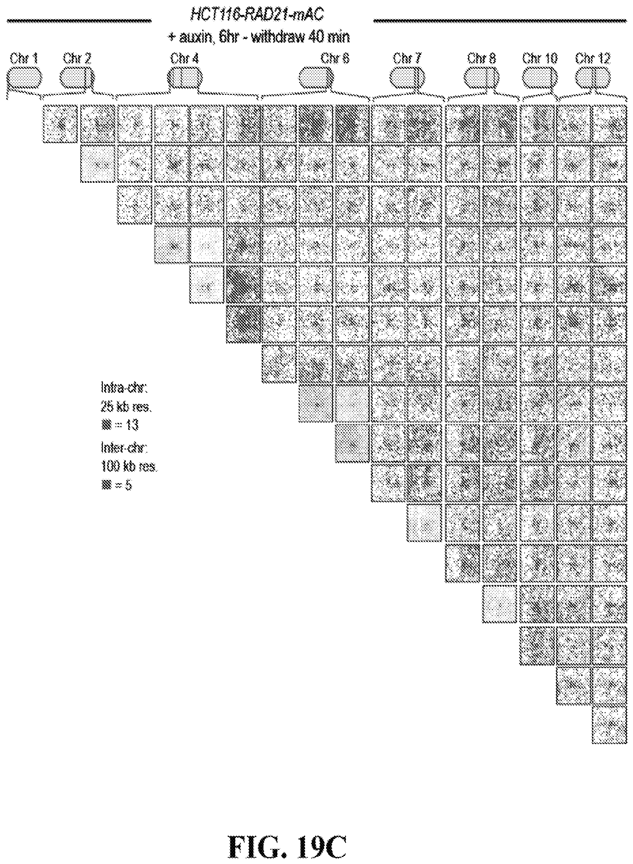

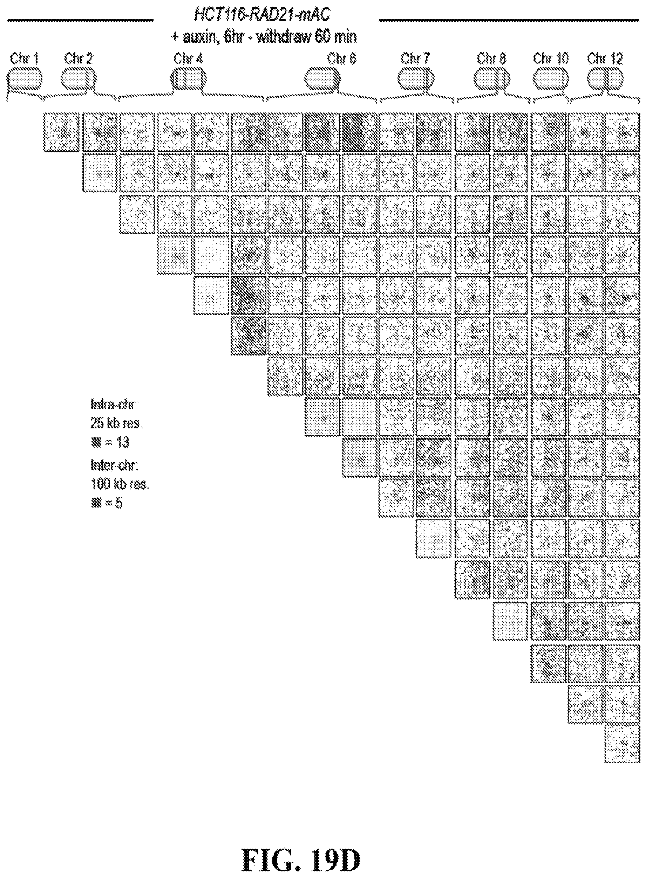

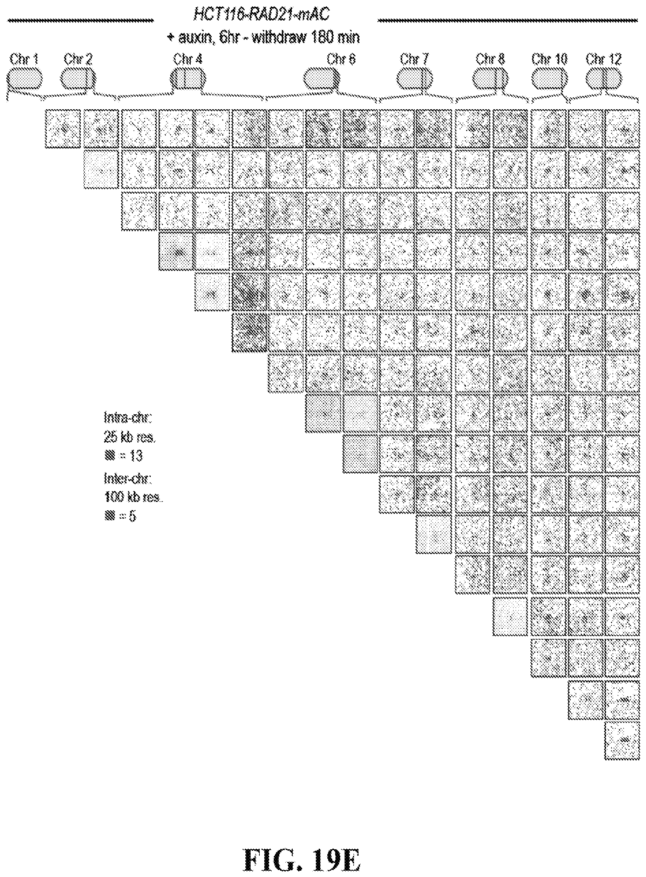

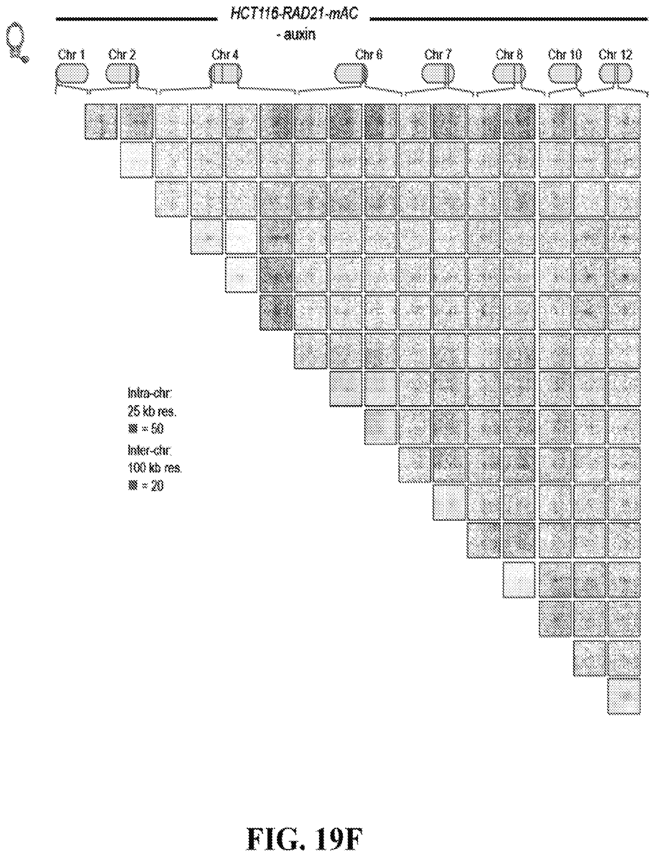

**FIG. 19** - **A large cohesin-independent clique across an auxin withdrawal time course.** The interactions between 17 cohesin-independent loop anchors spread across 8 chromosomes are shown along an auxin withdrawal time course (A: 6hr auxin treatment; B: 20min withdrawal of auxin; C: 40min withdrawal of auxin; D: 60 min withdrawal of auxin; E: 180 min withdrawal of auxin; F: no auxin treatment). Each matrix shows a 2 Mb by 2 Mb matrix centered on the respective anchors. Intrachromosomal interactions are shown at 25kb resolution with a maximum intensity of 50 reads (auxin treated and untreated maps) or 10 reads (40, 60, 180 min withdrawal maps); interchromosomal interactions are shown at 100kb resolution with a maximum intensity of 20 reads (auxin treated and untreated maps) or 4 reads (40, 60, 180 min withdrawal maps). Cohesin-independent links are rapidly weakened and lost upon withdrawal of auxin.

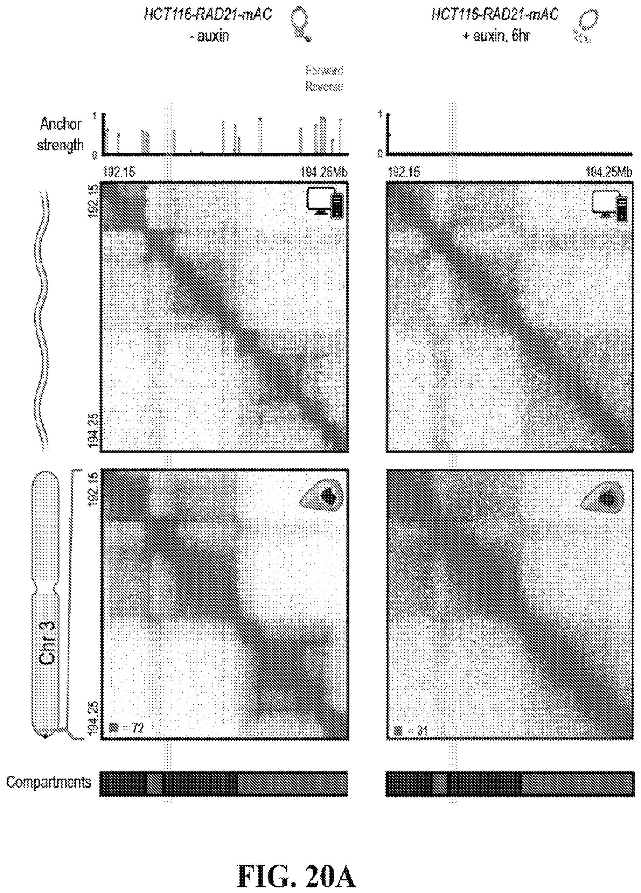

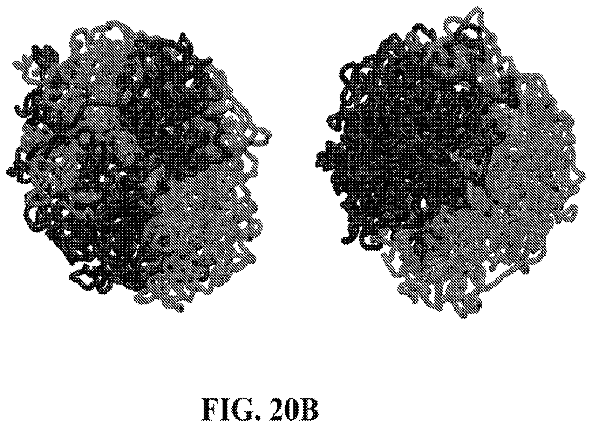

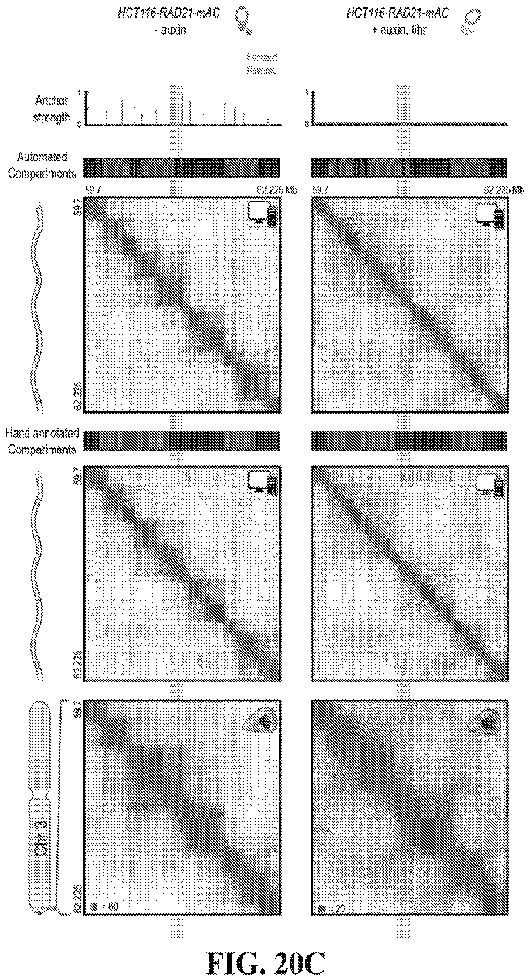

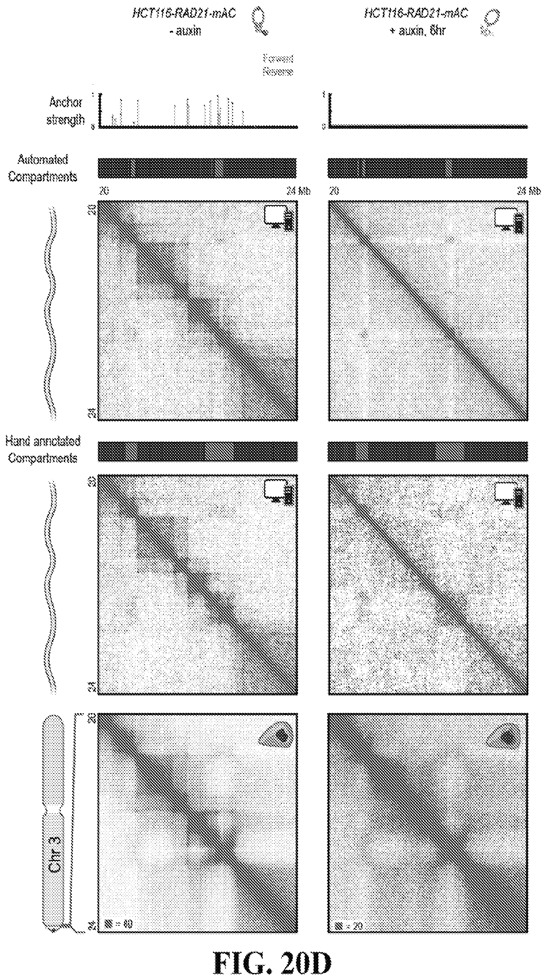

**FIG. 20** **- Simulations of extrusion and compartmentalization. (A)** Applicants use loop extrusion and compartmentalization to simulate a 2.1 Mb region on chromosome 3 in HCT-116 RAD21-mAC cells before (left) and after (right) auxin treatment. SMC1 ChIP-Seq signals are normalized and converted into binding probabilities for the simulated extrusion complex. Each peak is assigned a forward (green) or reverse (red) orientation based on the corresponding CTCF motif. Hi-C contact patterns in the treated map were used to determine the positions of compartment intervals (red and blue). The simulations yield an ensemble of polymer configurations. Applicants show contact maps from the simulated ensemble (top) and from the corresponding Hi-C experiments (bottom). The simulations accurately capture the positions of loops and domains, as well as the loss of loop domains after the depletion of cohesin. In addition, our simulation accurately captures compartmentalization patterns seen before and after auxin treatment. Notably, one of the loop domains spans multiple compartment intervals; the loci between the boundary of one of the compartment intervals and the loop anchor are highlighted (grey). **(B)** Examples of globules from simulations of compartmentalization with extrusion (left) and without (right). Notably, the globule without extrusion shows stronger segregation of compartment types. **(C)** Simulation of loop extrusion and compartmentalization in a 2.525 Mb region on chromosome 5 in HCT-116 RAD21mAC cells before (left) and after (right) auxin treatment. Compartment states were assigned either using an automated classification based on ChIP-Seq input data (top row), or a hand annotated compartment track (middle row). Notably, one of the loop domains spans multiple compartment intervals; the loci between the boundary of one of the compartment intervals and the loop anchor are highlighted (grey). **(D)** Simulations as in **(C),** for another region (chr4:20-24Mb). As in **(C),** simulations using an automated annotation of compartment state are shown in the top row, and simulations using a hand annotation are shown in the middle row. Real Hi-C data is shown in the bottom row.

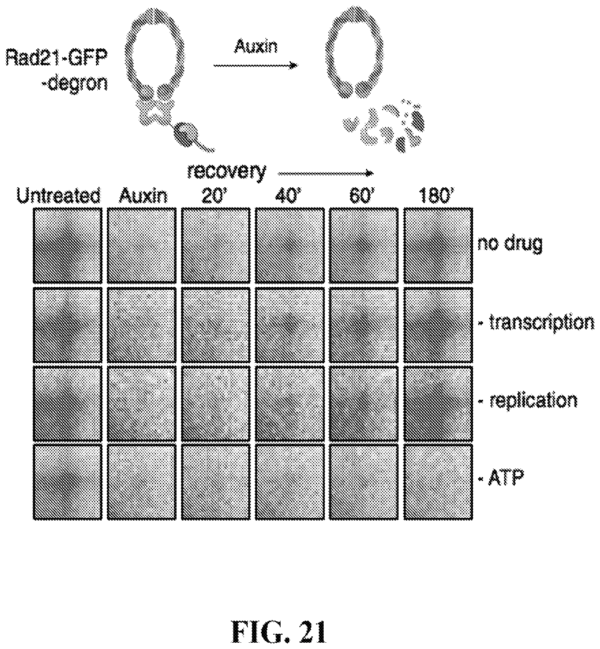

**FIG. 21** **- APA analysis of HCT-116 cells.** Rad21 is temporarily degraded and allowed to recover in the absence of ATP (treatment with oligomycin), transcription (treatment with flavopiridol), or replication (treatment with thymidine). Treatment with flavopiridol or thymidine does not block loop formation, but treatment with oligomycin does block loop formation.

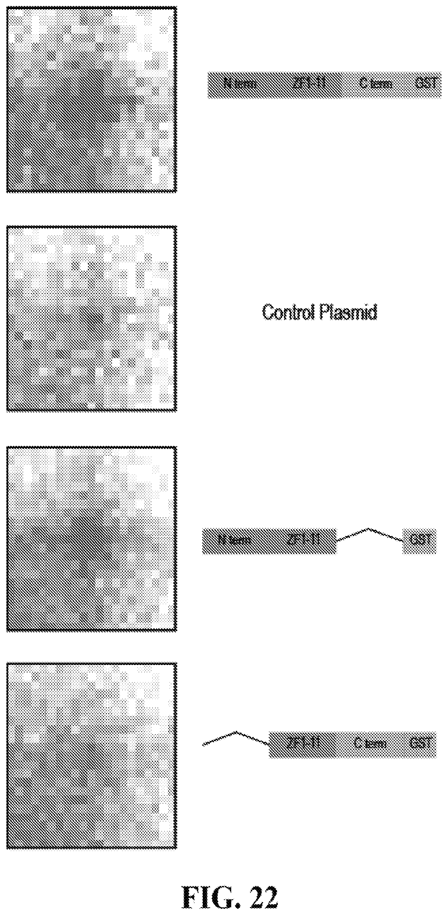

**FIG. 22** - **Rescue experiments in the CTCF-AID cell line.** WT CTCF and CTCF missing the C terminal domain rescue the looping phenotype. CTCF missing the N terminal domain is unable to halt cohesin and form loops.

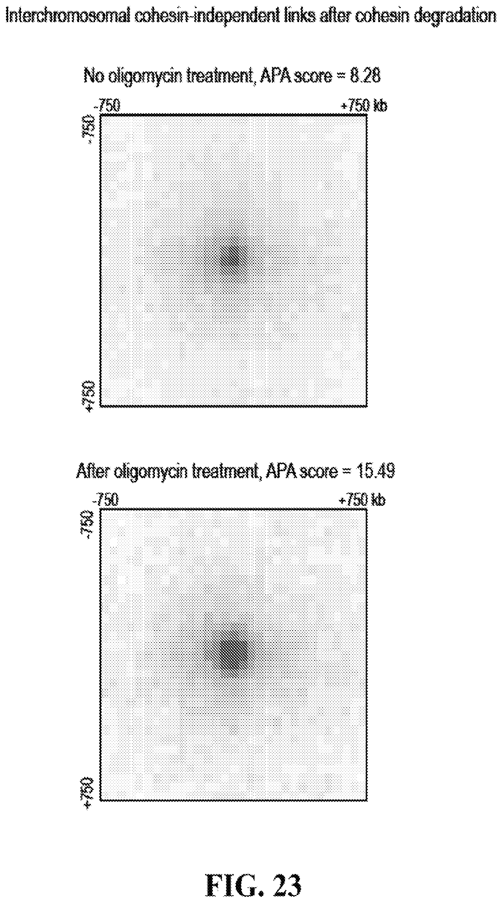

**FIG. 23** - **APA for cohesin-independent links in in situ Hi-C maps of HCT-116 RAD21-mAC cells after cohesin degradation by auxin addition for 6 hours,** without any additional treatment (top) and with additional treatment with oligomycin and 2DG to deplete ATP (bottom). Oligomycin and 2DG treatment to deplete ATP strengthens the interchromosomal cohesin-independent interactions between superenhancers (APA score of 8.28 [top] vs 15.49 [bottom]).
**FIG. 24** - **dCas9 can be used to reengineer chromatin loop and domain structures in an inducible manner. (A)** HI-C² contact map for the WT locus at chr8: 133.8-134.55 Mb in Hap1 cells. **(B)** left, HI-C contact map for the WT locus at chr6: 123.5-124.3 Mb in Hap1 cells. right, tiling of 7 dCas9/gRNAs from chr6: 123.925-123.930 Mb (>100kb from either loop anchor).

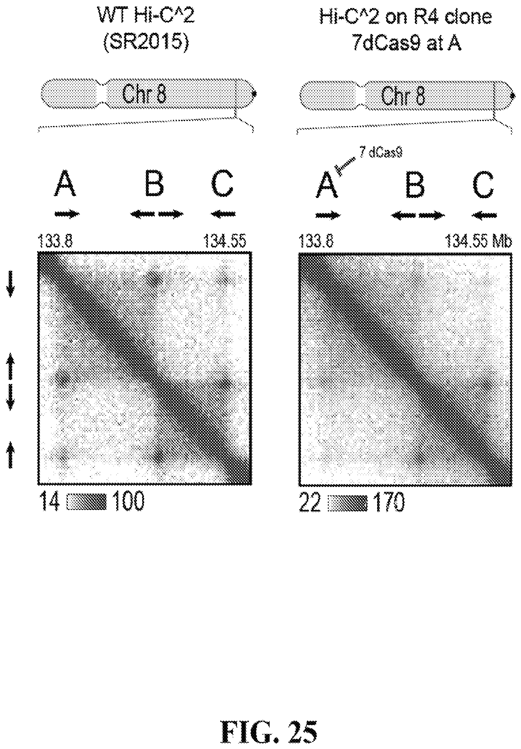

**FIG. 25** - **dCas9 can be used to reengineer chromatin loop and domain structures in an inducible manner.** HI-C² contact map for the WT locus at chr8: 133.8-134.55 Mb in Hap1 cells. left, no blocking. right blocking the A loop anchor (including the CTCF motif itself) with 7 dCas9/gRNAs.

The figures herein are for illustrative purposes only and are not necessarily drawn to scale. Color versions of the figures described above may be found in the publication Rao et al., Cohesin Loss Eliminates All Loop Domains, 2017, Cell 171, 305-320.

### DETAILED DESCRIPTION OF THE EXAMPLE EMBODIMENTS

### General Definitions

Unless defined otherwise, technical and scientific terms used herein have the same meaning as commonly understood by one of ordinary skill in the art to which this disclosure pertains. Definitions of common terms and techniques in molecular biology may be found in Molecular Cloning: A Laboratory Manual, 2nd edition (1989) (Sambrook, Fritsch, and Maniatis); Molecular Cloning: A Laboratory Manual, 4th edition (2012) (Green and Sambrook); Current Protocols in Molecular Biology (1987) (F.M. Ausubel et al. eds.); the series Methods in Enzymology (Academic Press, Inc.): PCR 2: A Practical Approach (1995) (M.J. MacPherson, B.D. Hames, and G.R. Taylor eds.): Antibodies, A Laboraotry Manual (1988) (Harlow and Lane, eds.): Antibodies A Laboraotry Manual, 2nd edition 2013 (E.A. Greenfield ed.); Animal Cell Culture (1987) (R.I. Freshney, ed.); Benjamin Lewin, Genes IX, published by Jones and Bartlet, 2008 (ISBN 0763752223); Kendrew et al. (eds.), The Encyclopedia of Molecular Biology, published by Blackwell Science Ltd., 1994 (ISBN 0632021829); Robert A. Meyers (ed.), Molecular Biology and Biotechnology: a Comprehensive Desk Reference, published by VCH Publishers, Inc., 1995 (ISBN 9780471185710); Singleton et al., Dictionary of Microbiology and Molecular Biology 2nd ed., J. Wiley & Sons (New York, N.Y. 1994), March, Advanced Organic Chemistry Reactions, Mechanisms and Structure 4th ed., John Wiley & Sons (New York, N.Y. 1992); and Marten H. Hofker and Jan van Deursen, Transgenic Mouse Methods and Protocols, 2nd edition (2011) .

As used herein, the singular forms "a", "an", and "the" include both singular and plural referents unless the context clearly dictates otherwise.

The term "optional" or "optionally" means that the subsequent described event, circumstance or substituent may or may not occur, and that the description includes instances where the event or circumstance occurs and instances where it does not.

The recitation of numerical ranges by endpoints includes all numbers and fractions subsumed within the respective ranges, as well as the recited endpoints.

The terms "about" or "approximately" as used herein when referring to a measurable value such as a parameter, an amount, a temporal duration, and the like, are meant to encompass variations of and from the specified value, such as variations of +/-10% or less, +/-5% or less, +/-1% or less, and +/-0.1% or less of and from the specified value, insofar such variations are appropriate to perform in the disclosed invention. It is to be understood that the value to which the modifier "about" or "approximately" refers is itself also specifically, and preferably, disclosed.

Unless otherwise noted, technical terms are used according to conventional usage. Definitions of common terms in molecular biology may be found in Benjamin Lewin, Genes IX, published by Jones and Bartlet, 2008 (ISBN 0763752223); Kendrew et al. (eds.), The Encyclopedia of Molecular Biology, published by Blackwell Science Ltd., 1994 (ISBN 0632021829); and Robert A. Meyers (ed.), Molecular Biology and Biotechnology: a Comprehensive Desk Reference, published by VCH Publishers, Inc., 1995 (ISBN 9780471185710).

The word "or" is intended to include "and" unless the context clearly indicates otherwise. The term "comprises" means "includes." In case of conflict, the present specification, including explanations of terms, will control.

Reference throughout this specification to "one embodiment", "an embodiment," "an example embodiment," means that a particular feature, structure or characteristic described in connection with the embodiment is included in at least one embodiment of the present invention. Thus, appearances of the phrases "in one embodiment," "in an embodiment," or "an example embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention. For example, in the appended claims, any of the claimed embodiments can be used in any combination.

Reference is made to WO2017106290 A1 ("Methods of detecting insulator dysfunction and oncogene activation for screening, diagnosis and treatment of patients in need thereof"); WO2017031370 A1 ("Methods and compositions for altering function and structure of chromatin loops and/or domains"-believed not be be prior art in the US as to this application in view of overlapping inventors and the present application being on file less than a year after publication); WO2017075294 A1 (Perturb seq); WO2016040476 A1 (Drop seq); WO2017164936 (single cell RNA seq); WO2011127150 A2; WO2011127150 A3; US20130090254 ("Gene-expression profiling with reduced numbers of transcript measurements"); and Rao et al., "Cohesin Loss Eliminates All Loop Domains," Cell 171:305-320 (October 5, 2017).

To facilitate review of the various embodiments of this disclosure, the following explanations of specific terms are provided:
**Amplification:** To increase the number of copies of a nucleic acid molecule, such as one or more end joined nucleic acid fragments that includes a junction, such as a ligation junction. The resulting amplification products are called "amplicons." Amplification of a nucleic acid molecule (such as a DNA or RNA molecule) refers to use of a technique that increases the number of copies of a nucleic acid molecule (including fragments).

An example of amplification is the polymerase chain reaction (PCR), in which a sample is contacted with a pair of oligonucleotide primers under conditions that allow for the hybridization of the primers to a nucleic acid template in the sample. The primers are extended under suitable conditions, dissociated from the template, re-annealed, extended, and dissociated to amplify the number of copies of the nucleic acid. This cycle can be repeated. The product of amplification can be characterized by such techniques as electrophoresis, restriction endonuclease cleavage patterns, oligonucleotide hybridization or ligation, and/or nucleic acid sequencing.

Other examples of *in vitro* amplification techniques include quantitative real-time PCR; reverse transcriptase PCR (RT-PCR); real-time PCR (rt PCR); real-time reverse transcriptase PCR (rt RT-PCR); nested PCR; strand displacement amplification (see U.S. Patent No. 5,744,311); transcription-free isothermal amplification (see U.S. Patent No. 6,033,881, repair chain reaction amplification (see WO 90/01069); ligase chain reaction amplification (see European patent publication EP-A-320 308); gap filling ligase chain reaction amplification (see U.S. Patent No. 5,427,930); coupled ligase detection and PCR (see U.S. Patent No. 6,027,889); and NASBA^{™} RNA transcription-free amplification (see U.S. Patent No. 6,025,134) amongst others.

**Binding or stable binding (of an oligonucleotide):** An oligonucleotide, such as a nucleic acid probe that specifically binds to a target junction in an end joined nucleic acid fragment, binds or stably binds to a target nucleic acid if a sufficient amount of the oligonucleotide forms base pairs or is hybridized to its target nucleic acid. For example depending in the hybridization conditions, there need not be complete matching between the probe and the nucleic acid target, for example there can be mismatch, or a nucleic acid bubble. Binding can be detected by either physical or functional properties.

**Binding site:** A region on a protein, DNA, or RNA to which other molecules stably bind. In one example, a binding site is the site on an end joined nucleic acid fragment.

**Biotin-14-CTP:** A biologically active analog of cytosine-5'-triphosphate that is readily incorporated into a nucleic acid by polymerase or a reverse transcriptase. In some examples, biotin-14-CTP is incorporated into a nucleic acid fragment that has a 3' overhang.

**Capture moieties:** Molecules or other substances that when attached to a nucleic acid molecule, such as an end joined nucleic acid, allow for the capture of the nucleic acid molecule through interactions of the capture moiety and something that the capture moiety binds to, such as a particular surface and/or molecule, such as a specific binding molecule that is capable of specifically binding to the capture moiety.

**Chromatin loop:** Chromatin fibers are arranged in living cells as independent loops anchored to the nuclear matrix or chromosomal scaffold. Specific DNA sequences act as anchors for these loops but it is not clear how flexible the anchors are. In order to fit DNA into the nucleus, it must be packaged into a highly compacted structure known as chromatin. In the first step of this process DNA is condensed into a 11 nm fiber that represents an approximate 6-fold level of compaction. This is achieved through nucleosome assembly, produced through interactions between DNA and histone proteins. Despite the extensive knowledge on the structure of the 11 nm nucleosome fiber, as well as metaphase chromosomes, the intermediate chromatin structures commonly described were largely hypothetical and had yet to be observed *in vivo.* The present invention has enabled to directly examine long-range interactions between chromosomal sequences *in situ.* This has produced convincing evidence that genes are configured into looped structures or chromatin loops that juxtapose regulatory elements to activate or repress transcription. The detection of loops *in situ* and the discovery how the majority of such loops is formed, now provides clear indiation of the factors that are involved in forming, maintaining and resolving such loops, and how they impact on gene expression. The present inventors have, through the use of *in situ* Hi-C technology, discovered that chromatin looping in the intact nucleus of a cell can be dynamically observed, quantified, and manipulated. Chromatin loop formation is the result of the presence of a pair of CTCF binding motifs in the convergent orientation on opposite strands of the DNA. Disruption of one of these motifs results in disappearance of the loop.

**Complementary:** A double-stranded DNA or RNA strand consists of two complementary strands of base pairs. Complementary binding occurs when the base of one nucleic acid molecule forms a hydrogen bond to the base of another nucleic acid molecule. Normally, the base adenine (A) is complementary to thymidine (T) and uracil (U), while cytosine (C) is complementary to guanine (G). For example, the sequence 5'-ATCG-3' of one ssDNA molecule can bond to 3'-TAGC-5' of another ssDNA to form a dsDNA. In this example, the sequence 5'-ATCG-3' is the reverse complement of 3'-TAGC-5'. Nucleic acid molecules can be complementary to each other even without complete hydrogen-bonding of all bases of each molecule. For example, hybridization with a complementary nucleic acid sequence can occur under conditions of differing stringency in which a complement will bind at some but not all nucleotide positions.

**Contacting:** Placement in direct physical association, including both in solid or liquid form, for example contacting a sample with a crosslinking agent or a probe..

**Control:** A reference standard. A control can be a known value or range of values indicative of basal levels or amounts or present in a tissue or a cell or populations thereof. A control can also be a cellular or tissue control, for example a tissue from a non-diseased state and/or exposed to different environmental conditions. A difference between a test sample and a control can be an increase or conversely a decrease. The difference can be a qualitative difference or a quantitative difference, for example a statistically significant difference.

**Covalently linked:** Refers to a covalent linkage between atoms by the formation of a covalent bond characterized by the sharing of pairs of electrons between atoms. In one example, a covalent link is a bond between an oxygen and a phosphorous, such as phosphodiester bonds in the backbone of a nucleic acid strand. In another example, a covalent link is one between a nucleic acid protein, another protein and/or nucleic acid that has been crosslinked by chemical means. In another example, a covalent link is one between fragmented nucleic acids.

**Crosslinking agent:** A chemical agent or even light, which facilitates the attachment of one molecule to another molecule. Crosslinking agents can be protein-nucleic acid crosslinking agents, nucleic acid-nucleic acid crosslinking agents, and protein-protein crosslinking agents. Examples of such agents are known in the art. In some embodiments, a crosslinking agent is a reversible crosslinking agent. In some embodiments, a crosslinking agent is a non-reversible crosslinking agent.

**CTCF:** Transcriptional repressor CTCF (UniProtKB P49711) also known as 11-zinc finger protein or CCCTC-binding factor is a transcription factor that in humans is encoded by the CTCF gene (Gene ID: 10664). This gene is a member of the BORIS + CTCF gene family and encodes a transcriptional regulator protein with 11 highly conserved zinc finger (ZF) domains. This nuclear protein is able to use different combinations of the ZF domains to bind different DNA target sequences and proteins. Depending upon the context of the site, the protein can bind a histone acetyltransferase (HAT)-containing complex and function as a transcriptional activator or bind a histone deacetylase (HDAC)-containing complex and function as a transcriptional repressor. If the protein is bound to a transcriptional insulator element, it can block communication between enhancers and upstream promoters, thereby regulating imprinted expression. Mutations in this gene have been associated with invasive breast cancers, prostate cancers, and Wilms' tumors. Alternatively spliced transcript variants encoding different isoforms have been found for this gene. Such variants and orthologs are in some embodiments incorporated in aspects of this invention. CTCF binds to a DNA sequence having sufficient sequence similarity (e.g. > 70% sequence similarity over the length of the sequence) to the consensus CTCF binding DNA sequence 5'-CCGCGNGGNGGCAG-3' (SEQ ID NO: 1) (in IUPAC notation), dubbed herein the CTCF binding motif or CTCF binding site. The binding to this sequence is defined by 11 zinc finger motifs in the CTCF protein structure. The binding of CTCF to DNA can be disrupted by CpG methylation of the binding site. The CpG sites or CG sites are regions of DNA where a cytosine nucleotide is followed by a guanine nucleotide in the linear sequence of bases along its 5' → 3' direction separated by only one phosphate (5'-C-phosphate-G-3'). Cytosines in CpG dinucleotides or CpG islands can be methylated to form 5-methylcytosine. The methyl group is added by DNA methyltransferases. Hence, site-specific methylation of the CTCF binding motif by methyltransferases can be used to disrupt binding of CTCF, and thereby loop formation.

**Detect:** To determine if an agent (such as a signal or particular nucleic acid or protein) is present or absent. In some examples, this can further include quantification in a sample, or a fraction of a sample, such as a particular cell or cells within a tissue.

**Detectable label:** A compound or composition that is conjugated directly or indirectly to another molecule to facilitate detection of that molecule. Specific, non-limiting examples of labels include fluorescent tags, enzymatic linkages, and radioactive isotopes and other physical tags, such as biotin. In some examples, a label is attached to a nucleic acid, such as an end-joined nucleic acid, to facilitate detection and/or isolation of the nucleic acid.

**DNA sequencing:** The process of determining the nucleotide order of a given DNA molecule. Generally, the sequencing can be performed using automated Sanger sequencing (AB13730xl genome analyzer), pyrosequencing on a solid support (454 sequencing, Roche), sequencing-by-synthesis with reversible terminations (ILLUMINA^{®} Genome Analyzer), sequencing-by-ligation (ABI SOLiD^{®}) or sequencing-by-synthesis with virtual terminators (HELISCOPE^{®}). In some embodiments, DNA sequencing is performed using a chain termination method developed by Frederick Sanger, and thus termed "Sanger based sequencing" or "SBS." This technique uses sequence-specific termination of a DNA synthesis reaction using modified nucleotide substrates. Extension is initiated at a specific site on the template DNA by using a short oligonucleotide primer complementary to the template at that region. The oligonucleotide primer is extended using DNA polymerase in the presence of the four deoxynucleotide bases (DNA building blocks), along with a low concentration of a chain terminating nucleotide (most commonly a di-deoxynucleotide). Limited incorporation of the chain terminating nucleotide by the DNA polymerase results in a series of related DNA fragments that are terminated only at positions where that particular nucleotide is present. The fragments are then size-separated by electrophoresis a polyacrylamide gel, or in a narrow glass tube (capillary) filled with a viscous polymer. An alternative to using a labeled primer is to use labeled terminators instead; this method is commonly called "dye terminator sequencing." "Pyrosequencing" is an array based method, which has been commercialized by 454 Life Sciences. In some embodiments of the array-based methods, single-stranded DNA is annealed to beads and amplified via EmPCR^{®}. These DNA-bound beads are then placed into wells on a fiber-optic chip along with enzymes that produce light in the presence of ATP. When free nucleotides are washed over this chip, light is produced as the PCR amplification occurs and ATP is generated when nucleotides join with their complementary base pairs. Addition of one (or more) nucleotide(s) results in a reaction that generates a light signal that is recorded, such as by the charge coupled device (CCD) camera, within the instrument. The signal strength is proportional to the number of nucleotides, for example, homopolymer stretches, incorporated in a single nucleotide flow.

**Domain:** A self-interacting segment of mammalian chromatin manifested as squares of enriched contacts revealed along the diagonal of a Hi-C contact map. The inventors have shown that a genome is partitioned into domains that are associated with particular patterns of histone marks that segregates into sub-compartments, distinguished by unique long-range contact patterns. Domain includes reference to superdomain and loop domain. A loop domain is a domain whose endpoints are anchored to form a chromatin loop. Loops are anchored at DNA sites bound by higher-order "loop anchor complexes" containing loop anchor proteins, including CTCF and cohesin, and other factors. Many loops demarcate domains; the vast majority of loops are anchored at a pair of convergent CTCF/RAD21/SMC3 binding sites. The pairs of CTCF motifs that anchor a loop are nearly all found in the convergent orientation. The inactive X chromosome (Xi) is found to be partitioned into two large "superdomains" whose boundary lies near the locus of the lncRNA DXZ4 (Chadwick, 2008). Applicants also detect a network of extremely long-range (7 - 74Mb) "superloops", the strongest of which are anchored at locations containing lncRNA genes (loc550643, XIST, DXZ4, and FIRRE). With the exception of XIST, all of these lncRNAs contain CTCF-binding tandem repeats that bind CTCF only on the inactive X.

**Exclusion domain:** A contact domain formed as a result of the formation of a loop by an extrusion complex between adjacent forward and reverse motifs in the convergent orientation, wherein a third CTCF motif downstream of the revers or upstream of the forward motif causes the an extrusion complex that lands in the interval between the two reverse or two forward motives is obstructed on both sides, tends to remain inside the Interval, thereby resulting in the formation of a domain.

**Fluorophore:** A chemical compound, which when excited by exposure to a particular stimulus such as a defined wavelength of light, emits light (fluoresces), for example at a different wavelength (such as a longer wavelength of light). Fluorophores are part of the larger class of luminescent compounds. Luminescent compounds include chemiluminescent molecules, which do not require a particular wavelength of light to luminesce, but rather use a chemical source of energy. Therefore, the use of chemiluminescent molecules (such as aequorin) eliminates the need for an external source of electromagnetic radiation, such as a laser.

Examples of particular fluorophores that can be used in the probes disclosed herein are provided in U.S. Patent No. 5,866,366 to Nazarenko et al., such as 4-acetamido-4'-isothiocyanatostilbene-2,2'disulfonic acid, acridine and derivatives such as acridine and acridine isothiocyanate, 5-(2'-aminoethyl)aminonaphthalene-1-sulfonic acid (EDANS), 4-amino-N-[3-vinylsulfonyl)phenyl]naphthalimide-3,5 disulfonate (Lucifer Yellow VS), N-(4-anilino-1-naphthyl)maleimide, anthranilamide, Brilliant Yellow, coumarin and derivatives such as coumarin, 7-amino-4-methylcoumarin (AMC, Coumarin 120), 7-amino-4-trifluoromethylcouluarin (Coumaran 151); cyanosine; 4',6-diaminidino-2-phenylindole (DAPI); 5', 5"-dibromopyrogallol-sulfonephthalein (Bromopyrogallol Red); 7-diethylamino-3-(4'-isothiocyanatophenyl)-4-methylcoumarin; diethylenetriamine pentaacetate; 4,4'-diisothiocyanatodihydro-stilbene-2,2'-disulfonic acid; 4,4'-diisothiocyanatostilbene-2,2'-disulfonic acid; 5-[dimethylamino]naphthalene-1-sulfonyl chloride (DNS, dansyl chloride); 4-dimethylaminophenylazophenyl-4'-isothiocyanate (DABITC); eosin and derivatives such as eosin and eosin isothiocyanate; erythrosin and derivatives such as erythrosin B and erythrosin isothiocyanate; ethidium; fluorescein and derivatives such as 5-carboxyfluorescein (FAM), 5-(4,6-dichlorotriazin-2-yl)aminofluorescein (DTAF), 2'7'-dimethoxy-4'5'-dichloro-6-carboxyfluorescein (JOE), fluorescein, fluorescein isothiocyanate (FITC), and QFITC (XRITC); fluorescamine; IR144; IR1446; Malachite Green isothiocyanate; 4-methylumbelliferone; ortho cresolphthalein; nitrotyrosine; pararosaniline; Phenol Red; B-phycoerythrin; o-phthaldialdehyde; pyrene and derivatives such as pyrene, pyrene butyrate and succinimidyl 1-pyrene butyrate; Reactive Red 4 (Cibacron^{™}. Brilliant Red 3B-A); rhodamine and derivatives such as 6-carboxy-X-rhodamine (ROX), 6-carboxyrhodamine (R6G), lissamine rhodamine B sulfonyl chloride, rhodamine (Rhod), rhodamine B, rhodamine 123, rhodamine X isothiocyanate, sulforhodamine B, sulforhodamine 101 and sulfonyl chloride derivative of sulforhodamine 101 (Texas Red); N,N,N',N'-tetramethyl-6-carboxyrhodamine (TAMRA); tetramethyl rhodamine; tetramethyl rhodamine isothiocyanate (TRITC); riboflavin; rosolic acid and terbium chelate derivatives; LightCycler Red 640; Cy5.5; and Cy56-carboxyfluorescein; 5-carboxyfluorescein (5-FAM); boron dipyrromethene difluoride (BODIPY); N,N,N',N'-tetramethyl-6-carboxyrhodamine (TAMRA); acridine, stilbene, -6-carboxy-fluorescein (HEX), TET (Tetramethyl fluorescein), 6-carboxy-X-rhodamine (ROX), Texas Red, 2',7'-dimethoxy-4',5'-dichloro-6-carboxyfluorescein (JOE), Cy3, Cy5, VIC^{®} (Applied Biosystems), LC Red 640, LC Red 705, Yakima yellow amongst others.

**Genome or nucleic acid editing and the like:** In some embodiments, editing of a genome, for example as described herein, includes inserting, deleting, or otherwise altering the nucleic acid sequence of the genome, for example in a cell. In certain embodiments this can include using a genome editing system, such as a CRISPR/Cas, system, a TALEN system, a ZFN system, a meganuclease and the like.

**High throughput technique:** Through a combination of robotics, data processing and control software, liquid handling devices, and detectors, high throughput techniques allows the rapid screening of potential reagents, conditions, or targets in a short period of time, for example in less than 24, less than 12, less than 6 hours, or even less than 1 hour.

**Hybridization:** Oligonucleotides and their analogs hybridize by hydrogen bonding, which includes Watson-Crick, Hoogsteen or reversed Hoogsteen hydrogen bonding, between complementary bases. Generally, nucleic acid consists of nitrogenous bases that are either pyrimidines (cytosine (C), uracil (U), and thymine (T)) or purines (adenine (A) and guanine (G)). These nitrogenous bases form hydrogen bonds between a pyrimidine and a purine, and the bonding of the pyrimidine to the purine is referred to as "base pairing." More specifically, A will hydrogen bond to T or U, and G will bond to C. "Complementary" refers to the base pairing that occurs between two distinct nucleic acid sequences or two distinct regions of the same nucleic acid sequence. "Specifically hybridizable" and "specifically complementary" are terms that indicate a sufficient degree of complementarity such that stable and specific binding occurs between the oligonucleotide (or it's analog) and the DNA, RNA, and or DNA-RNA hybrid target. The oligonucleotide or oligonucleotide analog need not be 100% complementary to its target sequence to be specifically hybridizable. An oligonucleotide or analog is specifically hybridizable when there is a sufficient degree of complementarity to avoid non-specific binding of the oligonucleotide or analog to non-target sequences under conditions where specific binding is desired. Such binding is referred to as specific hybridization.

**Isolated:** An "isolated" biological component (such as the end joined fragmented nucleic acids described herein) has been substantially separated or purified away from other biological components in the cell of the organism, in which the component naturally occurs, for example, extra-chromatin DNA and RNA, proteins and organelles. Nucleic acids and proteins that have been "isolated" include nucleic acids and proteins purified by standard purification methods, for example from a sample. The term also embraces nucleic acids and proteins prepared by recombinant expression in a host cell as well as chemically synthesized nucleic acids. It is understood that the term "isolated" does not imply that the biological component is free of trace contamination, and can include nucleic acid molecules that are at least 50% isolated, such as at least 75%, 80%, 90%, 95%, 98%, 99%, or even 100% isolated.

**Junction:** A site where two nucleic acid fragments or joined, for example using the methods described herein. A junction encodes information about the proximity of the nucleic acid fragments that participate in formation of the junction. For example, junction formation between to nucleic acid fragments indicates that these two nucleic acid sequences where in close proximity when the junction was formed, although they may not be in proximity in liner nucleic acid sequence space. Thus, a junction can define ling range interactions. In some embodiments, a junction is labeled, for example with a labeled nucleotide, for example to facilitate isolation of the nucleic acid molecule that includes the junction.

**Motif:** A nucleic acid sequence to which a protein will bind to directly (e.g. through a zinc finger) or indirectly (e.g. via a protein mediator). One example of a motif is a CTCF motif capable of binding CTCF.

**Nucleic acid (molecule or sequence):** A deoxyribonucleotide or ribonucleotide polymer including without limitation, cDNA, mRNA, genomic DNA, and synthetic (such as chemically synthesized) DNA or RNA or hybrids thereof. The nucleic acid can be double-stranded (ds) or single-stranded (ss). Where single-stranded, the nucleic acid can be the sense strand or the antisense strand. Nucleic acids can include natural nucleotides (such as A, T/U, C, and G), and can also include analogs of natural nucleotides, such as labeled nucleotides. Some examples of nucleic acids include the probes disclosed herein. The major nucleotides of DNA are deoxyadenosine 5 '-triphosphate (dATP or A), deoxyguanosine 5 '-triphosphate (dGTP or G), deoxycytidine 5 '-triphosphate (dCTP or C) and deoxythymidine 5 '-triphosphate (dTTP or T). The major nucleotides of RNA are adenosine 5 '-triphosphate (ATP or A), guanosine 5'-triphosphate (GTP or G), cytidine 5 '-triphosphate (CTP or C) and uridine 5'-triphosphate (UTP or U). Nucleotides include those nucleotides containing modified bases, modified sugar moieties, and modified phosphate backbones, for example as described in U.S. Patent No. 5,866,336 to Nazarenko et al.

Examples of modified base moieties which can be used to modify nucleotides at any position on its structure include, but are not limited to: 5-fluorouracil, 5-bromouracil, 5-chlorouracil, 5-iodouracil, hypoxanthine, xanthine, acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N~6-sopentenyladenine, 1 -methylguanine, 1-methylinosine, 2,2-dimethylguanine, 2-methyladenine, 2-methylguanine, 3-methylcytosine, 5 -methyl cytosine, N6-adenine, 7-methylguanine, 5-methylaminomethyluracil, methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid, pseudouracil, queosine, 2-thiocytosine, 5-methyl-2-thiouracil, 2-thiouracil, 4-thiouracil, 5-methyluracil, uracil-5-oxyacetic acid methylester, uracil-S-oxyacetic acid, 5-methyl-2-thiouracil, 3-(3-amino-3-N-2-carboxypropyl) uracil, 2,6-diaminopurine and biotinylated analogs, amongst others.

Examples of modified sugar moieties which may be used to modify nucleotides at any position on its structure include, but are not limited to arabinose, 2-fluoroarabinose, xylose, and hexose, or a modified component of the phosphate backbone, such as phosphorothioate, a phosphorodithioate, a phosphoramidothioate, a phosphoramidate, a phosphordiamidate, a methylphosphonate, an alkyl phosphotriester, or a formacetal or analog thereof.

**Loop anchor:** The present inventors herein describe new, one-kilobase-resolution contact maps of the human genome created by using *in situ* Hi-C, which couples DNA-DNA proximity ligation in intact nuclei (nuclear ligation assay) with high-throughput sequencing. The maps - containing over 15 billion contacts - allowed the inventors to annotate nearly 9,000 contact domains, which are contiguous genomic intervals in which there is an enhanced probability of contact among all loci. Contact domains range in size from tens of kilo bases to several megabases, with a median size of 185kb. The inventors found that many contact domains are also "loop domains" - that is, contact domains whose boundaries are demarcated by the endpoints of a chromatin loop. These endpoints are revealed in the contact maps from Hi-C data as numerous nodes (local maxima) that correspond to loop anchor loci at the boundary of a domain. The inventors found that contact domains often correspond to loops - that is, the two boundaries of the domain lie at the loop's two anchor loci, which are spatially proximate. The inventors dubbed this common configuration a "loop domain." The maps allowed the inventors to annotate over 10,000 loops. These loops typically lie between convergent DNA motifs (i.e., motifs pointing toward one another) that bind a complex containing CTCF and cohesin. Thus, each anchor site typically contains a motif that binds a complex containing CTCF and cohesin. The ubiquity of the convergent orientation or configuration of these CTCF binding motifs suggests that the binding of CTCF and cohesin is responsible for the formation of loops and domains at precise genomic coordinates. The CTCF binding motif is a loop anchor motif, forming a loop anchor for the "extrusion complex" containing CTCF and cohesin, which stabilizes the domain loop at its anchor point(s).

**Primers:** Short nucleic acid molecules, such as a DNA oligonucleotide, which can be annealed to a complementary target nucleic acid molecule by nucleic acid hybridization to form a hybrid between the primer and the target nucleic acid strand. A primer can be extended along the target nucleic acid molecule by a polymerase enzyme. Therefore, primers can be used to amplify a target nucleic acid molecule, wherein the sequence of the primer is specific for the target nucleic acid molecule, for example so that the primer will hybridize to the target nucleic acid molecule under very high stringency hybridization conditions. The specificity of a primer increases with its length. Thus, for example, a primer that includes 30 consecutive nucleotides will anneal to a target sequence with a higher specificity than a corresponding primer of only 15 nucleotides. Thus, to obtain greater specificity, probes and primers can be selected that include at least 5, 10, 15, 20, 25, 30, 35, 40, 45, 50 or more consecutive nucleotides. In particular examples, a primer is at least 15 nucleotides in length, such as at least 5 contiguous nucleotides complementary to a target nucleic acid molecule. Particular lengths of primers that can be used to practice the methods of the present disclosure include primers having at least 5, at least 10, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 45, at least 50, or more contiguous nucleotides complementary to the target nucleic acid molecule to be amplified, such as a primer of 5-60 nucleotides, 15-50 nucleotides, 15-30 nucleotides or greater. Primer pairs can be used for amplification of a nucleic acid sequence, for example, by PCR, or other nucleic-acid amplification methods known in the art. An "upstream" or "forward" primer is a primer 5' to a reference point on a nucleic acid sequence. A "downstream" or "reverse" primer is a primer 3' to a reference point on a nucleic acid sequence. In general, at least one forward and one reverse primer are included in an amplification reaction. PCR primer pairs can be derived from a known sequence, for example, by using computer programs intended for that purpose such as Primer (Version 0.5, ^{©} 1991, Whitehead Institute for Biomedical Research, Cambridge, MA). Methods for preparing and using primers are described in, for example, Sambrook et al. (1989) Molecular Cloning: A Laboratory Manual, Cold Spring Harbor, New York; Ausubel et al. (1987) Current Protocols in Molecular Biology, Greene Publ. Assoc. & Wiley-Intersciences.

**Probe:** A probe comprises an isolated nucleic acid capable of hybridizing to a target nucleic acid (such as end joined nucleic acid fragment). A detectable label or reporter molecule can be attached to a probe. Typical labels include radioactive isotopes, enzyme substrates, cofactors, ligands, chemiluminescent or fluorescent agents, haptens, and enzymes. Methods for labeling and guidance in the choice of labels appropriate for various purposes are discussed, for example, in Sambrook et al., Molecular Cloning: A Laboratory Manual, Cold Spring Harbor Laboratory Press (1989) and Ausubel et al., Current Protocols in Molecular Biology, Greene Publishing Associates and Wiley-Intersciences (1987). Probes are generally at least 5 nucleotides in length, such as at least 10, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45, at least 46, at least 47, at least 48, at least 49, at least 50 at least 51, at least 52, at least 53, at least 54, at least 55, at least 56, at least 57, at least 58, at least 59, at least 60, or more contiguous nucleotides complementary to the target nucleic acid molecule, such as 50-60 nucleotides, 20-50 nucleotides, 20-40 nucleotides, 20-30 nucleotides or greater.

**Targeting probe:** A probe that includes an isolated nucleic acid capable of hybridizing to a junction in a end joined nucleic acid fragment, wherein the probe specifically hybridizes to the end joined nucleic acid fragment both 5' and 3' of the site of the junction and spans the site of the junction.

**Target junction:** Any nucleic acid present or thought to be present in a sample that the information of a junction between an end joined nucleic acid fragment about which information would like to be obtained, such as its presence or absence.

**Sample:** A sample, such as a biological sample, that includes biological materials (such as nucleic acid and proteins, for example double-stranded nucleic acid binding proteins) obtained from an organism or a part thereof, such as a plant, animal, bacteria, and the like. In particular embodiments, the biological sample is obtained from an animal subject, such as a human subject. A biological sample is any solid or fluid sample obtained from, excreted by or secreted by any living organism, including without limitation, single celled organisms, such as bacteria, yeast, protozoans, and amebas among others, multicellular organisms (such as plants or animals, including samples from a healthy or apparently healthy human subject or a human patient affected by a condition or disease to be diagnosed or investigated, such as cancer). For example, a biological sample can be a biological fluid obtained from, for example, blood, plasma, serum, urine, bile, ascites, saliva, cerebrospinal fluid, aqueous or vitreous humor, or any bodily secretion, a transudate, an exudate (for example, fluid obtained from an abscess or any other site of infection or inflammation), or fluid obtained from a joint (for example, a normal joint or a joint affected by disease, such as a rheumatoid arthritis, osteoarthritis, gout or septic arthritis). A sample can also be a sample obtained from any organ or tissue (including a biopsy or autopsy specimen, such as a tumor biopsy) or can include a cell (whether a primary cell or cultured cell) or medium conditioned by any cell, tissue or organ.

**Specific Binding Agent:** An agent that binds substantially or preferentially only to a defined target such as a protein, enzyme, polysaccharide, oligonucleotide, DNA, RNA, recombinant vector or a small molecule. In an example, a "specific binding agent that specifically binds to the label" is capable of binding to a label that is covalently linked to a targeting probe. A nucleic acid-specific binding agent binds substantially only to the defined nucleic acid, such as DNA, or to a specific region within the nucleic acid, for example a nucleic acid probe. A protein-specific binding agent binds substantially only the defined protein, or to a specific region within the protein. For example, a "specific binding agent" includes antibodies and other agents that bind substantially to a specified polypeptide. Antibodies can be monoclonal or polyclonal antibodies that are specific for the polypeptide, as well as immunologically effective portions ("fragments") thereof. The determination that a particular agent binds substantially only to a specific polypeptide may readily be made by using or adapting routine procedures. One suitable in vitro assay makes use of the Western blotting procedure (described in many standard texts, including Harlow and Lane, Using Antibodies: A Laboratory Manual, CSHL, New York, 1999).

**Test agent:** Any agent that that is tested for its effects, for example its effects on a cell. In some embodiments, a test agent is a chemical compound, such as a chemotherapeutic agent, antibiotic, or even an agent with unknown biological properties.

**Tissue:** A plurality of functionally related cells. A tissue can be a suspension, a semisolid, or solid. Tissue includes cells collected from a subject such as blood, cervix, uterus, lymph nodes breast, skin, and other organs.

**Treatment:** "Treatment" or "treating," or "palliating" or "ameliorating" are used interchangeably. These terms refer to an approach for obtaining beneficial or desired results including but not limited to a therapeutic benefit and/or a prophylactic benefit. By therapeutic benefit is meant any therapeutically relevant improvement in or effect on one or more diseases, conditions, or symptoms under treatment. For prophylactic benefit, the compositions may be administered to a subject at risk of developing a particular disease, condition, or symptom, or to a subject reporting one or more of the physiological symptoms of a disease, even though the disease, condition, or symptom may not have yet been manifested.

**Under conditions that permit binding:** A phrase used to describe any environment that permits the desired activity, for example conditions under which two or more molecules, such as nucleic acid molecules and/or protein molecules, can bind.

### Overview

A major goal in modern biology is defining the interactions between different biological actors in vivo. Over the past few decades, major advances have been made in developing methods to identify the molecular interactions with any given protein. With nucleic acids and in particular genomic DNA it is difficult to determine the interactions in a cell in part because of enormity, at the sequence level, of genomic DNA in a cell. It is believed that genomic DNA adopts a fractal globule state in which the DNA organized in three dimensions such that functionally related genomic elements, for example enhancers and their target genes, are directly interacting or are located in very close spatial proximity. Such close physical proximity between such elements is further believed to play a role in genome biology both in normal development and homeostasis and in disease. During the cell cycle the particular proximity relationships change, further complicating the study of genome dynamics. Understanding, and perhaps controlling, these tertiary interactions at the nucleic acid level has enormous potential to further our understating of the complexities cellular dynamics and perhaps fostering the development of new classes of therapeutics. Thus, methods are needed to investigate these interactions. This disclosure meets those needs.

Moreover, the human genome folds to create thousands of intervals, called "contact domains," that exhibit enhanced contact frequency within themselves. "Loop domains" form because of tethering between two loci - almost always bound by CTCF and cohesin - lying on the same chromosome. "Compartment domains" form when genomic intervals with similar histone marks co-segregate. Here, Applicants explore the effects of degrading cohesin. All loop domains are eliminated, but neither compartment domains nor histone marks are affected. Loss of loop domains does not lead to widespread ectopic gene activation, but does affect a significant minority of active genes. In particular, cohesin loss causes superenhancers to co-localize, forming hundreds of links within and across chromosomes, and affecting the regulation of nearby genes. Applicants then restore cohesin and monitor the re-formation of each loop. Although re-formation rates vary greatly, many megabase-sized loops recovered in under an hour, consistent with a model where loop extrusion is rapid.

The results provide for novel methods to eliminate all or essentially all loops in a cell. In certain embodiments, all loops in a cell are eliminated by reducing or degrading one or more members of the cohesin complex or CTCF. The present invention also provides a method to engineer chromatin loops and contact domains in one or more target regions of chromatin DNA inside the nucleus of a cell based on the novel findings herein. In certain embodiments cohesin independent loops (e.g., superenhancers) are modulated. Additionally, the present invention provides for identifying loops for therapeutically targeting in disease (e.g., cancer).

Genes are located at a particular position on a particular chromosome, but the elements that regulate their activity can lie far away. Understanding these distal regulatory sequences is essential to understanding how genes turn on and off in a healthy person, and how this process goes awry in disease. But finding distal regulatory sequences has been an open problem for over 30 years.

Using the three-dimensional genome sequencing approach disclosed herein, it is possible to comprehensively identify all distal regulators of all genes in a sample population of cells. The in situ method, HiC, was used to anlyze global loop formation upon complete loss of the cohesin complex. The information available, will make it possible to assess the impact of candidate drugs on specific cellular circuits, hastening the process of drug discovery and for biological research in general. The information available will also enable the mapping of genomic structural and sequence variations. The methods as disclosed herein will further allow the modification of gene expression through modification of chromatin loops and domains, which finds application in all types of industry, and in medicine.

### In Situ Methods for Detecting Spatial Nucleic Acid Proximity

Disclosed herein is a method for detecting spatial proximity relationships between DNA *in situ* (see, e.g., Rao et al., A 3D map of the human genome at kilobase resolution reveals principles of chromatin looping, Cell. 2014 Dec 18;159(7):1665-80). By combining DNA-DNA proximity ligation with high throughput sequencing in order to measure how frequently positions in the human genome come into close physical proximity, the disclosed method can simultaneously map substantially all of the interactions of DNAs in a cell, including spatial arrangements of DNA. Some of the advantages of the disclosed method are that is can be completed on a small sample of cells, without dilution of the sample. This lack of dilution yields many more contacts than previous methods used to define DNA/DNA interactions, such as chromosome Conformation Capture (3C) and Hi-C technology (see Dekker et al., Science 295:1306-1311 (2002) and Lieberman-Aiden et al., Science 326:289-93 (2009).

In situ determination of nucleic acid proximity as described results in surprising superior results over the Hi-C protocol. The disclosed methods yield a result with greater complexity, which indicates more interactions that can be mapped and consequently more information. In addition, method disclosed herein provide more information on long distance intra-chromosomal contacts. These contacts are the most informative ones, as they can pin down the long-range interactions in the cell.

In order to determine the target for intervention, the methods for determining spatial proximity relationships between nucleic acid sequences are elemental. Further, the methods can also be used to monitor the result of interventions in chromatin looping. In the paragraphs below, the methods for detecting spatial nucleic acid proximity, agents for modulating chromatin 3D structure, and methods and tools for delivering such agents are discussed in detail, as will methods for intervention in chromatin loop formation using genome editing tools.

Disclosed herein are in situ methods for detecting spatial proximity relationships between nucleic acid sequences in a sample, such as DNA sequences, for example in a cell or multiple cells. Preferred methods include *in situ* Hi-C methods. The methods include providing a sample of one or more cells, nuclear extract, cellular milieu or system of nucleic acids of interest that include nucleic acids. In some embodiments, the spatial relationships in the cell is locked in, for example cross-linked or otherwise stabilized. For example, a sample of cells can be treated with a cross-linker to lock in the spatial information or relationship about the molecules in the cells, such as the DNA in the cell. The nucleic acids present are fragmented to yield nucleic acids with overhanging ends, such as a 5' overhanging end. The overhanging ends are then filled in, for example using a DNA polymerase, such as available from a commercial source. The filled in nucleic acid fragments are thus blunt ended at the end filled 5' end. The fragments are then end joined at the filled in end, for example, by ligation using a commercially available nucleic acid ligase, or otherwise attached to another fragment that is in close physical proximity. The ligation, or other attachment procedure, for example nick translation or strand displacement, creates one or more end joined nucleic acid fragments having a junction, for example a ligation junction, wherein the site of the junction, or at least within a few bases, includes one or more labeled nucleic acids, for example, one or more fragmented nucleic acids that have had their overhanging ends filled and joined together. While this step typically involves a ligase, it is contemplated that any means of joining the fragments can be used, for example any chemical or enzymatic means. Further, it is not necessary that the ends be joined in a typical 3'-5' ligation.

To identify the created ligation junction, a labeled nucleotide is used. In one example embodiment, one or more labeled nucleotides are incorporated into the ligated junction. For example, the overhanging ends may be filled in using a DNA polymerase that incorporates one or more labeled nucleotides during the filling in step described above.

In some embodiments, the nucleic acids are cross-linked, either directly, or indirectly, and the information about spatial relationships between the different DNA fragments in the cell, or cells, is maintained during this joining step, and substantially all of the end joined nucleic acid fragments formed at this step were in spatial proximity in the cell prior to the crosslinking step. Therefore, at this point the information about which sequences were in spatial proximity to other sequences in the cell is locked into the end joined fragments. It has been found however, that in some situations, it is not necessary to hold the nucleic acids in place using a chemical fixative or crosslinking agent. Thus in some embodiments, no crosslinking agent is used. In still other embodiments, the nucleic acids are held in position relative to each other by the application of non-crosslinking means, such as by using agar or other polymer to hold the nucleic acids in position.

The labeled nucleotide is present in the junction is used to isolate the one or more end joined nucleic acid fragments using the labeled nucleotide. The sequence is determined at the junction of the one or more end joined nucleic acid fragments, thereby detecting spatial proximity relationships between nucleic acid sequences in a cell. In some embodiments, such as for genome assembly, essentially all of the sequence of the end joined fragments is determined. In some embodiments, determining the sequence of the junction of the one or more end joined nucleic acid fragments includes nucleic acid sequencing. In some embodiments, determining the sequence of the junction of the one or more end joined nucleic acid fragments includes using a probe that specifically hybridizes to the nucleic acid sequences both 5' and 3' of the junction of the one or more end joined nucleic acid fragments, for example using an RNA probe, a DNA probe, a locked nucleic acid (LNA) probe, a peptide nucleic acid (PNA) probe, or a hybrid RNA-DNA probe. In exemplary embodiments of the disclosed method, the location is determined or identified for nucleic acid sequences both 5' and 3' of the ligation junction of the one or more end joined nucleic acid fragments relative to source genome and/or chromosome. In some embodiments, the junction identified is correlated with a disease state. In some embodiments, the junction identified is correlated with an environmental condition. In some embodiments, the sequenced end joined fragments are assembled to create an assembled genome or portion thereof, such as a chromosome or sub-fraction thereof. In some embodiments, information from one or more ligation junctions derived from a sample consisting of a mixture of cells from different organisms, such as mixture of microbes, is used to identify the organisms present in the sample and their relative proportions. In some example, the sample is derived from patient samples.

Typically, the end joined fragments are desired to be between about 100 and about 1000 bases in length, although longer and shorter fragments are contemplated. In some embodiments, the nucleic acid fragments are between about 100 and about 1000 bases in length, such as about 100, about 150, about 200, about 250, about 300, about 350, about 400, about 450, about 500, about 550, about 600, about 650, about 700, about 750, about 800, about 850, about 900, about 950 or about 1000 bases in length, for example form about 100 to about 1000, about 200 to about 800, about 500 to about 850, about 100 to about 500 and about 300 to about 775 base pairs in length and the like. In specific examples, end joined fragments are selected for sequence determination that are between about 300 and 500 base pairs in length.

In some embodiments, in order to create discrete portions of nucleic acid that can be joined together in subsequent steps of the methods, the nucleic acids present in the cells, such as cross-linked cells, are fragmented. The fragmentation can be done by a variety of methods, such as enzymatic and chemical cleavage. For example, DNA can be fragmented using an endonuclease that cuts a specific sequence of DNA and leaves behind a DNA fragment with a 5' overhang, thereby yielding fragmented DNA. In other examples an endonuclease can be selected that cuts the DNA at random spots and yields overhangs or blunt ends. In some embodiments, fragmenting the nucleic acid present in the one or more cells comprises enzymatic digestion with an endonuclease that leaves 5' overhanging ends. Enzymes that fragment, or cut, nucleic acids and yield an overhanging sequence are known in the art and can be obtained from such commercial sources as New England BioLabs^{®} and Promega^{®}. One of ordinary skill in the art can choose the restriction enzyme with out undue experimentation. One of ordinary skill in the art will appreciate that using different fragmentation techniques, such as different enzymes with different sequence requirements, will yield different fragmentation patterns and therefore different nucleic acid ends. The process of fragmenting the sample can yield ends that are capable of being joined.

In some embodiments, the end joined DNA that includes a labeled nucleotide is captured with a specific binding agent that specifically binds a capture moiety, such as biotin, on the labeled nucleotide. In some embodiments, the capture moiety is adsorbed or otherwise captured on a surface. In specific embodiments, the end target joined DNA is labeled with biotin, for instance by incorporation of biotin-14-CTP or other biotinylated nucleotide during the filling in of the 5' overhang, for example with a DNA polymerase, allowing capture by streptavidin. Other means for labeling, capturing, and detecting nucleic acid probes include: incorporation of aminoallyl-labeled nucleotides, incorporation of sulfhydryl-labeled nucleotides, incorporation of allyl- or azide-containing nucleotides, and many other methods described in Bioconjugate Techniques (2nd Ed), Greg T. Hermanson, Elsevier (2008). In some embodiments the specific binding agent has been immobilized for example on a solid support, thereby isolating the target nucleic molecule of interest. By "solid support or carrier" is intended any support capable of binding a targeting nucleic acid. Well-known supports or carriers include glass, polystyrene, polypropylene, polyethylene, dextran, nylon, amylases, natural and modified celluloses, polyacrylamides, agarose, gabbros and magnetite. The nature of the carrier can be either soluble to some extent or insoluble for the purposes of the present disclosure. The support material may have virtually any possible structural configuration so long as the coupled molecule is capable of binding to targeting probe. Thus, the support configuration may be spherical, as in a bead, or cylindrical, as in the inside surface of a test tube, or the external surface of a rod. Alternatively, the surface may be flat such as a sheet or test strip. After capture, these end joined nucleic acid fragments are available for further analysis, for example to determine the sequences that contributed to the information encoded by the ligation junction, which can be used to determine which DNA sequences are close in spatial proximity in the cell, for example to map the three dimensional structure of DNA in a cell such as genomic and/or chromatin bound DNA. In some embodiments, the sequence is determined by PCR, hybridization of a probe and/or sequencing, for example by sequencing using high-throughput paired end sequencing. In some embodiments determining the sequence at the one or more junctions of the one or more end joined nucleic acid fragments comprises nucleic acid sequencing, such as short-read sequencing technologies or long-read sequencing technologies. In some embodiments, nucleic acid sequencing is used to determine two or more junctions within an end-joined concatemer simultaneously.

In some embodiments, determining the sequence of a junction includes using a probe that specifically binds to the junction at the site of the two joined nucleic acid fragments. In particular embodiments, the probe specifically hybridizes to the junction both 5' and 3' of the site of the join and spans the site of the join. A probe that specifically binds to the junction at the site of the join can be selected based on known interactions, for example in a diagnostic setting where the presence of a particular target junction, or set of target junctions, has been correlated with a particular disease or condition. It is further contemplated that once a target junction is known, a probe for that target junction can be synthesized.

In some embodiments, the end joined nucleic acids are selectively amplified. In some examples, to selectively amplify the end joined nucleic acids, a 3' DNA adaptor and a 5' RNA, or conversely a 5' DNA adaptor and a 3' RNA adaptor can be ligated to the ends of the molecules can be used to mark the end joined nucleic acids. Using primers specific for these adaptors only end joined nucleic acids will be amplified during an amplification procedure such as PCR. In some embodiments, the target end joined nucleic acid is amplified using primers that specifically hybridize to the adaptor nucleic acid sequences present at the 3' and 5' ends of the end joined nucleic acids. In some embodiments, the non-ligated ends of the nucleic acids are end repaired. In some embodiments attaching sequencing adapters to the ends of the end ligated nucleic acid fragments.

In some embodiments, the cells are lysed to release the cellular contents, for example after crosslinking. In some examples the nuclei are lysed as well, while in other examples, the nuclei are maintained intact, which can then be isolated and optionally lysed, for example using an reagent that selectively targets the nuclei or other separation technique known in the art. In some examples, the sample is a sample of permeablized nuclei, multiple nuclei, isolated nuclei, synchronized cells, (such at various points in the cell cycle, for example metaphase) or acellular. In some embodiments, the nucleic acids present in the sample are purified, for example using ethanol precipitation. In example embodiments of the disclosed method the cells and/or cell nuclei are not subjected to mechanical lysis. In some example embodiments, the sample is not subjected to RNA degradation. In specific embodiments, the sample is not contacted with an exonuclease to remove of biotin from un-ligated ends. In some embodiments, the sample is not subjected to phenol/chloroform extraction.

In some embodiments of the disclosed method the nucleic acids present in the cell or cells are fixed in position relative to each other by chemical crosslinking, for example by contacting the cells with one or more chemical cross linkers. This treatment locks in the spatial relationships between portions of nucleic acids in a cell. Any method of fixing the nucleic acids in their positions can be used. In some embodiments, the cells are fixed, for example with a fixative, such as an aldehyde, for example formaldehyde or gluteraldehyde. In some embodiments, a sample of one or more cells is cross-linked with a cross-linker to maintain the spatial relationships in the cell. For example, a sample of cells can be treated with a cross-linker to lock in the spatial information or relationship about the molecules in the cells, such as the DNA and RNA in the cell. In other embodiments, the relative positions of the nucleic acid can be maintained without using crosslinking agents. For example the nucleic acids can be stabilized using spermine and spermidine (see Cullen et al., Science 261, 203 (1993). Other methods of maintaining the positional relationships of nucleic acids are known in the art. In some embodiments, nuclei are stabilized by embedding in a polymer such as agarose. In some embodiments, the cross-linker is a reversible cross-linker. In some embodiments, the cross-linker is reversed, for example after the fragments are joined. In specific examples, the nucleic acids are released from the cross-linked three-dimensional matrix by treatment with an agent, such as a proteinase, that degrade the proteinaceous material form the sample, thereby releasing the end ligated nucleic acids for further analysis, such as determination of the nucleic acid sequence. In specific embodiments, the sample is contacted with a proteinase, such as Proteinase K. In some embodiments of the disclosed methods, the cells are contacted with a crosslinking agent to provide the cross-linked cells. In some examples, the cells are contacted with a proteinnucleic acid crosslinking agent, a nucleic acid-nucleic acid crosslinking agent, a protein-protein crosslinking agent or any combination thereof. By this method, the nucleic acids present in the sample become resistant to special rearrangement and the spatial information about the relative locations of nucleic acids in the cell is maintained. In some examples, a cross-linker is a reversible -, such that the cross-linked molecules can be easily separated in subsequent steps of the method. In some examples, a cross-linker is a non-reversible cross-linker, such that the cross-linked molecules cannot be easily separated. In some examples, a cross-linker is light, such as UV light. In some examples, a cross linker is light activated. These cross-linkers include formaldehyde, disuccinimidyl glutarate, UV light, psoralens and their derivatives such as aminomethyltrioxsalen, glutaraldehyde, ethylene glycol bis[succinimidylsuccinate], bissulfosuccinimidyl suberate, 1-Ethyl-3-[3-dimethylaminopropyl]carbodiimide (EDC) bis[sulfosuccinimidyl] suberate (BS3) and other compounds known to those skilled in the art, including those described in the Thermo Scientific Pierce Crosslinking Technical Handbook, Thermo Scientific (2009) as available on the world wide web at piercenet.com/files/1601673_Crosslink_HB_Intl.pdf.

The disclosed methods are also particularly suited to monitoring disease states, such as disease state in an organism, for example a plant or an animal subject, such as a mammalian subject, for example a human subject. Certain disease states may be caused and/or characterized by the differential formation of certain target joins. For example, certain interactions may occur in a diseased cell but not in a normal cell. In other examples, certain interactions may occur in a normal cell but not in diseased cell. Thus, using the disclosed methods a profile of the interaction between DNA sequences in vivo, can be correlated with a disease state. The target join profile correlated with a disease can be used as a "fingerprint" to identify and/or diagnose a disease in a cell, by virtue of having a similar "fingerprint." In addition, the profile can be used to monitor a disease state, for example to monitor the response to a therapy, disease progression and/or make treatment decisions for subjects.

The ability to obtain an interaction profile allows for the diagnosis of a disease state, for example by comparison of the profile present in a sample with the correlated with a specific disease state, wherein a similarity in profile indicates a particular disease state.

Accordingly, aspects of the disclosed methods relate to diagnosing a disease state based on target junction profile correlated with a disease state, for example cancer, or an infection, such as a viral or bacterial infection. It is understood that a diagnosis of a disease state could be made for any organism, including without limitation plants, and animals, such as humans.

Aspects of the present disclosure relate to the correlation of an environmental stress or state with an target junction profile, such as a sample of cells, for example a culture of cells, can be exposed to an environmental stress, such as but not limited to heat shock, osmolarity, hypoxia, cold, oxidative stress, radiation, starvation, a chemical (for example a therapeutic agent or potential therapeutic agent) and the like. After the stress is applied, a representative sample can be subjected to analysis, for example at various time points, and compared to a control, such as a sample from an organism or cell, for example a cell from an organism, or a standard value.

In some embodiments, the disclosed methods can be used to screen chemical libraries for agents that modulate DNA interaction profiles, for example that alter the interaction profile from an abnormal one, for example correlated to a disease state to one indicative of a disease free state. By exposing cells, or fractions thereof, tissues, or even whole animals, to different members of the chemical libraries, and performing the methods described herein, different members of a chemical library can be screened for their effect on interaction profiles simultaneously in a relatively short amount of time, for example using a high throughput method.

In some embodiments, the sequence information determined by the disclosed methods may be used to phase polymorphisms and/or assemble individual haplotypes, distinguish between heterozygous and homozygous structural variations, resolve genomic structural genomic variation, including copy number variations, estimate the 1D distance between two fragments of DNA from the same chromosome, assess syntenic relationships between two or more organisms at arbitrary resolution, and/or generate phylogenetic trees and/or ancestral genomes.

In some embodiments, screening of test agents involves testing a combinatorial library containing a large number of potential modulator compounds. A combinatorial chemical library may be a collection of diverse chemical compounds generated by either chemical synthesis or biological synthesis, by combining a number of chemical "building blocks" such as reagents. For example, a linear combinatorial chemical library, such as a polypeptide library, is formed by combining a set of chemical building blocks (amino acids) in every possible way for a given compound length (for example the number of amino acids in a polypeptide compound). Millions of chemical compounds can be synthesized through such combinatorial mixing of chemical building blocks.

Appropriate agents can be contained in libraries, for example, synthetic or natural compounds in a combinatorial library. Numerous libraries are commercially available or can be readily produced; means for random and directed synthesis of a wide variety of organic compounds and biomolecules, including expression of randomized oligonucleotides, such as antisense oligonucleotides and oligopeptides, also are known. Alternatively, libraries of natural compounds in the form of bacterial, fungal, plant and animal extracts are available or can be readily produced. Additionally, natural or synthetically produced libraries and compounds are readily modified through conventional chemical, physical and biochemical means, and may be used to produce combinatorial libraries. Such libraries are useful for the screening of a large number of different compounds.

The compounds identified using the methods disclosed herein can serve as conventional "lead compounds" or can themselves be used as potential or actual therapeutics. In some instances, pools of candidate agents can be identified and further screened to determine which individual or sub-pools of agents in the collective have a desired activity.

Appropriate samples for use in the methods disclosed herein include any conventional biological sample obtained from an organism or a part thereof, such as a plant, animal, and the like. In particular embodiments, the biological sample is obtained from an animal subject, such as a human subject. A biological sample is any solid or fluid sample obtained from, excreted by or secreted by any living organism, including without limitation, single celled organisms, such as yeast, protozoans, and amoebas among others, multicellular organisms (such as plants or animals, including samples from a healthy or apparently healthy human subject or a human patient affected by a condition or disease to be diagnosed or investigated, such as cancer). For example, a biological sample can be a biological fluid obtained from, for example, blood, plasma, serum, urine, bile, ascites, saliva, cerebrospinal fluid, aqueous or vitreous humor, or any bodily secretion, a transudate, an exudate (for example, fluid obtained from an abscess or any other site of infection or inflammation), or fluid obtained from a joint (for example, a normal joint or a joint affected by disease, such as a rheumatoid arthritis, osteoarthritis, gout or septic arthritis). A sample can also be a sample obtained from any organ or tissue (including a biopsy or autopsy specimen, such as a tumor biopsy) or can include a cell (whether a primary cell or cultured cell) or medium conditioned by any cell, tissue or organ. Exemplary samples include, without limitation, cells, cell lysates, blood smears, cyto-centrifuge preparations, cytology smears, bodily fluids (e.g., blood, plasma, serum, saliva, sputum, urine, bronchoalveolar lavage, semen, etc.), tissue biopsies (e.g., tumor biopsies), fine-needle aspirates, and/or tissue sections (e.g., cryostat tissue sections and/or paraffin-embedded tissue sections). In other examples, the sample includes circulating tumor cells (which can be identified by cell surface markers). In particular examples, samples are used directly (e.g., fresh or frozen), or can be manipulated prior to use, for example, by fixation (e.g., using formalin) and/or embedding in wax (such as formalin-fixed paraffin-embedded (FFPE) tissue samples). It will be appreciated that any method of obtaining tissue from a subject can be utilized, and that the selection of the method used will depend upon various factors such as the type of tissue, age of the subject, or procedures available to the practitioner. Standard techniques for acquisition of such samples are available. See, for example Schluger et al., J. Exp. Med. 176:1327-33 (1992); Bigby et al., Am. Rev. Respir. Dis. 133:515-18 (1986); Kovacs et al., NEJM 318:589-93 (1988); and Ognibene et al., Am. Rev. Respir. Dis. 129:929-32 (1984).

This disclosure also provides integrated systems for high-throughput testing, or automated testing. The systems typically include a robotic armature that transfers fluid from a source to a destination, a controller that controls the robotic armature, a detector, a data storage unit that records detection, and an assay component such as a microtiter dish comprising a well having a reaction mixture for example media.

In some embodiments of the disclosed methods, determining the identity of a nucleic acid, such as a target junction, includes detection by nucleic acid hybridization. Nucleic acid hybridization involves providing a probe and target nucleic acid under conditions where the probe and its complementary target can form stable hybrid duplexes through complementary base pairing. The nucleic acids that do not form hybrid duplexes are then washed away leaving the hybridized nucleic acids to be detected, typically through detection of an attached detectable label. It is generally recognized that nucleic acids are denatured by increasing the temperature or decreasing the salt concentration of the buffer containing the nucleic acids. Under low stringency conditions (e.g., low temperature and/or high salt) hybrid duplexes (e.g., DNA:DNA, PNA:DNA, RNA:RNA, or RNA:DNA) will form even where the annealed sequences are not perfectly complementary. Thus, specificity of hybridization is reduced at lower stringency. Conversely, at higher stringency (e.g., higher temperature or lower salt) successful hybridization requires fewer mismatches. One of skill in the art will appreciate that hybridization conditions can be designed to provide different degrees of stringency.

In general, there is a tradeoff between hybridization specificity (stringency) and signal intensity. Thus, in one embodiment, the wash is performed at the highest stringency that produces consistent results and that provides a signal intensity greater than approximately 10% of the background intensity. Thus, the hybridized array may be washed at successively higher stringency solutions and read between each wash. Analysis of the data sets thus produced will reveal a wash stringency above which the hybridization pattern is not appreciably altered and which provides adequate signal for the particular oligonucleotide probes of interest. In some examples, RNA is detected using Northern blotting or in situ hybridization (Parker & Barnes, Methods in Molecular Biology 106:247-283, 1999); RNAse protection assays (Hod, Biotechniques 13:852-4, 1992); and PCR-based methods, such as reverse transcription polymerase chain reaction (RT-PCR) (Weis et al., Trends in Genetics 8:263-4, 1992).

In one embodiment, the hybridized nucleic acids are detected by detecting one or more labels attached to the sample nucleic acids. The labels can be incorporated by any of a number of methods. In one example, the label is simultaneously incorporated during the amplification step in the preparation of the sample nucleic acids. Thus, for example, polymerase chain reaction (PCR) with labeled primers or labeled nucleotides will provide a labeled amplification product. In one embodiment, transcription amplification, as described above, using a labeled nucleotide (such as fluorescein-labeled UTP and/or CTP) incorporates a label into the transcribed nucleic acids.

Detectable labels suitable for use include any composition detectable by spectroscopic, photochemical, biochemical, immunochemical, electrical, optical or chemical means. Useful labels include biotin for staining with labeled streptavidin conjugate, magnetic beads (for example DynabeadsTM), fluorescent dyes (for example, fluorescein, Texas red, rhodamine, green fluorescent protein, and the like), radiolabels (for example, 3 H, 125 I, 35 S, 14 C, or 32 P), enzymes (for example, horseradish peroxidase, alkaline phosphatase and others commonly used in an ELISA), and colorimetric labels such as colloidal gold or colored glass or plastic (for example, polystyrene, polypropylene, latex, etc.) beads. Patents teaching the use of such labels include U.S. Patent No. 3,817,837; U.S. Patent No. 3,850,752; U.S. Patent No. 3,939,350; U.S. Patent No. 3,996,345; U.S. Patent No. 4,277,437; U.S. Patent No. 4,275,149; and U.S. Patent No. 4,366,241.

Means of detecting such labels are also well known. Thus, for example, radiolabels may be detected using photographic film or scintillation counters, fluorescent markers may be detected using a photodetector to detect emitted light. Enzymatic labels are typically detected by providing the enzyme with a substrate and detecting the reaction product produced by the action of the enzyme on the substrate, and colorimetric labels are detected by simply visualizing the colored label.

The label may be added to the target (sample) nucleic acid(s) prior to, or after, the hybridization. So-called "direct labels" are detectable labels that are directly attached to or incorporated into the target (sample) nucleic acid prior to hybridization. In contrast, so-called "indirect labels" are joined to the hybrid duplex after hybridization. Often, the indirect label is attached to a binding moiety that has been attached to the target nucleic acid prior to the hybridization. Thus, for example, the target nucleic acid may be biotinylated before the hybridization. After hybridization, an avidin-conjugated fluorophore will bind the biotin bearing hybrid duplexes providing a label that is easily detected (see Laboratory Techniques in Biochemistry and Molecular Biology, Vol. 24: Hybridization With Nucleic Acid Probes, P. Tijssen, ed. Elsevier, N.Y., 1993).

In some embodiments, the identity of a nucleic acid is determined by DNA or RNA sequencing. Generally, the sequencing can be performed using automated Sanger sequencing (AB13730xl genome analyzer), pyrosequencing on a solid support (454 sequencing, Roche), sequencing-by-synthesis with reversible terminations (ILLUMINA^{®} Genome Analyzer), sequencing-by-ligation (ABI SOLiD^{®}) or sequencing-by-synthesis with virtual terminators (HELISCOPE^{®}); Moleculo sequencing (see Voskoboynik et al. eLife 2013 2:e00569 and US Patent Application No. 13/608,778, filed Sep 10, 2012); DNA nanoball sequencing; Single molecule real time (SMRT) sequencing; Nanopore DNA sequencing; Sequencing by hybridization; Sequencing with mass spectrometry; and Microfluidic Sanger sequencing. Examples of information that can be obtained from the disclosed methods and the analysis of the results thereof, include without limitation uni- or multiplex, 3 dimensional genome mapping, genome assembly, one dimensional genome mapping, the use of single nucleotide polymorphisms to phase genome maps, for example to determine the patterns of chromosome inactivation, such as for analysis of genomic imprinting, the use of specific junctions to determine karyotypes, including but not limited to chromosome number alterations (such as unisomies, uniparental disomies, and trisomies), translocations, inversions, duplications, deletions and other chromosomal rearrangements, the use of specific junctions correlated with disease to aid in diagnosis.

Furthermore, the methods disclosed herein can readily be combined with other techniques, such as hybrid capture after library generation (to target specific parts of the genome), chromatin immunoprecipitation after ligation (to examine the chromatin environment of regions associated with specific proteins), bisulfite treatment, (to probe the methylation state of DNA). For examples the information from one or more ligation junctions is used to infer and/or determine the three dimensional structure of the genome. In some embodiments, the information from one or more ligation junctions is used to simultaneously map protein-DNA interactions and DNA-DNA interactions or RNA-DNA interactions and DNA-DNA interactions. In some embodiments, the information from one or more ligation junctions is used to simultaneously map methylation and three-dimensional structure. In some embodiments, the information from more than one ligation junction is used to assemble whole genomes or parts of genomes. In some embodiments, the sample is treated to accentuate interactions between contiguous regions of the genome. In some embodiments, the cells in the sample are synchronized in metaphase.

In one example embodiment, hybrid capture after library generation comprises treating a library of end joined nucleic acid fragments generated using the methods described above with an agent that isolates end joined nucleic acid fragments comprising specific nucleic acid sequence (target sequence). In certain example embodiments, the specific nucleic acid sequence is at least 50, at least 60, at least 70, at least 80, at least 90, at least 100, at least 110, at least 120, at least 130, at least 140, at least 150, at least 160, at least 170, at least 180, at least 190, or at least 200 base pairs long. In certain example embodiments, the specific nucleic acid sequence is within at least 50, at least 60, at least 70, at least, 80, at least 90, or at least 100 base pairs, in either the 5' or 3' direction, of a restriction site. In certain example embodiments, the specific nucleic sequence comprises less than ten repetitive bases. In certain other example embodiments, the GC content of the specific nucleic acid sequence is between 25% and 80%, between 40% and 70%, or between 50% and 60%.

In certain example embodiments, the agent that isolates the end joined nucleic acid fragments comprising the specific nucleic acid sequence is a probe. The probe may be labeled. In certain example embodiments, the probe is radiolabeled, fluorescently-labeled, enzymatically-labeled, or chemically labeled. In certain other example embodiments, the probe may be labeled with a capture moiety, such as a biotin-label. Wherein, the probe is labeled with a capture moiety, the capture moiety may be used to isolate the end joined nucleic acid fragments using techniques such as those known in the art and described previously. The exact sequence of the isolated end-joined nucleic acid fragments may then be determined, for example, by sequencing as described previously.

Considering the wealth of information that can be gained using the methods described herein, with respect to genome architecture at the primary, secondary, tertiary and beyond (see Examples below), the methods disclosed herein can be used to apply genome engineering techniques for the treatment of disease as well as the study of biological questions. In some embodiments, the organizational structure of a genome is determined using the methods disclosed herein. For example the methods disclosed herein have been demonstrated (see Example 1) to generate very dense contact maps. In some examples sequences obtained using the methods disclosed herein are mapped to a genome of an organism, such as a animal, plant, fungi, or microorganism, for example a bacterial, yeast, virus and the like. In some examples, using single nucleotide polymorphisms (SNPs), diploid maps corresponding to each chromosomal homolog are constructed. These maps, as well as others that can be generated using the disclosed technology provide a picture, such as a three-dimensional picture, of genomic architecture with high resolution, such as a resolution of 1 kilobase or even lower, for example less then 500 bases.

As disclosed herein, the inventors have shown that a genome is partitioned into domains that are associated with particular patterns of histone marks that segregates into subcompartments, distinguished by unique long-range contact patterns. Using the maps, the inventors have identified ~10,000 distinct loops across the genome and studied their properties, including their strong association with gene activation. Using the maps constructed with the methods described herein as a starting place, targeted alterations in genome structure can be made.

Such genetic and epigenetic control of cells with genome engineering technologies enables a broad range of applications from basic biology to biotechnology and medicine. Manipulating transcriptional regulation or chromatin states at particular loci can reveal how genetic material is organized and utilized within a cell, illuminating relationships between the architecture of the genome and its functions. In addition, once the organization is determined, for example using the methods disclosed herein, manipulation of the genome can be used as a treatment for certain diseases as well as reconstruction of useful biological systems, for example for drug development processes and medical therapeutics. A series of programmable nucleasebased genome editing technologies have developed (see Hsu et al., Cell 157, June 5, 2014 1262-1278 for review). Among these, the clustered regularly interspaced short palindromic repeats (CRISPR)/CRISPR-associated (Cas) system provides for a preferred embodiment in aspects of this invention (see e.g.; Platt et al., Cell 159(2), 440-455 (2014); Shalem et al., Science 3 84-87 (2014); Le Cong et al., Science 339, 819 (2013); and WO2015/089486).

Disclosed herein are methods of altering or modulating the spatial proximity relationships between nucleic acids inside a cell. The methods include providing a sample of one or more cells comprising nucleic acids and providing one or more agents targeting one or more specific genomic regions of interest. The agents are introduced into the one or more cells in order to introduce or remove a sequence or nucleic acid/histone modification associated with a particular spatial proximity arrangement of nucleic acids. In some embodiments, the genomic regions of interest are identified with the methods disclosed herein. In some embodiments, a particular sequence is deleted/inserted in order to abrogate/establish a chromatin loop. In some embodiments, the chromatin loop is altered in a tissue specific manner. In some embodiments, the chromatin loop is involved in the regulation of the expression of a gene. In some embodiments, the chromatin loop or specific genomic regions participating in the chromatin loop are indicative of a disease or condition. In some embodiments, a particular sequence is deleted/inserted in order to abrogate/establish a chromatin domain with elevated contacts between all pairs of loci within a contiguous interval. In some embodiments, the chromatin domain is altered in a tissue specific manner. In some embodiments, the chromatin domain is involved in the regulation of the expression of a gene. In some embodiments, the chromatin domain or specific genomic regions participating in the chromatin domain are indicative of a disease or condition. In some embodiments, an agent is introduced to alter the histone modifications at a specific genomic region. In some embodiments, specific histone modifications are introduced at genomic region to target the region to a nuclear compartment. In some preferred embodiments, the agent introduced to target specific genomic regions is a CRISPR/Cas system.

The present invention thus provides methods of altering or modulating the spatial proximity relationships between nucleic acids inside a cell, wherein said methods include providing a sample of one or more cells comprising nucleic acids and providing one or more agents targeting one or more specific genomic regions of interest, wherein the agents are introduced into the one or more cells in order to introduce or remove a sequence or nucleic acid/histone modification associated with a particular spatial proximity arrangement of nucleic acids, and preferably wherein the one or more specific genomic regions of interest comprise at least one CTCF binding motif.

### Agents for Modulating 3D Chromatin Structure

Chromatin 3D stucture modulating agents in the context of the present invention are intended to interfere or manipulate the function of loop anchor motifs, such as CTCF motifs in any possible way. In certain embodiments, the modulating agent may reversibly eliminate all loops in a cell. By reversibly, the agent may eliminate all loops and then be removed (e.g., by washing) to allow loops to reform. In certain example embodiments, the present invention may block formation of a loop anchor or chromatin domain or induce formation of a loop anchor or chromatin domain at a targeted genomic location. For instance, a loop anchor motif can be altered, such as by mutating (including inverting) a binding motif so as to remove such a motif, or by adding new binding motifs in new locations within a loop domain, so as to reduce the size of an existing loop, so as to modify the size of an existing loop, or combinations thereof. Alternatively, the chromatin 3D structure modulating agent may bind a target region and mask a loop anchor motif, thereby preventing a loop anchor or chromatin domain from forming. The chromatin 3D structure modulating agent may bind a target region and cause a loop anchor of chromatin domain to form. For example, the chromatin 3D structure modulating agent may arrest an extrusion complext at the targeted genomic region faciliating the formation of a new loop anchor or chromatin domain. In certain embodiments, the loop modulating agent may be a degredation system (e.g., inducible degron system). In certain embodiments, the loop modulating agent may be a genetic modifying agent. In certain embodiments, loop modulating agent may be a small molecule.

### Genetic Modifying Agents

In certain embodiments, the one or more modulating agents may be a genetic modifying agent. The genetic modifying agent may comprise a CRISPR system, a zinc finger nuclease system, a TALEN, a meganuclease or RNAi system. In certain embodiments, a CRISPR system or RNAi targeting mRNA is used to temporarily disrupt CTCF or one or more cohesin complex proteins. In certain embodiments, a genetic modifying agent is used to target a functional domain to a specific loop for modulation (e.g., a cohesin independent loop).

Apart from altering the CTCF binding motif or introducing new CTCF binding motifs, it is also envisaged that binding and accumulation of a bulky DNA-binding agent, such as a Cas enzyme, Zinc finger protein, Tale protein, or fusion proteins thereof, optionally multiple Cas enzymes, at the genomic locus of interest, preferably at the loop anchors defined by two converging CTCF motifs, suffices to prevent proper loop formation and/or extrusion, and thereby alter loop architecture. Hence, as an example of such an embodiment, the agent may be a CRISPR/Cas vector system comprising one or more vectors encoding a Cas protein, preferably a Cas9 protein or a Cpf1 protein, such as a catalytically inactive Cas, and one or more guide RNAs, wherein said one or more guide RNAs are targeted to various genomic loci upstream or downstream of a loop anchor site, preferably outside a loop domain, wherein the bound Cas protein(s) form a bloc. The exact location may be optimized according to degree of loop modulation desired. This system provides for a programmable and reversible method for altering chromatin three dimensional (3D) structure in a cell. In such embodiments, it is preferred that the two active cutting sites (HNH and RuvC) of the Cas protein are disabled, so as to render it catalytically inactive, while its ability to home in on its target DNA are preserved. Such methods are known to one of skill in the art.

Preferred agents in the context of this invention comprise a CRISPR/Cas system. The CRISPR/Cas system does not require the generation of customized proteins to target specific sequences but rather a single Cas protein can be programmed by an RNA guide to recognize a specific DNA target, in other words the Cas protein can be recruited to a specific DNA target or genomic locus of interest using said RNA guide. Adding the CRISPR/Cas system to the repertoire of genome sequencing techniques and analysis methods significantly simplifies the methodology to alter genome structure.

CRISPR/Cas genome editing is preferably carried out with a Type II or type V CRISPR system.

In general, a CRISPR-Cas or CRISPR system as used in herein and in documents, such as WO 2014/093622 (PCT/US2013/074667), refers collectively to transcripts and other elements involved in the expression of or directing the activity of CRISPR-associated ("Cas") genes, including sequences encoding a Cas gene, a tracr (trans-activating CRISPR) sequence (e.g. tracrRNA or an active partial tracrRNA), a tracr-mate sequence (encompassing a "direct repeat" and a tracrRNA-processed partial direct repeat in the context of an endogenous CRISPR system), a guide sequence (also referred to as a "spacer" in the context of an endogenous CRISPR system), or "RNA(s)" as that term is herein used (e.g., RNA(s) to guide Cas, such as Cas9, e.g. CRISPR RNA and transactivating (tracr) RNA or a single guide RNA (sgRNA) (chimeric RNA)) or other sequences and transcripts from a CRISPR locus. In general, a CRISPR system is characterized by elements that promote the formation of a CRISPR complex at the site of a target sequence (also referred to as a protospacer in the context of an endogenous CRISPR system). See, e.g, Shmakov et al. (2015) "Discovery and Functional Characterization of Diverse Class 2 CRISPR-Cas Systems", Molecular Cell, DOI: dx.doi.org/10.1016/j.molcel.2015.10.008.

In certain embodiments, a protospacer adjacent motif (PAM) or PAM-like motif directs binding of the effector protein complex as disclosed herein to the target locus of interest. In some embodiments, the PAM may be a 5' PAM (i.e., located upstream of the 5' end of the protospacer). In other embodiments, the PAM may be a 3' PAM (i.e., located downstream of the 5' end of the protospacer). The term "PAM" may be used interchangeably with the term "PFS" or "protospacer flanking site" or "protospacer flanking sequence".

In a preferred embodiment, the CRISPR effector protein may recognize a 3' PAM. In certain embodiments, the CRISPR effector protein may recognize a 3' PAM which is 5'H, wherein H is A, C or U.

In the context of formation of a CRISPR complex, "target sequence" refers to a sequence to which a guide sequence is designed to have complementarity, where hybridization between a target sequence and a guide sequence promotes the formation of a CRISPR complex. A target sequence may comprise RNA polynucleotides. The term "target RNA" refers to a RNA polynucleotide being or comprising the target sequence. In other words, the target RNA may be a RNA polynucleotide or a part of a RNA polynucleotide to which a part of the gRNA, i.e. the guide sequence, is designed to have complementarity and to which the effector function mediated by the complex comprising CRISPR effector protein and a gRNA is to be directed. In some embodiments, a target sequence is located in the nucleus or cytoplasm of a cell.

In certain example embodiments, the CRISPR effector protein may be delivered using a nucleic acid molecule encoding the CRISPR effector protein. The nucleic acid molecule encoding a CRISPR effector protein, may advantageously be a codon optimized CRISPR effector protein. An example of a codon optimized sequence, is in this instance a sequence optimized for expression in eukaryote, e.g., humans (i.e. being optimized for expression in humans), or for another eukaryote, animal or mammal as herein discussed; see, e.g., SaCas9 human codon optimized sequence in WO 2014/093622 (PCT/US2013/074667). Whilst this is preferred, it will be appreciated that other examples are possible and codon optimization for a host species other than human, or for codon optimization for specific organs is known. In some embodiments, an enzyme coding sequence encoding a CRISPR effector protein is a codon optimized for expression in particular cells, such as eukaryotic cells. The eukaryotic cells may be those of or derived from a particular organism, such as a plant or a mammal, including but not limited to human, or non-human eukaryote or animal or mammal as herein discussed, e.g., mouse, rat, rabbit, dog, livestock, or non-human mammal or primate. In some embodiments, processes for modifying the germ line genetic identity of human beings and/or processes for modifying the genetic identity of animals which are likely to cause them suffering without any substantial medical benefit to man or animal, and also animals resulting from such processes, may be excluded. In general, codon optimization refers to a process of modifying a nucleic acid sequence for enhanced expression in the host cells of interest by replacing at least one codon (e.g. about or more than about 1, 2, 3, 4, 5, 10, 15, 20, 25, 50, or more codons) of the native sequence with codons that are more frequently or most frequently used in the genes of that host cell while maintaining the native amino acid sequence. Various species exhibit particular bias for certain codons of a particular amino acid. Codon bias (differences in codon usage between organisms) often correlates with the efficiency of translation of messenger RNA (mRNA), which is in turn believed to be dependent on, among other things, the properties of the codons being translated and the availability of particular transfer RNA (tRNA) molecules. The predominance of selected tRNAs in a cell is generally a reflection of the codons used most frequently in peptide synthesis. Accordingly, genes can be tailored for optimal gene expression in a given organism based on codon optimization. Codon usage tables are readily available, for example, at the "Codon Usage Database" available at kazusa.orjp/codon/ and these tables can be adapted in a number of ways. See Nakamura, Y., et al. "Codon usage tabulated from the international DNA sequence databases: status for the year 2000" Nucl. Acids Res. 28:292 (2000). Computer algorithms for codon optimizing a particular sequence for expression in a particular host cell are also available, such as Gene Forge (Aptagen; Jacobus, PA), are also available. In some embodiments, one or more codons (e.g. 1, 2, 3, 4, 5, 10, 15, 20, 25, 50, or more, or all codons) in a sequence encoding a Cas correspond to the most frequently used codon for a particular amino acid.

In certain embodiments, the methods as described herein may comprise providing a Cas transgenic cell in which one or more nucleic acids encoding one or more guide RNAs are provided or introduced operably connected in the cell with a regulatory element comprising a promoter of one or more gene of interest. As used herein, the term "Cas transgenic cell" refers to a cell, such as a eukaryotic cell, in which a Cas gene has been genomically integrated. The nature, type, or origin of the cell are not particularly limiting according to the present invention. Also the way the Cas transgene is introduced in the cell may vary and can be any method as is known in the art. In certain embodiments, the Cas transgenic cell is obtained by introducing the Cas transgene in an isolated cell. In certain other embodiments, the Cas transgenic cell is obtained by isolating cells from a Cas transgenic organism. By means of example, and without limitation, the Cas transgenic cell as referred to herein may be derived from a Cas transgenic eukaryote, such as a Cas knock-in eukaryote. Reference is made to WO 2014/093622 (PCT/US13/74667). Methods of US Patent Publication Nos. 20120017290 and 20110265198 assigned to Sangamo BioSciences, Inc. directed to targeting the Rosa locus may be modified to utilize the CRISPR Cas system of the present invention. Methods of US Patent Publication No. 20130236946 assigned to Cellectis directed to targeting the Rosa locus may also be modified to utilize the CRISPR Cas system of the present invention. By means of further example reference is made to Platt et. al. (Cell; 159(2):440-455 (2014)), describing a Cas9 knock-in mouse. The Cas transgene can further comprise a Lox-Stop-polyA-Lox(LSL) cassette thereby rendering Cas expression inducible by Cre recombinase. Alternatively, the Cas transgenic cell may be obtained by introducing the Cas transgene in an isolated cell. Delivery systems for transgenes are well known in the art. By means of example, the Cas transgene may be delivered in for instance eukaryotic cell by means of vector (e.g., AAV, adenovirus, lentivirus) and/or particle and/or nanoparticle delivery, as also described herein elsewhere.

It will be understood by the skilled person that the cell, such as the Cas transgenic cell, as referred to herein may comprise further genomic alterations besides having an integrated Cas gene or the mutations arising from the sequence specific action of Cas when complexed with RNA capable of guiding Cas to a target locus.

In certain aspects, the invention involves vectors, e.g. for delivering or introducing in a cell Cas and/or RNA capable of guiding Cas to a target locus (i.e. guide RNA), but also for propagating these components (e.g. in prokaryotic cells). A used herein, a "vector" is a tool that allows or facilitates the transfer of an entity from one environment to another. It is a replicon, such as a plasmid, phage, or cosmid, into which another DNA segment may be inserted so as to bring about the replication of the inserted segment. Generally, a vector is capable of replication when associated with the proper control elements. In general, the term "vector" refers to a nucleic acid molecule capable of transporting another nucleic acid to which it has been linked. Vectors include, but are not limited to, nucleic acid molecules that are single-stranded, double-stranded, or partially double-stranded; nucleic acid molecules that comprise one or more free ends, no free ends (e.g. circular); nucleic acid molecules that comprise DNA, RNA, or both; and other varieties of polynucleotides known in the art. One type of vector is a "plasmid," which refers to a circular double stranded DNA loop into which additional DNA segments can be inserted, such as by standard molecular cloning techniques. Another type of vector is a viral vector, wherein virally-derived DNA or RNA sequences are present in the vector for packaging into a virus (e.g. retroviruses, replication defective retroviruses, adenoviruses, replication defective adenoviruses, and adeno-associated viruses (AAVs)). Viral vectors also include polynucleotides carried by a virus for transfection into a host cell. Certain vectors are capable of autonomous replication in a host cell into which they are introduced (e.g. bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors are capable of directing the expression of genes to which they are operatively-linked. Such vectors are referred to herein as "expression vectors." Common expression vectors of utility in recombinant DNA techniques are often in the form of plasmids.

Recombinant expression vectors can comprise a nucleic acid of the invention in a form suitable for expression of the nucleic acid in a host cell, which means that the recombinant expression vectors include one or more regulatory elements, which may be selected on the basis of the host cells to be used for expression, that is operatively-linked to the nucleic acid sequence to be expressed. Within a recombinant expression vector, "operably linked" is intended to mean that the nucleotide sequence of interest is linked to the regulatory element(s) in a manner that allows for expression of the nucleotide sequence (e.g. in an in vitro transcription/translation system or in a host cell when the vector is introduced into the host cell). With regards to recombination and cloning methods, mention is made of U.S. patent application 10/815,730, published September 2, 2004 as US 2004-0171156 A1. Thus, the embodiments disclosed herein may also comprise transgenic cells comprising the CRISPR effector system. In certain example embodiments, the transgenic cell may function as an individual discrete volume. In other words samples comprising a masking construct may be delivered to a cell, for example in a suitable delivery vesicle and if the target is present in the delivery vesicle the CRISPR effector is activated and a detectable signal generated.

The vector(s) can include the regulatory element(s), e.g., promoter(s). The vector(s) can comprise Cas encoding sequences, and/or a single, but possibly also can comprise at least 3 or 8 or 16 or 32 or 48 or 50 guide RNA(s) (e.g., sgRNAs) encoding sequences, such as 1-2, 1-3, 1-4 1-5, 3-6, 3-7, 3-8, 3-9, 3-10, 3-8, 3-16, 3-30, 3-32, 3-48, 3-50 RNA(s) (e.g., sgRNAs). In a single vector there can be a promoter for each RNA (e.g., sgRNA), advantageously when there are up to about 16 RNA(s); and, when a single vector provides for more than 16 RNA(s), one or more promoter(s) can drive expression of more than one of the RNA(s), e.g., when there are 32 RNA(s), each promoter can drive expression of two RNA(s), and when there are 48 RNA(s), each promoter can drive expression of three RNA(s). By simple arithmetic and well established cloning protocols and the teachings in this disclosure one skilled in the art can readily practice the invention as to the RNA(s) for a suitable exemplary vector such as AAV, and a suitable promoter such as the U6 promoter. For example, the packaging limit of AAV is ~4.7 kb. The length of a single U6-gRNA (plus restriction sites for cloning) is 361 bp. Therefore, the skilled person can readily fit about 12-16, e.g., 13 U6-gRNA cassettes in a single vector. This can be assembled by any suitable means, such as a golden gate strategy used for TALE assembly (genome-engineering.org/taleffectors/). The skilled person can also use a tandem guide strategy to increase the number of U6-gRNAs by approximately 1.5 times, e.g., to increase from 12-16, e.g., 13 to approximately 18-24, e.g., about 19 U6-gRNAs. Therefore, one skilled in the art can readily reach approximately 18-24, e.g., about 19 promoter-RNAs, e.g., U6-gRNAs in a single vector, e.g., an AAV vector. A further means for increasing the number of promoters and RNAs in a vector is to use a single promoter (e.g., U6) to express an array of RNAs separated by cleavable sequences. And an even further means for increasing the number of promoter-RNAs in a vector, is to express an array of promoter-RNAs separated by cleavable sequences in the intron of a coding sequence or gene; and, in this instance it is advantageous to use a polymerase II promoter, which can have increased expression and enable the transcription of long RNA in a tissue specific manner. (see, e.g., nar.oxfordjournals.org/content/34/7/e53.short and nature.com/mt/joumal/v16/n9/abs/mt2008144a.html). In an advantageous embodiment, AAV may package U6 tandem gRNA targeting up to about 50 genes. Accordingly, from the knowledge in the art and the teachings in this disclosure the skilled person can readily make and use vector(s), e.g., a single vector, expressing multiple RNAs or guides under the control or operatively or functionally linked to one or more promoters-especially as to the numbers of RNAs or guides discussed herein, without any undue experimentation.

The guide RNA(s) encoding sequences and/or Cas encoding sequences, can be functionally or operatively linked to regulatory element(s) and hence the regulatory element(s) drive expression. The promoter(s) can be constitutive promoter(s) and/or conditional promoter(s) and/or inducible promoter(s) and/or tissue specific promoter(s). The promoter can be selected from the group consisting of RNA polymerases, pol I, pol II, pol III, T7, U6, H1, retroviral Rous sarcoma virus (RSV) LTR promoter, the cytomegalovirus (CMV) promoter, the SV40 promoter, the dihydrofolate reductase promoter, the β-actin promoter, the phosphoglycerol kinase (PGK) promoter, and the EF1α promoter. An advantageous promoter is the promoter is U6.

Additional effectors for use according to the invention can be identified by their proximity to cas1 genes, for example, though not limited to, within the region 20 kb from the start of the cas1 gene and 20 kb from the end of the cas1 gene. In certain embodiments, the effector protein comprises at least one HEPN domain and at least 500 amino acids, and wherein the C2c2 effector protein is naturally present in a prokaryotic genome within 20 kb upstream or downstream of a Cas gene or a CRISPR array. Non-limiting examples of Cas proteins include Cas1, Cas1B, Cas2, Cas3, Cas4, Cas5, Cas6, Cas7, Cas8, Cas9 (also known as Csn1 and Csx12), Cas10, Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, homologues thereof, or modified versions thereof. In certain example embodiments, the C2c2 effector protein is naturally present in a prokaryotic genome within 20kb upstream or downstream of a Cas 1 gene. The terms "orthologue" (also referred to as "ortholog" herein) and "homologue" (also referred to as "homolog" herein) are well known in the art. By means of further guidance, a "homologue" of a protein as used herein is a protein of the same species which performs the same or a similar function as the protein it is a homologue of. Homologous proteins may but need not be structurally related, or are only partially structurally related. An "orthologue" of a protein as used herein is a protein of a different species which performs the same or a similar function as the protein it is an orthologue of. Orthologous proteins may but need not be structurally related, or are only partially structurally related.

### Guide Molecules

The methods described herein may be used to screen inhibition of CRISPR systems employing different types of guide molecules. As used herein, the term "guide sequence" and "guide molecule" in the context of a CRISPR-Cas system, comprises any polynucleotide sequence having sufficient complementarity with a target nucleic acid sequence to hybridize with the target nucleic acid sequence and direct sequence-specific binding of a nucleic acid-targeting complex to the target nucleic acid sequence. The guide sequences made using the methods disclosed herein may be a full-length guide sequence, a truncated guide sequence, a full-length sgRNA sequence, a truncated sgRNA sequence, or an E+F sgRNA sequence. In some embodiments, the degree of complementarity of the guide sequence to a given target sequence, when optimally aligned using a suitable alignment algorithm, is about or more than about 50%, 60%, 75%, 80%, 85%, 90%, 95%, 97.5%, 99%, or more. In certain example embodiments, the guide molecule comprises a guide sequence that may be designed to have at least one mismatch with the target sequence, such that a RNA duplex formed between the guide sequence and the target sequence. Accordingly, the degree of complementarity is preferably less than 99%. For instance, where the guide sequence consists of 24 nucleotides, the degree of complementarity is more particularly about 96% or less. In particular embodiments, the guide sequence is designed to have a stretch of two or more adjacent mismatching nucleotides, such that the degree of complementarity over the entire guide sequence is further reduced. For instance, where the guide sequence consists of 24 nucleotides, the degree of complementarity is more particularly about 96% or less, more particularly, about 92% or less, more particularly about 88% or less, more particularly about 84% or less, more particularly about 80% or less, more particularly about 76% or less, more particularly about 72% or less, depending on whether the stretch of two or more mismatching nucleotides encompasses 2, 3, 4, 5, 6 or 7 nucleotides, etc. In some embodiments, aside from the stretch of one or more mismatching nucleotides, the degree of complementarity, when optimally aligned using a suitable alignment algorithm, is about or more than about 50%, 60%, 75%, 80%, 85%, 90%, 95%, 97.5%, 99%, or more. Optimal alignment may be determined with the use of any suitable algorithm for aligning sequences, non-limiting example of which include the Smith-Waterman algorithm, the Needleman-Wunsch algorithm, algorithms based on the Burrows-Wheeler Transform (e.g., the Burrows Wheeler Aligner), ClustalW, Clustal X, BLAT, Novoalign (Novocraft Technologies; available at www.novocraft.com), ELAND (Illumina, San Diego, CA), SOAP (available at soap.genomics.org.cn), and Maq (available at maq.sourceforge.net). The ability of a guide sequence (within a nucleic acid-targeting guide RNA) to direct sequence-specific binding of a nucleic acid -targeting complex to a target nucleic acid sequence may be assessed by any suitable assay. For example, the components of a nucleic acid-targeting CRISPR system sufficient to form a nucleic acid-targeting complex, including the guide sequence to be tested, may be provided to a host cell having the corresponding target nucleic acid sequence, such as by transfection with vectors encoding the components of the nucleic acid-targeting complex, followed by an assessment of preferential targeting (e.g., cleavage) within the target nucleic acid sequence, such as by Surveyor assay as described herein. Similarly, cleavage of a target nucleic acid sequence (or a sequence in the vicinity thereof) may be evaluated in a test tube by providing the target nucleic acid sequence, components of a nucleic acid-targeting complex, including the guide sequence to be tested and a control guide sequence different from the test guide sequence, and comparing binding or rate of cleavage at or in the vicinity of the target sequence between the test and control guide sequence reactions. Other assays are possible, and will occur to those skilled in the art. A guide sequence, and hence a nucleic acid-targeting guide RNA may be selected to target any target nucleic acid sequence.

In certain embodiments, the guide sequence or spacer length of the guide molecules is from 15 to 50 nt. In certain embodiments, the spacer length of the guide RNA is at least 15 nucleotides. In certain embodiments, the spacer length is from 15 to 17 nt, e.g., 15, 16, or 17 nt, from 17 to 20 nt, e.g., 17, 18, 19, or 20 nt, from 20 to 24 nt, e.g., 20, 21, 22, 23, or 24 nt, from 23 to 25 nt, e.g., 23, 24, or 25 nt, from 24 to 27 nt, e.g., 24, 25, 26, or 27 nt, from 27-30 nt, e.g., 27, 28, 29, or 30 nt, from 30-35 nt, e.g., 30, 31, 32, 33, 34, or 35 nt, or 35 nt or longer. In certain example embodiment, the guide sequence is 15, 16, 17,18, 19, 20, 21, 22, 23, 24, 25, 26, 27, 28, 29, 30, 31, 32, 33, 34, 35, 36, 37, 38, 39 40, 41, 42, 43, 44, 45, 46, 47 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 nt.

In some embodiments, the guide sequence is an RNA sequence of between 10 to 50 nt in length, but more particularly of about 20-30 nt advantageously about 20 nt, 23-25 nt or 24 nt. The guide sequence is selected so as to ensure that it hybridizes to the target sequence. This is described more in detail below. Selection can encompass further steps which increase efficacy and specificity.

In some embodiments, the guide sequence has a canonical length (e.g., about 15-30 nt) is used to hybridize with the target RNA or DNA. In some embodiments, a guide molecule is longer than the canonical length (e.g., >30 nt) is used to hybridize with the target RNA or DNA, such that a region of the guide sequence hybridizes with a region of the RNA or DNA strand outside of the Cas-guide target complex. This can be of interest where additional modifications, such deamination of nucleotides is of interest. In alternative embodiments, it is of interest to maintain the limitation of the canonical guide sequence length.

In some embodiments, the sequence of the guide molecule (direct repeat and/or spacer) is selected to reduce the degree secondary structure within the guide molecule. In some embodiments, about or less than about 75%, 50%, 40%, 30%, 25%, 20%, 15%, 10%, 5%, 1%, or fewer of the nucleotides of the nucleic acid-targeting guide RNA participate in selfcomplementary base pairing when optimally folded. Optimal folding may be determined by any suitable polynucleotide folding algorithm. Some programs are based on calculating the minimal Gibbs free energy. An example of one such algorithm is mFold, as described by Zuker and Stiegler (Nucleic Acids Res. 9 (1981), 133-148). Another example folding algorithm is the online webserver RNAfold, developed at Institute for Theoretical Chemistry at the University of Vienna, using the centroid structure prediction algorithm (see e.g., A.R. Gruber et al., 2008, Cell 106(1): 23-24; and PA Carr and GM Church, 2009, Nature Biotechnology 27(12): 1151-62).

In some embodiments, it is of interest to reduce the susceptibility of the guide molecule to RNA cleavage, such as to cleavage by Cas13. Accordingly, in particular embodiments, the guide molecule is adjusted to avoide cleavage by Cas13 or other RNA-cleaving enzymes.

In certain embodiments, the guide molecule comprises non-naturally occurring nucleic acids and/or non-naturally occurring nucleotides and/or nucleotide analogs, and/or chemically modifications. Preferably, these non-naturally occurring nucleic acids and non-naturally occurring nucleotides are located outside the guide sequence. Non-naturally occurring nucleic acids can include, for example, mixtures of naturally and non-naturally occurring nucleotides. Non-naturally occurring nucleotides and/or nucleotide analogs may be modified at the ribose, phosphate, and/or base moiety. In an embodiment of the invention, a guide nucleic acid comprises ribonucleotides and non-ribonucleotides. In one such embodiment, a guide comprises one or more ribonucleotides and one or more deoxyribonucleotides. In an embodiment of the invention, the guide comprises one or more non-naturally occurring nucleotide or nucleotide analog such as a nucleotide with phosphorothioate linkage, a locked nucleic acid (LNA) nucleotides comprising a methylene bridge between the 2' and 4' carbons of the ribose ring, or bridged nucleic acids (BNA). Other examples of modified nucleotides include 2'-O-methyl analogs, 2'-deoxy analogs, or 2'-fluoro analogs. Further examples of modified bases include, but are not limited to, 2-aminopurine, 5-bromo-uridine, pseudouridine, inosine, 7-methylguanosine. Examples of guide RNA chemical modifications include, without limitation, incorporation of 2' -O-methyl (M), 2' -O-methyl 3' phosphorothioate (MS), *S-*constrained ethyl(cEt), or 2' -O-methyl 3' thioPACE (MSP) at one or more terminal nucleotides. Such chemically modified guides can comprise increased stability and increased activity as compared to unmodified guides, though on-target vs. off-target specificity is not predictable. (See, Hendel, 2015, Nat Biotechnol. 33(9):985-9, doi: 10.1038/nbt.3290, published online 29 June 2015 Ragdarm et al., 0215, PNAS, E7110-E7111; Allerson et al., J. Med. Chem. 2005, 48:901-904; Bramsen et al., Front. Genet., 2012, 3:154; Deng et al., PNAS, 2015, 112:11870-11875; Sharma et al., MedChemComm., 2014, 5:1454-1471; Hendel et al., Nat. Biotechnol. (2015) 33(9): 985-989; Li et al., Nature Biomedical Engineering, 2017, 1, 0066 DOI:10.1038/s41551-017-0066). In some embodiments, the 5' and/or 3' end of a guide RNA is modified by a variety of functional moieties including fluorescent dyes, polyethylene glycol, cholesterol, proteins, or detection tags. (See Kelly et al., 2016, J. Biotech. 233:74-83). In certain embodiments, a guide comprises ribonucleotides in a region that binds to a target RNA and one or more deoxyribonucletides and/or nucleotide analogs in a region that binds to Cas13. In an embodiment of the invention, deoxyribonucleotides and/or nucleotide analogs are incorporated in engineered guide structures, such as, without limitation, stem-loop regions, and the seed region. For Cas13 guide, in certain embodiments, the modification is not in the 5'-handle of the stem-loop regions. Chemical modification in the 5'-handle of the stem-loop region of a guide may abolish its function (see Li, et al., Nature Biomedical Engineering, 2017, 1:0066). In certain embodiments, at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, or 75 nucleotides of a guide is chemically modified. In some embodiments, 3-5 nucleotides at either the 3' or the 5' end of a guide is chemically modified. In some embodiments, only minor modifications are introduced in the seed region, such as 2'-F modifications. In some embodiments, 2'-F modification is introduced at the 3' end of a guide. In certain embodiments, three to five nucleotides at the 5' and/or the 3' end of the guide are chemicially modified with 2'-O-methyl (M), 2'-O-methyl 3' phosphorothioate (MS), S-constrained ethyl(cEt), or 2'-O-methyl 3' thioPACE (MSP). Such modification can enhance genome editing efficiency (see Hendel et al., Nat. Biotechnol. (2015) 33(9): 985-989). In certain embodiments, all of the phosphodiester bonds of a guide are substituted with phosphorothioates (PS) for enhancing levels of gene disruption. In certain embodiments, more than five nucleotides at the 5' and/or the 3' end of the guide are chemicially modified with 2'-O-Me, 2'-F or *S-*constrained ethyl(cEt). Such chemically modified guide can mediate enhanced levels of gene disruption (see Ragdarm et al., 0215, PNAS, E7110-E7111). In an embodiment of the invention, a guide is modified to comprise a chemical moiety at its 3' and/or 5' end. Such moieties include, but are not limited to amine, azide, alkyne, thio, dibenzocyclooctyne (DBCO), or Rhodamine. In certain embodiment, the chemical moiety is conjugated to the guide by a linker, such as an alkyl chain. In certain embodiments, the chemical moiety of the modified guide can be used to attach the guide to another molecule, such as DNA, RNA, protein, or nanoparticles. Such chemically modified guide can be used to identify or enrich cells generically edited by a CRISPR system (see Lee et al., eLife, 2017, 6:e25312, DOI:10.7554).

In some embodiments, the modification to the guide is a chemical modification, an insertion, a deletion or a split. In some embodiments, the chemical modification includes, but is not limited to, incorporation of 2'-O-methyl (M) analogs, 2'-deoxy analogs, 2-thiouridine analogs, N6-methyladenosine analogs, 2'-fluoro analogs, 2-aminopurine, 5-bromo-uridine, pseudouridine (Ψ), N1-methylpseudouridine (me1Ψ), 5-methoxyuridine(5moU), inosine, 7-methylguanosine, 2'-O-methyl 3'phosphorothioate (MS), S-constrained ethyl(cEt), phosphorothioate (PS), or 2'-O-methyl 3'thioPACE (MSP). In some embodiments, the guide comprises one or more of phosphorothioate modifications. In certain embodiments, at least 1, 2, 3, 4, 5, 6, 7, 8, 9, 10, 11, 12, 13, 14, 15, 16, 17, 18, 19, 20, or 25 nucleotides of the guide are chemically modified. In certain embodiments, one or more nucleotides in the seed region are chemically modified. In certain embodiments, one or more nucleotides in the 3'-terminus are chemically modified. In certain embodiments, none of the nucleotides in the 5'-handle is chemically modified. In some embodiments, the chemical modification in the seed region is a minor modification, such as incorporation of a 2'-fluoro analog. In a specific embodiment, one nucleotide of the seed region is replaced with a 2'-fluoro analog. In some embodiments, 5 to 10 nucleotides in the 3'-terminus are chemically modified. Such chemical modifications at the 3'-terminus of the Cas13 CrRNA may improve Cas13 activity. In a specific embodiment, 1, 2, 3, 4, 5, 6, 7, 8, 9 or 10 nucleotides in the 3'-terminus are replaced with 2'-fluoro analogues. In a specific embodiment, 1, 2, 3, 4, 5, 6, 7, 8, 9 or 10 nucleotides in the 3'-terminus are replaced with 2'- O-methyl (M) analogs.

In some embodiments, the loop of the 5'-handle of the guide is modified. In some embodiments, the loop of the 5'-handle of the guide is modified to have a deletion, an insertion, a split, or chemical modifications. In certain embodiments, the modified loop comprises 3, 4, or 5 nucleotides. In certain embodiments, the loop comprises the sequence of UCUU, UUUU, UAUU, or UGUU.

In some embodiments, the guide molecule forms a stemloop with a separate non-covalently linked sequence, which can be DNA or RNA. In particular embodiments, the sequences forming the guide are first synthesized using the standard phosphoramidite synthetic protocol (Herdewijn, P., ed., Methods in Molecular Biology Col 288, Oligonucleotide Synthesis: Methods and Applications, Humana Press, New Jersey (2012)). In some embodiments, these sequences can be functionalized to contain an appropriate functional group for ligation using the standard protocol known in the art (Hermanson, G. T., Bioconjugate Techniques, Academic Press (2013)). Examples of functional groups include, but are not limited to, hydroxyl, amine, carboxylic acid, carboxylic acid halide, carboxylic acid active ester, aldehyde, carbonyl, chlorocarbonyl, imidazolylcarbonyl, hydrozide, semicarbazide, thio semicarbazide, thiol, maleimide, haloalkyl, sufonyl, ally, propargyl, diene, alkyne, and azide. Once this sequence is functionalized, a covalent chemical bond or linkage can be formed between this sequence and the direct repeat sequence. Examples of chemical bonds include, but are not limited to, those based on carbamates, ethers, esters, amides, imines, amidines, aminotrizines, hydrozone, disulfides, thioethers, thioesters, phosphorothioates, phosphorodithioates, sulfonamides, sulfonates, fulfones, sulfoxides, ureas, thioureas, hydrazide, oxime, triazole, photolabile linkages, C-C bond forming groups such as Diels-Alder cyclo-addition pairs or ring-closing metathesis pairs, and Michael reaction pairs.

In some embodiments, these stem-loop forming sequences can be chemically synthesized. In some embodiments, the chemical synthesis uses automated, solid-phase oligonucleotide synthesis machines with 2'-acetoxyethyl orthoester (2'-ACE) (Scaringe et al., J. Am. Chem. Soc. (1998) 120: 11820-11821; Scaringe, Methods Enzymol. (2000) 317: 3-18) or 2'-thionocarbamate (2'-TC) chemistry (Dellinger et al., J. Am. Chem. Soc. (2011) 133: 11540-11546; Hendel et al., Nat. Biotechnol. (2015) 33:985-989).

In certain embodiments, the guide molecule comprises (1) a guide sequence capable of hybridizing to a target locus and (2) a tracr mate or direct repeat sequence whereby the direct repeat sequence is located upstream (i.e., 5') from the guide sequence. In a particular embodiment the seed sequence (i.e. the sequence essential critical for recognition and/or hybridization to the sequence at the target locus) of th guide sequence is approximately within the first 10 nucleotides of the guide sequence.

In a particular embodiment the guide molecule comprises a guide sequence linked to a direct repeat sequence, wherein the direct repeat sequence comprises one or more stem loops or optimized secondary structures. In particular embodiments, the direct repeat has a minimum length of 16 nts and a single stem loop. In further embodiments the direct repeat has a length longer than 16 nts, preferably more than 17 nts, and has more than one stem loops or optimized secondary structures. In particular embodiments the guide molecule comprises or consists of the guide sequence linked to all or part of the natural direct repeat sequence. A typical Type V or Type VI CRISPR-cas guide molecule comprises (in 3' to 5' direction or in 5' to 3' direction): a guide sequence a first complimentary stretch (the "repeat"), a loop (which is typically 4 or 5 nucleotides long), a second complimentary stretch (the "anti-repeat" being complimentary to the repeat), and a poly A (often poly U in RNA) tail (terminator). In certain embodiments, the direct repeat sequence retains its natural architecture and forms a single stem loop. In particular embodiments, certain aspects of the guide architecture can be modified, for example by addition, subtraction, or substitution of features, whereas certain other aspects of guide architecture are maintained. Preferred locations for engineered guide molecule modifications, including but not limited to insertions, deletions, and substitutions include guide termini and regions of the guide molecule that are exposed when complexed with the CRISPR-Cas protein and/or target, for example the stemloop of the direct repeat sequence.

In particular embodiments, the stem comprises at least about 4bp comprising complementary X and Y sequences, although stems of more, e.g., 5, 6, 7, 8, 9, 10, 11 or 12 or fewer, e.g., 3, 2, base pairs are also contemplated. Thus, for example X2-10 and Y2-10 (wherein X and Y represent any complementary set of nucleotides) may be contemplated. In one aspect, the stem made of the X and Y nucleotides, together with the loop will form a complete hairpin in the overall secondary structure; and, this may be advantageous and the amount of base pairs can be any amount that forms a complete hairpin. In one aspect, any complementary X:Y basepairing sequence (e.g., as to length) is tolerated, so long as the secondary structure of the entire guide molecule is preserved. In one aspect, the loop that connects the stem made of X:Y basepairs can be any sequence of the same length (e.g., 4 or 5 nucleotides) or longer that does not interrupt the overall secondary structure of the guide molecule. In one aspect, the stemloop can further comprise, e.g. an MS2 aptamer. In one aspect, the stem comprises about 5-7bp comprising complementary X and Y sequences, although stems of more or fewer basepairs are also contemplated. In one aspect, non-Watson Crick basepairing is contemplated, where such pairing otherwise generally preserves the architecture of the stemloop at that position.

In particular embodiments the natural hairpin or stemloop structure of the guide molecule is extended or replaced by an extended stemloop. It has been demonstrated that extension of the stem can enhance the assembly of the guide molecule with the CRISPR-Cas proten (Chen et al. Cell. (2013); 155(7): 1479-1491). In particular embodiments the stem of the stemloop is extended by at least 1, 2, 3, 4, 5 or more complementary basepairs (i.e. corresponding to the addition of 2,4, 6, 8, 10 or more nucleotides in the guide molecule). In particular embodiments these are located at the end of the stem, adjacent to the loop of the stemloop.

In particular embodiments, the susceptibility of the guide molecule to RNAses or to decreased expression can be reduced by slight modifications of the sequence of the guide molecule which do not affect its function. For instance, in particular embodiments, premature termination of transcription, such as premature transcription of U6 Pol-III, can be removed by modifying a putative Pol-III terminator (4 consecutive U's) in the guide molecules sequence. Where such sequence modification is required in the stemloop of the guide molecule, it is preferably ensured by a basepair flip.

In a particular embodiment, the direct repeat may be modified to comprise one or more protein-binding RNA aptamers. In a particular embodiment, one or more aptamers may be included such as part of optimized secondary structure. Such aptamers may be capable of binding a bacteriophage coat protein as detailed further herein.

In some embodiments, the guide molecule forms a duplex with a target RNA comprising at least one target cytosine residue to be edited. Upon hybridization of the guide RNA molecule to the target RNA, the cytidine deaminase binds to the single strand RNA in the duplex made accessible by the mismatch in the guide sequence and catalyzes deamination of one or more target cytosine residues comprised within the stretch of mismatching nucleotides.

A guide sequence, and hence a nucleic acid-targeting guide RNA may be selected to target any target nucleic acid sequence. The target sequence may be mRNA.

In certain embodiments, the target sequence should be associated with a PAM (protospacer adjacent motif) or PFS (protospacer flanking sequence or site); that is, a short sequence recognized by the CRISPR complex. Depending on the nature of the CRISPR-Cas protein, the target sequence should be selected such that its complementary sequence in the DNA duplex (also referred to herein as the non-target sequence) is upstream or downstream of the PAM. In the embodiments of the present invention where the CRISPR-Cas protein is a Cas13 protein, the compelementary sequence of the target sequence is downstream or 3' of the PAM or upstream or 5' of the PAM. The precise sequence and length requirements for the PAM differ depending on the Cas13 protein used, but PAMs are typically 2-5 base pair sequences adjacent the protospacer (that is, the target sequence). Examples of the natural PAM sequences for different Cas13 orthologues are provided herein below and the skilled person will be able to identify further PAM sequences for use with a given Cas13 protein.

Further, engineering of the PAM Interacting (PI) domain may allow programing of PAM specificity, improve target site recognition fidelity, and increase the versatility of the CRISPR-Cas protein, for example as described for Cas9 in Kleinstiver BP et al. Engineered CRISPR-Cas9 nucleases with altered PAM specificities. Nature. 2015 Jul 23;523(7561):481-5. doi: 10.1038/nature14592. As further detailed herein, the skilled person will understand that Cas13 proteins may be modified analogously.

In particular embodiment, the guide is an escorted guide. By "escorted" is meant that the CRISPR-Cas system or complex or guide is delivered to a selected time or place within a cell, so that activity of the CRISPR-Cas system or complex or guide is spatially or temporally controlled. For example, the activity and destination of the 3 CRISPR-Cas system or complex or guide may be controlled by an escort RNA aptamer sequence that has binding affinity for an aptamer ligand, such as a cell surface protein or other localized cellular component. Alternatively, the escort aptamer may for example be responsive to an aptamer effector on or in the cell, such as a transient effector, such as an external energy source that is applied to the cell at a particular time.

The escorted CRISPR-Cas systems or complexes have a guide molecule with a functional structure designed to improve guide molecule structure, architecture, stability, genetic expression, or any combination thereof. Such a structure can include an aptamer.

Aptamers are biomolecules that can be designed or selected to bind tightly to other ligands, for example using a technique called systematic evolution of ligands by exponential enrichment (SELEX; Tuerk C, Gold L: "Systematic evolution of ligands by exponential enrichment: RNA ligands to bacteriophage T4 DNA polymerase." Science 1990, 249:505-510). Nucleic acid aptamers can for example be selected from pools of random-sequence oligonucleotides, with high binding affinities and specificities for a wide range of biomedically relevant targets, suggesting a wide range of therapeutic utilities for aptamers (Keefe, Anthony D., Supriya Pai, and Andrew Ellington. "Aptamers as therapeutics." Nature Reviews Drug Discovery 9.7 (2010): 537-550). These characteristics also suggest a wide range of uses for aptamers as drug delivery vehicles (Levy-Nissenbaum, Etgar, et al. "Nanotechnology and aptamers: applications in drug delivery." Trends in biotechnology 26.8 (2008): 442-449; and, Hicke BJ, Stephens AW. "Escort aptamers: a delivery service for diagnosis and therapy." J Clin Invest 2000, 106:923-928.). Aptamers may also be constructed that function as molecular switches, responding to a que by changing properties, such as RNA aptamers that bind fluorophores to mimic the activity of green flourescent protein (Paige, Jeremy S., Karen Y. Wu, and Samie R. Jaffrey. "RNA mimics of green fluorescent protein." Science 333.6042 (2011): 642-646). It has also been suggested that aptamers may be used as components of targeted siRNA therapeutic delivery systems, for example targeting cell surface proteins (Zhou, Jiehua, and John J. Rossi. "Aptamer-targeted cell-specific RNA interference." Silence 1.1 (2010): 4).

Accordingly, in particular embodiments, the guide molecule is modified, e.g., by one or more aptamer(s) designed to improve guide molecule delivery, including delivery across the cellular membrane, to intracellular compartments, or into the nucleus. Such a structure can include, either in addition to the one or more aptamer(s) or without such one or more aptamer(s), moiety(ies) so as to render the guide molecule deliverable, inducible or responsive to a selected effector. The invention accordingly comprehends an guide molecule that responds to normal or pathological physiological conditions, including without limitation pH, hypoxia, O₂ concentration, temperature, protein concentration, enzymatic concentration, lipid structure, light exposure, mechanical disruption (e.g. ultrasound waves), magnetic fields, electric fields, or electromagnetic radiation.

Light responsiveness of an inducible system may be achieved via the activation and binding of cryptochrome-2 and CIB1. Blue light stimulation induces an activating conformational change in cryptochrome-2, resulting in recruitment of its binding partner CIB1. This binding is fast and reversible, achieving saturation in <15 sec following pulsed stimulation and returning to baseline <15 min after the end of stimulation. These rapid binding kinetics result in a system temporally bound only by the speed of transcription/translation and transcript/protein degradation, rather than uptake and clearance of inducing agents. Crytochrome-2 activation is also highly sensitive, allowing for the use of low light intensity stimulation and mitigating the risks of phototoxicity. Further, in a context such as the intact mammalian brain, variable light intensity may be used to control the size of a stimulated region, allowing for greater precision than vector delivery alone may offer.

The invention contemplates energy sources such as electromagnetic radiation, sound energy or thermal energy to induce the guide. Advantageously, the electromagnetic radiation is a component of visible light. In a preferred embodiment, the light is a blue light with a wavelength of about 450 to about 495 nm. In an especially preferred embodiment, the wavelength is about 488 nm. In another preferred embodiment, the light stimulation is via pulses. The light power may range from about 0-9 mW/cm². In a preferred embodiment, a stimulation paradigm of as low as 0.25 sec every 15 sec should result in maximal activation.

The chemical or energy sensitive guide may undergo a conformational change upon induction by the binding of a chemical source or by the energy allowing it act as a guide and have the Cas13 CRISPR-Cas system or complex function. The invention can involve applying the chemical source or energy so as to have the guide function and the Cas13 CRISPR-Cas system or complex function; and optionally further determining that the expression of the genomic locus is altered.

There are several different designs of this chemical inducible system: 1. ABI-PYL based system inducible by Abscisic Acid (ABA) (see, e.g., stke.sciencemag.org/cgi/content/abstract/sigtrans;4/164/rs2), 2. FKBP-FRB based system inducible by rapamycin (or related chemicals based on rapamycin) (see, e.g., www.nature.com/nmeth/journal/v2/n6/full/nmeth763.html). 3. GID1-GAI based system inducible by Gibberellin (GA) (see, e.g., www.nature.com/nchembio/journal/v8/n5/full/nchembio.922.html).

A chemical inducible system can be an estrogen receptor (ER) based system inducible by 4-hydroxytamoxifen (4OHT) (see, e.g., www.pnas.org/content/104/3/1027.abstract). A mutated ligand-binding domain of the estrogen receptor called ERT2 translocates into the nucleus of cells upon binding of 4-hydroxytamoxifen. In further embodiments of the invention any naturally occurring or engineered derivative of any nuclear receptor, thyroid hormone receptor, retinoic acid receptor, estrogren receptor, estrogenrelated receptor, glucocorticoid receptor, progesterone receptor, androgen receptor may be used in inducible systems analogous to the ER based inducible system.

Another inducible system is based on the design using Transient receptor potential (TRP) ion channel based system inducible by energy, heat or radio-wave (see, e.g., www.sciencemag.org/content/336/6081/604). These TRP family proteins respond to different stimuli, including light and heat. When this protein is activated by light or heat, the ion channel will open and allow the entering of ions such as calcium into the plasma membrane. This influx of ions will bind to intracellular ion interacting partners linked to a polypeptide including the guide and the other components of the Cas13 CRISPR-Cas complex or system, and the binding will induce the change of sub-cellular localization of the polypeptide, leading to the entire polypeptide entering the nucleus of cells. Once inside the nucleus, the guide protein and the other components of the Cas13 CRISPR-Cas complex will be active and modulating target gene expression in cells.

While light activation may be an advantageous embodiment, sometimes it may be disadvantageous especially for in vivo applications in which the light may not penetrate the skin or other organs. In this instance, other methods of energy activation are contemplated, in particular, electric field energy and/or ultrasound which have a similar effect.

Electric field energy is preferably administered substantially as described in the art, using one or more electric pulses of from about 1 Volt/cm to about 10 kVolts/cm under *in vivo* conditions. Instead of or in addition to the pulses, the electric field may be delivered in a continuous manner. The electric pulse may be applied for between 1 µs and 500 milliseconds, preferably between 1 µs and 100 milliseconds. The electric field may be applied continuously or in a pulsed manner for 5 about minutes.

As used herein, 'electric field energy' is the electrical energy to which a cell is exposed. Preferably the electric field has a strength of from about 1 Volt/cm to about 10 kVolts/cm or more under *in vivo* conditions (see WO97/49450).

As used herein, the term "electric field" includes one or more pulses at variable capacitance and voltage and including exponential and/or square wave and/or modulated wave and/or modulated square wave forms. References to electric fields and electricity should be taken to include reference the presence of an electric potential difference in the environment of a cell. Such an environment may be set up by way of static electricity, alternating current (AC), direct current (DC), etc, as known in the art. The electric field may be uniform, non-uniform or otherwise, and may vary in strength and/or direction in a time dependent manner.

Single or multiple applications of electric field, as well as single or multiple applications of ultrasound are also possible, in any order and in any combination. The ultrasound and/or the electric field may be delivered as single or multiple continuous applications, or as pulses (pulsatile delivery).

Electroporation has been used in both *in vitro* and *in vivo* procedures to introduce foreign material into living cells. With *in vitro* applications, a sample of live cells is first mixed with the agent of interest and placed between electrodes such as parallel plates. Then, the electrodes apply an electrical field to the cell/implant mixture. Examples of systems that perform *in vitro* electroporation include the Electro Cell Manipulator ECM600 product, and the Electro Square Porator T820, both made by the BTX Division of Genetronics, Inc (see U.S. Pat. No 5,869,326).

The known electroporation techniques (both *in vitro* and *in vivo*) function by applying a brief high voltage pulse to electrodes positioned around the treatment region. The electric field generated between the electrodes causes the cell membranes to temporarily become porous, whereupon molecules of the agent of interest enter the cells. In known electroporation applications, this electric field comprises a single square wave pulse on the order of 1000 V/cm, of about 100 .mu.s duration. Such a pulse may be generated, for example, in known applications of the Electro Square Porator T820.

Preferably, the electric field has a strength of from about 1 V/cm to about 10 kV/cm under *in vitro* conditions. Thus, the electric field may have a strength of 1 V/cm, 2 V/cm, 3 V/cm, 4 V/cm, 5 V/cm, 6 V/cm, 7 V/cm, 8 V/cm, 9 V/cm, 10 V/cm, 20 V/cm, 50 V/cm, 100 V/cm, 200 V/cm, 300 V/cm, 400 V/cm, 500 V/cm, 600 V/cm, 700 V/cm, 800 V/cm, 900 V/cm, 1 kV/cm, 2 kV/cm, 5 kV/cm, 10 kV/cm, 20 kV/cm, 50 kV/cm or more. More preferably from about 0.5 kV/cm to about 4.0 kV/cm under *in vitro* conditions. Preferably the electric field has a strength of from about 1 V/cm to about 10 kV/cm under *in vivo* conditions. However, the electric field strengths may be lowered where the number of pulses delivered to the target site are increased. Thus, pulsatile delivery of electric fields at lower field strengths is envisaged.

Preferably the application of the electric field is in the form of multiple pulses such as double pulses of the same strength and capacitance or sequential pulses of varying strength and/or capacitance. As used herein, the term "pulse" includes one or more electric pulses at variable capacitance and voltage and including exponential and/or square wave and/or modulated wave/square wave forms.

Preferably the electric pulse is delivered as a waveform selected from an exponential wave form, a square wave form, a modulated wave form and a modulated square wave form.

A preferred embodiment employs direct current at low voltage. Thus, Applicants disclose the use of an electric field which is applied to the cell, tissue or tissue mass at a field strength of between 1V/cm and 20V/cm, for a period of 100 milliseconds or more, preferably 15 minutes or more.

Ultrasound is advantageously administered at a power level of from about 0.05 W/cm2 to about 100 W/cm2. Diagnostic or therapeutic ultrasound may be used, or combinations thereof.

As used herein, the term "ultrasound" refers to a form of energy which consists of mechanical vibrations the frequencies of which are so high they are above the range of human hearing. Lower frequency limit of the ultrasonic spectrum may generally be taken as about 20 kHz. Most diagnostic applications of ultrasound employ frequencies in the range 1 and 15 MHz' (From Ultrasonics in Clinical Diagnosis, P. N. T. Wells, ed., 2nd. Edition, Publ. Churchill Livingstone [Edinburgh, London & NY, 1977]).

Ultrasound has been used in both diagnostic and therapeutic applications. When used as a diagnostic tool ("diagnostic ultrasound"), ultrasound is typically used in an energy density range of up to about 100 mW/cm2 (FDA recommendation), although energy densities of up to 750 mW/cm2 have been used. In physiotherapy, ultrasound is typically used as an energy source in a range up to about 3 to 4 W/cm2 (WHO recommendation). In other therapeutic applications, higher intensities of ultrasound may be employed, for example, HIFU at 100 W/cm up to 1 kW/cm2 (or even higher) for short periods of time. The term "ultrasound" as used in this specification is intended to encompass diagnostic, therapeutic and focused ultrasound.

Focused ultrasound (FUS) allows thermal energy to be delivered without an invasive probe (see Morocz et al 1998 Journal of Magnetic Resonance Imaging Vol.8, No. 1, pp.136-142. Another form of focused ultrasound is high intensity focused ultrasound (HIFU) which is reviewed by Moussatov et al in Ultrasonics (1998) Vol.36, No.8, pp.893-900 and TranHuuHue et al in Acustica (1997) Vol.83, No.6, pp.1103-1106.

Preferably, a combination of diagnostic ultrasound and a therapeutic ultrasound is employed. This combination is not intended to be limiting, however, and the skilled reader will appreciate that any variety of combinations of ultrasound may be used. Additionally, the energy density, frequency of ultrasound, and period of exposure may be varied.

Preferably the exposure to an ultrasound energy source is at a power density of from about 0.05 to about 100 Wcm-2. Even more preferably, the exposure to an ultrasound energy source is at a power density of from about 1 to about 15 Wcm-2.

Preferably the exposure to an ultrasound energy source is at a frequency of from about 0.015 to about 10.0 MHz. More preferably the exposure to an ultrasound energy source is at a frequency of from about 0.02 to about 5.0 MHz or about 6.0 MHz. Most preferably, the ultrasound is applied at a frequency of 3 MHz.

Preferably the exposure is for periods of from about 10 milliseconds to about 60 minutes. Preferably the exposure is for periods of from about 1 second to about 5 minutes. More preferably, the ultrasound is applied for about 2 minutes. Depending on the particular target cell to be disrupted, however, the exposure may be for a longer duration, for example, for 15 minutes.

Advantageously, the target tissue is exposed to an ultrasound energy source at an acoustic power density of from about 0.05 Wcm-2 to about 10 Wcm-2 with a frequency ranging from about 0.015 to about 10 MHz (see WO 98/52609). However, alternatives are also possible, for example, exposure to an ultrasound energy source at an acoustic power density of above 100 Wcm-2, but for reduced periods of time, for example, 1000 Wcm-2 for periods in the millisecond range or less.

Preferably the application of the ultrasound is in the form of multiple pulses; thus, both continuous wave and pulsed wave (pulsatile delivery of ultrasound) may be employed in any combination. For example, continuous wave ultrasound may be applied, followed by pulsed wave ultrasound, or vice versa. This may be repeated any number of times, in any order and combination. The pulsed wave ultrasound may be applied against a background of continuous wave ultrasound, and any number of pulses may be used in any number of groups.

Preferably, the ultrasound may comprise pulsed wave ultrasound. In a highly preferred embodiment, the ultrasound is applied at a power density of 0.7 Wcm-2 or 1.25 Wcm-2 as a continuous wave. Higher power densities may be employed if pulsed wave ultrasound is used.

Use of ultrasound is advantageous as, like light, it may be focused accurately on a target. Moreover, ultrasound is advantageous as it may be focused more deeply into tissues unlike light. It is therefore better suited to whole-tissue penetration (such as but not limited to a lobe of the liver) or whole organ (such as but not limited to the entire liver or an entire muscle, such as the heart) therapy. Another important advantage is that ultrasound is a non-invasive stimulus which is used in a wide variety of diagnostic and therapeutic applications. By way of example, ultrasound is well known in medical imaging techniques and, additionally, in orthopedic therapy. Furthermore, instruments suitable for the application of ultrasound to a subject vertebrate are widely available and their use is well known in the art.

In particular embodiments, the guide molecule is modified by a secondary structure to increase the specificity of the CRISPR-Cas system and the secondary structure can protect against exonuclease activity and allow for 5' additions to the guide sequence also referred to herein as a protected guide molecule.

In one aspect, the invention provides for hybridizing a "protector RNA" to a sequence of the guide molecule, wherein the "protector RNA" is an RNA strand complementary to the 3' end of the guide molecule to thereby generate a partially double-stranded guide RNA. In an embodiment of the invention, protecting mismatched bases (i.e. the bases of the guide molecule which do not form part of the guide sequence) with a perfectly complementary protector sequence decreases the likelihood of target RNA binding to the mismatched basepairs at the 3' end. In particular embodiments of the invention, additional sequences comprising an extented length may also be present within the guide molecule such that the guide comprises a protector sequence within the guide molecule. This "protector sequence" ensures that the guide molecule comprises a "protected sequence" in addition to an "exposed sequence" (comprising the part of the guide sequence hybridizing to the target sequence). In particular embodiments, the guide molecule is modified by the presence of the protector guide to comprise a secondary structure such as a hairpin. Advantageously there are three or four to thirty or more, e.g., about 10 or more, contiguous base pairs having complementarity to the protected sequence, the guide sequence or both. It is advantageous that the protected portion does not impede thermodynamics of the CRISPR-Cas system interacting with its target. By providing such an extension including a partially double stranded guide moleucle, the guide molecule is considered protected and results in improved specific binding of the CRISPR-Cas complex, while maintaining specific activity.

In particular embodiments, use is made of a truncated guide (tru-guide), i.e. a guide molecule which comprises a guide sequence which is truncated in length with respect to the canonical guide sequence length. As described by Nowak et al. (Nucleic Acids Res (2016) 44 (20): 9555-9564), such guides may allow catalytically active CRISPR-Cas enzyme to bind its target without cleaving the target RNA. In particular embodiments, a truncated guide is used which allows the binding of the target but retains only nickase activity of the CRISPR-Cas enzyme.

### CRISPR RNA-Targeting Effector Proteins

In one example embodiment, the CRISPR system effector protein is an RNA-targeting effector protein. In certain embodiments, the CRISPR system effector protein is a Type VI CRISPR system targeting RNA (e.g., Cas13a, Cas13b, Cas13c or Cas13d). Example RNA-targeting effector proteins include Cas13b and C2c2 (now known as Cas13a). It will be understood that the term "C2c2" herein is used interchangeably with "Cas13a". "C2c2" is now referred to as "Cas13a", and the terms are used interchangeably herein unless indicated otherwise. As used herein, the term "Cas13" refers to any Type VI CRISPR system targeting RNA (e.g., Cas13a, Cas13b, Cas13c or Cas13d). When the CRISPR protein is a C2c2 protein, a tracrRNA is not required. C2c2 has been described in Abudayyeh et al. (2016) "C2c2 is a single-component programmable RNA-guided RNA-targeting CRISPR effector"; Science; DOI: 10.1126/science.aaf5573; and Shmakov et al. (2015) "Discovery and Functional Characterization of Diverse Class 2 CRISPR-Cas Systems", Molecular Cell, DOI: dx.doi.org/10.1016/j.molcel.2015.10.008. Cas13b has been described in Smargon et al. (2017) "Cas13b Is a Type VI-B CRISPR-Associated RNA-Guided RNases Differentially Regulated by Accessory Proteins Csx27 and Csx28," Molecular Cell. 65, 1-13; dx.doi.org/10.1016/j.molcel.2016.12.023.

In some embodiments, one or more elements of a nucleic acid-targeting system is derived from a particular organism comprising an endogenous CRISPR RNA-targeting system. In certain example embodiments, the effector protein CRISPR RNA-targeting system comprises at least one HEPN domain, including but not limited to the HEPN domains described herein, HEPN domains known in the art, and domains recognized to be HEPN domains by comparison to consensus sequence motifs. Several such domains are provided herein. In one non-limiting example, a consensus sequence can be derived from the sequences of C2c2 or Cas13b orthologs provided herein. In certain example embodiments, the effector protein comprises a single HEPN domain. In certain other example embodiments, the effector protein comprises two HEPN domains.

In one example embodiment, the effector protein comprise one or more HEPN domains comprising a RxxxxH motif sequence. The RxxxxH motif sequence can be, without limitation, from a HEPN domain described herein or a HEPN domain known in the art. RxxxxH motif sequences further include motif sequences created by combining portions of two or more HEPN domains. As noted, consensus sequences can be derived from the sequences of the orthologs disclosed in U.S. Provisional Patent Application 62/432,240 entitled "Novel CRISPR Enzymes and Systems," U.S. Provisional Patent Application 62/471,710 entitled "Novel Type VI CRISPR Orthologs and Systems" filed on March 15, 2017, and U.S. Provisional Patent Application entitled "Novel Type VI CRISPR Orthologs and Systems," labeled as attorney docket number 47627-05-2133 and filed on April 12, 2017.

In certain other example embodiments, the CRISPR system effector protein is a C2c2 nuclease. The activity of C2c2 may depend on the presence of two HEPN domains. These have been shown to be RNase domains, *i.e.* nuclease (in particular an endonuclease) cutting RNA. C2c2 HEPN may also target DNA, or potentially DNA and/or RNA. On the basis that the HEPN domains of C2c2 are at least capable of binding to and, in their wild-type form, cutting RNA, then it is preferred that the C2c2 effector protein has RNase function. Regarding C2c2 CRISPR systems, reference is made to U.S. Provisional 62/351,662 filed on June 17, 2016 and U.S. Provisional 62/376,377 filed on August 17, 2016. Reference is also made to U.S. Provisional 62/351,803 filed on June 17, 2016. Reference is also made to U.S. Provisional entitled "Novel Crispr Enzymes and Systems" filed December 8, 2016 bearing Broad Institute No. 10035.PA4 and Attorney Docket No. 47627.03.2133. Reference is further made to East-Seletsky et al. "Two distinct RNase activities of CRISPR-C2c2 enable guide-RNA processing and RNA detection" Nature doi:10/1038/nature19802 and Abudayyeh et al. "C2c2 is a single-component programmable RNA-guided RNA targeting CRISPR effector" bioRxiv doi:10.1101/054742.

In certain embodiments, the C2c2 effector protein is from an organism of a genus selected from the group consisting of: Leptotrichia, Listeria, Corynebacter, Sutterella, Legionella, Treponema, Filifactor, Eubacterium, Streptococcus, Lactobacillus, Mycoplasma, Bacteroides, Flaviivola, Flavobacterium, Sphaerochaeta, Azospirillum, Gluconacetobacter, Neisseria, Roseburia, Parvibaculum, Staphylococcus, Nitratifractor, Mycoplasma, Campylobacter, and Lachnospira, or the C2c2 effector protein is an organism selected from the group consisting of: Leptotrichia shahii, Leptotrichia. wadei, Listeria seeligeri, Clostridium aminophilum, Carnobacterium gallinarum, Paludibacter propionicigenes, Listeria weihenstephanensis, or the C2c2 effector protein is a L. wadei F0279 or L. wadei F0279 (Lw2) C2C2 effector protein. In another embodiment, the one or more guide RNAs are designed to detect a single nucleotide polymorphism, splice variant of a transcript, or a frameshift mutation in a target RNA or DNA.

In certain example embodiments, the RNA-targeting effector protein is a Type VI-B effector protein, such as Cas13b and Group 29 or Group 30 proteins. In certain example embodiments, the RNA-targeting effector protein comprises one or more HEPN domains. In certain example embodiments, the RNA-targeting effector protein comprises a C-terminal HEPN domain, a N-terminal HEPN domain, or both. Regarding example Type VI-B effector proteins that may be used in the context of this invention, reference is made to US Application No. 15/331,792 entitled "Novel CRISPR Enzymes and Systems" and filed October 21, 2016, International Patent Application No. PCT/US2016/058302 entitled "Novel CRISPR Enzymes and Systems", and filed October 21, 2016, and Smargon et al. "Cas13b is a Type VI-B CRISPR-associated RNA-Guided RNase differentially regulated by accessory proteins Csx27 and Csx28" Molecular Cell, 65, 1-13 (2017); dx.doi.org/10.1016/j.molcel.2016.12.023, and U.S. Provisional Application No. to be assigned, entitled "Novel Cas13b Orthologues CRISPR Enzymes and System" filed March 15, 2017. In particular embodiments, the Cas13b enzyme is derived from *Bergeyella zoohelcum.*

In certain example embodiments, the RNA-targeting effector protein is a Cas13c effector protein as disclosed in U.S. Provisional Patent Application No. 62/525,165 filed June 26, 2017, and PCT Application No. US 2017/047193 filed August 16, 2017.

In some embodiments, one or more elements of a nucleic acid-targeting system is derived from a particular organism comprising an endogenous CRISPR RNA-targeting system. In certain embodiments, the CRISPR RNA-targeting system is found in *Eubacterium* and *Ruminococcus.* In certain embodiments, the effector protein comprises targeted and collateral ssRNA cleavage activity. In certain embodiments, the effector protein comprises dual HEPN domains. In certain embodiments, the effector protein lacks a counterpart to the Helical-1 domain of Cas13a. In certain embodiments, the effector protein is smaller than previously characterized class 2 CRISPR effectors, with a median size of 928 aa. This median size is 190 aa (17%) less than that of Cas13c, more than 200 aa (18%) less than that of Cas13b, and more than 300 aa (26%) less than that of Cas13a. In certain embodiments, the effector protein has no requirement for a flanking sequence (e.g., PFS, PAM).

In certain embodiments, the effector protein locus structures include a WYL domain containing accessory protein (so denoted after three amino acids that were conserved in the originally identified group of these domains; see, e.g., WYL domain IPR026881). In certain embodiments, the WYL domain accessory protein comprises at least one helix-turn-helix (HTH) or ribbon-helix-helix (RHH) DNA-binding domain. In certain embodiments, the WYL domain containing accessory protein increases both the targeted and the collateral ssRNA cleavage activity of the RNA-targeting effector protein. In certain embodiments, the WYL domain containing accessory protein comprises an N-terminal RHH domain, as well as a pattern of primarily hydrophobic conserved residues, including an invariant tyrosine-leucine doublet corresponding to the original WYL motif. In certain embodiments, the WYL domain containing accessory protein is WYL1. WYL1 is a single WYL-domain protein associated primarily with *Ruminococcus.*

In other example embodiments, the Type VI RNA-targeting Cas enzyme is Cas13d. In certain embodiments, Cas13d is *Eubacterium siraeum* DSM 15702 (EsCas13d) or *Ruminococcus* sp. N15.MGS-57 (RspCas13d) (see, e.g., Yan et al., Cas13d Is a Compact RNA-Targeting Type VI CRISPR Effector Positively Modulated by a WYL-Domain-Containing Accessory Protein, Molecular Cell (2018), doi.org/10.1016/j.molcel.2018.02.028). RspCas13d and EsCas13d have no flanking sequence requirements (e.g., PFS, PAM).

### Cas13 RNA Editing

In one aspect, the invention provides a method of modifying or editing a target transcript in a eukaryotic cell. In some embodiments, the method comprises allowing a CRISPR-Cas effector module complex to bind to the target polynucleotide to effect RNA base editing, wherein the CRISPR-Cas effector module complex comprises a Cas effector module complexed with a guide sequence hybridized to a target sequence within said target polynucleotide, wherein said guide sequence is linked to a direct repeat sequence. In some embodiments, the Cas effector module comprises a catalytically inactive CRISPR-Cas protein. In some embodiments, the guide sequence is designed to introduce one or more mismatches to the RNA/RNA duplex formed between the target sequence and the guide sequence. In particular embodiments, the mismatch is an A-C mismatch. In some embodiments, the Cas effector may associate with one or more functional domains (e.g. via fusion protein or suitable linkers). In some embodiments, the effector domain comprises one or more cytindine or adenosine deaminases that mediate endogenous editing of via hydrolytic deamination. In particular embodiments, the effector domain comprises the adenosine deaminase acting on RNA (ADAR) family of enzymes. In particular embodiments, the adenosine deaminase protein or catalytic domain thereof capable of deaminating adenosine or cytidine in RNA or is an RNA specific adenosine deaminase and/or is a bacterial, human, cephalopod, or Drosophila adenosine deaminase protein or catalytic domain thereof, preferably TadA, more preferably ADAR, optionally huADAR, optionally (hu)ADAR1 or (hu)ADAR2, preferably huADAR2 or catalytic domain thereof.

The present application relates to modifying a target RNA sequence of interest (see, e.g, Cox et al., Science. 2017 Nov 24;358(6366):1019-1027). Using RNA-targeting rather than DNA targeting offers several advantages relevant for therapeutic development. First, there are substantial safety benefits to targeting RNA: there will be fewer off-target events because the available sequence space in the transcriptome is significantly smaller than the genome, and if an off-target event does occur, it will be transient and less likely to induce negative side effects. Second, RNA-targeting therapeutics will be more efficient because they are cell-type independent and not have to enter the nucleus, making them easier to deliver.

A further aspect relates to the method and composition as envisaged herein for use in prophylactic or therapeutic treatment, preferably wherein said target locus of interest is within a human or animal and to methods of modifying an Adenine or Cytidine in a target RNA sequence of interest, comprising delivering to said target RNA, the composition as described herein. In particular embodiments, the CRISPR system and the adenonsine deaminase, or catalytic domain thereof, are delivered as one or more polynucleotide molecules, as a ribonucleoprotein complex, optionally via particles, vesicles, or one or more viral vectors. In particular embodiments, compositions for use in therapy are provided. In particular embodiments, when the target is a human or animal target, the method is carried out ex vivo or in vitro.

A further aspect relates to the method as envisaged herein for use in prophylactic or therapeutic treatment, preferably wherein said target of interest is within a human or animal and to methods of modifying an Adenine or Cytidine in a target RNA sequence of interest, comprising delivering to said target RNA, the composition as described herein. In particular embodiments, the CRISPR system and the adenonsine deaminase, or catalytic domain thereof, are delivered as one or more polynucleotide molecules, as a ribonucleoprotein complex, optionally via particles, vesicles, or one or more viral vectors.

In one aspect (described but not claimed herein), a method of generating a eukaryotic cell comprising a modified or edited gene is provided. In some embodiments, the method comprises (a) introducing one or more vectors into a eukaryotic cell, wherein the one or more vectors drive expression of one or more of: Cas effector module, and a guide sequence linked to a direct repeat sequence, wherein the Cas effector module associate one or more effector domains that mediate base editing, and (b) allowing a CRISPR-Cas effector module complex to bind to a target polynucleotide to effect base editing of the target polynucleotide within said disease gene, wherein the CRISPR-Cas effector module complex comprises a Cas effector module complexed with the guide sequence that is hybridized to the target sequence within the target polynucleotide, wherein the guide sequence may be designed to introduce one or more mismatches between the RNA/RNA duplex formed between the guide sequence and the target sequence. In particular embodiments, the mismatch is an A-C mismatch. In some embodiments, the Cas effector may associate with one or more functional domains (e.g. via fusion protein or suitable linkers). In some embodiments, the effector domain comprises one or more cytidine or adenosine deaminases that mediate endogenous editing of via hydrolytic deamination. In particular embodiments, the effector domain comprises the adenosine deaminase acting on RNA (ADAR) family of enzymes. In particular embodiments, the adenosine deaminase protein or catalytic domain thereof capable of deaminating adenosine or cytidine in RNA or is an RNA specific adenosine deaminase and/or is a bacterial, human, cephalopod, or Drosophila adenosine deaminase protein or catalytic domain thereof, preferably TadA, more preferably ADAR, optionally huADAR, optionally (hu)ADAR1 or (hu)ADAR2, preferably huADAR2 or catalytic domain thereof.

### dCas9 and Functional Effectors

Unlike CRISPR-Cas-mediated gene knockout, which permanently eliminates expression by mutating the gene at the DNA level, CRISPR-Cas knockdown allows for temporary reduction of gene expression through the use of artificial transcription factors. Mutating key residues in cleavage domains of the Cas protein results in the generation of a catalytically inactive Cas protein. A catalytically inactive Cas protein complexes with a guide RNA and localizes to the DNA sequence specified by that guide RNA's targeting domain, however, it does not cleave the target DNA. Fusion of the inactive Cas protein to an effector domain also referred to herein as a functional domain, e.g., a transcription repression domain, enables recruitment of the effector to any DNA site specified by the guide RNA.

In certain example embodiments, inactivated Cas protein may be delivered to one or more target regions. For example, using the *in situ* Hi-C methods described herein chromatin loop domains may be identified in a sample. From the Hi-C contact map target genome regions may be identified where abrogation of a loop or insertion of a new chromatin loop is desired. Accordingly an inactived Cas protein may be delivered to the target region. In certain example embodiments, the inactivated Cas protein binds to or proximate to an existing loop anchor motif to mask or otherwise interfer with formation of a chromatin loop anchor. In certain other example embodiments, one or more inactivated Cas proteins may be delivered to one or more target regions such that upon binding the one or more inactivated Cas proteins arrest an chromatin loop extrusion complex resulting in formation of a new chromatin loop or modification of an existing chromatin loop (i.e. increasing or descreasing the size of a given chromatin domain).

The inactivated CRISPR/Cas protein may have associated (e.g., via fusion protein) one or more functional domains, including for example, one or more domains from the group comprising, consisting essentially of, or consisting of methylase activity, demethylase activity, transcription activation activity, transcription repression activity, transcription release factor activity, histone modification activity (e.g., histone acetylation activity, histone deacetylation activity), RNA cleavage activity, DNA cleavage activity, nucleic acid binding activity, and molecular switches (e.g., light inducible). Preferred domains are Fok1, VP64, P65, HSF1, MyoD1. In the event that Fok1 is provided, it is advantageous that multiple Fok1 functional domains are provided to allow for a functional dimer and that guide RNAs are designed to provide proper spacing for functional use (Fok1) as specifically described in Tsai et al. Nature Biotechnology, Vol. 32, Number 6, June 2014). The adaptor protein may utilize known linkers to attach such functional domains. In certain example embodiments, the inactivated Cas protein may be fused to one or more of CTCF, SA1/2, Smc3, Smc1, cohesin and Rad21, such that binding of the fusion protein causes a loop anchor to form or an extrusion complext arrest at the site of inactivated Cas binding. When more than one functional domain is included, the functional domains may be the same or different.

In general, the positioning of the one or more functional domain on the inactivated CRISPR/Cas protein is one which allows for correct spatial orientation for the functional domain to affect the target with the attributed functional effect. For example, if the functional domain is a transcription activator (e.g., VP64 or p65), the transcription activator is placed in a spatial orientation which allows it to affect the transcription of the target. Likewise, a transcription repressor will be advantageously positioned to affect the transcription of the target, and a nuclease (e.g., Fok1) will be advantageously positioned to cleave or partially cleave the target. This may include positions other than the N- / C- terminus of the CRISPR protein.

In certain embodiments, Cas protein may be fused to a transcriptional repression domain and recruited to the promoter region of a gene. Especially for gene repression, it is contemplated herein that blocking the binding site of an endogenous transcription factor would aid in downregulating gene expression. In another embodiment, an inactive Cas protein can be fused to a chromatin modifying protein. Altering chromatin status can result in decreased expression of the target gene. This § could be adapted to describe the "Road block" embodiment described by Erez

In an embodiment, a guide RNA molecule can be targeted to a known transcription response elements (e.g., promoters, enhancers, etc.), a known upstream activating sequences, and/or sequences of unknown or known function that are suspected of being able to control expression of the target DNA. Idem: adapt to refer to regions with the motifs of interest

In some methods, a target polynucleotide can be inactivated to effect the modification of the expression in a cell. For example, upon the binding of a CRISPR complex to a target sequence in a cell, the target polynucleotide is inactivated such that the sequence is not transcribed, the coded protein is not produced, or the sequence does not function as the wild-type sequence does. For example, a protein or microRNA coding sequence may be inactivated such that the protein is not produced. idem

A further aspect relates to an isolated cell obtained or obtainable from the methods described herein comprising the composition described herein or progeny of said modified cell, preferably wherein said cell comprises a hypoxanthine or a guanine in replace of said Adenine in said target RNA of interest compared to a corresponding cell not subjected to the method. In particular embodiments, the cell is a eukaryotic cell, preferably a human or non-human animal cell, optionally a therapeutic T cell or an antibody-producing B-cell.

In some embodiments, the modified cell is a therapeutic T cell, such as a T cell suitable for adoptive cell transfer therapies (e.g., CAR-T therapies). The modification may result in one or more desirable traits in the therapeutic T cell, as described further herein.

The invention further relates to a method for cell therapy, comprising administering to a patient in need thereof the modified cell described herein, wherein the presence of the modified cell remedies a disease in the patient.

The present invention may be further illustrated and extended based on aspects of CRISPR-Cas development and use as set forth in the following articles and particularly as relates to delivery of a CRISPR protein complex and uses of an RNA guided endonuclease in cells and organisms:
➢ Multiplex genome engineering using CRISPR-Cas systems. Cong, L., Ran, F.A., Cox, D., Lin, S., Barretto, R., Habib, N., Hsu, P.D., Wu, X., Jiang, W., Marraffini, L.A., & Zhang, F. Science Feb 15;339(6121):819-23 (2013);
➢ RNA-guided editing of bacterial genomes using CRISPR-Cas systems. Jiang W., Bikard D., Cox D., Zhang F, Marraffini LA. Nat Biotechnol Mar;31(3):233-9 (2013);
➢ One-Step Generation of Mice Carrying Mutations in Multiple Genes by CRISPR-Cas-Mediated Genome Engineering. Wang H., Yang H., Shivalila CS., Dawlaty MM., Cheng AW., Zhang F., Jaenisch R. Cell May 9;153(4):910-8 (2013);
➢Optical control of mammalian endogenous transcription and epigenetic states. Konermann S, Brigham MD, Trevino AE, Hsu PD, Heidenreich M, Cong L, Platt RJ, Scott DA, Church GM, Zhang F. Nature. Aug 22;500(7463):472-6. doi: 10.1038/Nature12466. Epub 2013 Aug 23 (2013);
➢ Double Nicking by RNA-Guided CRISPR Cas9 for Enhanced Genome Editing Specificity. Ran, FA., Hsu, PD., Lin, CY., Gootenberg, JS., Konermann, S., Trevino, AE., Scott, DA., Inoue, A., Matoba, S., Zhang, Y., & Zhang, F. Cell Aug 28. pii: S0092-8674(13)01015-5 (2013-A);
➢ DNA targeting specificity of RNA-guided Cas9 nucleases. Hsu, P., Scott, D., Weinstein, J., Ran, FA., Konermann, S., Agarwala, V., Li, Y., Fine, E., Wu, X., Shalem, O., Cradick, TJ., Marraffini, LA., Bao, G., & Zhang, F. Nat Biotechnol doi:10.1038/nbt.2647 (2013);
➢Genome engineering using the CRISPR-Cas9 system. Ran, FA., Hsu, PD., Wright, J., Agarwala, V., Scott, DA., Zhang, F. Nature Protocols Nov;8(11):2281-308 (2013-B);
➢ Genome-Scale CRISPR-Cas9 Knockout Screening in Human Cells. Shalem, O., Sanjana, NE., Hartenian, E., Shi, X., Scott, DA., Mikkelson, T., Heckl, D., Ebert, BL., Root, DE., Doench, JG., Zhang, F. Science Dec 12. (2013);
➢ Crystal structure of cas9 in complex with guide RNA and target DNA. Nishimasu, H., Ran, FA., Hsu, PD., Konermann, S., Shehata, SI., Dohmae, N., Ishitani, R., Zhang, F., Nureki, O. Cell Feb 27, 156(5):935-49 (2014);
➢ Genome-wide binding of the CRISPR endonuclease Cas9 in mammalian cells. Wu X., Scott DA., Kriz AJ., Chiu AC., Hsu PD., Dadon DB., Cheng AW., Trevino AE., Konermann S., Chen S., Jaenisch R., Zhang F., Sharp PA. Nat Biotechnol. Apr 20. doi: 10.1038/nbt.2889 (2014);
➢ CRISPR-Cas9 Knockin Mice for Genome Editing and Cancer Modeling. Platt RJ, Chen S, Zhou Y, Yim MJ, Swiech L, Kempton HR, Dahlman JE, Parnas O, Eisenhaure TM, Jovanovic M, Graham DB, Jhunjhunwala S, Heidenreich M, Xavier RJ, Langer R, Anderson DG, Hacohen N, Regev A, Feng G, Sharp PA, Zhang F. Cell 159(2): 440-455 DOI: 10.1016/j.cell.2014.09.014(2014);
➢Development and Applications of CRISPR-Cas9 for Genome Engineering, Hsu PD, Lander ES, Zhang F., Cell. Jun 5;157(6):1262-78 (2014).
➢ Genetic screens in human cells using the CRISPR-Cas9 system, Wang T, Wei JJ, Sabatini DM, Lander ES., Science. January 3; 343(6166): 80-84. doi:10.1126/science.1246981 (2014);
➢Rational design of highly active sgRNAs for CRISPR-Cas9-mediated gene inactivation, Doench JG, Hartenian E, Graham DB, Tothova Z, Hegde M, Smith I, Sullender M, Ebert BL, Xavier RJ, Root DE., (published online 3 September 2014) Nat Biotechnol. Dec;32(12):1262-7 (2014);
➢ In vivo interrogation of gene function in the mammalian brain using CRISPR-Cas9, Swiech L, Heidenreich M, Banerjee A, Habib N, Li Y, Trombetta J, Sur M, Zhang F., (published online 19 October 2014) Nat Biotechnol. Jan;33(1):102-6 (2015);
➢Genome-scale transcriptional activation by an engineered CRISPR-Cas9 complex, Konermann S, Brigham MD, Trevino AE, Joung J, Abudayyeh OO, Barcena C, Hsu PD, Habib N, Gootenberg JS, Nishimasu H, Nureki O, Zhang F., Nature. Jan 29;517(7536):583-8 (2015).
➢A split-Cas9 architecture for inducible genome editing and transcription modulation, Zetsche B, Volz SE, Zhang F., (published online 02 February 2015) Nat Biotechnol. Feb;33(2):139-42 (2015);
➢Genome-wide CRISPR Screen in a Mouse Model of Tumor Growth and Metastasis, Chen S, Sanjana NE, Zheng K, Shalem O, Lee K, Shi X, Scott DA, Song J, Pan JQ, Weissleder R, Lee H, Zhang F, Sharp PA. Cell 160, 1246-1260, March 12, 2015 (multiplex screen in mouse), and
➢ In vivo genome editing using Staphylococcus aureus Cas9, Ran FA, Cong L, Yan WX, Scott DA, Gootenberg JS, Kriz AJ, Zetsche B, Shalem O, Wu X, Makarova KS, Koonin EV, Sharp PA, Zhang F., (published online 01 April 2015), Nature. Apr 9;520(7546):186-91 (2015).
➢ Shalem et al., "High-throughput functional genomics using CRISPR-Cas9," Nature Reviews Genetics 16, 299-311 (May 2015).
➢Xu et al., "Sequence determinants of improved CRISPR sgRNA design," Genome Research 25, 1147-1157 (August 2015).
➢ Parnas et al., "A Genome-wide CRISPR Screen in Primary Immune Cells to Dissect Regulatory Networks," Cell 162, 675-686 (July 30, 2015).
➢Ramanan et al., CRISPR-Cas9 cleavage of viral DNA efficiently suppresses hepatitis B virus," Scientific Reports 5:10833. doi: 10.1038/srep10833 (June 2, 2015)
➢ Nishimasu et al., Crystal Structure of Staphylococcus aureus Cas9," Cell 162, 1113-1126 (Aug. 27, 2015)
➢ BCL11A enhancer dissection by Cas9-mediated in situ saturating mutagenesis, Canver et al., Nature 527(7577):192-7 (Nov. 12, 2015) doi: 10.1038/nature15521. Epub 2015 Sep 16.
➢ Cpf1 Is a Single RNA-Guided Endonuclease of a Class 2 CRISPR-Cas System, Zetsche et al., Cell 163, 759-71 (Sep 25, 2015).
➢ Discovery and Functional Characterization of Diverse Class 2 CRISPR-Cas Systems, Shmakov et al., Molecular Cell, 60(3), 385-397 doi: 10.1016/j.molcel.2015.10.008 Epub October 22, 2015.
➢ Rationally engineered Cas9 nucleases with improved specificity, Slaymaker et al., Science 2016 Jan 1 351(6268): 84-88 doi: 10.1126/science.aad5227. Epub 2015 Dec 1.
➢ Gao et al, "Engineered Cpf1 Enzymes with Altered PAM Specificities," bioRxiv 091611; doi: http://dx.doi.org/10.1101/091611 (Dec. 4, 2016).
➢ Cox et al., "RNA editing with CRISPR-Cas13," Science. 2017 Nov 24;358(6366):1019-1027. doi: 10.1126/science.aaq0180. Epub 2017 Oct 25.

Each of which is discussed briefly below:
➢ Cong *et al.* engineered type II CRISPR-Cas systems for use in eukaryotic cells based on both *Streptococcus thermophilus* Cas9 and also *Streptococcus pyogenes* Cas9 and demonstrated that Cas9 nucleases can be directed by short RNAs to induce precise cleavage of DNA in human and mouse cells. Their study further showed that Cas9 as converted into a nicking enzyme can be used to facilitate homology-directed repair in eukaryotic cells with minimal mutagenic activity. Additionally, their study demonstrated that multiple guide sequences can be encoded into a single CRISPR array to enable simultaneous editing of several at endogenous genomic loci sites within the mammalian genome, demonstrating easy programmability and wide applicability of the RNA-guided nuclease technology. This ability to use RNA to program sequence specific DNA cleavage in cells defined a new class of genome engineering tools. These studies further showed that other CRISPR loci are likely to be transplantable into mammalian cells and can also mediate mammalian genome cleavage. Importantly, it can be envisaged that several aspects of the CRISPR-Cas system can be further improved to increase its efficiency and versatility.
➢ Jiang *et al.* used the clustered, regularly interspaced, short palindromic repeats (CRISPR)-associated Cas9 endonuclease complexed with dual-RNAs to introduce precise mutations in the genomes of *Streptococcus pneumoniae* and *Escherichia coli.* The approach relied on dual-RNA:Cas9-directed cleavage at the targeted genomic site to kill unmutated cells and circumvents the need for selectable markers or counter-selection systems. The study reported reprogramming dual-RNA:Cas9 specificity by changing the sequence of short CRISPR RNA (crRNA) to make single- and multinucleotide changes carried on editing templates. The study showed that simultaneous use of two crRNAs enabled multiplex mutagenesis. Furthermore, when the approach was used in combination with recombineering, in *S. pneumoniae,* nearly 100% of cells that were recovered using the described approach contained the desired mutation, and in E. *coli,* 65% that were recovered contained the mutation.
➢ Wang *et al.* (2013) used the CRISPR-Cas system for the one-step generation of mice carrying mutations in multiple genes which were traditionally generated in multiple steps by sequential recombination in embryonic stem cells and/or time-consuming intercrossing of mice with a single mutation. The CRISPR-Cas system will greatly accelerate the *in vivo* study of functionally redundant genes and of epistatic gene interactions.
➢Konermann *et al.* (2013) addressed the need in the art for versatile and robust technologies that enable optical and chemical modulation of DNA-binding domains based CRISPR Cas9 enzyme and also Transcriptional Activator Like Effectors
➢ Ran *et al.* (2013-A) described an approach that combined a Cas9 nickase mutant with paired guide RNAs to introduce targeted double-strand breaks. This addresses the issue of the Cas9 nuclease from the microbial CRISPR-Cas system being targeted to specific genomic loci by a guide sequence, which can tolerate certain mismatches to the DNA target and thereby promote undesired off-target mutagenesis. Because individual nicks in the genome are repaired with high fidelity, simultaneous nicking *via* appropriately offset guide RNAs is required for double-stranded breaks and extends the number of specifically recognized bases for target cleavage. The authors demonstrated that using paired nicking can reduce off-target activity by 50- to 1,500-fold in cell lines and to facilitate gene knockout in mouse zygotes without sacrificing on-target cleavage efficiency. This versatile strategy enables a wide variety of genome editing applications that require high specificity.
➢Hsu *et al.* (2013) characterized SpCas9 targeting specificity in human cells to inform the selection of target sites and avoid off-target effects. The study evaluated >700 guide RNA variants and SpCas9-induced indel mutation levels at >100 predicted genomic off-target loci in 293T and 293FT cells. The authors that SpCas9 tolerates mismatches between guide RNA and target DNA at different positions in a sequence-dependent manner, sensitive to the number, position and distribution of mismatches. The authors further showed that SpCas9-mediated cleavage is unaffected by DNA methylation and that the dosage of SpCas9 and guide RNA can be titrated to minimize off-target modification. Additionally, to facilitate mammalian genome engineering applications, the authors reported providing a web-based software tool to guide the selection and validation of target sequences as well as off-target analyses.
➢ Ran *et al.* (2013-B) described a set of tools for Cas9-mediated genome editing *via* nonhomologous end joining (NHEJ) or homology-directed repair (HDR) in mammalian cells, as well as generation of modified cell lines for downstream functional studies. To minimize off-target cleavage, the authors further described a double-nicking strategy using the Cas9 nickase mutant with paired guide RNAs. The protocol provided by the authors experimentally derived guidelines for the selection of target sites, evaluation of cleavage efficiency and analysis of off-target activity. The studies showed that beginning with target design, gene modifications can be achieved within as little as 1-2 weeks, and modified clonal cell lines can be derived within 2-3 weeks.
➢ Shalem *et al.* described a new way to interrogate gene function on a genome-wide scale. Their studies showed that delivery of a genome-scale CRISPR-Cas9 knockout (GeCKO) library targeted 18,080 genes with 64,751 unique guide sequences enabled both negative and positive selection screening in human cells. First, the authors showed use of the GeCKO library to identify genes essential for cell viability in cancer and pluripotent stem cells. Next, in a melanoma model, the authors screened for genes whose loss is involved in resistance to vemurafenib, a therapeutic that inhibits mutant protein kinase BRAF. Their studies showed that the highest-ranking candidates included previously validated genes NF1 and MED12 as well as novel hits NF2, CUL3, TADA2B, and TADA1. The authors observed a high level of consistency between independent guide RNAs targeting the same gene and a high rate of hit confirmation, and thus demonstrated the promise of genome-scale screening with Cas9.
➢ Nishimasu *et al.* reported the crystal structure of *Streptococcus pyogenes* Cas9 in complex with sgRNA and its target DNA at 2.5 A° resolution. The structure revealed a bilobed architecture composed of target recognition and nuclease lobes, accommodating the sgRNA:DNA heteroduplex in a positively charged groove at their interface. Whereas the recognition lobe is essential for binding sgRNA and DNA, the nuclease lobe contains the HNH and RuvC nuclease domains, which are properly positioned for cleavage of the complementary and non-complementary strands of the target DNA, respectively. The nuclease lobe also contains a carboxyl-terminal domain responsible for the interaction with the protospacer adjacent motif (PAM). This high-resolution structure and accompanying functional analyses have revealed the molecular mechanism of RNA-guided DNA targeting by Cas9, thus paving the way for the rational design of new, versatile genome-editing technologies.
➢ Wu *et al.* mapped genome-wide binding sites of a catalytically inactive Cas9 (dCas9) from *Streptococcus pyogenes* loaded with single guide RNAs (sgRNAs) in mouse embryonic stem cells (mESCs). The authors showed that each of the four sgRNAs tested targets dCas9 to between tens and thousands of genomic sites, frequently characterized by a 5-nucleotide seed region in the sgRNA and an NGG protospacer adjacent motif (PAM). Chromatin inaccessibility decreases dCas9 binding to other sites with matching seed sequences; thus 70% of off-target sites are associated with genes. The authors showed that targeted sequencing of 295 dCas9 binding sites in mESCs transfected with catalytically active Cas9 identified only one site mutated above background levels. The authors proposed a two-state model for Cas9 binding and cleavage, in which a seed match triggers binding but extensive pairing with target DNA is required for cleavage.
➢ Platt *et al.* established a Cre-dependent Cas9 knockin mouse. The authors demonstrated *in vivo* as well as *ex vivo* genome editing using adeno-associated virus (AAV)-, lentivirus-, or particle-mediated delivery of guide RNA in neurons, immune cells, and endothelial cells.
➢Hsu *et al.* (2014) is a review article that discusses generally CRISPR-Cas9 history from yogurt to genome editing, including genetic screening of cells.
➢ Wang *et al.* (2014) relates to a pooled, loss-of-function genetic screening approach suitable for both positive and negative selection that uses a genome-scale lentiviral single guide RNA (sgRNA) library.
➢Doench *et al.* created a pool of sgRNAs, tiling across all possible target sites of a panel of six endogenous mouse and three endogenous human genes and quantitatively assessed their ability to produce null alleles of their target gene by antibody staining and flow cytometry. The authors showed that optimization of the PAM improved activity and also provided an on-line tool for designing sgRNAs.
➢ Swiech *et al.* demonstrate that AAV-mediated SpCas9 genome editing can enable reverse genetic studies of gene function in the brain.
➢Konermann *et al.* (2015) discusses the ability to attach multiple effector domains, e.g., transcriptional activator, functional and epigenomic regulators at appropriate positions on the guide such as stem or tetraloop with and without linkers.
➢Zetsche *et al.* demonstrates that the Cas9 enzyme can be split into two and hence the assembly of Cas9 for activation can be controlled.
➢ Chen *et al.* relates to multiplex screening by demonstrating that a genome-wide *in vivo* CRISPR-Cas9 screen in mice reveals genes regulating lung metastasis.
➢Ran *et al.* (2015) relates to SaCas9 and its ability to edit genomes and demonstrates that one cannot extrapolate from biochemical assays.
➢ Shalem *et al.* (2015) described ways in which catalytically inactive Cas9 (dCas9) fusions are used to synthetically repress (CRISPRi) or activate (CRISPRa) expression, showing. advances using Cas9 for genome-scale screens, including arrayed and pooled screens, knockout approaches that inactivate genomic loci and strategies that modulate transcriptional activity.
➢Xu *et al.* (2015) assessed the DNA sequence features that contribute to single guide RNA (sgRNA) efficiency in CRISPR-based screens. The authors explored efficiency of CRISPR-Cas9 knockout and nucleotide preference at the cleavage site. The authors also found that the sequence preference for CRISPRi/a is substantially different from that for CRISPR-Cas9 knockout.
➢ Parnas *et al.* (2015) introduced genome-wide pooled CRISPR-Cas9 libraries into dendritic cells (DCs) to identify genes that control the induction of tumor necrosis factor (Tnf) by bacterial lipopolysaccharide (LPS). Known regulators of Tlr4 signaling and previously unknown candidates were identified and classified into three functional modules with distinct effects on the canonical responses to LPS.
➢Ramanan *et al* (2015) demonstrated cleavage of viral episomal DNA (cccDNA) in infected cells. The HBV genome exists in the nuclei of infected hepatocytes as a 3.2kb double-stranded episomal DNA species called covalently closed circular DNA (cccDNA), which is a key component in the HBV life cycle whose replication is not inhibited by current therapies. The authors showed that sgRNAs specifically targeting highly conserved regions of HBV robustly suppresses viral replication and depleted cccDNA.
➢ Nishimasu *et al.* (2015) reported the crystal structures of SaCas9 in complex with a single guide RNA (sgRNA) and its double-stranded DNA targets, containing the 5'-TTGAAT-3' PAM and the 5'-TTGGGT-3' PAM. A structural comparison of SaCas9 with SpCas9 highlighted both structural conservation and divergence, explaining their distinct PAM specificities and orthologous sgRNA recognition.
➢ Canver *et al.* (2015) demonstrated a CRISPR-Cas9-based functional investigation of non-coding genomic elements. The authors we developed pooled CRISPR-Cas9 guide RNA libraries to perform *in situ* saturating mutagenesis of the human and mouse BCL11A enhancers which revealed critical features of the enhancers.
➢ Zetsche et al. (2015) reported characterization of Cpf1, a class 2 CRISPR nuclease from Francisella novicida U112 having features distinct from Cas9. Cpf1 is a single RNA-guided endonuclease lacking tracrRNA, utilizes a T-rich protospacer-adjacent motif, and cleaves DNA via a staggered DNA double-stranded break.
➢ Shmakov et al. (2015) reported three distinct Class 2 CRISPR-Cas systems. Two system CRISPR enzymes (C2c1 and C2c3) contain RuvC-like endonuclease domains distantly related to Cpf1. Unlike Cpf1, C2c1 depends on both crRNA and tracrRNA for DNA cleavage. The third enzyme (C2c2) contains two predicted HEPN RNase domains and is tracrRNA independent.
➢ Slaymaker et al (2016) reported the use of structure-guided protein engineering to improve the specificity of Streptococcus pyogenes Cas9 (SpCas9). The authors developed "enhanced specificity" SpCas9 (eSpCas9) variants which maintained robust on-target cleavage with reduced off-target effects.
➢ Cox et al., (2017) reported the use of catalytically inactive Cas13 (dCas13) to direct adenosine-to-inosine deaminase activity by ADAR2 (adenosine deaminase acting on RNA type 2) to transcripts in mammalian cells. The system, referred to as RNA Editing for Programmable A to I Replacement (REPAIR), has no strict sequence constraints and can be used to edit full-length transcripts. The authors further engineered the system to create a high-specificity variant and minimized the system to facilitate viral delivery.

The methods and tools provided herein are may be designed for use with or Cas13, a type II nuclease that does not make use of tracrRNA. Orthologs of Cas13 have been identified in different bacterial species as described herein. Further type II nucleases with similar properties can be identified using methods described in the art (Shmakov et al. 2015, 60:385-397; Abudayeh et al. 2016, Science, 5;353(6299)). In particular embodiments, such methods for identifying novel CRISPR effector proteins may comprise the steps of selecting sequences from the database encoding a seed which identifies the presence of a CRISPR Cas locus, identifying loci located within 10 kb of the seed comprising Open Reading Frames (ORFs) in the selected sequences, selecting therefrom loci comprising ORFs of which only a single ORF encodes a novel CRISPR effector having greater than 700 amino acids and no more than 90% homology to a known CRISPR effector. In particular embodiments, the seed is a protein that is common to the CRISPR-Cas system, such as Cas1. In further embodiments, the CRISPR array is used as a seed to identify new effector proteins.

Also, "Dimeric CRISPR RNA-guided FokI nucleases for highly specific genome editing", Shengdar Q. Tsai, Nicolas Wyvekens, Cyd Khayter, Jennifer A. Foden, Vishal Thapar, Deepak Reyon, Mathew J. Goodwin, Martin J. Aryee, J. Keith Joung Nature Biotechnology 32(6): 569-77 (2014), relates to dimeric RNA-guided FokI Nucleases that recognize extended sequences and can edit endogenous genes with high efficiencies in human cells.

With respect to general information on CRISPR/Cas Systems, components thereof, and delivery of such components, including methods, materials, delivery vehicles, vectors, particles, and making and using thereof, including as to amounts and formulations, as well as CRISPR-Cas-expressing eukaryotic cells, CRISPR-Cas expressing eukaryotes, such as a mouse, reference is made to: US Patents Nos. 8,999,641, 8,993,233, 8,697,359, 8,771,945, 8,795,965, 8,865,406, 8,871,445, 8,889,356, 8,889,418, 8,895,308, 8,906,616, 8,932,814, and 8,945,839; US Patent Publications US 2014-0310830 (US App. Ser. No. 14/105,031), US 2014-0287938 A1 (U.S. App. Ser. No. 14/213,991), US 2014-0273234 A1 (U.S. App. Ser. No. 14/293,674), US2014-0273232 A1 (U.S. App. Ser. No. 14/290,575), US 2014-0273231 (U.S. App. Ser. No. 14/259,420), US 2014-0256046 A1 (U.S. App. Ser. No. 14/226,274), US 2014-0248702 A1 (U.S. App. Ser. No. 14/258,458), US 2014-0242700 A1 (U.S. App. Ser. No. 14/222,930), US 2014-0242699 A1 (U.S. App. Ser. No. 14/183,512), US 2014-0242664 A1 (U.S. App. Ser. No. 14/104,990), US 2014-0234972 A1 (U.S. App. Ser. No. 14/183,471), US 2014-0227787 A1 (U.S. App. Ser. No. 14/256,912), US 2014-0189896 A1 (U.S. App. Ser. No. 14/105,035), US 2014-0186958 (U.S. App. Ser. No. 14/105,017), US 2014-0186919 A1 (U.S. App. Ser. No. 14/104,977), US 2014-0186843 A1 (U.S. App. Ser. No. 14/104,900), US 2014-0179770 A1 (U.S. App. Ser. No. 14/104,837) and US 2014-0179006 A1 (U.S. App. Ser. No. 14/183,486), US 2014-0170753 (US App Ser No 14/183,429); US 2015-0184139 (U.S. App. Ser. No. 14/324,960); 14/054,414 European Patent Applications EP 2 771 468 (EP13818570.7), EP 2 764 103 (EP13824232.6), and EP 2 784 162 (EP14170383.5); and PCT Patent Publications WO2014/093661 (PCT/US2013/074743), WO2014/093694 (PCT/US2013/074790), WO2014/093595 (PCT/US2013/074611), WO2014/093718 (PCT/US2013/074825), WO2014/093709 (PCT/US2013/074812), WO2014/093622 (PCT/US2013/074667), WO2014/093635 (PCT/US2013/074691), WO2014/093655 (PCT/US2013/074736), WO2014/093712 (PCT/US2013/074819), WO2014/093701 (PCT/US2013/074800), WO2014/018423 (PCT/US2013/051418), WO2014/204723 (PCT/US2014/041790), WO2014/204724 (PCT/US2014/041800), WO2014/204725 (PCT/US2014/041803), WO2014/204726 (PCT/US2014/041804), WO2014/204727 (PCT/US2014/041806), WO2014/204728 (PCT/US2014/041808), WO2014/204729 (PCT/US2014/041809), WO2015/089351 (PCT/US2014/069897), WO2015/089354 (PCT/US2014/069902), WO2015/089364 (PCT/US2014/069925), WO2015/089427 (PCT/US2014/070068), WO2015/089462 (PCT/US2014/070127), WO2015/089419 (PCT/US2014/070057), WO2015/089465 (PCT/US2014/070135), WO2015/089486 (PCT/US2014/070175), WO2015/058052 (PCT/US2014/061077), WO2015/070083 (PCT/US2014/064663), WO2015/089354 (PCT/US2014/069902), WO2015/089351 (PCT/US2014/069897), WO2015/089364 (PCT/US2014/069925), WO2015/089427 (PCT/US2014/070068), WO2015/089473 (PCT/US2014/070152), WO2015/089486 (PCT/US2014/070175), WO2016/049258 (PCT/US2015/051830), WO2016/094867 (PCT/US2015/065385), WO2016/094872 (PCT/US2015/065393), WO2016/094874 (PCT/US2015/065396), WO2016/106244 (PCT/US2015/067177).

Mention is also made of US application 62/180,709, 17-Jun-15, PROTECTED GUIDE RNAS (PGRNAS); US application 62/091,455, filed, 12-Dec-14, PROTECTED GUIDE RNAS (PGRNAS); US application 62/096,708, 24-Dec-14, PROTECTED GUIDE RNAS (PGRNAS); US applications 62/091,462, 12-Dec-14, 62/096,324, 23-Dec-14, 62/180,681, 17-Jun-2015, and 62/237,496, 5-Oct-2015, DEAD GUIDES FOR CRISPR TRANSCRIPTION FACTORS; US application 62/091,456, 12-Dec-14 and 62/180,692, 17-Jun-2015, ESCORTED AND FUNCTIONALIZED GUIDES FOR CRISPR-CAS SYSTEMS; US application 62/091,461, 12-Dec-14, DELIVERY, USE AND THERAPEUTIC APPLICATIONS OF THE CRISPR-CAS SYSTEMS AND COMPOSITIONS FOR GENOME EDITING AS TO HEMATOPOETIC STEM CELLS (HSCs); US application 62/094,903, 19-Dec-14, UNBIASED IDENTIFICATION OF DOUBLE-STRAND BREAKS AND GENOMIC REARRANGEMENT BY GENOME-WISE INSERT CAPTURE SEQUENCING; US application 62/096,761, 24-Dec-14, ENGINEERING OF SYSTEMS, METHODS AND OPTIMIZED ENZYME AND GUIDE SCAFFOLDS FOR SEQUENCE MANIPULATION; US application 62/098,059, 30-Dec-14, 62/181,641, 18-Jun-2015, and 62/181,667, 18-Jun-2015, RNA-TARGETING SYSTEM; US application 62/096,656, 24-Dec-14 and 62/181,151, 17-Jun-2015, CRISPR HAVING OR ASSOCIATED WITH DESTABILIZATION DOMAINS; US application 62/096,697, 24-Dec-14, CRISPR HAVING OR ASSOCIATED WITH AAV; US application 62/098,158, 30-Dec-14, ENGINEERED CRISPR COMPLEX INSERTIONAL TARGETING SYSTEMS; US application 62/151,052, 22-Apr-15, CELLULAR TARGETING FOR EXTRACELLULAR EXOSOMAL REPORTING; US application 62/054,490, 24-Sep-14, DELIVERY, USE AND THERAPEUTIC APPLICATIONS OF THE CRISPR-CAS SYSTEMS AND COMPOSITIONS FOR TARGETING DISORDERS AND DISEASES USING PARTICLE DELIVERY COMPONENTS; US application 61/939,154, 12-F EB-14, SYSTEMS, METHODS AND COMPOSITIONS FOR SEQUENCE MANIPULATION WITH OPTIMIZED FUNCTIONAL CRISPR-CAS SYSTEMS; US application 62/055,484, 25-Sep-14, SYSTEMS, METHODS AND COMPOSITIONS FOR SEQUENCE MANIPULATION WITH OPTIMIZED FUNCTIONAL CRISPR-CAS SYSTEMS; US application 62/087,537, 4-Dec-14, SYSTEMS, METHODS AND COMPOSITIONS FOR SEQUENCE MANIPULATION WITH OPTIMIZED FUNCTIONAL CRISPR-CAS SYSTEMS; US application 62/054,651, 24-Sep-14, DELIVERY, USE AND THERAPEUTIC APPLICATIONS OF THE CRISPR-CAS SYSTEMS AND COMPOSITIONS FOR MODELING COMPETITION OF MULTIPLE CANCER MUTATIONS IN VIVO; US application 62/067,886, 23-Oct-14, DELIVERY, USE AND THERAPEUTIC APPLICATIONS OF THE CRISPR-CAS SYSTEMS AND COMPOSITIONS FOR MODELING COMPETITION OF MULTIPLE CANCER MUTATIONS IN VIVO; US applications 62/054,675, 24-Sep-14 and 62/181,002, 17-Jun-2015, DELIVERY, USE AND THERAPEUTIC APPLICATIONS OF THE CRISPR-CAS SYSTEMS AND COMPOSITIONS IN NEURONAL CELLS/TISSUES; US application 62/054,528, 24-Sep-14, DELIVERY, USE AND THERAPEUTIC APPLICATIONS OF THE CRISPR-CAS SYSTEMS AND COMPOSITIONS IN IMMUNE DISEASES OR DISORDERS; US application 62/055,454, 25-Sep-14, DELIVERY, USE AND THERAPEUTIC APPLICATIONS OF THE CRISPR-CAS SYSTEMS AND COMPOSITIONS FOR TARGETING DISORDERS AND DISEASES USING CELL PENETRATION PEPTIDES (CPP); US application 62/055,460, 25-Sep-14, MULTIFUNCTIONAL-CRISPR COMPLEXES AND/OR OPTIMIZED ENZYME LINKED FUNCTIONAL-CRISPR COMPLEXES; US application 62/087,475, 4-Dec-14 and 62/181,690, 18-Jun-2015, FUNCTIONAL SCREENING WITH OPTIMIZED FUNCTIONAL CRISPR-CAS SYSTEMS; US application 62/055,487, 25-Sep-14, FUNCTIONAL SCREENING WITH OPTIMIZED FUNCTIONAL CRISPR-CAS SYSTEMS; US application 62/087,546, 4-Dec-14 and 62/181,687, 18-Jun-2015, MULTIFUNCTIONAL CRISPR COMPLEXES AND/OR OPTIMIZED ENZYME LINKED FUNCTIONAL-CRISPR COMPLEXES; and US application 62/098,285, 30-Dec-14, CRISPR MEDIATED IN VIVO MODELING AND GENETIC SCREENING OF TUMOR GROWTH AND METASTASIS.

Mention is made of US applications 62/181,659, 18-Jun-2015 and 62/207,318, 19-Aug-2015, ENGINEERING AND OPTIMIZATION OF SYSTEMS, METHODS, ENZYME AND GUIDE SCAFFOLDS OF CAS9 ORTHOLOGS AND VARIANTS FOR SEQUENCE MANIPULATION. Mention is made of US applications 62/181,663, 18-Jun-2015 and 62/245,264, 22-Oct-2015, NOVEL CRISPR ENZYMES AND SYSTEMS, US applications 62/181,675, 18-Jun-2015, 62/285,349, 22-Oct-2015, 62/296,522, 17-Feb-2016, and 62/320,231, 8-Apr-2016, NOVEL CRISPR ENZYMES AND SYSTEMS, US application 62/232,067, 24-Sep-2015, US Application 14/975,085, 18-Dec-2015, European application No. 16150428.7, US application 62/205,733, 16-Aug-2015, US application 62/201,542, 5-Aug-2015, US application 62/193,507, 16-Jul-2015, and US application 62/181,739, 18-Jun-2015, each entitled NOVEL CRISPR ENZYMES AND SYSTEMS and of US application 62/245,270, 22-Oct-2015, NOVEL CRISPR ENZYMES AND SYSTEMS. Mention is also made of US application 61/939,256, 12-Feb-2014, and WO 2015/089473 (PCT/US2014/070152), 12-Dec-2014, each entitled ENGINEERING OF SYSTEMS, METHODS AND OPTIMIZED GUIDE COMPOSITIONS WITH NEW ARCHITECTURES FOR SEQUENCE MANIPULATION. Mention is also made of PCT/US2015/045504, 15-Aug-2015, US application 62/180,699, 17-Jun-2015, and US application 62/038,358, 17-Aug-2014, each entitled GENOME EDITING USING CAS9 NICKASES.

Each of these patents, patent publications, and applications, and all documents cited therein or during their prosecution ("appln cited documents") and all documents cited or referenced in the appln cited documents, together with any instructions, descriptions, product specifications, and product sheets for any products mentioned therein may be employed in the practice of the invention.

In particular embodiments, pre-complexed guide RNA and CRISPR effector protein, (optionally, adenosine deaminase fused to a CRISPR protein or an adaptor) are delivered as a ribonucleoprotein (RNP). RNPs have the advantage that they lead to rapid editing effects even more so than the RNA method because this process avoids the need for transcription. An important advantage is that both RNP delivery is transient, reducing off-target effects and toxicity issues. Efficient genome editing in different cell types has been observed by Kim et al. (2014, Genome Res. 24(6):1012-9), Paix et al. (2015, Genetics 204(1):47-54), Chu et al. (2016, BMC Biotechnol. 16:4), and Wang et al. (2013, Cell. 9;153(4):910-8).

In particular embodiments, the ribonucleoprotein is delivered by way of a polypeptide-based shuttle agent as described in WO2016161516. WO2016161516 describes efficient transduction of polypeptide cargos using synthetic peptides comprising an endosome leakage domain (ELD) operably linked to a cell penetrating domain (CPD), to a histidine-rich domain and a CPD. Similarly these polypeptides can be used for the delivery of CRISPR-effector based RNPs in eukaryotic cells.

### Tale Systems

As disclosed herein editing can be made by way of the transcription activator-like effector nucleases (TALENs) system. Transcription activator-like effectors (TALEs) can be engineered to bind practically any desired DNA sequence. Exemplary methods of genome editing using the TALEN system can be found for example in Cermak T. Doyle EL. Christian M. Wang L. Zhang Y. Schmidt C, et al. Efficient design and assembly of custom TALEN and other TAL effector-based constructs for DNA targeting. Nucleic Acids Res. 2011;39:e82; Zhang F. Cong L. Lodato S. Kosuri S. Church GM. Arlotta P Efficient construction of sequence-specific TAL effectors for modulating mammalian transcription. Nat Biotechnol. 2011;29:149-153 and US Patent Nos. 8,450,471, 8,440,431 and 8,440,432.

In advantageous embodiments of the invention, the methods provided herein use isolated, non-naturally occurring, recombinant or engineered DNA binding proteins that comprise TALE monomers as a part of their organizational structure that enable the targeting of nucleic acid sequences with improved efficiency and expanded specificity.

Naturally occurring TALEs or "wild type TALEs" are nucleic acid binding proteins secreted by numerous species of proteobacteria. TALE polypeptides contain a nucleic acid binding domain composed of tandem repeats of highly conserved monomer polypeptides that are predominantly 33, 34 or 35 amino acids in length and that differ from each other mainly in amino acid positions 12 and 13. In advantageous embodiments the nucleic acid is DNA. As used herein, the term "polypeptide monomers", or "TALE monomers" will be used to refer to the highly conserved repetitive polypeptide sequences within the TALE nucleic acid binding domain and the term "repeat variable di-residues" or "RVD" will be used to refer to the highly variable amino acids at positions 12 and 13 of the polypeptide monomers. As provided throughout the disclosure, the amino acid residues of the RVD are depicted using the IUPAC single letter code for amino acids. A general representation of a TALE monomer which is comprised within the DNA binding domain is X1-11-(X12X13)-X14-33 or 34 or 35, where the subscript indicates the amino acid position and X represents any amino acid. X12X13 indicate the RVDs. In some polypeptide monomers, the variable amino acid at position 13 is missing or absent and in such polypeptide monomers, the RVD consists of a single amino acid. In such cases the RVD may be alternatively represented as X*, where X represents X12 and (*) indicates that X13 is absent. The DNA binding domain comprises several repeats of TALE monomers and this may be represented as (X1-11-(X12X13)-X14-33 or 34 or 35)z, where in an advantageous embodiment, z is at least 5 to 40. In a further advantageous embodiment, z is at least 10 to 26.

The TALE monomers have a nucleotide binding affinity that is determined by the identity of the amino acids in its RVD. For example, polypeptide monomers with an RVD of NI preferentially bind to adenine (A), polypeptide monomers with an RVD of NG preferentially bind to thymine (T), polypeptide monomers with an RVD of HD preferentially bind to cytosine (C) and polypeptide monomers with an RVD of NN preferentially bind to both adenine (A) and guanine (G). In yet another embodiment of the invention, polypeptide monomers with an RVD of IG preferentially bind to T. Thus, the number and order of the polypeptide monomer repeats in the nucleic acid binding domain of a TALE determines its nucleic acid target specificity. In still further embodiments of the invention, polypeptide monomers with an RVD of NS recognize all four base pairs and may bind to A, T, G or C. The structure and function of TALEs is further described in, for example, Moscou et al., Science 326:1501 (2009); Boch et al., Science 326:1509-1512 (2009); and Zhang et al., Nature Biotechnology 29:149-153 (2011).

The TALE polypeptides used in methods of the invention are isolated, non-naturally occurring, recombinant or engineered nucleic acid-binding proteins that have nucleic acid or DNA binding regions containing polypeptide monomer repeats that are designed to target specific nucleic acid sequences.

As described herein, polypeptide monomers having an RVD of HN or NH preferentially bind to guanine and thereby allow the generation of TALE polypeptides with high binding specificity for guanine containing target nucleic acid sequences. In a preferred embodiment of the invention, polypeptide monomers having RVDs RN, NN, NK, SN, NH, KN, HN, NQ, HH, RG, KH, RH and SS preferentially bind to guanine. In a much more advantageous embodiment of the invention, polypeptide monomers having RVDs RN, NK, NQ, HH, KH, RH, SS and SN preferentially bind to guanine and thereby allow the generation of TALE polypeptides with high binding specificity for guanine containing target nucleic acid sequences. In an even more advantageous embodiment of the invention, polypeptide monomers having RVDs HH, KH, NH, NK, NQ, RH, RN and SS preferentially bind to guanine and thereby allow the generation of TALE polypeptides with high binding specificity for guanine containing target nucleic acid sequences. In a further advantageous embodiment, the RVDs that have high binding specificity for guanine are RN, NH RH and KH. Furthermore, polypeptide monomers having an RVD of NV preferentially bind to adenine and guanine. In more preferred embodiments of the invention, polypeptide monomers having RVDs of H*, HA, KA, N*, NA, NC, NS, RA, and S* bind to adenine, guanine, cytosine and thymine with comparable affinity.

The predetermined N-terminal to C-terminal order of the one or more polypeptide monomers of the nucleic acid or DNA binding domain determines the corresponding predetermined target nucleic acid sequence to which the TALE polypeptides will bind. As used herein the polypeptide monomers and at least one or more half polypeptide monomers are "specifically ordered to target" the genomic locus or gene of interest. In plant genomes, the natural TALE-binding sites always begin with a thymine (T), which may be specified by a cryptic signal within the non-repetitive N-terminus of the TALE polypeptide; in some cases this region may be referred to as repeat 0. In animal genomes, TALE binding sites do not necessarily have to begin with a thymine (T) and TALE polypeptides may target DNA sequences that begin with T, A, G or C. The tandem repeat of TALE monomers always ends with a half-length repeat or a stretch of sequence that may share identity with only the first 20 amino acids of a repetitive full length TALE monomer and this half repeat may be referred to as a half-monomer (FIG. 8), which is included in the term "TALE monomer". Therefore, it follows that the length of the nucleic acid or DNA being targeted is equal to the number of full polypeptide monomers plus two.

As described in Zhang et al., Nature Biotechnology 29:149-153 (2011), TALE polypeptide binding efficiency may be increased by including amino acid sequences from the "capping regions" that are directly N-terminal or C-terminal of the DNA binding region of naturally occurring TALEs into the engineered TALEs at positions N-terminal or C-terminal of the engineered TALE DNA binding region. Thus, in certain embodiments, the TALE polypeptides described herein further comprise an N-terminal capping region and/or a C-terminal capping region.

An exemplary amino acid sequence of a N-terminal capping region is:

M D P I R S R T P S P A R E L L S G P Q P D G V Q P T A D R G V S P
P A G G P L D G L P A R R T M S R T R L P S P P A P S P A F S A D S
F S D L L R Q F D P S L F N T S L F D S L P P F G A H H T E A A T G
E W D E V Q S G L R A A D A P P P T M R V A V T A A R P P R A K P A
P R R R A A Q P S D A S P A A Q V D L R T L G Y S Q Q Q Q E K I K P
K V R S T V A Q H H E A L V G H G F T H A H I V A L S Q H P A A L G
T V A V K Y Q D M I A A L P E A T H E A I V G V G K Q W S G A R A L
E A L L T V A G E L R G P P L Q L D T G Q L L K I A K R G G V T A V
E A V H A W R N A L T G A P L N (SEQ ID NO: 2)

An exemplary amino acid sequence of a C-terminal capping region is:

R P A L E S I V A Q L S R P D P A L A A L T N D H L V A L A C L G

GRPALDAVKKGLPHAPALIKRTNRRIPERTSHR
VADHAQVVRVLGFFQCHSHPAQAFDDAMTQFGM
SRHGLLQLFRRVGVTELEARSGTLPPASQRWDR
ILQASGMKRAKPSPTSTQTPDQASLHAFADSLE
RDLDAPSPMHEGDQTRAS (SEQ ID NO: 3)

As used herein the predetermined "N-terminus" to "C terminus" orientation of the N-terminal capping region, the DNA binding domain comprising the repeat TALE monomers and the C-terminal capping region provide structural basis for the organization of different domains in the d-TALEs or polypeptides of the invention.

The entire N-terminal and/or C-terminal capping regions are not necessary to enhance the binding activity of the DNA binding region. Therefore, in certain embodiments, fragments of the N-terminal and/or C-terminal capping regions are included in the TALE polypeptides described herein.

In certain embodiments, the TALE polypeptides described herein contain a N-terminal capping region fragment that included at least 10, 20, 30, 40, 50, 54, 60, 70, 80, 87, 90, 94, 100, 102, 110, 117, 120, 130, 140, 147, 150, 160, 170, 180, 190, 200, 210, 220, 230, 240, 250, 260 or 270 amino acids of an N-terminal capping region. In certain embodiments, the N-terminal capping region fragment amino acids are of the C-terminus (the DNA-binding region proximal end) of an N-terminal capping region. As described in Zhang et al., Nature Biotechnology 29:149-153 (2011), N-terminal capping region fragments that include the C-terminal 240 amino acids enhance binding activity equal to the full length capping region, while fragments that include the C-terminal 147 amino acids retain greater than 80% of the efficacy of the full length capping region, and fragments that include the C-terminal 117 amino acids retain greater than 50% of the activity of the full-length capping region.

In some embodiments, the TALE polypeptides described herein contain a C-terminal capping region fragment that included at least 6, 10, 20, 30, 37, 40, 50, 60, 68, 70, 80, 90, 100, 110, 120, 127, 130, 140, 150, 155, 160, 170, 180 amino acids of a C-terminal capping region. In certain embodiments, the C-terminal capping region fragment amino acids are of the N-terminus (the DNA-binding region proximal end) of a C-terminal capping region. As described in Zhang et al., Nature Biotechnology 29:149-153 (2011), C-terminal capping region fragments that include the C-terminal 68 amino acids enhance binding activity equal to the full length capping region, while fragments that include the C-terminal 20 amino acids retain greater than 50% of the efficacy of the full length capping region.

In certain embodiments, the capping regions of the TALE polypeptides described herein do not need to have identical sequences to the capping region sequences provided herein. Thus, in some embodiments, the capping region of the TALE polypeptides described herein have sequences that are at least 50%, 60%, 70%, 80%, 85%, 90%, 91%, 92%, 93%, 94%, 95%, 96%, 97%, 98% or 99% identical or share identity to the capping region amino acid sequences provided herein. Sequence identity is related to sequence homology. Homology comparisons may be conducted by eye, or more usually, with the aid of readily available sequence comparison programs. These commercially available computer programs may calculate percent (%) homology between two or more sequences and may also calculate the sequence identity shared by two or more amino acid or nucleic acid sequences. In some preferred embodiments, the capping region of the TALE polypeptides described herein have sequences that are at least 95% identical or share identity to the capping region amino acid sequences provided herein.

Sequence homologies may be generated by any of a number of computer programs known in the art, which include but are not limited to BLAST or FASTA. Suitable computer program for carrying out alignments like the GCG Wisconsin Bestfit package may also be used. Once the software has produced an optimal alignment, it is possible to calculate % homology, preferably % sequence identity. The software typically does this as part of the sequence comparison and generates a numerical result.

In advantageous embodiments described herein, the TALE polypeptides of the invention include a nucleic acid binding domain linked to the one or more effector domains. The terms "effector domain" or "regulatory and functional domain" refer to a polypeptide sequence that has an activity other than binding to the nucleic acid sequence recognized by the nucleic acid binding domain. By combining a nucleic acid binding domain with one or more effector domains, the polypeptides of the invention may be used to target the one or more functions or activities mediated by the effector domain to a particular target DNA sequence to which the nucleic acid binding domain specifically binds.

In some embodiments of the TALE polypeptides described herein, the activity mediated by the effector domain is a biological activity. For example, in some embodiments the effector domain is a transcriptional inhibitor (i.e., a repressor domain), such as an mSin interaction domain (SID). SID4X domain or a Krüppel-associated box (KRAB) or fragments of the KRAB domain. In some embodiments the effector domain is an enhancer of transcription (i.e. an activation domain), such as the VP16, VP64 or p65 activation domain. In some embodiments, the nucleic acid binding is linked, for example, with an effector domain that includes but is not limited to a transposase, integrase, recombinase, resolvase, invertase, protease, DNA methyltransferase, DNA demethylase, histone acetylase, histone deacetylase, nuclease, transcriptional repressor, transcriptional activator, transcription factor recruiting, protein nuclear-localization signal or cellular uptake signal.

In some embodiments, the effector domain is a protein domain which exhibits activities which include but are not limited to transposase activity, integrase activity, recombinase activity, resolvase activity, invertase activity, protease activity, DNA methyltransferase activity, DNA demethylase activity, histone acetylase activity, histone deacetylase activity, nuclease activity, nuclear-localization signaling activity, transcriptional repressor activity, transcriptional activator activity, transcription factor recruiting activity, or cellular uptake signaling activity. Other preferred embodiments of the invention may include any combination the activities described herein.

### ZN-Finger Nucleases

Other preferred tools for genome editing for use in the context of this invention include zinc finger systems. One type of programmable DNA-binding domain is provided by artificial zinc-finger (ZF) technology, which involves arrays of ZF modules to target new DNA-binding sites in the genome. Each finger module in a ZF array targets three DNA bases. A customized array of individual zinc finger domains is assembled into a ZF protein (ZFP).

ZFPs can comprise a functional domain. The first synthetic zinc finger nucleases (ZFNs) were developed by fusing a ZF protein to the catalytic domain of the Type IIS restriction enzyme FokI. (Kim, Y. G. et al., 1994, Chimeric restriction endonuclease, Proc. Natl. Acad. Sci. U.S.A. 91, 883-887; Kim, Y. G. et al., 1996, Hybrid restriction enzymes: zinc finger fusions to Fok I cleavage domain. Proc. Natl. Acad. Sci. U.S.A. 93, 1156-1160). Increased cleavage specificity can be attained with decreased off target activity by use of paired ZFN heterodimers, each targeting different nucleotide sequences separated by a short spacer. (Doyon, Y. et al., 2011, Enhancing zinc-finger-nuclease activity with improved obligate heterodimeric architectures. Nat. Methods 8, 74-79). ZFPs can also be designed as transcription activators and repressors and have been used to target many genes in a wide variety of organisms. Exemplary methods of genome editing using ZFNs can be found for example in U.S. Patent Nos. 6,534,261, 6,607,882, 6,746,838, 6,794,136, 6,824,978, 6,866,997, 6,933,113, 6,979,539, 7,013,219, 7,030,215, 7,220,719, 7,241,573, 7,241,574, 7,585,849, 7,595,376, 6,903,185, and 6,479,626.

### Meganucleases

As disclosed herein editing can be made by way of meganucleases, which are endodeoxyribonucleases characterized by a large recognition site (double-stranded DNA sequences of 12 to 40 base pairs). Exemplary method for using meganucleases can be found in US Patent Nos: 8,163,514; 8,133,697; 8,021,867; 8,119,361; 8,119,381; 8,124,369; and 8,129,134.

### RNAi

In certain embodiments, the genetic modifying agent is RNAi (e.g., shRNA). As used herein, "gene silencing" or "gene silenced" in reference to an activity of an RNAi molecule, for example a siRNA or miRNA refers to a decrease in the mRNA level in a cell for a target gene by at least about 5%, about 10%, about 20%, about 30%, about 40%, about 50%, about 60%, about 70%, about 80%, about 90%, about 95%, about 99%, about 100% of the mRNA level found in the cell without the presence of the miRNA or RNA interference molecule. In one preferred embodiment, the mRNA levels are decreased by at least about 70%, about 80%, about 90%, about 95%, about 99%, about 100%.

As used herein, the term "RNAi" refers to any type of interfering RNA, including but not limited to, siRNAi, shRNAi, endogenous microRNA and artificial microRNA. For instance, it includes sequences previously identified as siRNA, regardless of the mechanism of down-stream processing of the RNA (i.e. although siRNAs are believed to have a specific method of in vivo processing resulting in the cleavage of mRNA, such sequences can be incorporated into the vectors in the context of the flanking sequences described herein). The term "RNAi" can include both gene silencing RNAi molecules, and also RNAi effector molecules which activate the expression of a gene.

As used herein, a "siRNA" refers to a nucleic acid that forms a double stranded RNA, which double stranded RNA has the ability to reduce or inhibit expression of a gene or target gene when the siRNA is present or expressed in the same cell as the target gene. The double stranded RNA siRNA can be formed by the complementary strands. In one embodiment, a siRNA refers to a nucleic acid that can form a double stranded siRNA. The sequence of the siRNA can correspond to the full-length target gene, or a subsequence thereof. Typically, the siRNA is at least about 15-50 nucleotides in length (e.g., each complementary sequence of the double stranded siRNA is about 15-50 nucleotides in length, and the double stranded siRNA is about 15-50 base pairs in length, preferably about 19-30 base nucleotides, preferably about 20-25 nucleotides in length, e.g., 20, 21 , 22, 23, 24, 25, 26, 27, 28, 29, or 30 nucleotides in length).

As used herein "shRNA" or "small hairpin RNA" (also called stem loop) is a type of siRNA. In one embodiment, these shRNAs are composed of a short, e.g. about 19 to about 25 nucleotide, antisense strand, followed by a nucleotide loop of about 5 to about 9 nucleotides, and the analogous sense strand. Alternatively, the sense strand can precede the nucleotide loop structure and the antisense strand can follow.

The terms "microRNA" or "miRNA" are used interchangeably herein are endogenous RNAs, some of which are known to regulate the expression of protein-coding genes at the posttranscriptional level. Endogenous microRNAs are small RNAs naturally present in the genome that are capable of modulating the productive utilization of mRNA. The term artificial microRNA includes any type of RNA sequence, other than endogenous microRNA, which is capable of modulating the productive utilization of mRNA. MicroRNA sequences have been described in publications such as Lim, et al., Genes & Development, 17, p. 991 - 1008 (2003), Lim et al Science 299, 1540 (2003), Lee and Ambros Science, 294, 862 (2001), Lau et al., Science 294, 858-861 (2001), Lagos-Quintana et al, Current Biology, 12, 735-739 (2002), Lagos Quintana et al, Science 294, 853- 857 (2001), and Lagos-Quintana et al, RNA, 9, 175-179 (2003). Multiple microRNAs can also be incorporated into a precursor molecule. Furthermore, miRNA-like stem-loops can be expressed in cells as a vehicle to deliver artificial miRNAs and short interfering RNAs (siRNAs) for the purpose of modulating the expression of endogenous genes through the miRNA and or RNAi pathways.

As used herein, "double stranded RNA" or "dsRNA" refers to RNA molecules that are comprised of two strands. Double-stranded molecules include those comprised of a single RNA molecule that doubles back on itself to form a two-stranded structure. For example, the stem loop structure of the progenitor molecules from which the single-stranded miRNA is derived, called the pre-miRNA (Bartel et al. 2004. Cell 1 16:281 -297), comprises a dsRNA molecule.

### Small Molecules

In certain embodiments, the one or more agents comprises a small molecule inhibitor, small molecule degrader (e.g., PROTAC), genetic modifying agent, antibody, antibody fragment, antibody-like protein scaffold, aptamer, protein, or any combination thereof.

In certain embodiments, the one or more agents is a small molecule. The term "small molecule" refers to compounds, preferably organic compounds, with a size comparable to those organic molecules generally used in pharmaceuticals. The term excludes biological macromolecules (e.g., proteins, peptides, nucleic acids, etc.). Preferred small organic molecules range in size up to about 5000 Da, e.g., up to about 4000, preferably up to 3000 Da, more preferably up to 2000 Da, even more preferably up to about 1000 Da, e.g., up to about 900, 800, 700, 600 or up to about 500 Da. In certain embodiments, the small molecule may act as an antagonist or agonist (e.g., blocking an enzyme active site or activating a receptor by binding to a ligand binding site).

One type of small molecule applicable to the present invention is a degrader molecule. Proteolysis Targeting Chimera (PROTAC) technology is a rapidly emerging alternative therapeutic strategy with the potential to address many of the challenges currently faced in modem drug development programs. PROTAC technology employs small molecules that recruit target proteins for ubiquitination and removal by the proteasome (see, e.g., Bondeson and Crews, Targeted Protein Degradation by Small Molecules, Annu Rev Pharmacol Toxicol. 2017 Jan 6; 57: 107-123; and Lai et al., Modular PROTAC Design for the Degradation of Oncogenic BCR-ABL Angew Chem Int Ed Engl. 2016 Jan 11; 55(2): 807-810). Specific small molecule degraders targeting bromodomain and extra-terminal (BET) family proteins, consisting of BRD2, BRD3, BRD4, and testis-specific BRDT members (e.g., BETd-260/ZBC260) are specifically applicable for targeting the acetylated superenhancers that co-localize and form links within and across chromosomes (see, e.g., Zhou et al., Discovery of a Small-Molecule Degrader of Bromodomain and Extra- Terminal (BET) Proteins with Picomolar Cellular Potencies and Capable of Achieving Tumor Regression. J. Med. Chem. 2018, 61, 462-481).

In certain embodiments, drugs targeting epigenetic proteins are applicable to chromatin loop formation or regulation (e.g., for screening molecules that modulate chromatin looping and for modulating chromatin loops). Small molecules targeting epigenetic proteins are currently being developed and/or used in the clinic to treat disease (see, e.g., Qi et al., HEDD: the human epigenetic drug database. Database, 2016, 1-10; and Ackloo et al., Chemical probes targeting epigenetic proteins: Applications beyond oncology. Epigenetics 2017, VOL. 12, NO. 5, 378-400). In certain embodiments, the one or more agents comprise a histone acetylation inhibitor, histone deacetylase (HDAC) inhibitor, histone lysine methylation inhibitor, histone lysine demethylation inhibitor, DNA methyltransferase (DNMT) inhibitor, inhibitor of acetylated histone binding proteins, inhibitor of methylated histone binding proteins, sirtuin inhibitor, protein arginine methyltransferase inhibitor or kinase inhibitor. In certain embodiments, any small molecule exhibiting the functional activity described above may be used in the present invention. In certain embodiments, the DNA methyltransferase (DNMT) inhibitor is selected from the group consisting of azacitidine (5-azacytidine), decitabine (5-aza-2'-deoxycytidine), EGCG (epigallocatechin-3-gallate), zebularine, hydralazine, and procainamide. In certain embodiments, the histone acetylation inhibitor is C646. In certain embodiments, the histone deacetylase (HDAC) inhibitor is selected from the group consisting of vorinostat, givinostat, panobinostat, belinostat, entinostat, CG-1521, romidepsin, ITF-A, ITF-B, valproic acid, OSU-HDAC-44, HC-toxin, magnesium valproate, plitidepsin, tasquinimod, sodium butyrate, mocetinostat, carbamazepine, SB939, CHR-2845, CHR-3996, JNJ-26481585, sodium phenylbutyrate, pivanex, abexinostat, resminostat, dacinostat, droxinostat, and trichostatin A (TSA). In certain embodiments, the histone lysine demethylation inhibitor is selected from the group consisting of pargyline, clorgyline, bizine, GSK2879552, GSK-J4, KDM5-C70, JIB-04, and tranylcypromine. In certain embodiments, the histone lysine methylation inhibitor is selected from the group consisting of EPZ-6438, GSK126, CPI-360, CPI-1205, CPI-0209, DZNep, GSK343, EI1, BIX-01294, UNC0638, EPZ004777, GSK343, UNC1999 and UNC0224. In certain embodiments, the inhibitor of acetylated histone binding proteins is selected from the group consisting of AZD5153 (see e.g., Rhyasen et al., AZD5153: A Novel Bivalent BET Bromodomain Inhibitor Highly Active against Hematologic Malignancies, Mol Cancer Ther. 2016 Nov;15(11):2563-2574. Epub 2016 Aug 29), PFI-1, CPI-203, CPI-0610, RVX-208, OTX015, I-BET151, I-BET762, I-BET-726, dBET1, ARV-771, ARV-825, BETd-260/ZBC260 and MZ1. In certain embodiments, the inhibitor of methylated histone binding proteins is selected from the group consisting of UNC669 and UNC1215. In certain embodiments, the sirtuin inhibitor comprises nicotinamide.

### Tools and Methods for Delivery of Certain Chromatin 3D Structure Modulating Agents

Through this disclosure, knowledge in the art, and ithe above disclosure regarding delivery of CRISPR-Cas systems, Zinc Finger sytems, TALEs, and other modulating agents, or components thereof, or nucleic acid molecules thereof (including, for instance HDR template), or nucleic acid molecules encoding or providing components thereof, may be delivered by a delivery system herein described both generally and in detail.

Vector delivery, e.g., plasmid, viral delivery: the chromatin 3D structure modulating agents, can be delivered using any suitable vector, e.g., plasmid or viral vectors, such as adeno associated virus (AAV), lentivirus, adenovirus or other viral vector types, or combinations thereof. In some embodiments, the vector, e.g., plasmid or viral vector is delivered to the tissue of interest by, for example, an intramuscular injection, while other times the delivery is via intravenous, transdermal, intranasal, oral, mucosal, or other delivery methods. Such delivery may be either via a single dose, or multiple doses. One skilled in the art understands that the actual dosage to be delivered herein may vary greatly depending upon a variety of factors, such as the vector choice, the target cell, organism, or tissue, the general condition of the subject to be treated, the degree of transformation/modification sought, the administration route, the administration mode, the type of transformation/modification sought, etc.

Such a dosage may further contain, for example, a carrier (water, saline, ethanol, glycerol, lactose, sucrose, calcium phosphate, gelatin, dextran, agar, pectin, peanut oil, sesame oil, etc.), a diluent, a pharmaceutically-acceptable carrier (e.g., phosphate-buffered saline), a pharmaceutically-acceptable excipient, and/or other compounds known in the art. The dosage may further contain one or more pharmaceutically acceptable salts such as, for example, a mineral acid salt such as a hydrochloride, a hydrobromide, a phosphate, a sulfate, etc.; and the salts of organic acids such as acetates, propionates, malonates, benzoates, etc. Additionally, auxiliary substances, such as wetting or emulsifying agents, pH buffering substances, gels or gelling materials, flavorings, colorants, microspheres, polymers, suspension agents, etc. may also be present herein. In addition, one or more other conventional pharmaceutical ingredients, such as preservatives, humectants, suspending agents, surfactants, antioxidants, anticaking agents, fillers, chelating agents, coating agents, chemical stabilizers, etc. may also be present, especially if the dosage form is a reconstitutable form. Suitable exemplary ingredients include microcrystalline cellulose, carboxymethylcellulose sodium, polysorbate 80, phenylethyl alcohol, chlorobutanol, potassium sorbate, sorbic acid, sulfur dioxide, propyl gallate, the parabens, ethyl vanillin, glycerin, phenol, parachlorophenol, gelatin, albumin and a combination thereof. A thorough discussion of pharmaceutically acceptable excipients is available in REMINGTON'S PHARMACEUTICAL SCIENCES (Mack Pub. Co., N.J. 1991).

In an embodiment, herein the delivery is via an adenovirus, which may be at a single booster dose containing at least 1 x 10⁵ particles (also referred to as particle units, pu) of adenoviral vector. In an embodiment herein, the dose preferably is at least about 1 x 10⁶ particles (for example, about 1 x 10⁶ - 1 x 10¹² particles), more preferably at least about 1 x 10⁷ particles, more preferably at least about 1 x 10⁸ particles (e.g., about 1 x 10⁸ - 1 x 10¹¹ particles or about 1 x 10⁸ - 1 x 10¹² particles), and most preferably at least about 1 x 10⁰ particles (e.g., about 1 x 10⁹-1 x 10¹⁰ particles or about 1 x 10⁹-1 x 10¹² particles), or even at least about 1 x 10¹⁰ particles (e.g., about 1 x 10¹⁰-1 x 10¹² particles) of the adenoviral vector. Alternatively, the dose comprises no more than about 1 x 10¹⁴ particles, preferably no more than about 1 x 1013 particles, even more preferably no more than about 1 x 10¹² particles, even more preferably no more than about 1 x 10¹¹ particles, and most preferably no more than about 1 x 10¹⁰ particles (e.g., no more than about 1 x 109 articles). Thus, the dose may contain a single dose of adenoviral vector with, for example, about 1 x 106 particle units (pu), about 2 x 106 pu, about 4 x 106 pu, about 1 x 107 pu, about 2 x 107 pu, about 4 x 107 pu, about 1 x 108 pu, about 2 x 108 pu, about 4 x 108 pu, about 1 x 109 pu, about 2 x 109 pu, about 4 x 109 pu, about 1 x 1010 pu, about 2 x 1010 pu, about 4 x 1010 pu, about 1 x 1011 pu, about 2 x 1011 pu, about 4 x 1011 pu, about 1 x 1012 pu, about 2 x 1012 pu, or about 4 x 1012 pu of adenoviral vector. See, for example, the adenoviral vectors in U.S. Patent No. 8,454,972 B2 to Nabel, et. al., granted on June 4, 2013; and the dosages at col 29, lines 36-58 thereof. In an embodiment herein, the adenovirus is delivered via multiple doses.

In an embodiment herein, the delivery is via an AAV. A therapeutically effective dosage for in vivo delivery of the AAV to a human is believed to be in the range of from about 20 to about 50 ml of saline solution containing from about 1 x 1010 to about 1 x 1010 functional AAV/ml solution. The dosage may be adjusted to balance the therapeutic benefit against any side effects. In an embodiment herein, the AAV dose is generally in the range of concentrations of from about 1 x 105 to 1 x 1050 genomes AAV, from about 1 x 108 to 1 x 1020 genomes AAV, from about 1 x 1010 to about 1 x 1016 genomes, or about 1 x 1011 to about 1 x 1016 genomes AAV. A human dosage may be about 1 x 1013 genomes AAV. Such concentrations may be delivered in from about 0.001 ml to about 100 ml, about 0.05 to about 50 ml, or about 10 to about 25 ml of a carrier solution. Other effective dosages can be readily established by one of ordinary skill in the art through routine trials establishing dose response curves. See, for example, U.S. Patent No. 8,404,658 B2 to Hajjar, et al., granted on March 26, 2013, at col. 27, lines 45-60.

In an embodiment herein the delivery is via a plasmid. In such plasmid compositions, the dosage should be a sufficient amount of plasmid to elicit a response. For instance, suitable quantities of plasmid DNA in plasmid compositions can be from about 0.1 to about 2 mg, or from about 1 µg to about 10 µg per 70 kg individual. Plasmids of the invention will generally comprise (i) a promoter; (ii) a sequence encoding a CRISPR enzyme, operably linked to said promoter; (iii) a selectable marker; (iv) an origin of replication; and (v) a transcription terminator downstream of and operably linked to (ii). The plasmid can also encode the RNA components of a CRISPR complex, but one or more of these may instead be encoded on a different vector.

The doses herein are based on an average 70 kg individual. The frequency of administration is within the ambit of the medical or veterinary practitioner (e.g., physician, veterinarian), or scientist skilled in the art. It is also noted that mice used in experiments are typically about 20g and from mice experiments one can scale up to a 70 kg individual.

In some embodiments the RNA molecules of the invention are delivered in liposome or lipofectin formulations and the like and can be prepared by methods well known to those skilled in the art. Such methods are described, for example, in U.S. Pat. Nos. 5,593,972, 5,589,466, and 5,580,859. Delivery systems aimed specifically at the enhanced and improved delivery of siRNA into mammalian cells have been developed, (see, for example, Shen et al FEBS Let. 2003, 539:111-114; Xia et al., Nat. Biotech. 2002, 20:1006-1010; Reich et al., Mol. Vision. 2003, 9: 210-216; Sorensen et al., J. Mol. Biol. 2003, 327: 761-766; Lewis et al., Nat. Gen. 2002, 32: 107-108 and Simeoni et al., NAR 2003, 31, 11: 2717-2724) and may be applied to the present invention. siRNA has recently been successfully used for inhibition of gene expression in primates (see for example. Tolentino et al., Retina 24(4):660 which may also be applied to the present invention.

Indeed, RNA delivery is a useful method of in vivo delivery. It is possible to deliver a CRISPR/Cas protein and one or more RNA polynucleotides (for instance, guide RNA and/or HR repair template) into cells using liposomes or particles. Thus delivery of the CRISPR enzyme, such as a Cas9 and/or delivery of the RNAs of the invention may be in RNA form and via microvesicles, liposomes or particles as described above. For example, Cas protein encoding mRNA and gRNA can be packaged into liposomal particles for delivery in vivo. Liposomal transfection reagents such as lipofectamine from Life Technologies and other reagents on the market can effectively deliver RNA molecules into the liver.

Means of delivery of RNA also preferred include delivery of RNA via nanoparticles (Cho, S., Goldberg, M., Son, S., Xu, Q., Yang, F., Mei, Y., Bogatyrev, S., Langer, R. and Anderson, D., Lipid-like nanoparticles for small interfering RNA delivery to endothelial cells, Advanced Functional Materials, 19: 3112-3118, 2010) or exosomes (Schroeder, A., Levins, C., Cortez, C., Langer, R., and Anderson, D., Lipid-based nanotherapeutics for siRNA delivery, Journal of Internal Medicine, 267: 9-21, 2010, PMID: 20059641). Indeed, exosomes have been shown to be particularly useful in delivery siRNA, a system with some parallels to the CRISPR system. For instance, El-Andaloussi S, et al. ("Exosome-mediated delivery of siRNA in vitro and in vivo." Nat Protoc. 2012 Dec;7(12):2112-26. doi: 10.1038/nprot.2012.131. Epub 2012 Nov 15.) describe how exosomes are promising tools for drug delivery across different biological barriers and can be harnessed for delivery of siRNA in vitro and in vivo. Their approach is to generate targeted exosomes through transfection of an expression vector, comprising an exosomal protein fused with a peptide ligand. The exosomes are then purify and characterized from transfected cell supernatant, then RNA is loaded into the exosomes. Delivery or administration according to the invention can be performed with exosomes, in particular but not limited to the brain. Vitamin E (α-tocopherol) may be conjugated with CRISPR Cas and delivered to the brain along with high density lipoprotein (HDL), for example in a similar manner as was done by Uno et al. (HUMAN GENE THERAPY 22:711-719 (June 2011)) for delivering short-interfering RNA (siRNA) to the brain. Mice were infused via Osmotic minipumps (model 1007D; Alzet, Cupertino, CA) filled with phosphate-buffered saline (PBS) or free TocsiBACE or Toc-siBACE/HDL and connected with Brain Infusion Kit 3 (Alzet). A brain-infusion cannula was placed about 0.5mm posterior to the bregma at midline for infusion into the dorsal third ventricle. Uno et al. found that as little as 3 nmol of Toc-siRNA with HDL could induce a target reduction in comparable degree by the same ICV infusion method. A similar dosage of CRISPR Cas conjugated to α-tocopherol and co-administered with HDL targeted to the brain may be contemplated for humans in the present invention, for example, about 3 nmol to about 3 µmol of CRISPR Cas targeted to the brain may be contemplated. Zou et al. ((HUMAN GENE THERAPY 22:465-475 (April 2011)) describes a method of lentiviral-mediated delivery of short-hairpin RNAs targeting PKCγ for in vivo gene silencing in the spinal cord of rats. Zou et al. administered about 10 µl of a recombinant lentivirus having a titer of 1 x 109 transducing units (TU)/ml by an intrathecal catheter. A similar dosage of CRISPR Cas expressed in a lentiviral vector targeted to the brain may be contemplated for humans in the present invention, for example, about 10-50 ml of CRISPR Cas targeted to the brain in a lentivirus having a titer of 1 x 109 transducing units (TU)/ml may be contemplated.

In terms of local delivery to the brain, this can be achieved in various ways. For instance, material can be delivered intrastriatally e.g. by injection. Injection can be performed stereotactically via a craniotomy.

### Adeno associated virus (AAV)

Cas9 and one or more guide RNA can be delivered using adeno associated virus (AAV), lentivirus, adenovirus or other plasmid or viral vector types, in particular, using formulations and doses from, for example, US Patents Nos. 8,454,972 (formulations, doses for adenovirus), 8,404,658 (formulations, doses for AAV) and 5,846,946 (formulations, doses for DNA plasmids) and from clinical trials and publications regarding the clinical trials involving lentivirus, AAV and adenovirus. For examples, for AAV, the route of administration, formulation and dose can be as in US Patent No. 8,454,972 and as in clinical trials involving AAV. For Adenovirus, the route of administration, formulation and dose can be as in US Patent No. 8,404,658 and as in clinical trials involving adenovirus. For plasmid delivery, the route of administration, formulation and dose can be as in US Patent No 5,846,946 and as in clinical studies involving plasmids. Doses may be based on or extrapolated to an average 70 kg individual (e.g. a male adult human), and can be adjusted for patients, subjects, mammals of different weight and species. Frequency of administration is within the ambit of the medical or veterinary practitioner (e.g., physician, veterinarian), depending on usual factors including the age, sex, general health, other conditions of the patient or subject and the particular condition or symptoms being addressed. The viral vectors can be injected into the tissue of interest. For cell-type specific genome modification, the expression of Cas9 can be driven by a cell-type specific promoter. For example, liver-specific expression might use the Albumin promoter and neuron-specific expression (e.g. for targeting CNS disorders) might use the Synapsin I promoter.

In terms of *in vivo* delivery, AAV is advantageous over other viral vectors for a couple of reasons:
- Low toxicity (this may be due to the purification method not requiring ultra centrifugation of cell particles that can activate the immune response)
- Low probability of causing insertional mutagenesis because it doesn't integrate into the host genome.

### Lentivirus

Lentiviruses are complex retroviruses that have the ability to infect and express their genes in both mitotic and post-mitotic cells. The most commonly known lentivirus is the human immunodeficiency virus (HIV), which uses the envelope glycoproteins of other viruses to target a broad range of cell types.

Lentiviruses may be prepared as follows. After cloning pCasES10 (which contains a lentiviral transfer plasmid backbone), HEK293FT at low passage (p=5) were seeded in a T-75 flask to 50% confluence the day before transfection in DMEM with 10% fetal bovine serum and without antibiotics. After 20 hours, media was changed to OptiMEM (serum-free) media and transfection was done 4 hours later. Cells were transfected with 10 µg of lentiviral transfer plasmid (pCasES10) and the following packaging plasmids: 5 µg of pMD2.G (VSV-g pseudotype), and 7.5ug of psPAX2 (gag/pol/rev/tat). Transfection was done in 4mL OptiMEM with a cationic lipid delivery agent (50uL Lipofectamine 2000 and 100ul Plus reagent). After 6 hours, the media was changed to antibiotic-free DMEM with 10% fetal bovine serum. These methods use serum during cell culture, but serum-free methods are preferred.

Lentivirus may be purified as follows. Viral supernatants were harvested after 48 hours. Supernatants were first cleared of debris and filtered through a 0.45um low protein binding (PVDF) filter. They were then spun in a ultracentrifuge for 2 hours at 24,000 rpm. Viral pellets were resuspended in 50ul of DMEM overnight at 4C. They were then aliquotted and immediately frozen at -80°C.

In another embodiment, minimal non-primate lentiviral vectors based on the equine infectious anemia virus (EIAV) are also contemplated, especially for ocular gene therapy (see, e.g., Balagaan, J Gene Med 2006; 8: 275 - 285). In another embodiment, RetinoStat^{®}, an equine infectious anemia virus-based lentiviral gene therapy vector that expresses angiostatic proteins endostatin and angiostatin that is delivered via a subretinal injection for the treatment of the web form of age-related macular degeneration is also contemplated (see, e.g., Binley et al., HUMAN GENE THERAPY 23:980-991 (September 2012)) and this vector may be modified for the CRISPR-Cas system of the present invention.

In another embodiment, self-inactivating lentiviral vectors with an siRNA targeting a common exon shared by HIV tat/rev, a nucleolar-localizing TAR decoy, and an anti-CCR5-specific hammerhead ribozyme (see, e.g., DiGiusto et al. (2010) Sci Transl Med 2:36ra43) may be used/and or adapted to the CRISPR-Cas system of the present invention. A minimum of 2.5 × 106 CD34+ cells per kilogram patient weight may be collected and prestimulated for 16 to 20 hours in X-VIVO 15 medium (Lonza) containing 2 µmol/L-glutamine, stem cell factor (100 ng/ml), Flt-3 ligand (Flt-3L) (100 ng/ml), and thrombopoietin (10 ng/ml) (CellGenix) at a density of 2 × 106 cells/ml. Prestimulated cells may be transduced with lentiviral at a multiplicity of infection of 5 for 16 to 24 hours in 75-cm2 tissue culture flasks coated with fibronectin (25 mg/cm2) (RetroNectin, Takara Bio Inc.).

Lentiviral vectors have been disclosed as in the treatment for Parkinson's Disease, see, e.g., US Patent Publication No. 20120295960 and US Patent Nos. 7303910 and 7351585. Lentiviral vectors have also been disclosed for the treatment of ocular diseases, see e.g., US Patent Publication Nos. 20060281180, 20090007284, US20110117189; US20090017543; US20070054961, US20100317109. Lentiviral vectors have also been disclosed for delivery to the brain, see, e.g., US Patent Publication Nos. US20110293571; US20110293571, US20040013648, US20070025970, US20090111106 and US Patent No. US7259015.

### RNA delivery

RNA delivery: The chromatin 3D structure modulating agents, such asthe CRISPR protein, and/or any other of the components of the CRISPR/Cas system, for instance a guide RNA, can also be delivered in the form of RNA. Cas enzyme encoding mRNA can be generated using in vitro transcription. For example, Cas9 mRNA can be synthesized using a PCR cassette containing the following elements: T7_promoter-kozak sequence (GCCACC)-Cas9-3' UTR from beta globin-polyA tail (a string of 120 or more adenines). The cassette can be used for transcription by T7 polymerase. Guide RNAs can also be transcribed using in vitro transcription from a cassette containing T7_promoter-GG-guide RNA sequence.

To enhance expression and reduce possible toxicity, the CRISPR enzyme-coding sequence and/or the guide RNA can be modified to include one or more modified nucleoside e.g. using pseudo-U or 5-Methyl-C.

mRNA delivery methods are especially promising for liver delivery currently.

Much clinical work on RNA delivery has focused on RNAi or antisense, but these systems can be adapted for delivery of RNA for implementing the present invention. References below to RNAi etc. should be read accordingly.

### Particle delivery systems and/or formulations:

Several types of particle delivery systems and/or formulations are known to be useful in a diverse spectrum of biomedical applications. In general, a particle is defined as a small object that behaves as a whole unit with respect to its transport and properties. Particles are further classified according to diameter Coarse particles cover a range between 2,500 and 10,000 nanometers. Fine particles are sized between 100 and 2,500 nanometers. Ultrafine particles, or nanoparticles, are generally between 1 and 100 nanometers in size. The basis of the 100-nm limit is the fact that novel properties that differentiate particles from the bulk material typically develop at a critical length scale of under 100 nm.

As used herein, a particle delivery system/formulation is defined as any biological delivery system/formulation which includes a particle in accordance with the present invention. A particle in accordance with the present invention is any entity having a greatest dimension (e.g. diameter) of less than 100 microns (µm). In some embodiments, inventive particles have a greatest dimension of less than 10 µm. In some embodiments, inventive particles have a greatest dimension of less than 2000 nanometers (nm). In some embodiments, inventive particles have a greatest dimension of less than 1000 nanometers (nm). In some embodiments, inventive particles have a greatest dimension of less than 900 nm, 800 nm, 700 nm, 600 nm, 500 nm, 400 nm, 300 nm, 200 nm, or 100 nm. Typically, inventive particles have a greatest dimension (e.g., diameter) of 500 nm or less. In some embodiments, inventive particles have a greatest dimension (e.g., diameter) of 250 nm or less. In some embodiments, inventive particles have a greatest dimension (e.g., diameter) of 200 nm or less. In some embodiments, inventive particles have a greatest dimension (e.g., diameter) of 150 nm or less. In some embodiments, inventive particles have a greatest dimension (e.g., diameter) of 100 nm or less. Smaller particles, e.g., having a greatest dimension of 50 nm or less are used in some embodiments of the invention. In some embodiments, inventive particles have a greatest dimension ranging between 25 nm and 200 nm.

Particle characterization (including e.g., characterizing morphology, dimension, etc.) is done using a variety of different techniques. Common techniques are electron microscopy (TEM, SEM), atomic force microscopy (AFM), dynamic light scattering (DLS), X-ray photoelectron spectroscopy (XPS), powder X-ray diffraction (XRD), Fourier transform infrared spectroscopy (FTIR), matrix-assisted laser desorption/ionization time-of-flight mass spectrometry(MALDI-TOF), ultraviolet-visible spectroscopy, dual polarisation interferometry and nuclear magnetic resonance (NMR). Characterization (dimension measurements) may be made as to native particles (i.e., preloading) or after loading of the cargo (herein cargo refers to e.g., one or more components of CRISPR-Cas system e.g., CRISPR enzyme or mRNA or guide RNA, or any combination thereof, and may include additional carriers and/or excipients) to provide particles of an optimal size for delivery for any in vitro, ex vivo and/or in vivo application of the present invention. In certain preferred embodiments, particle dimension (e.g., diameter) characterization is based on measurements using dynamic laser scattering (DLS). Mention is made of US Patent No. 8,709,843; US Patent No. 6,007,845; US Patent No. 5,855,913; US Patent No. 5,985,309; US. Patent No. 5,543,158; and the publication by James E. Dahlman and Carmen Barnes et al. Nature Nanotechnology (2014) published online 11 May 2014, doi:10.1038/nnano.2014.84, concerning particles, methods of making and using them and measurements thereof.

Particles delivery systems within the scope of the present invention may be provided in any form, including but not limited to solid, semi-solid, emulsion, or colloidal particles. As such any of the delivery systems described herein, including but not limited to, e.g., lipid-based systems, liposomes, micelles, microvesicles, exosomes, or gene gun may be provided as particle delivery systems within the scope of the present invention.

The chromatin 3D structure modulating agents, such as but not limited to CRISPR protein mRNA and guide RNA may be delivered simultaneously using particles or lipid envelopes; for instance, CRISPR enzyme and RNA of the invention, e.g., as a complex, can be delivered via a particle as in Dahlman et al., WO2015089419 A2 and documents cited therein, such as 7C1 (see, e.g., James E. Dahlman and Carmen Barnes et al. Nature Nanotechnology (2014) published online 11 May 2014, doi:10.1038/nnano.2014.84), e.g., delivery particle comprising lipid or lipidoid and hydrophilic polymer, e.g., cationic lipid and hydrophilic polymer, for instance wherein the the cationic lipid comprises 1,2-dioleoyl-3-trimethylammonium-propane (DOTAP) or 1,2-ditetradecanoyl-sn-glycero-3-phosphocholine (DMPC) and/or wherein the hydrophilic polymer comprises ethylene glycol or polyethylene glycol (PEG); and/or wherein the particle further comprises cholesterol (e.g., particle from formulation 1 = DOTAP 100, DMPC 0, PEG 0, Cholesterol 0; formulation number 2 = DOTAP 90, DMPC 0, PEG 10, Cholesterol 0; formulation number 3 = DOTAP 90, DMPC 0, PEG 5, Cholesterol 5), wherein particles are formed using an efficient, multistep process wherein first, effector protein and RNA are mixed together, e.g., at a 1:1 molar ratio, e.g., at room temperature, e.g., for 30 minutes, e.g., in sterile, nuclease free 1X PBS; and separately, DOTAP, DMPC, PEG, and cholesterol as applicable for the formulation are dissolved in alcohol, e.g., 100% ethanol; and, the two solutions are mixed together to form particles containing the complexes).

For example, Su X, Fricke J, Kavanagh DG, Irvine DJ ("In vitro and in vivo mRNA delivery using lipid-enveloped pH-responsive polymer nanoparticles" Mol Pharm. 2011 Jun 6;8(3):774-87. doi: 10.1021/mp100390w. Epub 2011 Apr 1) describes biodegradable core-shell structured particles with a poly(β-amino ester) (PBAE) core enveloped by a phospholipid bilayer shell. These were developed for in vivo mRNA delivery. The pH-responsive PBAE component was chosen to promote endosome disruption, while the lipid surface layer was selected to minimize toxicity of the polycation core. Such are, therefore, preferred for delivering RNA of the present invention.

In one embodiment, particles based on self assembling bioadhesive polymers are contemplated, which may be applied to oral delivery of peptides, intravenous delivery of peptides and nasal delivery of peptides, all to the brain. Other embodiments, such as oral absorption and ocular delivery of hydrophobic drugs are also contemplated. The molecular envelope technology involves an engineered polymer envelope which is protected and delivered to the site of the disease (see, e.g., Mazza, M. et al. ACSNano, 2013. 7(2): 1016-1026; Siew, A., et al. Mol Pharm, 2012. 9(1):14-28; Lalatsa, A., et al. J Contr Rel, 2012. 161(2):523-36; Lalatsa, A., et al., Mol Pharm, 2012. 9(6):1665-80; Lalatsa, A., et al. Mol Pharm, 2012. 9(6):1764-74; Garrett, N.L., et al. J Biophotonics, 2012. 5(5-6):458-68; Garrett, N.L., et al. J Raman Spect, 2012. 43(5):681-688; Ahmad, S., et al. J Royal Soc Interface 2010. 7:S423-33; Uchegbu, I.F. Expert Opin Drug Deliv, 2006. 3(5):629-40; Qu, X.,et al. Biomacromolecules, 2006. 7(12):3452-9 and Uchegbu, I.F., et al. Int J Pharm, 2001. 224:185-199). Doses of about 5 mg/kg are contemplated, with single or multiple doses, depending on the target tissue.

In one embodiment, particles that can deliver RNA to a cancer cell to stop tumor growth developed by Dan Anderson's lab at MIT may be used/and or adapted to the CRISPR Cas system of the present invention. In particular, the Anderson lab developed fully automated, combinatorial systems for the synthesis, purification, characterization, and formulation of new biomaterials and nanoformulations. See, e.g., Alabi et al., Proc Natl Acad Sci U S A. 2013 Aug 6;110(32):12881-6; Zhang et al., Adv Mater. 2013 Sep 6;25(33):4641-5; Jiang et al., Nano Lett. 2013 Mar 13;13(3):1059-64; Karagiannis et al., ACS Nano. 2012 Oct 23;6(10):8484-7; Whitehead et al., ACS Nano. 2012 Aug 28;6(8):6922-9 and Lee et al., Nat Nanotechnol. 2012 Jun 3;7(6):389-93.

US patent application 20110293703 relates to lipidoid compounds are also particularly useful in the administration of polynucleotides, which may be applied to deliver the CRISPR Cas system of the present invention. In one aspect, the aminoalcohol lipidoid compounds are combined with an agent to be delivered to a cell or a subject to form microparticles, particles, liposomes, or micelles. The agent to be delivered by the particles, liposomes, or micelles may be in the form of a gas, liquid, or solid, and the agent may be a polynucleotide, protein, peptide, or small molecule. The minoalcohol lipidoid compounds may be combined with other aminoalcohol lipidoid compounds, polymers (synthetic or natural), surfactants, cholesterol, carbohydrates, proteins, lipids, etc. to form the particles. These particles may then optionally be combined with a pharmaceutical excipient to form a pharmaceutical composition.

US Patent Publication No. 20110293703 also provides methods of preparing the aminoalcohol lipidoid compounds. One or more equivalents of an amine are allowed to react with one or more equivalents of an epoxide-terminated compound under suitable conditions to form an aminoalcohol lipidoid compound of the present invention. In certain embodiments, all the amino groups of the amine are fully reacted with the epoxide-terminated compound to form tertiary amines. In other embodiments, all the amino groups of the amine are not fully reacted with the epoxide-terminated compound to form tertiary amines thereby resulting in primary or secondary amines in the aminoalcohol lipidoid compound. These primary or secondary amines are left as is or may be reacted with another electrophile such as a different epoxide-terminated compound. As will be appreciated by one skilled in the art, reacting an amine with less than excess of epoxide-terminated compound will result in a plurality of different aminoalcohol lipidoid compounds with various numbers of tails. Certain amines may be fully functionalized with two epoxide-derived compound tails while other molecules will not be completely functionalized with epoxide-derived compound tails. For example, a diamine or polyamine may include one, two, three, or four epoxide-derived compound tails off the various amino moieties of the molecule resulting in primary, secondary, and tertiary amines. In certain embodiments, all the amino groups are not fully functionalized. In certain embodiments, two of the same types of epoxide-terminated compounds are used. In other embodiments, two or more different epoxide-terminated compounds are used. The synthesis of the aminoalcohol lipidoid compounds is performed with or without solvent, and the synthesis may be performed at higher temperatures ranging from 30-100 °C., preferably at approximately 50-90 °C. The prepared aminoalcohol lipidoid compounds may be optionally purified. For example, the mixture of aminoalcohol lipidoid compounds may be purified to yield an aminoalcohol lipidoid compound with a particular number of epoxide-derived compound tails. Or the mixture may be purified to yield a particular stereo- or regioisomer. The aminoalcohol lipidoid compounds may also be alkylated using an alkyl halide (e.g., methyl iodide) or other alkylating agent, and/or they may be acylated.

US Patent Publication No. 20110293703 also provides libraries of aminoalcohol lipidoid compounds prepared by the inventive methods. These aminoalcohol lipidoid compounds may be prepared and/or screened using high-throughput techniques involving liquid handlers, robots, microtiter plates, computers, etc. In certain embodiments, the aminoalcohol lipidoid compounds are screened for their ability to transfect polynucleotides or other agents (e.g., proteins, peptides, small molecules) into the cell.

US Patent Publication No. 20130302401 relates to a class of poly(beta-amino alcohols) (PBAAs) has been prepared using combinatorial polymerization. The inventive PBAAs may be used in biotechnology and biomedical applications as coatings (such as coatings of films or multilayer films for medical devices or implants), additives, materials, excipients, non-biofouling agents, micropatterning agents, and cellular encapsulation agents. When used as surface coatings, these PBAAs elicited different levels of inflammation, both in vitro and in vivo, depending on their chemical structures. The large chemical diversity of this class of materials allowed us to identify polymer coatings that inhibit macrophage activation in vitro. Furthermore, these coatings reduce the recruitment of inflammatory cells, and reduce fibrosis, following the subcutaneous implantation of carboxylated polystyrene microparticles. These polymers may be used to form polyelectrolyte complex capsules for cell encapsulation. The invention may also have many other biological applications such as antimicrobial coatings, DNA or siRNA delivery, and stem cell tissue engineering. The teachings of US Patent Publication No. 20130302401 may be applied to the CRISPR Cas system of the present invention.

In another embodiment, lipid particles (LNPs) are contemplated. An antitransthyretin small interfering RNA has been encapsulated in lipid particles and delivered to humans (see, e.g., Coelho et al., N Engl J Med 2013;369:819-29), and such a ssystem may be adapted and applied to the CRISPR Cas system of the present invention. Doses of about 0.01 to about 1 mg per kg of body weight administered intravenously are contemplated. Medications to reduce the risk of infusion-related reactions are contemplated, such as dexamethasone, acetampinophen, diphenhydramine or cetirizine, and ranitidine are contemplated. Multiple doses of about 0.3 mg per kilogram every 4 weeks for five doses are also contemplated.

LNPs have been shown to be highly effective in delivering siRNAs to the liver (see, e.g., Tabernero et al., Cancer Discovery, April 2013, Vol. 3, No. 4, pages 363-470) and are therefore contemplated for delivering RNA encoding CRISPR Cas to the liver. A dosage of about four doses of 6 mg/kg of the LNP every two weeks may be contemplated. Tabernero et al. demonstrated that tumor regression was observed after the first 2 cycles of LNPs dosed at 0.7 mg/kg, and by the end of 6 cycles the patient had achieved a partial response with complete regression of the lymph node metastasis and substantial shrinkage of the liver tumors. A complete response was obtained after 40 doses in this patient, who has remained in remission and completed treatment after receiving doses over 26 months. Two patients with RCC and extrahepatic sites of disease including kidney, lung, and lymph nodes that were progressing following prior therapy with VEGF pathway inhibitors had stable disease at all sites for approximately 8 to 12 months, and a patient with PNET and liver metastases continued on the extension study for 18 months (36 doses) with stable disease.

However, the charge of the LNP must be taken into consideration. As cationic lipids combined with negatively charged lipids to induce nonbilayer structures that facilitate intracellular delivery. Because charged LNPs are rapidly cleared from circulation following intravenous injection, ionizable cationic lipids with pKa values below 7 were developed (see, e.g., Rosin et al, Molecular Therapy, vol. 19, no. 12, pages 1286-2200, Dec. 2011). Negatively charged polymers such as RNA may be loaded into LNPs at low pH values (e.g., pH 4) where the ionizable lipids display a positive charge. However, at physiological pH values, the LNPs exhibit a low surface charge compatible with longer circulation times. Four species of ionizable cationic lipids have been focused upon, namely 1,2-dilineoyl-3-dimethylammonium-propane (DLinDAP), 1,2-dilinoleyloxy-3-N,N-dimethylaminopropane (DLinDMA), 1,2-dilinoleyloxy-keto-N,N-dimethyl-3-aminopropane (DLinKDMA), and 1,2-dilinoleyl-4-(2-dimethylaminoethyl)-[1,3]-dioxolane (DLinKC2-DMA). It has been shown that LNP siRNA systems containing these lipids exhibit remarkably different gene silencing properties in hepatocytes in vivo, with potencies varying according to the series DLinKC2-DMA>DLinKDMA>DLinDMA>>DLinDAP employing a Factor VII gene silencing model (see, e.g., Rosin et al, Molecular Therapy, vol. 19, no. 12, pages 1286-2200, Dec. 2011). A dosage of 1 µg/ml of LNP or CRISPR-Cas RNA in or associated with the LNP may be contemplated, especially for a formulation containing DLinKC2-DMA.

Preparation of LNPs and encapsulation of the chromatin 3D structure modulating agents may be used/and or adapted from Rosin et al, Molecular Therapy, vol. 19, no. 12, pages 1286-2200, Dec. 2011). The cationic lipids 1,2-dilineoyl-3-dimethylammonium-propane (DLinDAP), 1,2-dilinoleyloxy-3-N,N-dimethylaminopropane (DLinDMA), 1,2-dilinoleyloxyketo-N,N-dimethyl-3-aminopropane (DLinK-DMA), 1,2-dilinoleyl-4-(2-dimethylaminoethyl)-[1,3]-dioxolane (DLinKC2-DMA), (3-o-[2"-(methoxypolyethyleneglycol 2000) succinoyl]-1,2-dimyristoyl-sn-glycol (PEG-S-DMG), and R-3-[(ω-methoxy-poly(ethylene glycol)2000) carbamoyl]-1,2-dimyristyloxlpropyl-3-amine (PEG-C-DOMG) may be provided by Tekmira Pharmaceuticals (Vancouver, Canada) or synthesized. Cholesterol may be purchased from Sigma (St Louis, MO). The specific CRISPR Cas RNA may be encapsulated in LNPs containing DLinDAP, DLinDMA, DLinK-DMA, and DLinKC2-DMA (cationic lipid:DSPC:CHOL: PEGS-DMG or PEG-C-DOMG at 40:10:40:10 molar ratios). When required, 0.2% SP-DiOC18 (Invitrogen, Burlington, Canada) may be incorporated to assess cellular uptake, intracellular delivery, and biodistribution. Encapsulation may be performed by dissolving lipid mixtures comprised of cationic lipid:DSPC:cholesterol:PEG-c-DOMG (40:10:40:10 molar ratio) in ethanol to a final lipid concentration of 10 mmol/l. This ethanol solution of lipid may be added drop-wise to 50 mmol/l citrate, pH 4.0 to form multilamellar vesicles to produce a final concentration of 30% ethanol vol/vol. Large unilamellar vesicles may be formed following extrusion of multilamellar vesicles through two stacked 80 nm Nuclepore polycarbonate filters using the Extruder (Northern Lipids, Vancouver, Canada). Encapsulation may be achieved by adding RNA dissolved at 2 mg/ml in 50 mmol/l citrate, pH 4.0 containing 30% ethanol vol/vol drop-wise to extruded preformed large unilamellar vesicles and incubation at 31 °C for 30 minutes with constant mixing to a final RNA/lipid weight ratio of 0.06/1 wt/wt. Removal of ethanol and neutralization of formulation buffer were performed by dialysis against phosphate-buffered saline (PBS), pH 7.4 for 16 hours using Spectra/Por 2 regenerated cellulose dialysis membranes. Particle size distribution may be determined by dynamic light scattering using a NICOMP 370 particle sizer, the vesicle/intensity modes, and Gaussian fitting (Nicomp Particle Sizing, Santa Barbara, CA). The particle size for all three LNP systems may be ~70 nm in diameter. RNA encapsulation efficiency may be determined by removal of free RNA using VivaPureD MiniH columns (Sartorius Stedim Biotech) from samples collected before and after dialysis. The encapsulated RNA may be extracted from the eluted particles and quantified at 260 nm. RNA to lipid ratio was determined by measurement of cholesterol content in vesicles using the Cholesterol E enzymatic assay from Wako Chemicals USA (Richmond, VA). In conjunction with the herein discussion of LNPs and PEG lipids, PEGylated liposomes or LNPs are likewise suitable for delivery of a CRISPR-Cas system or components thereof.

Preparation of large LNPs may be used/and or adapted from Rosin et al, Molecular Therapy, vol. 19, no. 12, pages 1286-2200, Dec. 2011. A lipid premix solution (20.4 mg/ml total lipid concentration) may be prepared in ethanol containing DLinKC2-DMA, DSPC, and cholesterol at 50:10:38.5 molar ratios. Sodium acetate may be added to the lipid premix at a molar ratio of 0.75:1 (sodium acetate:DLinKC2-DMA). The lipids may be subsequently hydrated by combining the mixture with 1.85 volumes of citrate buffer (10 mmol/l, pH 3.0) with vigorous stirring, resulting in spontaneous liposome formation in aqueous buffer containing 35% ethanol. The liposome solution may be incubated at 37 °C to allow for time-dependent increase in particle size. Aliquots may be removed at various times during incubation to investigate changes in liposome size by dynamic light scattering (Zetasizer Nano ZS, Malvern Instruments, Worcestershire, UK). Once the desired particle size is achieved, an aqueous PEG lipid solution (stock = 10 mg/ml PEG-DMG in 35% (vol/vol) ethanol) may be added to the liposome mixture to yield a final PEG molar concentration of 3.5% of total lipid. Upon addition of PEG-lipids, the liposomes should their size, effectively quenching further growth. RNA may then be added to the empty liposomes at an RNA to total lipid ratio of approximately 1:10 (wt:wt), followed by incubation for 30 minutes at 37 °C to form loaded LNPs. The mixture may be subsequently dialyzed overnight in PBS and filtered with a 0.45-µm syringe filter.

Spherical Nucleic Acid (SNA^{™}) constructs and other particles (particularly gold particles) are also contemplated as a means to delivery CRISPR-Cas system to intended targets. Significant data show that AuraSense Therapeutics' Spherical Nucleic Acid (SNA^{™}) constructs, based upon nucleic acid-functionalized gold particles, are useful.

Literature that may be employed in conjunction with herein teachings include: Cutler et al., J. Am. Chem. Soc. 2011 133:9254-9257, Hao et al., Small. 2011 7:3158-3162, Zhang et al., ACS Nano. 2011 5:6962-6970, Cutler et al., J. Am. Chem. Soc. 2012 134:1376-1391, Young et al., Nano Lett. 2012 12:3867-71, Zheng et al., Proc. Natl. Acad. Sci. USA. 2012 109:11975-80, Mirkin, Nanomedicine 2012 7:635-638 Zhang et al., J. Am. Chem. Soc. 2012 134:16488-1691, Weintraub, Nature 2013 495:S14-S16, Choi et al., Proc. Natl. Acad. Sci. USA. 2013 110(19):7625-7630, Jensen et al., Sci. Transl. Med. 5, 209ra152 (2013) and Mirkin, et al., Small, 10:186-192.

Self-assembling particles with RNA may be constructed with polyethyleneimine (PEI) that is PEGylated with an Arg-Gly-Asp (RGD) peptide ligand attached at the distal end of the polyethylene glycol (PEG). This system has been used, for example, as a means to target tumor neovasculature expressing integrins and deliver siRNA inhibiting vascular endothelial growth factor receptor-2 (VEGF R2) expression and thereby achieve tumor angiogenesis (see, e.g., Schiffelers et al., Nucleic Acids Research, 2004, Vol. 32, No. 19). Nanoplexes may be prepared by mixing equal volumes of aqueous solutions of cationic polymer and nucleic acid to give a net molar excess of ionizable nitrogen (polymer) to phosphate (nucleic acid) over the range of 2 to 6. The electrostatic interactions between cationic polymers and nucleic acid resulted in the formation of polyplexes with average particle size distribution of about 100 nm, hence referred to here as nanoplexes. A dosage of about 100 to 200 mg of CRISPR Cas is envisioned for delivery in the self-assembling particles of Schiffelers et al.

The nanoplexes of Bartlett et al. (PNAS, September 25, 2007,vol. 104, no. 39) may also be applied to the present invention. The nanoplexes of Bartlett et al. are prepared by mixing equal volumes of aqueous solutions of cationic polymer and nucleic acid to give a net molar excess of ionizable nitrogen (polymer) to phosphate (nucleic acid) over the range of 2 to 6. The electrostatic interactions between cationic polymers and nucleic acid resulted in the formation of polyplexes with average particle size distribution of about 100 nm, hence referred to here as nanoplexes. The DOTA-siRNA of Bartlett et al. was synthesized as follows: 1,4,7,10-tetraazacyclododecane-1,4,7,10-tetraacetic acid mono(N-hydroxysuccinimide ester) (DOTA-NHSester) was ordered from Macrocyclics (Dallas, TX). The amine modified RNA sense strand with a 100-fold molar excess of DOTA-NHS-ester in carbonate buffer (pH 9) was added to a microcentrifuge tube. The contents were reacted by stirring for 4 h at room temperature. The DOTA-RNAsense conjugate was ethanol-precipitated, resuspended in water, and annealed to the unmodified antisense strand to yield DOTA-siRNA. All liquids were pretreated with Chelex-100 (Bio-Rad, Hercules, CA) to remove trace metal contaminants. Tf-targeted and nontargeted siRNA particles may be formed by using cyclodextrin-containing polycations. Typically, particles were formed in water at a charge ratio of 3 (+/-) and an siRNA concentration of 0.5 g/liter. One percent of the adamantane-PEG molecules on the surface of the targeted particles were modified with Tf (adamantane-PEG-Tf). The particles were suspended in a 5% (wt/vol) glucose carrier solution for injection.

Davis et al. (Nature, Vol 464, 15 April 2010) conducts a RNA clinical trial that uses a targeted particle-delivery system (clinical trial registration number NCT00689065). Patients with solid cancers refractory to standard-of-care therapies are administered doses of targeted particles on days 1, 3, 8 and 10 of a 21-day cycle by a 30-min intravenous infusion. The particles consist of a synthetic delivery system containing: (1) a linear, cyclodextrin-based polymer (CDP), (2) a human transferrin protein (TF) targeting ligand displayed on the exterior of the particle to engage TF receptors (TFR) on the surface of the cancer cells, (3) a hydrophilic polymer (polyethylene glycol (PEG) used to promote particle stability in biological fluids), and (4) siRNA designed to reduce the expression of the RRM2 (sequence used in the clinic was previously denoted siR2B+5). The TFR has long been known to be upregulated in malignant cells, and RRM2 is an established anti-cancer target. These particles (clinical version denoted as CALAA-01) have been shown to be well tolerated in multi-dosing studies in non-human primates. Although a single patient with chronic myeloid leukaemia has been administered siRNAby liposomal delivery, Davis et al.'s clinical trial is the initial human trial to systemically deliver siRNA with a targeted delivery system and to treat patients with solid cancer. To ascertain whether the targeted delivery system can provide effective delivery of functional siRNA to human tumours, Davis et al. investigated biopsies from three patients from three different dosing cohorts; patients A, B and C, all of whom had metastatic melanoma and received CALAA-01 doses of 18, 24 and 30 mg m-2 siRNA, respectively. Similar doses may also be contemplated for the the chromatin 3D structure modulating agents of the present invention. The delivery of the invention may be achieved with particles containing a linear, cyclodextrin-based polymer (CDP), a human transferrin protein (TF) targeting ligand displayed on the exterior of the particle to engage TF receptors (TFR) on the surface of the cancer cells and/or a hydrophilic polymer (for example, polyethylene glycol (PEG) used to promote particle stability in biological fluids).

It is preferred to have the chromatin 3D structure modulating agents, such as one or more components of CRISPR complex, e.g., CRISPR protein or mRNA or guide RNA delivered using particles or lipid envelopes. Other delivery systems or vectors are may be used in conjunction with the particle aspects of the invention.

In general, a "nanoparticle" refers to any particle having a diameter of less than 1000 nm. In certain preferred embodiments, nanoparticles of the invention have a greatest dimension (e.g., diameter) of 500 nm or less. In other preferred embodiments, nanoparticles of the invention have a greatest dimension ranging between 25 nm and 200 nm. In other preferred embodiments, nanoparticles of the invention have a greatest dimension of 100 nm or less. In other preferred embodiments, particles of the invention have a greatest dimension ranging between 35 nm and 60 nm. In other preferred embodiments, the particles of the invention are not nanoparticles.

Particles encompassed in the present invention may be provided in different forms, e.g., as solid particles (e.g., metal such as silver, gold, iron, titanium), non-metal, lipid-based solids, polymers), suspensions of particles, or combinations thereof. Metal, dielectric, and semiconductor particles may be prepared, as well as hybrid structures (e.g., core-shell particles). Particles made of semiconducting material may also be labeled quantum dots if they are small enough (typically sub 10 nm) that quantization of electronic energy levels occurs. Such nanoscale particles are used in biomedical applications as drug carriers or imaging agents and may be adapted for similar purposes in the present invention.

Semi-solid and soft particles have been manufactured, and are within the scope of the present invention. A prototype particle of semi-solid nature is the liposome. Various types of liposome particles are currently used clinically as delivery systems for anticancer drugs and vaccines. Particles with one half hydrophilic and the other half hydrophobic are termed Janus particles and are particularly effective for stabilizing emulsions. They can self-assemble at water/oil interfaces and act as solid surfactants.

US Patent No. 8,709,843, provides a drug delivery system for targeted delivery of therapeutic agent-containing particles to tissues, cells, and intracellular compartments. The invention provides targeted particles comprising comprising polymer conjugated to a surfactant, hydrophilic polymer or lipid. US Patent No. 6,007,845, provides particles which have a core of a multiblock copolymer formed by covalently linking a multifunctional compound with one or more hydrophobic polymers and one or more hydrophilic polymers, and conatin a biologically active material. US Patent No. 5,855,913, provides a particulate composition having aerodynamically light particles having a tap density of less than 0.4 g/cm3 with a mean diameter of between 5 µm and 30 µ m, incorporating a surfactant on the surface thereof for drug delivery to the pulmonary system. US Patent No. 5,985,309, provides particles incorporating a surfactant and/or a hydrophilic or hydrophobic complex of a positively or negatively charged therapeutic or diagnostic agent and a charged molecule of opposite charge for delivery to the pulmonary system. US. Patent No. 5,543,158, provides biodegradable injectable particles having a biodegradable solid core containing a biologically active material and poly(alkylene glycol) moieties on the surface. WO2012135025 (also published as US20120251560), describes conjugated polyethyleneimine (PEI) polymers and conjugated aza-macrocycles (collectively referred to as "conjugated lipomer" or "lipomers"). In certain embodiments, it can envisioned that such conjugated lipomers can be used in the context of the CRISPR-Cas system to achieve in vitro, ex vivo and in vivo genomic perturbations to modify gene expression, including modulation of protein expression.

In one embodiment, the particle may be epoxide-modified lipid-polymer, advantageously 7C1 (see, e.g., James E. Dahlman and Carmen Barnes et al. Nature Nanotechnology (2014) published online 11 May 2014, doi:10.1038/nnano.2014.84). C71 was synthesized by reacting C15 epoxide-terminated lipids with PEI600 at a 14:1 molar ratio, and was formulated with C14PEG2000 to produce particles (diameter between 35 and 60 nm) that were stable in PBS solution for at least 40 days.

An epoxide-modified lipid-polymer may be utilized to deliver the CRISPR-Cas system of the present invention to pulmonary, cardiovascular or renal cells, however, one of skill in the art may adapt the system to deliver to other target organs. Dosage ranging from about 0.05 to about 0.6 mg/kg are envisioned. Dosages over several days or weeks are also envisioned, with a total dosage of about 2 mg/kg.

### Exosomes

Exosomes are endogenous nano-vesicles that transport RNAs and proteins, and which can deliver agents to the brain and other target organs. To reduce immunogenicity, Alvarez-Erviti et al. (2011, Nat Biotechnol 29: 341) used self-derived dendritic cells for exosome production. Targeting to the brain was achieved by engineering the dendritic cells to express Lamp2b, an exosomal membrane protein, fused to the neuron-specific RVG peptide. Purified exosomes were loaded with exogenous RNA by electroporation. Intravenously injected RVG-targeted exosomes delivered GAPDH siRNA specifically to neurons, microglia, oligodendrocytes in the brain, resulting in a specific gene knockdown. Pre-exposure to RVG exosomes did not attenuate knockdown, and non-specific uptake in other tissues was not observed. The therapeutic potential of exosome-mediated siRNA delivery was demonstrated by the strong mRNA (60%) and protein (62%) knockdown of BACE1, a therapeutic target in Alzheimer's disease.

To obtain a pool of immunologically inert exosomes, Alvarez-Erviti et al. harvested bone marrow from inbred C57BL/6 mice with a homogenous major histocompatibility complex (MHC) haplotype. As immature dendritic cells produce large quantities of exosomes devoid of T-cell activators such as MHC-II and CD86, Alvarez-Erviti et al. selected for dendritic cells with granulocyte/macrophage-colony stimulating factor (GM-CSF) for 7 d. Exosomes were purified from the culture supernatant the following day using well-established ultracentrifugation protocols. The exosomes produced were physically homogenous, with a size distribution peaking at 80 nm in diameter as determined by particle tracking analysis (NTA) and electron microscopy. Alvarez-Erviti et al. obtained 6-12 µg of exosomes (measured based on protein concentration) per 106 cells.

Next, Alvarez-Erviti et al. investigated the possibility of loading modified exosomes with exogenous cargoes using electroporation protocols adapted for nanoscale applications. As electroporation for membrane particles at the nanometer scale is not well-characterized, non-specific Cy5-labeled RNA was used for the empirical optimization of the electroporation protocol. The amount of encapsulated RNA was assayed after ultracentrifugation and lysis of exosomes. Electroporation at 400 V and 125 µF resulted in the greatest retention of RNA and was used for all subsequent experiments.

Alvarez-Erviti et al. administered 150 µg of each BACE1 siRNA encapsulated in 150 µg of RVG exosomes to normal C57BL/6 mice and compared the knockdown efficiency to four controls: untreated mice, mice injected with RVG exosomes only, mice injected with BACE1 siRNA complexed to an in vivo cationic liposome reagent and mice injected with BACE1 siRNA complexed to RVG-9R, the RVG peptide conjugated to 9 D-arginines that electrostatically binds to the siRNA. Cortical tissue samples were analyzed 3 d after administration and a significant protein knockdown (45%, P < 0.05, versus 62%, P < 0.01) in both siRNA-RVG-9R-treated and siRNARVG exosome-treated mice was observed, resulting from a significant decrease in BACE1 mRNA levels (66% [+ or -] 15%, P < 0.001 and 61% [+ or -] 13% respectively, P < 0.01). Moreover, Applicants demonstrated a significant decrease (55%, P < 0.05) in the total [beta]-amyloid 1-42 levels, a main component of the amyloid plaques in Alzheimer's pathology, in the RVG-exosome-treated animals. The decrease observed was greater than the β-amyloid 1-40 decrease demonstrated in normal mice after intraventricular injection of BACE1 inhibitors. Alvarez-Erviti et al. carried out 5'-rapid amplification of cDNA ends (RACE) on BACE1 cleavage product, which provided evidence of RNAi-mediated knockdown by the siRNA.

Finally, Alvarez-Erviti et al. investigated whether RNA-RVG exosomes induced immune responses in vivo by assessing IL-6, IP-10, TNFα and IFN-α serum concentrations. Following exosome treatment, nonsignificant changes in all cytokines were registered similar to siRNA-transfection reagent treatment in contrast to siRNA-RVG-9R, which potently stimulated IL-6 secretion, confirming the immunologically inert profile of the exosome treatment. Given that exosomes encapsulate only 20% of siRNA, delivery with RVG-exosome appears to be more efficient than RVG-9R delivery as comparable mRNA knockdown and greater protein knockdown was achieved with fivefold less siRNA without the corresponding level of immune stimulation. This experiment demonstrated the therapeutic potential of RVG-exosome technology, which is potentially suited for long-term silencing of genes related to neurodegenerative diseases. The exosome delivery system of Alvarez-Erviti et al. may be applied to deliver the CRISPR-Cas system of the present invention to therapeutic targets, especially neurodegenerative diseases. A dosage of about 100 to 1000 mg of CRISPR Cas encapsulated in about 100 to 1000 mg of RVG exosomes may be contemplated for the present invention.

El-Andaloussi et al. (Nature Protocols 7,2112-2126(2012)) discloses how exosomes derived from cultured cells can be harnessed for delivery of RNA in vitro and in vivo. This protocol first describes the generation of targeted exosomes through transfection of an expression vector, comprising an exosomal protein fused with a peptide ligand. Next, El-Andaloussi et al. explain how to purify and characterize exosomes from transfected cell supernatant. Next, El-Andaloussi et al. detail crucial steps for loading RNA into exosomes. Finally, El-Andaloussi et al. outline how to use exosomes to efficiently deliver RNA in vitro and in vivo in mouse brain. Examples of anticipated results in which exosome-mediated RNA delivery is evaluated by functional assays and imaging are also provided. The entire protocol takes ~3 weeks. Delivery or administration according to the invention may be performed using exosomes produced from self-derived dendritic cells. From the herein teachings, this can be employed in the practice of the invention.

In another embodiment, the plasma exosomes of Wahlgren et al. (Nucleic Acids Research, 2012, Vol. 40, No. 17 e130) are contemplated. Exosomes are nano-sized vesicles (30-90nm in size) produced by many cell types, including dendritic cells (DC), B cells, T cells, mast cells, epithelial cells and tumor cells. These vesicles are formed by inward budding of late endosomes and are then released to the extracellular environment upon fusion with the plasma membrane. Because exosomes naturally carry RNA between cells, this property may be useful in gene therapy, and from this disclosure can be employed in the practice of the instant invention.

Exosomes from plasma can be prepared by centrifugation of buffy coat at 900g for 20 min to isolate the plasma followed by harvesting cell supernatants, centrifuging at 300g for 10 min to eliminate cells and at 16 500g for 30 min followed by filtration through a 0.22 mm filter. Exosomes are pelleted by ultracentrifugation at 120 000g for70 min. Chemical transfection of siRNA into exosomes is carried out according to the manufacturer's instructions in RNAi Human/Mouse Starter Kit (Quiagen, Hilden, Germany). siRNA is added to 100 ml PBS at a final concentration of 2 mmol/ml. After adding HiPerFect transfection reagent, the mixture is incubated for 10 min at RT. In order to remove the excess of micelles, the exosomes are re-isolated using aldehyde/sulfate latex beads. The chemical transfection of CRISPR Cas into exosomes may be conducted similarly to siRNA. The exosomes may be co-cultured with monocytes and lymphocytes isolated from the peripheral blood of healthy donors. Therefore, it may be contemplated that exosomes containing CRISPR Cas may be introduced to monocytes and lymphocytes of and autologously reintroduced into a human. Accordingly, delivery or administration according to the invention may beperformed using plasma exosomes.

### Liposomes

Delivery or administration according to the invention can be performed with liposomes. Liposomes are spherical vesicle structures composed of a uni- or multilamellar lipid bilayer surrounding internal aqueous compartments and a relatively impermeable outer lipophilic phospholipid bilayer. Liposomes have gained considerable attention as drug delivery carriers because they are biocompatible, nontoxic, can deliver both hydrophilic and lipophilic drug molecules, protect their cargo from degradation by plasma enzymes, and transport their load across biological membranes and the blood brain barrier (BBB) (see, e.g., Spuch and Navarro, Journal of Drug Delivery, vol. 2011, Article ID 469679, 12 pages, 2011. doi:10.1155/2011/469679 for review).

Liposomes can be made from several different types of lipids; however, phospholipids are most commonly used to generate liposomes as drug carriers. Although liposome formation is spontaneous when a lipid film is mixed with an aqueous solution, it can also be expedited by applying force in the form of shaking by using a homogenizer, sonicator, or an extrusion apparatus (see, e.g., Spuch and Navarro, Journal of Drug Delivery, vol. 2011, Article ID 469679, 12 pages, 2011. doi:10.1155/2011/469679 for review).

Several other additives may be added to liposomes in order to modify their structure and properties. For instance, either cholesterol or sphingomyelin may be added to the liposomal mixture in order to help stabilize the liposomal structure and to prevent the leakage of the liposomal inner cargo. Further, liposomes are prepared from hydrogenated egg phosphatidylcholine or egg phosphatidylcholine, cholesterol, and dicetyl phosphate, and their mean vesicle sizes were adjusted to about 50 and 100 nm. (see, e.g., Spuch and Navarro, Journal of Drug Delivery, vol. 2011, Article ID 469679, 12 pages, 2011. doi:10.1155/2011/469679 for review).

A liposome formulation may be mainly comprised of natural phospholipids and lipids such as 1,2-distearoryl-sn-glycero-3-phosphatidyl choline (DSPC), sphingomyelin, egg phosphatidylcholines and monosialoganglioside. Since this formulation is made up of phospholipids only, liposomal formulations have encountered many challenges, one of the ones being the instability in plasma. Several attempts to overcome these challenges have been made, specifically in the manipulation of the lipid membrane. One of these attempts focused on the manipulation of cholesterol. Addition of cholesterol to conventional formulations reduces rapid release of the encapsulated bioactive compound into the plasma or 1,2-dioleoyl-sn-glycero-3-phosphoethanolamine (DOPE) increases the stability (see, e.g., Spuch and Navarro, Journal of Drug Delivery, vol. 2011, Article ID 469679, 12 pages, 2011. doi: 10.1155/2011/469679 for review).

In a particularly advantageous embodiment, Trojan Horse liposomes (also known as Molecular Trojan Horses) are desirable and protocols may be found at cshprotocols.cshlp.org/content/2010/4/pdb.prot5407.long. These particles allow delivery of a transgene to the entire brain after an intravascular injection. Without being bound by limitation, it is believed that neutral lipid particles with specific antibodies conjugated to surface allow crossing of the blood brain barrier via endocytosis. Applicant postulates utilizing Trojan Horse Liposomes to deliver the CRISPR family of nucleases to the brain via an intravascular injection, which would allow whole brain transgenic animals without the need for embryonic manipulation. About 1-5 g of DNA or RNA may be contemplated for in vivo administration in liposomes.

In another embodiment, the the chromatin 3D structure modulating agents such as the CRISPR Cas system may be administered in liposomes, such as a stable nucleic-acid-lipid particle (SNALP) (see, e.g., Morrissey et al., Nature Biotechnology, Vol. 23, No. 8, August 2005). Daily intravenous injections of about 1, 3 or 5 mg/kg/day of a specific CRISPR Cas targeted in a SNALP are contemplated. The daily treatment may be over about three days and then weekly for about five weeks. In another embodiment, a specific CRISPR Cas encapsulated SNALP) administered by intravenous injection to at doses of about 1 or 2.5 mg/kg are also contemplated (see, e.g., Zimmerman et al., Nature Letters, Vol. 441, 4 May 2006). The SNALP formulation may contain the lipids 3-N-[(wmethoxypoly(ethylene glycol) 2000) carbamoyl] - 1,2-dimyristyloxy-propylamine (PEG-C-DMA), 1,2-dilinoleyloxy-N,N-dimethyl-3-aminopropane (DLinDMA), 1,2-distearoyl-sn-glycero-3-phosphocholine (DSPC) and cholesterol, in a 2:40:10:48 molar per cent ratio (see, e.g., Zimmerman et al., Nature Letters, Vol. 441, 4 May 2006).

In another embodiment, stable nucleic-acid-lipid particles (SNALPs) have proven to be effective delivery molecules to highly vascularized HepG2-derived liver tumors but not in poorly vascularized HCT-116 derived liver tumors (see, e.g., Li, Gene Therapy (2012) 19, 775-780). The SNALP liposomes may be prepared by formulating D-Lin-DMA and PEG-C-DMA with distearoylphosphatidylcholine (DSPC), Cholesterol and siRNA using a 25:1 lipid/siRNA ratio and a 48/40/10/2 molar ratio of Cholesterol/D-Lin-DMA/DSPC/PEG-C-DMA. The resulted SNALP liposomes are about 80-100 nm in size.

In yet another embodiment, a SNALP may comprise synthetic cholesterol (Sigma-Aldrich, St Louis, MO, USA), dipalmitoylphosphatidylcholine (Avanti Polar Lipids, Alabaster, AL, USA), 3-N-[(w-methoxy poly(ethylene glycol)2000)carbamoyl]-1,2-dimyrestyloxypropylamine, and cationic 1,2-dilinoleyloxy-3-N,Ndimethylaminopropane (see, e.g., Geisbert et al., Lancet 2010; 375: 1896-905). A dosage of about 2 mg/kg total CRISPR Cas per dose administered as, for example, a bolus intravenous infusion may be contemplated.

In yet another embodiment, a SNALP may comprise synthetic cholesterol (Sigma-Aldrich), 1,2-distearoyl-sn-glycero-3-phosphocholine (DSPC; Avanti Polar Lipids Inc.), PEG-cDMA, and 1,2-dilinoleyloxy-3-(N;N-dimethyl)aminopropane (DLinDMA) (see, e.g., Judge, J. Clin. Invest. 119:661-673 (2009)). Formulations used for in vivo studies may comprise a final lipid/RNA mass ratio of about 9:1.

The safety profile of RNAi nanomedicines has been reviewed by Barros and Gollob of Alnylam Pharmaceuticals (see, e.g., Advanced Drug Delivery Reviews 64 (2012) 1730-1737). The stable nucleic acid lipid particle (SNALP) is comprised of four different lipids - an ionizable lipid (DLinDMA) that is cationic at low pH, a neutral helper lipid, cholesterol, and a diffusible polyethylene glycol (PEG)-lipid. The particle is approximately 80 nm in diameter and is charge-neutral at physiologic pH. During formulation, the ionizable lipid serves to condense lipid with the anionic RNA during particle formation. When positively charged under increasingly acidic endosomal conditions, the ionizable lipid also mediates the fusion of SNALP with the endosomal membrane enabling release of RNA into the cytoplasm. The PEG-lipid stabilizes the particle and reduces aggregation during formulation, and subsequently provides a neutral hydrophilic exterior that improves pharmacokinetic properties.

To date, two clinical programs have been initiated using SNALP formulations with RNA. Tekmira Pharmaceuticals recently completed a phase I single-dose study of SNALP-ApoB in adult volunteers with elevated LDL cholesterol. ApoB is predominantly expressed in the liver and jejunum and is essential for the assembly and secretion of VLDL and LDL. Seventeen subjects received a single dose of SNALP-ApoB (dose escalation across 7 dose levels). There was no evidence of liver toxicity (anticipated as the potential dose-limiting toxicity based on preclinical studies). One (of two) subjects at the highest dose experienced flu-like symptoms consistent with immune system stimulation, and the decision was made to conclude the trial.

Alnylam Pharmaceuticals has similarly advanced ALN-TTR01, which employs the SNALP technology described above and targets hepatocyte production of both mutant and wildtype TTR to treat TTR amyloidosis (ATTR). Three ATTR syndromes have been described: familial amyloidotic polyneuropathy (FAP) and familial amyloidotic cardiomyopathy (FAC) - both caused by autosomal dominant mutations in TTR; and senile systemic amyloidosis (SSA) cause by wildtype TTR. A placebo-controlled, single dose-escalation phase I trial of ALN-TTR01 was recently completed in patients with ATTR. ALN-TTR01 was administered as a 15-minute IV infusion to 31 patients (23 with study drug and 8 with placebo) within a dose range of 0.01 to 1.0 mg/kg (based on siRNA). Treatment was well tolerated with no significant increases in liver function tests. Infusion-related reactions were noted in 3 of 23 patients at≥0.4 mg/kg; all responded to slowing of the infusion rate and all continued on study. Minimal and transient elevations of serum cytokines IL-6, IP-10 and IL-1ra were noted in two patients at the highest dose of 1 mg/kg (as anticipated from preclinical and NHP studies). Lowering of serum TTR, the expected pharmacodynamics effect of ALN-TTR01, was observed at 1 mg/kg.

In yet another embodiment, a SNALP may be made by solubilizing a cationic lipid, DSPC, cholesterol and PEG-lipid e.g., in ethanol, e.g., at a molar ratio of 40:10:40:10, respectively (see, Semple et al., Nature Niotechnology, Volume 28 Number 2 February 2010, pp. 172-177). The lipid mixture was added to an aqueous buffer (50 mM citrate, pH 4) with mixing to a final ethanol and lipid concentration of 30% (vol/vol) and 6.1 mg/ml, respectively, and allowed to equilibrate at 22 °C for 2 min before extrusion. The hydrated lipids were extruded through two stacked 80 nm pore-sized filters (Nuclepore) at 22 °C using a Lipex Extruder (Northern Lipids) until a vesicle diameter of 70-90 nm, as determined by dynamic light scattering analysis, was obtained. This generally required 1-3 passes. The siRNA (solubilized in a 50 mM citrate, pH 4 aqueous solution containing 30% ethanol) was added to the pre-equilibrated (35 °C) vesicles at a rate of ~5 ml/min with mixing. After a final target siRNA/lipid ratio of 0.06 (wt/wt) was reached, the mixture was incubated for a further 30 min at 35 °C to allow vesicle reorganization and encapsulation of the siRNA. The ethanol was then removed and the external buffer replaced with PBS (155 mM NaCl, 3 mM Na2HPO4, 1 mM KH2PO4, pH 7.5) by either dialysis or tangential flow diafiltration. siRNA were encapsulated in SNALP using a controlled step-wise dilution method process. The lipid constituents of KC2-SNALP were DLin-KC2-DMA (cationic lipid), dipalmitoylphosphatidylcholine (DPPC; Avanti Polar Lipids), synthetic cholesterol (Sigma) and PEG-C-DMA used at a molar ratio of 57.1:7.1:34.3:1.4. Upon formation of the loaded particles, SNALP were dialyzed against PBS and filter sterilized through a 0.2 µm filter before use. Mean particle sizes were 75-85 nm and 90-95% of the siRNA was encapsulated within the lipid particles. The final siRNA/lipid ratio in formulations used for in vivo testing was ~0.15 (wt/wt). LNP-siRNA systems containing Factor VII siRNA were diluted to the appropriate concentrations in sterile PBS immediately before use and the formulations were administered intravenously through the lateral tail vein in a total volume of 10 ml/kg. This method and these delivery systems may be extrapolated to the CRISPR Cas system of the present invention.

### Other Lipids

Other cationic lipids, such as amino lipid 2,2-dilinoleyl-4-dimethylaminoethyl-[1,3]-dioxolane (DLin-KC2-DMA) may be utilized to encapsulate the chromatin 3D structure modulating agents such as CRISPR Cas or components thereof or nucleic acid molecule(s) coding therefor e.g., similar to SiRNA (see, e.g., Jayaraman, Angew. Chem. Int. Ed. 2012, 51, 8529 -8533), and hence may be employed in the practice of the invention. A preformed vesicle with the following lipid composition may be contemplated: amino lipid, distearoylphosphatidylcholine (DSPC), cholesterol and (R)-2,3-bis(octadecyloxy) propyl-1-(methoxy poly(ethylene glycol)2000)propylcarbamate (PEG-lipid) in the molar ratio 40/10/40/10, respectively, and a FVII siRNA/total lipid ratio of approximately 0.05 (w/w). To ensure a narrow particle size distribution in the range of 70-90 nm and a low polydispersity index of 0.11+0.04 (n=56), the particles may be extruded up to three times through 80 nm membranes prior to adding the CRISPR Cas RNA. Particles containing the highly potent amino lipid 16 may be used, in which the molar ratio of the four lipid components 16, DSPC, cholesterol and PEG-lipid (50/10/38.5/1.5) which may be further optimized to enhance in vivo activity.

Michael S D Kormann et al. ("Expression of therapeutic proteins after delivery of chemically modified mRNA in mice: Nature Biotechnology, Volume:29, Pages: 154-157 (2011)) describes the use of lipid envelopes to deliver RNA. Use of lipid envelopes is also preferred in the present invention.

In another embodiment, lipids may be formulated with the the chromatin 3D structure modulating agents, such as the CRISPR Cas system, of the present invention to form lipid particles (LNPs). Lipids include, but are not limited to, DLin-KC2-DMA4, C12-200 and colipids disteroylphosphatidyl choline, cholesterol, and PEG-DMG may be formulated with CRISPR Cas instead of siRNA (see, e.g., Novobrantseva, Molecular Therapy-Nucleic Acids (2012) 1, e4; doi:10.1038/mtna.2011.3) using a spontaneous vesicle formation procedure. The component molar ratio may be about 50/10/38.5/1.5 (DLin-KC2-DMA or C12-200/disteroylphosphatidyl choline/cholesterol/PEG-DMG). The final lipid:siRNA weight ratio may be ~12:1 and 9:1 in the case of DLin-KC2-DMA and C12-200 lipid particles (LNPs), respectively. The formulations may have mean particle diameters of ~80 nm with >90% entrapment efficiency. A 3 mg/kg dose may be contemplated.

Tekmira has a portfolio of approximately 95 patent families, in the U.S. and abroad, that are directed to various aspects of LNPs and LNP formulations (see, e.g., U.S. Pat. Nos. 7,982,027; 7,799,565; 8,058,069; 8,283,333; 7,901,708; 7,745,651; 7,803,397; 8,101,741; 8,188,263; 7,915,399; 8,236,943 and 7,838,658 and European Pat. Nos 1766035; 1519714; 1781593 and 1664316), all of which may be used and/or adapted to the present invention.

The the chromatin 3D structure modulating agents such as the CRISPR Cas system or components thereof or nucleic acid molecule(s) coding therefor may be delivered encapsulated in PLGA Microspheres such as that further described in US published applications 20130252281 and 20130245107 and 20130244279 (assigned to Moderna Therapeutics) which relate to aspects of formulation of compositions comprising modified nucleic acid molecules which may encode a protein, a protein precursor, or a partially or fully processed form of the protein or a protein precursor. The formulation may have a molar ratio 50:10:38.5:1.5-3.0 (cationic lipid:fusogenic lipid:cholesterol:PEG lipid). The PEG lipid may be selected from, but is not limited to PEG-c-DOMG, PEG-DMG. The fusogenic lipid may be DSPC. See also, Schrum et al., Delivery and Formulation of Engineered Nucleic Acids, US published application 20120251618.

Nanomerics' technology addresses bioavailability challenges for a broad range of therapeutics, including low molecular weight hydrophobic drugs, peptides, and nucleic acid based therapeutics (plasmid, siRNA, miRNA). Specific administration routes for which the technology has demonstrated clear advantages include the oral route, transport across the blood-brain-barrier, delivery to solid tumours, as well as to the eye. See, e.g., Mazza et al., 2013, ACS Nano. 2013 Feb 26;7(2):1016-26; Uchegbu and Siew, 2013, J Pharm Sci. 102(2):305-10 and Lalatsa et al., 2012, J Control Release. 2012 Jul 20; 161(2):523-36.

US Patent Publication No. 20050019923 describes cationic dendrimers for delivering bioactive molecules, such as polynucleotide molecules, peptides and polypeptides and/or pharmaceutical agents, to a mammalian body. The dendrimers are suitable for targeting the delivery of the bioactive molecules to, for example, the liver, spleen, lung, kidney or heart (or even the brain). Dendrimers are synthetic 3-dimensional macromolecules that are prepared in a step-wise fashion from simple branched monomer units, the nature and functionality of which can be easily controlled and varied. Dendrimers are synthesised from the repeated addition of building blocks to a multifunctional core (divergent approach to synthesis), or towards a multifunctional core (convergent approach to synthesis) and each addition of a 3-dimensional shell of building blocks leads to the formation of a higher generation of the dendrimers. Polypropylenimine dendrimers start from a diaminobutane core to which is added twice the number of amino groups by a double Michael addition of acrylonitrile to the primary amines followed by the hydrogenation of the nitriles. This results in a doubling of the amino groups. Polypropylenimine dendrimers contain 100% protonable nitrogens and up to 64 terminal amino groups (generation 5, DAB 64). Protonable groups are usually amine groups which are able to accept protons at neutral pH. The use of dendrimers as gene delivery agents has largely focused on the use of the polyamidoamine. and phosphorous containing compounds with a mixture of amine/amide or N--P(O2)S as the conjugating units respectively with no work being reported on the use of the lower generation polypropylenimine dendrimers for gene delivery. Polypropylenimine dendrimers have also been studied as pH sensitive controlled release systems for drug delivery and for their encapsulation of guest molecules when chemically modified by peripheral amino acid groups. The cytotoxicity and interaction of polypropylenimine dendrimers with DNA as well as the transfection efficacy of DAB 64 has also been studied.

US Patent Publication No. 20050019923 is based upon the observation that, contrary to earlier reports, cationic dendrimers, such as polypropylenimine dendrimers, display suitable properties, such as specific targeting and low toxicity, for use in the targeted delivery of bioactive molecules, such as genetic material. In addition, derivatives of the cationic dendrimer also display suitable properties for the targeted delivery of bioactive molecules. See also, Bioactive Polymers, US published application 20080267903, which discloses "Various polymers, including cationic polyamine polymers and dendrimeric polymers, are shown to possess antiproliferative activity, and may therefore be useful for treatment of disorders characterised by undesirable cellular proliferation such as neoplasms and tumours, inflammatory disorders (including autoimmune disorders), psoriasis and atherosclerosis. The polymers may be used alone as active agents, or as delivery vehicles for other therapeutic agents, such as drug molecules or nucleic acids for gene therapy. In such cases, the polymers' own intrinsic antitumour activity may complement the activity of the agent to be delivered." The disclosures of these patent publications may be employed in conjunction with herein teachings for delivery of CRISPR Cas system(s) or component(s) thereof or nucleic acid molecule(s) coding therefor.

### Supercharged proteins

Supercharged proteins are a class of engineered or naturally occurring proteins with unusually high positive or negative net theoretical charge and may be employed in delivery of the chromatin 3D structure modulating agents, such as the CRISPR Cas system(s) or component(s) thereof or nucleic acid molecule(s) coding therefor. Both supernegatively and superpositively charged proteins exhibit a remarkable ability to withstand thermally or chemically induced aggregation. Superpositively charged proteins are also able to penetrate mammalian cells. Associating cargo with these proteins, such as plasmid DNA, RNA, or other proteins, can enable the functional delivery of these macromolecules into mammalian cells both in vitro and in vivo. David Liu's lab reported the creation and characterization of supercharged proteins in 2007 (Lawrence et al., 2007, Journal of the American Chemical Society 129, 10110-10112).

The nonviral delivery of RNA and plasmid DNA into mammalian cells are valuable both for research and therapeutic applications (Akinc et al., 2010, Nat. Biotech. 26, 561-569). Purified +36 GFP protein (or other superpositively charged protein) is mixed with RNAs in the appropriate serum-free media and allowed to complex prior addition to cells. Inclusion of serum at this stage inhibits formation of the supercharged protein-RNA complexes and reduces the effectiveness of the treatment. The following protocol has been found to be effective for a variety of cell lines (McNaughton et al., 2009, Proc. Natl. Acad. Sci. USA 106, 6111-6116) (However, pilot experiments varying the dose of protein and RNA should be performed to optimize the procedure for specific cell lines): (1) One day before treatment, plate 1 x 105 cells per well in a 48-well plate. (2) On the day of treatment, dilute purified +36 GFP protein in serumfree media to a final concentration 200nM. Add RNA to a final concentration of 50nM. Vortex to mix and incubate at room temperature for 10min. (3) During incubation, aspirate media from cells and wash once with PBS. (4) Following incubation of +36 GFP and RNA, add the protein-RNA complexes to cells. (5) Incubate cells with complexes at 37 °C for 4h. (6) Following incubation, aspirate the media and wash three times with 20 U/mL heparin PBS. Incubate cells with serum-containing media for a further 48h or longer depending upon the assay for activity. (7) Analyze cells by immunoblot, qPCR, phenotypic assay, or other appropriate method.

David Liu's lab has further found +36 GFP to be an effective plasmid delivery reagent in a range of cells. As plasmid DNA is a larger cargo than siRNA, proportionately more +36 GFP protein is required to effectively complex plasmids. For effective plasmid delivery Applicants have developed a variant of +36 GFP bearing a C-terminal HA2 peptide tag, a known endosome-disrupting peptide derived from the influenza virus hemagglutinin protein. The following protocol has been effective in a variety of cells, but as above it is advised that plasmid DNA and supercharged protein doses be optimized for specific cell lines and delivery applications: (1) One day before treatment, plate 1 x 105 per well in a 48-well plate. (2) On the day of treatment, dilute purified p36 GFP protein in serumfree media to a final concentration 2 mM. Add 1mg of plasmid DNA. Vortex to mix and incubate at room temperature for 10min. (3) During incubation, aspirate media from cells and wash once with PBS. (4) Following incubation of p36 GFP and plasmid DNA, gently add the protein-DNA complexes to cells. (5) Incubate cells with complexes at 37 C for 4h. (6) Following incubation, aspirate the media and wash with PBS. Incubate cells in serum-containing media and incubate for a further 24-48h. (7) Analyze plasmid delivery (e.g., by plasmid-driven gene expression) as appropriate. See also, e.g., McNaughton et al., Proc. Natl. Acad. Sci. USA 106, 6111-6116 (2009); Cronican et al., ACS Chemical Biology 5, 747-752 (2010); Cronican et al., Chemistry & Biology 18, 833-838 (2011); Thompson et al., Methods in Enzymology 503, 293-319 (2012); Thompson, D.B., et al., Chemistry & Biology 19 (7), 831-843 (2012). The methods of the super charged proteins may be used and/or adapted for delivery of the CRISPR Cas system of the present invention. These systems of Dr. Lui and documents herein in inconjunction with herein teachints can be employed in the delivery of CRISPR Cas system(s) or component(s) thereof or nucleic acid molecule(s) coding therefor.

### Cell Penetrating Peptides (CPPs)

In yet another embodiment, cell penetrating peptides (CPPs) are contemplated for the delivery of the the chromatin 3D structure modulating agents , such the CRISPR Cas system as described above. CPPs are short peptides that facilitate cellular uptake of various molecular cargo (from nanosize particles to small chemical molecules and large fragments of DNA). The term "cargo" as used herein includes but is not limited to the group consisting of therapeutic agents, diagnostic probes, peptides, nucleic acids, antisense oligonucleotides, plasmids, proteins, particles, liposomes, chromophores, small molecules and radioactive materials. In aspects of the invention, the cargo may also comprise any component of the CRISPR Cas system or the entire functional CRISPR Cas system. Aspects of the present invention further provide methods for delivering a desired cargo into a subject comprising: (a) preparing a complex comprising the cell penetrating peptide of the present invention and a desired cargo, and (b) orally, intraarticularly, intraperitoneally, intrathecally, intrarterially, intranasally, intraparenchymally, subcutaneously, intramuscularly, intravenously, dermally, intrarectally, or topically administering the complex to a subject. The cargo is associated with the peptides either through chemical linkage via covalent bonds or through non-covalent interactions.

The function of the CPPs are to deliver the cargo into cells, a process that commonly occurs through endocytosis with the cargo delivered to the endosomes of living mammalian cells. Cell-penetrating peptides are of different sizes, amino acid sequences, and charges but all CPPs have one distinct characteristic, which is the ability to translocate the plasma membrane and facilitate the delivery of various molecular cargoes to the cytoplasm or an organelle. CPP translocation may be classified into three main entry mechanisms: direct penetration in the membrane, endocytosis-mediated entry, and translocation through the formation of a transitory structure. CPPs have found numerous applications in medicine as drug delivery agents in the treatment of different diseases including cancer and virus inhibitors, as well as contrast agents for cell labeling. Examples of the latter include acting as a carrier for GFP, MRI contrast agents, or quantum dots. CPPs hold great potential as in vitro and in vivo delivery vectors for use in research and medicine. CPPs typically have an amino acid composition that either contains a high relative abundance of positively charged amino acids such as lysine or arginine or has sequences that contain an alternating pattern of polar/charged amino acids and non-polar, hydrophobic amino acids. These two types of structures are referred to as polycationic or amphipathic, respectively. A third class of CPPs are the hydrophobic peptides, containing only apolar residues, with low net charge or have hydrophobic amino acid groups that are crucial for cellular uptake. One of the initial CPPs discovered was the trans-activating transcriptional activator (Tat) from Human Immunodeficiency Virus 1 (HIV-1) which was found to be efficiently taken up from the surrounding media by numerous cell types in culture. Since then, the number of known CPPs has expanded considerably and small molecule synthetic analogues with more effective protein transduction properties have been generated. CPPs include but are not limited to Penetratin, Tat (48-60), Transportan, and (R-AhX-R4) (Ahx=aminohexanoyl).

US Patent 8,372,951, provides a CPP derived from eosinophil cationic protein (ECP) which exhibits highly cell-penetrating efficiency and low toxicity. Aspects of delivering the CPP with its cargo into a vertebrate subject are also provided. Further aspects of CPPs and their delivery are described in U. S. patents 8,575,305; 8;614,194 and 8,044,019.

### Implantable devices

In another embodiment, implantable devices are also contemplated for delivery of the the chromatin 3D structure modulating agents or nucleic acid molecule(s) coding therefor. For example, US Patent Publication 20110195123 discloses an implantable medical device which elutes a drug locally and in prolonged period is provided, including several types of such a device, the treatment modes of implementation and methods of implantation. The device comprising of polymeric substrate, such as a matrix for example, that is used as the device body, and drugs, and in some cases additional scaffolding materials, such as metals or additional polymers, and materials to enhance visibility and imaging. An implantable delivery device can be advantageous in providing release locally and over a prolonged period, where drug is released directly to the extracellular matrix (ECM) of the diseased area such as tumor, inflammation, degeneration or for symptomatic objectives, or to injured smooth muscle cells, or for prevention. One kind of drug is RNA, as disclosed above, and this system may be used/and or adapted to the the chromatin 3D structure modulating agents such as the CRISPR Cas systemenvisaged herein. The modes of implantation in some embodiments are existing implantation procedures that are developed and used today for other treatments, including brachytherapy and needle biopsy. In such cases the dimensions of the new implant described in this invention are similar to the original implant. Typically a few devices are implanted during the same treatment procedure.

As described in US Patent Publication 20110195123, there is provided a drug delivery implantable or insertable system, including systems applicable to a cavity such as the abdominal cavity and/or any other type of administration in which the drug delivery system is not anchored or attached, comprising a biostable and/or degradable and/or bioabsorbable polymeric substrate, which may for example optionally be a matrix. It should be noted that the term "insertion" also includes implantation. The drug delivery system is preferably implemented as a "Loder" as described in US Patent Publication 20110195123.

The polymer or plurality of polymers are biocompatible, incorporating an agent and/or plurality of agents, enabling the release of agent at a controlled rate, wherein the total volume of the polymeric substrate, such as a matrix for example, in some embodiments is optionally and preferably no greater than a maximum volume that permits a therapeutic level of the agent to be reached. As a non-limiting example, such a volume is preferably within the range of 0.1 m3 to 1000 mm3, as required by the volume for the agent load. The Loder may optionally be larger, for example when incorporated with a device whose size is determined by functionality, for example and without limitation, a knee joint, an intra-uterine or cervical ring and the like.

The drug delivery system (for delivering the composition) is designed in some embodiments to preferably employ degradable polymers, wherein the main release mechanism is bulk erosion; or in some embodiments, non degradable, or slowly degraded polymers are used, wherein the main release mechanism is diffusion rather than bulk erosion, so that the outer part functions as membrane, and its internal part functions as a drug reservoir, which practically is not affected by the surroundings for an extended period (for example from about a week to about a few months). Combinations of different polymers with different release mechanisms may also optionally be used. The concentration gradient at the surface is preferably maintained effectively constant during a significant period of the total drug releasing period, and therefore the diffusion rate is effectively constant (termed "zero mode" diffusion). By the term "constant" it is meant a diffusion rate that is preferably maintained above the lower threshold of therapeutic effectiveness, but which may still optionally feature an initial burst and/or may fluctuate, for example increasing and decreasing to a certain degree. The diffusion rate is preferably so maintained for a prolonged period, and it can be considered constant to a certain level to optimize the therapeutically effective period, for example the effective silencing period.

The drug delivery system optionally and preferably is designed to shield the nucleotide based therapeutic agent from degradation, whether chemical in nature or due to attack from enzymes and other factors in the body of the subject.

The drug delivery system as described in US Patent Publication 20110195123 is optionally associated with sensing and/or activation appliances that are operated at and/or after implantation of the device, by non and/or minimally invasive methods of activation and/or acceleration/deceleration, for example optionally including but not limited to thermal heating and cooling, laser beams, and ultrasonic, including focused ultrasound and/or RF (radiofrequency) methods or devices.

According to some embodiments of US Patent Publication 20110195123, the site for local delivery may optionally include target sites characterized by high abnormal proliferation of cells, and suppressed apoptosis, including tumors, active and or chronic inflammation and infection including autoimmune diseases states, degenerating tissue including muscle and nervous tissue, chronic pain, degenerative sites, and location of bone fractures and other wound locations for enhancement of regeneration of tissue, and injured cardiac, smooth and striated muscle.

The site for implantation of the composition, or target site, preferably features a radius, area and/or volume that is sufficiently small for targeted local delivery. For example, the target site optionally has a diameter in a range of from about 0.1 mm to about 5 cm.

The location of the target site is preferably selected for maximum therapeutic efficacy. For example, the composition of the drug delivery system (optionally with a device for implantation as described above) is optionally and preferably implanted within or in the proximity of a tumor environment, or the blood supply associated thereof.

For example the composition (optionally with the device) is optionally implanted within or in the proximity to pancreas, prostate, breast, liver, via the nipple, within the vascular system and so forth.

The target location is optionally selected from the group consisting of (as non-limiting examples only, as optionally any site within the body may be suitable for implanting a Loder): 1. brain at degenerative sites like in Parkinson or Alzheimer disease at the basal ganglia, white and gray matter; 2. spine as in the case of amyotrophic lateral sclerosis (ALS); 3. uterine cervix to prevent HPV infection; 4. active and chronic inflammatory joints; 5. dermis as in the case of psoriasis; 6. sympathetic and sensoric nervous sites for analgesic effect; 7. Intra osseous implantation; 8. acute and chronic infection sites; 9. Intra vaginal; 10. Inner ear--auditory system, labyrinth of the inner ear, vestibular system; 11. Intra tracheal; 12. Intra-cardiac; coronary, epicardiac; 13. urinary bladder; 14. biliary system; 15. parenchymal tissue including and not limited to the kidney, liver, spleen; 16. lymph nodes; 17. salivary glands; 18. dental gums; 19. Intra-articular (into joints); 20. Intra-ocular; 21. Brain tissue; 22. Brain ventricles; 23. Cavities, including abdominal cavity (for example but without limitation, for ovary cancer); 24. Intra esophageal and 25. Intra rectal.

Optionally insertion of the system (for example a device containing the composition) is associated with injection of material to the ECM at the target site and the vicinity of that site to affect local pH and/or temperature and/or other biological factors affecting the diffusion of the drug and/or drug kinetics in the ECM, of the target site and the vicinity of such a site.

Optionally, according to some embodiments, the release of said agent could be associated with sensing and/or activation appliances that are operated prior and/or at and/or after insertion, by non and/or minimally invasive and/or else methods of activation and/or acceleration/deceleration, including laser beam, radiation, thermal heating and cooling, and ultrasonic, including focused ultrasound and/or RF (radiofrequency) methods or devices, and chemical activators.

According to other embodiments of US Patent Publication 20110195123, the drug preferably comprises a RNA, for example for localized cancer cases in breast, pancreas, brain, kidney, bladder, lung, and prostate as described below. Although exemplified with RNAi, many drugs are applicable to be encapsulated in Loder, and can be used in association with this invention, as long as such drugs can be encapsulated with the Loder substrate, such as a matrix for example, and this system may be used and/or adapted to deliver the the chromatin 3D structure modulating agents such as the CRISPR Cas systems envisaged in the context of the present invention.

As another example of a specific application, neuro and muscular degenerative diseases develop due to abnormal gene expression. Local delivery of RNAs may have therapeutic properties for interfering with such abnormal gene expression. Local delivery of anti apoptotic, anti inflammatory and anti degenerative drugs including small drugs and macromolecules may also optionally be therapeutic. In such cases the Loder is applied for prolonged release at constant rate and/or through a dedicated device that is implanted separately. All of this may be used and/or adapted to the the chromatin 3D structure modulating agents of the present invention.

As yet another example of a specific application, psychiatric and cognitive disorders are treated with gene modifiers. Gene knockdown is a treatment option. Loders locally delivering agents to central nervous system sites are therapeutic options for psychiatric and cognitive disorders including but not limited to psychosis, bi-polar diseases, neurotic disorders and behavioral maladies. The Loders could also deliver locally drugs including small drugs and macromolecules upon implantation at specific brain sites. All of this may be used and/or adapted to the the chromatin 3D structure modulating agents of the present invention.

As another example of a specific application, silencing of innate and/or adaptive immune mediators at local sites enables the prevention of organ transplant rejection. Local delivery of RNAs and immunomodulating reagents with the Loder implanted into the transplanted organ and/or the implanted site renders local immune suppression by repelling immune cells such as CD8 activated against the transplanted organ. All of this may be used/and or adapted to the the chromatin 3D structure modulating agents of the present invention.

As another example of a specific application, vascular growth factors including VEGFs and angiogenin and others are essential for neovascularization. Local delivery of the factors, peptides, peptidomimetics, or suppressing their repressors is an important therapeutic modality; silencing the repressors and local delivery of the factors, peptides, macromolecules and small drugs stimulating angiogenesis with the Loder is therapeutic for peripheral, systemic and cardiac vascular disease.

The method of insertion, such as implantation, may optionally already be used for other types of tissue implantation and/or for insertions and/or for sampling tissues, optionally without modifications, or alternatively optionally only with non-major modifications in such methods. Such methods optionally include but are not limited to brachytherapy methods, biopsy, endoscopy with and/or without ultrasound, such as ERCP, stereotactic methods into the brain tissue, Laparoscopy, including implantation with a laparoscope into joints, abdominal organs, the bladder wall and body cavities.

Implantable device technology herein discussed can be employed with herein teachings and hence by this disclosure and the knowledge in the art, the chromatin 3D structure modulating agents or nucleic acid molecules thereof or encoding or providing components may be delivered via an implantable device.

One of the other major advances enabled by the methods disclosed herein, is de novo assembly genome. As shown in FIG. 10, the combination of the disclosed methods and high through put sequencing can be used to assemble genomes de novo. The image at top represents the correct assembly of human chromosome 20. At bottom is shown a de novo assembly of human chromosome 20 from 100kb fragments, created using data generated with the methods disclosed herein. With the exception of a few small inversions, the assembly is perfect. The maps allow the creation of de novo genome assemblies without the use of mate pair reads.

### Modifying Gene Expression and Disease Treatment

A method of the invention may be used to create a plant, an animal or cell that may be used to model and/or study genetic or epigenetic conditions of interest, such as a through a model of mutations of interest or a as a disease model. In certain embodiments, mutations present in a chromatin loop associated factor may be used in complementation assays as described further herein. As used herein, "disease" refers to a disease, disorder, or indication in a subject. For example, a method of the invention may be used to create an animal or cell that comprises a modification in one or more nucleic acid sequences associated with a disease, or a plant, animal or cell in which the expression of one or more nucleic acid sequences associated with a disease are altered. Such a nucleic acid sequence may encode a disease associated protein sequence or may be a disease associated control sequence. Accordingly, it is understood that in embodiments of the invention, a plant, subject, patient, organism or cell can be a non-human subject, patient, organism or cell. Thus, the invention provides a plant, animal or cell, produced by the present methods, or a progeny thereof. The progeny may be a clone of the produced plant or animal, or may result from sexual reproduction by crossing with other individuals of the same species to introgress further desirable traits into their offspring. The cell may be *in vivo* or *ex vivo* in the cases of multicellular organisms, particularly animals or plants. In the instance where the cell is in cultured, a cell line may be established if appropriate culturing conditions are met and preferably if the cell is suitably adapted for this purpose (for instance a stem cell). Bacterial cell lines produced by the invention are also envisaged. Hence, cell lines are also envisaged.

The terms "subject," "individual," and "patient" are used interchangeably herein to refer to a vertebrate, preferably a mammal, more preferably a human. Mammals include, but are not limited to, murines, simians, humans, farm animals, sport animals, and pets. Tissues, cells and their progeny of a biological entity obtained in vivo or cultured in vitro are also encompassed.

The terms "therapeutic agent", "therapeutic capable agent" or "treatment agent" are used interchangeably and refer to a molecule or compound that confers some beneficial effect upon administration to a subject. The beneficial effect includes enablement of diagnostic determinations; amelioration of a disease, symptom, disorder, or pathological condition; reducing or preventing the onset of a disease, symptom, disorder or condition; and generally counteracting a disease, symptom, disorder or pathological condition.

As used herein, "treatment" or "treating," or "palliating" or "ameliorating" are used interchangeably. These terms refer to an approach for obtaining beneficial or desired results including but not limited to a therapeutic benefit and/or a prophylactic benefit. By therapeutic benefit is meant any therapeutically relevant improvement in or effect on one or more diseases, conditions, or symptoms under treatment. For prophylactic benefit, the compositions may be administered to a subject at risk of developing a particular disease, condition, or symptom, or to a subject reporting one or more of the physiological symptoms of a disease, even though the disease, condition, or symptom may not have yet been manifested.

The term "effective amount" or "therapeutically effective amount" refers to the amount of an agent that is sufficient to effect beneficial or desired results. The therapeutically effective amount may vary depending upon one or more of: the subject and disease condition being treated, the weight and age of the subject, the severity of the disease condition, the manner of administration and the like, which can readily be determined by one of ordinary skill in the art. The term also applies to a dose that will provide an image for detection by any one of the imaging methods described herein. The specific dose may vary depending on one or more of: the particular agent chosen, the dosing regimen to be followed, whether it is administered in combination with other compounds, timing of administration, the tissue to be imaged, and the physical delivery system in which it is carried.

The practice of the present invention employs, unless otherwise indicated, conventional techniques of immunology, biochemistry, chemistry, molecular biology, microbiology, cell biology, genomics and recombinant DNA, which are within the skill of the art. See Sambrook, Fritsch and Maniatis, MOLECULAR CLONING: A LABORATORY MANUAL, 2nd edition (1989); CURRENT PROTOCOLS IN MOLECULAR BIOLOGY (F. M. Ausubel, et al. eds., (1987)); the series METHODS IN ENZYMOLOGY (Academic Press, Inc.): PCR 2: A PRACTICAL APPROACH (M.J. MacPherson, B.D. Hames and G.R. Taylor eds. (1995)), Harlow and Lane, eds. (1988) ANTIBODIES, A LABORATORY MANUAL, and ANIMAL CELL CULTURE (R.I. Freshney, ed. (1987)).

In some embodiments, the methods described herein are used to produce a non-human transgenic animal or transgenic plant having altered gene expression due to chromatin loop or domain modification. In some embodiments, the transgenic animal is a mammal, such as a mouse, rat, or rabbit. In certain embodiments, the organism or subject is a plant. In certain embodiments, the organism or subject or plant is algae. Methods for producing transgenic plants and animals are known in the art, and generally begin with a method of cell transfection, such as described herein. Transgenic animals are also provided, as are transgenic plants, especially crops and algae. The transgenic animal or plant may be useful in applications outside of providing a disease model. These may include food or feed production through expression of, for instance, higher protein, carbohydrate, nutrient or vitamins levels than would normally be seen in the wildtype. In this regard, transgenic plants, especially pulses and tubers, and animals, especially mammals such as livestock (cows, sheep, goats and pigs), but also poultry and edible insects, are preferred.

Transgenic algae or other plants such as rape may be particularly useful in the production of vegetable oils or biofuels such as alcohols (especially methanol and ethanol), for instance. These may be engineered to express or overexpress high levels of oil or alcohols for use in the oil or biofuel industries.

In one aspect, the invention provides for methods of modifying the expression of a target polynucleotide in a eukaryotic cell. In some embodiments, the method comprises allowing a CRISPR complex to bind to the target polynucleotide associated with chromatin extrusion and loop formation, to effect cleavage of said target polynucleotide thereby modifying the target polynucleotide, wherein the CRISPR complex comprises a CRISPR protein complexed with a guide sequence hybridized to a target sequence within said target polynucleotide.

In one aspect, the invention provides a method of modifying expression of a polynucleotide in a eukaryotic cell. In some embodiments, the method comprises allowing a CRISPR complex to bind to the polynucleotide, such as a CTCF binding motif, such that said binding results in chromatin loop or domain modification, thereby altering locus interaction and increased or decreased expression of a polynucleotide in said loop or domain; wherein the CRISPR complex comprises a CRISPR protein complexed with a guide RNA comprising a guide sequence hybridized to a target sequence within said polynucleotide,.

With recent advances in crop genomics, the ability to use the methods disclosed herein to perform efficient and cost effective gene editing and manipulation will allow the rapid selection and comparison of single and and multiplexed genetic manipulations to transform such genomes for improved production and enhanced traits. In this regard reference is made to US patents and publications: US Patent No. 6,603,061 - Agrobacterium-Mediated Plant Transformation Method; US Patent No. 7,868,149 - Plant Genome Sequences and Uses Thereof and US 2009/0100536 - Transgenic Plants with Enhanced Agronomic Traits. In the practice of the invention, the contents and disclosure of Morrell et al "Crop genomics:advances and applications" Nat Rev Genet. 2011 Dec 29;13(2):85-96. In an advantageous embodiment of the invention, the methods disclosed herein are used to engineer microalgae (Example 14). Accordingly, reference herein to animal cells may also apply, mutatis mutandis, to plant cells unless otherwise apparent.

In one aspect, the invention provides for methods of modifying a target polynucleotide in a eukaryotic cell, which may be in vivo, ex vivo or in vitro. In some embodiments, the method comprises sampling a cell or population of cells from a human or non-human animal or plant (including micro-algae), and modifying the cell or cells. Culturing may occur at any stage ex vivo. The cell or cells may even be re-introduced into the non-human animal or plant (including micro-algae). Methods in aspects of this invention may thus include ex vivo methods or in vitro methods.

In one aspect, the invention provides kits containing any one or more of the elements disclosed in the above methods and compositions. In some embodiments, the kit comprises a vector system and instructions for using the kit.. In some embodiments, the kit includes instructions in one or more languages, for example in more than one language.

In some embodiments, a kit comprises one or more reagents for use in a process utilizing one or more of the elements described herein. Reagents may be provided in any suitable container. For example, a kit may provide one or more reaction or storage buffers. Reagents may be provided in a form that is usable in a particular assay, or in a form that requires addition of one or more other components before use (e.g. in concentrate or lyophilized form). A buffer can be any buffer, including but not limited to a sodium carbonate buffer, a sodium bicarbonate buffer, a borate buffer, a Tris buffer, a MOPS buffer, a HEPES buffer, and combinations thereof. In some embodiments, the buffer is alkaline. In some embodiments, the buffer has a pH from about 7 to about 10. In some embodiments, the kit comprises one or more oligonucleotides corresponding to a guide sequence for insertion into a vector so as to operably link the guide sequence and a regulatory element. In some embodiments, the kit comprises a homologous recombination template polynucleotide.

In one aspect, the invention provides methods for using one or more elements of a CRISPR system. The CRISPR complex of the invention provides an effective means for modifying a chromatin looping. The CRISPR complex of the invention has a wide variety of utility including modifying (e.g., deleting, inserting, translocating, inactivating, activating) a target polynucleotide in a multiplicity of cell types. As such the CRISPR complex for use in the invention has a broad spectrum of applications in, e.g., gene therapy, drug screening, disease diagnosis, and prognosis. An exemplary CRISPR complex comprises a CRISPR enzyme complexed with a guide RNA comprising a guide sequence hybridized to a target sequence within the target polynucleotide.

The target polynucleotide in a chromatin loop or domain, envisaged to be modified by methods of this invention, by virtue of which the expression of said target polynucleotide is modified, can be any polynucleotide endogenous or exogenous to the eukaryotic cell. For example, the target polynucleotide can be a polynucleotide residing in the nucleus of the eukaryotic cell. The target polynucleotide can be a sequence coding a gene product (e.g., a protein) or a non-coding sequence (e.g., a regulatory polynucleotide or a junk DNA).

The target polynucleotide in a chromatin loop or domain, modified by methods of this invention, by virtue of which the expression of said target polynucleotide is modified, may include a number of disease-associated genes and polynucleotides as well as signaling biochemical pathway-associated genes and polynucleotides as listed in US provisional patent applications 61/736,527 and 61/748,427 having Broad reference BI-2011/008/WSGR Docket No. 44063-701.101and BI-2011/008/WSGR Docket No. 44063-701.102 respectively, both entitled SYSTEMS METHODS AND COMPOSITIONS FOR SEQUENCE MANIPULATION filed on December 12, 2012 and January 2, 2013, respectively.

Examples of target polynucleotides include a sequence associated with a signaling biochemical pathway, e.g., a signaling biochemical pathway-associated gene or polynucleotide. Examples of target polynucleotides include a disease associated gene or polynucleotide. A "disease-associated" gene or polynucleotide refers to any gene or polynucleotide which is yielding transcription or translation products at an abnormal level or in an abnormal form in cells derived from a disease-affected tissues compared with tissues or cells of a non disease control. It may be a gene that becomes expressed at an abnormally high level; it may be a gene that becomes expressed at an abnormally low level, where the altered expression correlates with the occurrence and/or progression of the disease. A disease-associated gene also refers to a gene possessing mutation(s) or genetic variation that is directly responsible or is in linkage disequilibrium with a gene(s) that is responsible for the etiology of a disease. The transcribed or translated products may be known or unknown, and may be at a normal or abnormal level.

Examples of disease-associated genes and polynucleotides are available from McKusick-Nathans Institute of Genetic Medicine, Johns Hopkins University (Baltimore, Md.) and National Center for Biotechnology Information, National Library of Medicine (Bethesda, Md.), available on the World Wide Web.

Examples of disease-associated genes and polynucleotides are listed in Tables A and B. Disease specific information is available from McKusick-Nathans Institute of Genetic Medicine, Johns Hopkins University (Baltimore, Md.) and National Center for Biotechnology Information, National Library of Medicine (Bethesda, Md.), available on the World Wide Web. Examples of signaling biochemical pathway-associated genes and polynucleotides are listed in Table C.

Mutations in these genes and pathways can result in production of improper proteins or proteins in improper amounts which affect function. Such genes, proteins and pathways may be the target polynucleotide of a CRISPR complex.

**Table A**

| DISEASE/DISORDER | GENES |
|---|---|
| Neoplasia | PTEN; ATM; ATR; EGFR; ERBB2; ERBB3; ERBB4; Notch1; Notch2; Notch3; Notch4; AKT; AKT2; AKT3; |
| | HIF; HIF1a; HIF3a; Met; HRG; Bcl2; PPAR alpha; PPAR gamma; WT1 (Wilms Tumor); FGF Receptor Family members (5 members: 1, 2, 3, 4, 5); CDKN2a; APC; RB (retinoblastoma); MEN1; VHL; BRCA1; BRCA2; AR (Androgen Receptor); TSG101; IGF; IGF Receptor; Igf1 (4 variants); Igf2 (3 variants); Igf 1 Receptor; Igf 2 Receptor; Bax; Bcl2; caspases family (9 members: 1, 2, 3, 4, 6, 7, 8, 9, 12); Kras; Apc |
| Age-related Macular Degeneration | Abcr; Ccl2; Cc2; cp (ceruloplasmin); Timp3; cathepsinD; Vldlr; Ccr2 |
| Schizophrenia Disorders | Neuregulin1 (Nrg1); Erb4 (receptor for Neuregulin); Complexin1 (Cplx1); Tphl Tryptophan hydroxylase; Tph2, Tryptophan hydroxylase 2; Neurexin 1; GSK3; GSK3a; GSK3b, 5-HTT (Slc6a4); COMT; DRD (Drdla); SLC6A3; DAOA; DTNBP1; Dao (Dao1) |
| Trinucleotide Repeat Disorders | HTT (Huntington's Dx); SBMA/SMAX1/AR (Kennedy's Dx); FXN/X25 (Friedrich's Ataxia); ATX3 (Machado-Joseph's Dx); ATXN1 and ATXN2 (spinocerebellar ataxias); DMPK (myotonic dystrophy); Atrophin-1 and Atn1 (DRPLA Dx); CBP (Creb-BP - global instability); VLDLR (Alzheimer's); Atxn7; Atxn10 |
| Fragile X Syndrome | FMR2; FXR1; FXR2; mGLUR5 |
| Secretase Related Disorders | APH-1 (alpha and beta); Presenilin (Psen1); nicastrin (Ncstn); PEN-2 |
| Others | Nos1; Parp1; Nat1; Nat2 |
| Prion - related disorders | Prp |
| ALS | SOD1; ALS2; STEX; FUS; TARDBP; VEGF (VEGF-a; VEGF-b; VEGF-c) |
| Drug addiction | Prkce (alcohol); Drd2; Drd4; ABAT (alcohol); GRIA2; Grm5; Grin1; Htrlb; Grin2a; Drd3; Pdyn; Gria1 (alcohol) |
| Autism | Mecp2; BZRAP1; MDGA2; Sema5A; Neurexin 1; Fragile X (FMR2 (AFF2); FXR1; FXR2; Mglur5) |
| Alzheimer's Disease | E1; CHIP; UCH; UBB; Tau; LRP; PICALM; Clusterin; |
| | PS1; SORL1; CR1; Vldlr; Uba1; Uba3; CHIP28 (Aqp1, Aquaporin 1); Uchl1; Uchl3; APP |
| Inflammation | IL-10; IL-1 (IL-1a; IL-1b); IL-13; IL-17 (IL-17a (CTLA8); IL-17b; IL-17c; IL-17d; IL-17f); II-23; Cx3cr1; ptpn22; TNFa; NOD2/CARD15 for IBD; IL-6; IL-12 (IL-12a; IL-12b); CTLA4; Cx3c11 |
| Parkinson's Disease | x-Synuclein; DJ-1; LRRK2; Parkin; PINK1 |

**Table B:**

| DISEASE/DISORDER | GENES |
|---|---|
| Blood and coagulation diseases and disorders | Anemia (CDAN1, CDA1, RPS19, DBA, PKLR, PK1, NT5C3, UMPH1, PSN1, RHAG, RH50A, NRAMP2, SPTB, ALAS2, ANH1, ASB, ABCB7, ABC7, ASAT); Bare lymphocyte syndrome (TAPBP, TPSN, TAP2, ABCB3, PSF2, RING11, MHC2TA, C2TA, RFX5, RFXAP, RFX5), Bleeding disorders (TBXA2R, P2RX1, P2X1); Factor H and factor H-like 1 (HF1, CFH, HUS); Factor V and factor VIII (MCFD2); Factor VII deficiency (F7); Factor X deficiency (F10); Factor XI deficiency (F11); Factor XII deficiency (F12, HAF); Factor XIIIA deficiency (F13A1, F13A); Factor XIIIB deficiency (F13B); Fanconi anemia (FANCA, FACA, FA1, FA, FAA, FAAP95, FAAP90, FLJ34064, FANCB, FANCC, FACC, BRCA2, FANCD1, FANCD2, FANCD, FACD, FAD, FANCE, FACE, FANCF, XRCC9, FANCG, BRIP1, BACH1, FANCJ, PHF9, FANCL, FANCM, KIAA1596); Hemophagocytic lymphohistiocytosis disorders (PRF1, HPLH2, UNC13D, MUNC13-4, HPLH3, HLH3, FHL3); Hemophilia A (F8, F8C, HEMA); Hemophilia B (F9, HEMB), Hemorrhagic disorders (PI, ATT, F5); Leukocyde deficiencies and disorders (ITGB2, CD18, LCAMB, LAD, EIF2B1, EIF2BA, EIF2B2, EIF2B3, EIF2B5, LVWM, CACH, CLE, EIF2B4); Sickle cell anemia (HBB); |
| Cell dysregulation and oncology diseases and disorders | Thalassemia (HBA2, HBB, HBD, LCRB, HBA1). B-cell non-Hodgkin lymphoma (BCL7A, BCL7); Leukemia (TAL1, TCL5, SCL, TAL2, FLT3, NBS1, NBS, ZNFN1A1, IK1, LYF1, HOXD4, HOX4B, BCR, CML, PHL, ALL, ARNT, KRAS2, RASK2, GMPS, AF10, ARHGEF12, LARG, KIAA0382, CALM, CLTH, CEBPA, CEBP, CHIC2, BTL, FLT3, KIT, PBT, LPP, NPM1, NUP214, D9S46E, CAN, CAIN, RUNX1, CBFA2, AML1, WHSC1L1, NSD3, FLT3, AF1Q, NPM1, NUMA1, ZNF145, PLZF, PML, MYL, STAT5B, AF10, CALM, CLTH, ARL11, ARLTS1, P2RX7, P2X7, BCR, CML, PHL, ALL, GRAF, NF1, VRNF, WSS, NFNS, PTPN11, PTP2C, SHP2, NS1, BCL2, CCND1, PRAD1, BCL1, TCRA, GATA1, GF1, ERYF1, NFE1, ABL1, NQO1, DIA4, NMOR1, NUP214, D9S46E, CAN, CAIN). |
| Inflammation and immune related diseases and disorders | AIDS (KIR3DL1, NKAT3, NKB1, AMB11, KIR3DS1, IFNG, CXCL12, SDF1); Autoimmune lymphoproliferative syndrome (TNFRSF6, APT1, FAS, CD95, ALPS1A); Combined immunodeficiency, (IL2RG, SCIDX1, SCIDX, IMD4); HIV-1 (CCL5, SCYA5, D17S136E, TCP228), HIV susceptibility or infection (IL10, CSIF, CMKBR2, CCR2, CMKBR5, CCCKR5 (CCR5)); Immunodeficiencies (CD3E, CD3G, AICDA, AID, HIGM2, TNFRSF5, CD40, UNG, DGU, HIGM4, TNFSF5, CD40LG, HIGM1, IGM, FOXP3, IPEX, AIID, XPID, PIDX, TNFRSF14B, TACI); Inflammation (IL-10, IL-1 (IL-1a, IL-1b), IL-13, IL-17 (IL-17a (CTLA8), IL-17b, IL-17c, IL-17d, IL-17f), II-23, Cx3cr1, ptpn22, TNFa, NOD2/CARD15 for IBD, IL-6, IL-12 (IL-12a, IL-12b), CTLA4, Cx3c11); Severe combined immunodeficiencies (SCIDs)(JAK3, JAKL, DCLRE1C, ARTEMIS, SCIDA, RAG1, RAG2, ADA, PTPRC, CD45, LCA, IL7R, CD3D, T3D, IL2RG, SCIDX1, SCIDX, IMD4). |
| Metabolic, liver, kidney and protein diseases and disorders | Amyloid neuropathy (TTR, PALB); Amyloidosis (APOA1, APP, AAA, CVAP, AD1, GSN, FGA, LYZ, TTR, PALB); Cirrhosis (KRT18, KRT8, CIRH1A, NAIC, TEX292, KIAA1988); Cystic fibrosis (CFTR, ABCC7, CF, MRP7); Glycogen storage diseases (SLC2A2, GLUT2, G6PC, G6PT, G6PT1, GAA, LAMP2, LAMPB, AGL, GDE, GBE1, GYS2, PYGL, PFKM); Hepatic adenoma, 142330 (TCF1, HNF1A, MODY3), Hepatic failure, early onset, and neurologic disorder (SCOD1, SCO1), Hepatic lipase deficiency (LIPC), Hepatoblastoma, cancer and carcinomas (CTNNB1, PDGFRL, PDGRL, PRLTS, AXIN1, AXIN, CTNNB1, TP53, P53, LFS1, IGF2R, MPRI, MET, CASP8, MCH5; Medullary cystic kidney disease (UMOD, HNFJ, FJHN, MCKD2, ADMCKD2); Phenylketonuria (PAH, PKU1, QDPR, DHPR, PTS); Polycystic kidney and hepatic disease (FCYT, PKHD1, ARPKD, PKD1, PKD2, PKD4, PKDTS, PRKCSH, G19P1, PCLD, SEC63). |
| Muscular / Skeletal diseases and disorders | Becker muscular dystrophy (DMD, BMD, MYF6), Duchenne Muscular Dystrophy (DMD, BMD); Emery-Dreifuss muscular dystrophy (LMNA, LMN1, EMD2, FPLD, CMD1A, HGPS, LGMD1B, LMNA, LMN1, EMD2, FPLD, CMD1A); Facioscapulohumeral muscular dystrophy (FSHMD1A, FSHD1A); Muscular dystrophy (FKRP, MDC1C, LGMD2I, LAMA2, LAMM, LARGE, KIAA0609, MDC1D, FCMD, TTID, MYOT, CAPN3, CANP3, DYSF, LGMD2B, SGCG, LGMD2C, DMDA1, SCG3, SGCA, ADL, DAG2, LGMD2D, DMDA2, SGCB, LGMD2E, SGCD, SGD, LGMD2F, CMD1L, TCAP, LGMD2G, CMD1N, TRIM32, HT2A, LGMD2H, FKRP, MDC1C, LGMD2I, TTN, CMD1G, TMD, LGMD2J, POMT1, CAV3, LGMD1C, SEPN1, SELN, RSMD1, PLEC1, PLTN, EBS1); Osteopetrosis (LRP5, BMND1, LRP7, LR3, OPPG, VBCH2, CLCN7, CLC7, OPTA2, |
| | OSTM1, GL, TCIRG1, TIRC7, OC116, OPTB1); Muscular atrophy (VAPB, VAPC, ALS8, SMN1, SMA1, SMA2, SMA3, SMA4, BSCL2, SPG17, GARS, SMAD1, CMT2D, HEXB, IGHMBP2, SMUBP2, CATF1, SMARD1). |
| Neurological and neuronal diseases and disorders | ALS (SOD1, ALS2, STEX, FUS, TARDBP, VEGF (VEGF-a, VEGF-b, VEGF-c); Alzheimer disease (APP, AAA, CVAP, AD1, APOE, AD2, PSEN2, AD4, STM2, APBB2, FE65L1, NOS3, PLAU, URK, ACE, DCP1, ACE1, MPO, PACIP1, PAXIP1L, PTIP, A2M, BLMH, BMH, PSEN1, AD3); Autism (Mecp2, BZRAP1, MDGA2, Sema5A, Neurexin 1, GLO1, MECP2, RTT, PPMX, MRX16, MRX79, NLGN3, NLGN4, KIAA1260, AUTSX2); Fragile X Syndrome (FMR2, FXR1, FXR2, mGLUR5); Huntington's disease and disease like disorders (HD, IT15, PRNP, PRIP, JPH3, JP3, HDL2, TBP, SCA17); Parkinson disease (NR4A2, NURR1, NOT, TINUR, SNCAIP, TBP, SCA17, SNCA, NACP, PARK1, PARK4, DJ1, PARK7, LRRK2, PARK8, PINK1, PARK6, UCHL1, PARK5, SNCA, NACP, PARK1, PARK4, PRKN, PARK2, PDJ, DBH, NDUFV2); Rett syndrome (MECP2, RTT, PPMX, MRX16, MRX79, CDKL5, STK9, MECP2, RTT, PPMX, MRX16, MRX79, x-Synuclein, DJ-1); Schizophrenia (Neuregulin1 (Nrg1), Erb4 (receptor for Neuregulin), Complexin1 (Cplx1), Tph1 Tryptophan hydroxylase, Tph2, Tryptophan hydroxylase 2, Neurexin 1, GSK3, GSK3a, GSK3b, 5-HTT (Slc6a4), COMT, DRD (Drdla), SLC6A3, DAOA, DTNBP1, Dao (Dao1)); Secretase Related Disorders (APH-1 (alpha and beta), Presenilin (Psen1), nicastrin, (Ncstn), PEN-2, Nos1, Parp1, Nat1, Nat2); Trinucleotide Repeat Disorders (HTT (Huntington's Dx), SBMA/SMAX1/AR (Kennedy's Dx), FXN/X25 (Friedrich's Ataxia), ATX3 (Machado- Joseph's Dx), ATXN1 and ATXN2 (spinocerebellar ataxias), DMPK |
| | (myotonic dystrophy), Atrophin-1 and Atn1 (DRPLA Dx), CBP (Creb-BP - global instability), VLDLR (Alzheimer's), Atxn7, Atxn10). |
| Occular diseases and disorders | Age-related macular degeneration (Abcr, Ccl2, Cc2, cp (ceruloplasmin), Timp3, cathepsinD, Vldlr, Ccr2); Cataract (CRYAA, CRYA1, CRYBB2, CRYB2, PITX3, BFSP2, CP49, CP47, CRYAA, CRYA1, PAX6, AN2, MGDA, CRYBA1, CRYB1, CRYGC, CRYG3, CCL, LIM2, MP19, CRYGD, CRYG4, BFSP2, CP49, CP47, HSF4, CTM, HSF4, CTM, MIP, AQP0, CRYAB, CRYA2, CTPP2, CRYBB1, CRYGD, CRYG4, CRYBB2, CRYB2, CRYGC, CRYG3, CCL, CRYAA, CRYA1, GJA8, CX50, CAE1, GJA3, CX46, CZP3, CAE3, CCM1, CAM, KRIT1); Corneal clouding and dystrophy (APOA1, TGFBI, CSD2, CDGG1, CSD, BIGH3, CDG2, TACSTD2, TROP2, M1S1, VSX1, RINX, PPCD, PPD, KTCN, COL8A2, FECD, PPCD2, PIP5K3, CFD); Cornea plana congenital (KERA, CNA2); Glaucoma (MYOC, TIGR, GLC1A, JOAG, GPOA, OPTN, GLC1E, FIP2, HYPL, NRP, CYP1B1, GLC3A, OPA1, NTG, NPG, CYP1B1, GLC3A); Leber congenital amaurosis (CRB1, RP12, CRX, CORD2, CRD, RPGRIP1, LCA6, CORD9, RPE65, RP20, AIPL1, LCA4, GUCY2D, GUC2D, LCA1, CORD6, RDH12, LCA3); Macular dystrophy (ELOVL4, ADMD, STGD2, STGD3, RDS, RP7, PRPH2, PRPH, AVMD, AOFMD, VMD2). |

**Table C:**

| CELLULAR FUNCTION | GENES |
|---|---|
| PI3K/AKT Signaling | PRKCE; ITGAM; ITGA5; IRAK1; PRKAA2; EIF2AK2; PTEN; EIF4E; PRKCZ; GRK6; MAPK1; TSC1; PLK1; AKT2; IKBKB; PIK3CA; CDK8; CDKN1B; NFKB2; BCL2; PIK3CB; PPP2R1A; MAPK8; BCL2L1; MAPK3; TSC2; ITGA1; KRAS; EIF4EBP1; RELA; PRKCD; |
| | NOS3; PRKAA1; MAPK9; CDK2; PPP2CA; PIM1; ITGB7; YWHAZ; ILK; TP53; RAF1; IKBKG; RELB; DYRK1A; CDKN1A; ITGB1; MAP2K2; JAK1; AKT1; JAK2; PIK3R1; CHUK; PDPK1; PPP2R5C; CTNNB 1; MAP2K1; NFKB 1; PAK3; ITGB3; CCND1; GSK3A; FRAP1; SFN; ITGA2; TTK; CSNK1A1; BRAF; GSK3B; AKT3; FOXO1; SGK; HSP90AA1; RPS6KB1 |
| ERK/MAPK Signaling | PRKCE; ITGAM; ITGA5; HSPB1; IRAK1; PRKAA2; EIF2AK2; RAC1; RAP1A; TLN1; EIF4E; ELK1; GRK6; MAPK1; RAC2; PLK1; AKT2; PIK3CA; CDK8; CREB1; PRKCI; PTK2; FOS; RPS6KA4; PIK3CB; PPP2R1A; PIK3C3; MAPK8; MAPK3; ITGA1; ETS1; KRAS; MYCN; EIF4EBP1; PPARG; PRKCD; PRKAA1; MAPK9; SRC; CDK2; PPP2CA; PIM1; PIK3C2A; ITGB7; YWHAZ; PPP1CC; KSR1; PXN; RAF1; FYN; DYRK1A; ITGB1; MAP2K2; PAK4; PIK3R1; STAT3; PPP2R5C; MAP2K1; PAK3; ITGB3; ESR1; ITGA2; MYC; TTK; CSNK1A1; CRKL; BRAF; ATF4; PRKCA; SRF; STAT1; SGK |
| Glucocorticoid Receptor Signaling | RAC1; TAF4B; EP300; SMAD2; TRAF6; PCAF; ELK1; PK1; SMAD3; AKT2; IKBKB; NCOR2; UBE2I; PIK3CA; CREB1; FOS; HSPA5; NFKB2; BCL2; MAP3K14; STAT5B; PIK3CB; PIK3C3; MAPK8; BCL2L1; MAPK3; TSC22D3; MAPK10; NRIP1; KRAS; MAPK13; RELA; STAT5A; MAPK9; NOS2A; PBX1; NR3C1; PIK3C2A; CDKN1C; TRAF2; SERPINE1; NCOA3; MAPK14; TNF; RAF1; IKBKG; MAP3K7; CREBBP; CDKN1A; MAP2K2; JAK1; IL8; NCOA2; AKT1; JAK2; PIK3R1; CHUK; STAT3; MAP2K1; NFKB1; TGFBR1; ESR1; SMAD4; CEBPB; JUN; AR; AKT3; CCL2; MMP1; STAT1; IL6; HSP90AA1 |
| Axonal Guidance Signaling | PRKCE; ITGAM; ROCK1; ITGA5; CXCR4; ADAM 12; IGF1; RAC1; RAP1A; EIF4E; PRKCZ; NRP1; NTRK2; |
| | ARHGEF7; SMO; ROCK2; MAPK1; PGF; RAC2; PTPN 11; GNAS; AKT2; PIK3CA; ERBB2; PRKCI; PTK2; CFL1; GNAQ; PIK3CB; CXCL12; PIK3C3; WNT11; PRKD1; GNB2L1; ABL1; MAPK3; ITGA1; KRAS; RHOA; PRKCD; PIK3C2A; ITGB7; GLI2; PXN; VASP; RAF1; FYN; ITGB1; MAP2K2; PAK4; ADAM 17; AKT1; PIK3R1; GLI1; WNT5A; ADAM10; MAP2K1; PAK3; ITGB3; CDC42; VEGFA; ITGA2; EPHA8; CRKL; RND1; GSK3B; AKT3; PRKCA |
| Ephrin Receptor Signaling | PRKCE; ITGAM; ROCK1; ITGA5; CXCR4; IRAK1; PRKAA2; EIF2AK2; RAC1; RAP1A; GRK6; ROCK2; MAPK1; PGF; RAC2; PTPN11; GNAS; PLK1; AKT2; DOK1; CDK8; CREB1; PTK2; CFL1; GNAQ; MAP3K14; CXCL12; MAPK8; GNB2L1; ABL1; MAPK3; ITGA1; KRAS; RHOA; PRKCD; PRKAA1; MAPK9; SRC; CDK2; PIM1; ITGB7; PXN; RAF1; FYN; DYRK1A; ITGB1; MAP2K2; PAK4; AKT1; JAK2; STAT3; ADAM10; MAP2K1; PAK3; ITGB3; CDC42; VEGFA; ITGA2; EPHA8; TTK; CSNK1A1; CRKL; BRAF; PTPN13; ATF4; AKT3; SGK |
| Actin Cytoskeleton SIgnaling | ACTN4; PRKCE; ITGAM; ROCK1; ITGA5; IRAK1; PRKAA2; EIF2AK2; RAC1; INS; ARHGEF7; GRK6; ROCK2; MAPK1; RAC2; PLK1; AKT2; PIK3CA; CDK8; PTK2; CFL1; PIK3CB; MYH9; DIAPH1; PIK3C3; MAPK8; F2R; MAPK3; SLC9Al; ITGA1; KRAS; RHOA; PRKCD; PRKAA1; MAPK9; CDK2; PIM1; PIK3C2A; ITGB7; PPP1CC; PXN; VIL2; RAF1; GSN; DYRK1A; ITGB1; MAP2K2; PAK4; PIP5K1A; PIK3R1; MAP2K1; PAK3; ITGB3; CDC42; APC; ITGA2; TTK; CSNK1A1; CRKL; BRAF; VAV3; SGK |
| Huntington's Disease Signaling | PRKCE; IGF1; EP300; RCOR1; PRKCZ; HDAC4; TGM2; MAPK1; CAPNS1; AKT2; EGFR; NCOR2; SP1; CAPN2; PIK3CA; HDAC5; CREB1; PRKCI; HSPA5; REST; |
| | GNAQ; PIK3CB; PIK3C3; MAPK8; IGF1R; PRKD1; GNB2L1; BCL2L1; CAPN1; MAPK3; CASP8; HDAC2; HDAC7A; PRKCD; HDAC11; MAPK9; HDAC9; PIK3C2A; HDAC3; TP53; CASP9; CREBBP; AKT1; PIK3R1; PDPK1; CASP1; APAF1; FRAP1; CASP2; JUN; BAX; ATF4; AKT3; PRKCA; CLTC; SGK; HDAC6; CASP3 |
| Apoptosis Signaling | PRKCE; ROCK1; BID; IRAK1; PRKAA2; EIF2AK2; BAK1; BIRC4; GRK6; MAPK1; CAPNS1; PLK1; AKT2; IKBKB; CAPN2; CDK8; FAS; NFKB2; BCL2; MAP3K14; MAPK8; BCL2L1; CAPN1; MAPK3; CASP8; KRAS; RELA; PRKCD; PRKAA1; MAPK9; CDK2; PIM1; TP53; TNF; RAF1; IKBKG; RELB; CASP9; DYRK1A; MAP2K2; CHUK; APAF1; MAP2K1; NFKB1; PAK3; LMNA; CASP2; BIRC2; TTK; CSNK1A1; BRAF; BAX; PRKCA; SGK; CASP3; BIRC3; PARP1 |
| B Cell Receptor Signaling | RAC1; PTEN; LYN; ELK1; MAPK1; RAC2; PTPN11; AKT2; IKBKB; PIK3CA; CREB1; SYK; NFKB2; CAMK2A; MAP3K14; PIK3CB; PIK3C3; MAPK8; BCL2L1; ABL1; MAPK3; ETS1; KRAS; MAPK13; RELA; PTPN6; MAPK9; EGR1; PIK3C2A; BTK; MAPK14; RAF1; IKBKG; RELB; MAP3K7; MAP2K2; AKT1; PIK3R1; CHUK; MAP2K1; NFKB1; CDC42; GSK3A; FRAP1; BCL6; BCL10; JUN; GSK3B; ATF4; AKT3; VAV3; RPS6KB1 |
| Leukocyte Extravasation Signaling | ACTN4; CD44; PRKCE; ITGAM; ROCK1; CXCR4; CYBA; RAC1; RAP1A; PRKCZ; ROCK2; RAC2; PTPN11; MMP14; PIK3CA; PRKCI; PTK2; PIK3CB; CXCL12; PIK3C3; MAPK8; PRKD1; ABL1; MAPK10; CYBB; MAPK13; RHOA; PRKCD; MAPK9; SRC; PIK3C2A; BTK; MAPK14; NOX1; PXN; VIL2; VASP; ITGB1; MAP2K2; CTNND1; PIK3R1; CTNNB1; CLDN1; CDC42; F11R; ITK; CRKL; VAV3; CTTN; |
| | PRKCA; MMP1; MMP9 |
| Integrin Signaling | ACTN4; ITGAM; ROCK1; ITGA5; RAC1; PTEN; RAP1A; TLN1; ARHGEF7; MAPK1; RAC2; CAPNS1; AKT2; CAPN2; PIK3CA; PTK2; PIK3CB; PIK3C3; MAPK8; CAV1; CAPN1; ABL1; MAPK3; ITGA1; KRAS; RHOA; SRC; PIK3C2A; ITGB7; PPP1CC; ILK; PXN; VASP; RAF1; FYN; ITGB1; MAP2K2; PAK4; AKT1; PIK3R1; TNK2; MAP2K1; PAK3; ITGB3; CDC42; RND3; ITGA2; CRKL; BRAF; GSK3B; AKT3 |
| Acute Phase Response Signaling | IRAK1; SOD2; MYD88; TRAF6; ELK1; MAPK1; PTPN11; AKT2; IKBKB; PIK3CA; FOS; NFKB2; MAP3K14; PIK3CB; MAPK8; RIPK1; MAPK3; IL6ST; KRAS; MAPK13; IL6R; RELA; SOCS1; MAPK9; FTL; NR3C1; TRAF2; SERPINE1; MAPK14; TNF; RAF1; PDK1; IKBKG; RELB; MAP3K7; MAP2K2; AKT1; JAK2; PIK3R1; CHUK; STAT3; MAP2K1; NFKB1; FRAP1; CEBPB; JUN; AKT3; IL1R1; IL6 |
| PTEN Signaling | ITGAM; ITGA5; RAC1; PTEN; PRKCZ; BCL2L11; MAPK1; RAC2; AKT2; EGFR; IKBKB; CBL; PIK3CA; CDKN1B; PTK2; NFKB2; BCL2; PIK3CB; BCL2L1; MAPK3; ITGA1; KRAS; ITGB7; ILK; PDGFRB; INSR; RAF1; IKBKG; CASP9; CDKN1A; ITGB1; MAP2K2; AKT1; PIK3R1; CHUK; PDGFRA; PDPK1; MAP2K1; NFKB1; ITGB3; CDC42; CCND1; GSK3A; ITGA2; GSK3B; AKT3; FOXO1; CASP3; RPS6KB1 |
| p53 Signaling | PTEN; EP300; BBC3; PCAF; FASN; BRCA1; GADD45A; BIRC5; AKT2; PIK3CA; CHEK1; TP53INP1; BCL2; PIK3CB; PIK3C3; MAPK8; THBS1; ATR; BCL2L1; E2F1; PMAIP1; CHEK2; TNFRSF10B; TP73; RB1; HDAC9; CDK2; PIK3C2A; MAPK14; TP53; LRDD; CDKN1A; HIPK2; AKT1; PIK3R1; RRM2B; APAF1; CTNNB1; SIRT1; CCND1; PRKDC; ATM; SFN; CDKN2A; JUN; SNAI2; GSK3B; BAX; AKT3 |
| Aryl Hydrocarbon Receptor Signaling | HSPB1; EP300; FASN; TGM2; RXRA; MAPK1; NQO1; NCOR2; SP1; ARNT; CDKN1B; FOS; CHEK1; SMARCA4; NFKB2; MAPK8; ALDH1A1; ATR; E2F1; MAPK3; NRIP1; CHEK2; RELA; TP73; GSTP1; RB1; SRC; CDK2; AHR; NFE2L2; NCOA3; TP53; TNF; CDKN1A; NCOA2; APAF1; NFKB1; CCND1; ATM; ESR1; CDKN2A; MYC; JUN; ESR2; BAX; IL6; CYP1B1; HSP90AA1 |
| Xenobiotic Metabolism Signaling | PRKCE; EP300; PRKCZ; RXRA; MAPK1; NQO1; NCOR2; PIK3CA; ARNT; PRKCI; NFKB2; CAMK2A; PIK3CB; PPP2R1A; PIK3C3; MAPK8; PRKD1; ALDH1A1; MAPK3; NRIP1; KRAS; MAPK13; PRKCD; GSTP1; MAPK9; NOS2A; ABCB1; AHR; PPP2CA; FTL; NFE2L2; PIK3C2A; PPARGC1A; MAPK14; TNF; RAF1; CREBBP; MAP2K2; PIK3R1; PPP2R5C; MAP2K1; NFKB1; KEAP1; PRKCA; EIF2AK3; IL6; CYP1B1; HSP90AA1 |
| SAPK/JNK Signaling | PRKCE; IRAK1; PRKAA2; EIF2AK2; RAC1; ELK1; GRK6; MAPK1; GADD45A; RAC2; PLK1; AKT2; PIK3CA; FADD; CDK8; PIK3CB; PIK3C3; MAPK8; RIPK1; GNB2L1; IRS1; MAPK3; MAPK10; DAXX; KRAS; PRKCD; PRKAA1; MAPK9; CDK2; PIM1; PIK3C2A; TRAF2; TP53; LCK; MAP3K7; DYRK1A; MAP2K2; PIK3R1; MAP2K1; PAK3; CDC42; JUN; TTK; CSNK1A1; CRKL; BRAF; SGK |
| PPAr/RXR Signaling | PRKAA2; EP300; INS; SMAD2; TRAF6; PPARA; FASN; RXRA; MAPK1; SMAD3; GNAS; IKBKB; NCOR2; ABCA1; GNAQ; NFKB2; MAP3K14; STAT5B; MAPK8; IRS1; MAPK3; KRAS; RELA; PRKAA1; PPARGC1A; NCOA3; MAPK14; INSR; RAF1; IKBKG; RELB; MAP3K7; CREBBP; MAP2K2; JAK2; CHUK; MAP2K1; NFKB1; TGFBR1; SMAD4; JUN; IL1R1; PRKCA; IL6; HSP90AA1; ADIPOQ |
| NF-KB Signaling | IRAK1; EIF2AK2; EP300; INS; MYD88; PRKCZ; TRAF6; TBK1; AKT2; EGFR; IKBKB; PIK3CA; BTRC; NFKB2; MAP3K14; PIK3CB; PIK3C3; MAPK8; RIPK1; HDAC2; KRAS; RELA; PIK3C2A; TRAF2; TLR4; PDGFRB; TNF; INSR; LCK; IKBKG; RELB; MAP3K7; CREBBP; AKT1; PIK3R1; CHUK; PDGFRA; NFKB1; TLR2; BCL10; GSK3B; AKT3; TNFAIP3; IL1R1 |
| Neuregulin Signaling | ERBB4; PRKCE; ITGAM; ITGA5; PTEN; PRKCZ; ELK1; MAPK1; PTPN11; AKT2; EGFR; ERBB2; PRKCI; CDKN1B; STAT5B; PRKD1; MAPK3; ITGA1; KRAS; PRKCD; STAT5A; SRC; ITGB7; RAF1; ITGB1; MAP2K2; ADAM17; AKT1; PIK3R1; PDPK1; MAP2K1; ITGB3; EREG; FRAP1; PSEN1; ITGA2; MYC; NRG1; CRKL; AKT3; PRKCA; HSP90AA1; RPS6KB1 |
| Wnt & Beta catenin Signaling | CD44; EP300; LRP6; DVL3; CSNK1E; GJA1; SMO; AKT2; PIN1; CDH1; BTRC; GNAQ; MARK2; PPP2R1A; WNT11; SRC; DKK1; PPP2CA; SOX6; SFRP2; ILK; LEF1; SOX9; TP53; MAP3K7; CREBBP; TCF7L2; AKT1; PPP2R5C; WNTSA; LRP5; CTNNB1; TGFBR1; CCND1; GSK3A; DVL1; APC; CDKN2A; MYC; CSNK1A1; GSK3B; AKT3; SOX2 |
| Insulin Receptor Signaling | PTEN; INS; EIF4E; PTPN1; PRKCZ; MAPK1; TSC1; PTPN11; AKT2; CBL; PIK3CA; PRKCI; PIK3CB; PIK3C3; MAPK8; IRS1; MAPK3; TSC2; KRAS; EIF4EBP1; SLC2A4; PIK3C2A; PPP1CC; INSR; RAF1; FYN; MAP2K2; JAK1; AKT1; JAK2; PIK3R1; PDPK1; MAP2K1; GSK3A; FRAP1; CRKL; GSK3B; AKT3; FOXO1; SGK; RPS6KB1 |
| IL-6 Signaling | HSPB1; TRAF6; MAPKAPK2; ELK1; MAPK1; PTPN11; IKBKB; FOS; NFKB2; MAP3K14; MAPK8; MAPK3; MAPK10; IL6ST; KRAS; MAPK13; IL6R; RELA; SOCS1; MAPK9; ABCB1; TRAF2; MAPK14; TNF; RAF1; IKBKG; RELB; MAP3K7; MAP2K2; IL8; JAK2; |
| | CHUK; STAT3; MAP2K1; NFKB1; CEBPB; JUN; IL1R1; SRF; IL6 |
| Hepatic Cholestasis | PRKCE; IRAK1; INS; MYD88; PRKCZ; TRAF6; PPARA; RXRA; IKBKB; PRKCI; NFKB2; MAP3K14; MAPK8; PRKD1; MAPK10; RELA; PRKCD; MAPK9; ABCB1; TRAF2; TLR4; TNF; INSR; IKBKG; RELB; MAP3K7; IL8; CHUK; NR1H2; TJP2; NFKB1; ESR1; SREBF1; FGFR4; JUN; IL1R1; PRKCA; IL6 |
| IGF-1 Signaling | IGF1; PRKCZ; ELK1; MAPK1; PTPN11; NEDD4; AKT2; PIK3CA; PRKCI; PTK2; FOS; PIK3CB; PIK3C3; MAPK8; IGF1R; IRS1; MAPK3; IGFBP7; KRAS; PIK3C2A; YWHAZ; PXN; RAF1; CASP9; MAP2K2; AKT1; PIK3R1; PDPK1; MAP2K1; IGFBP2; SFN; JUN; CYR61; AKT3; FOXO1; SRF; CTGF; RPS6KB1 |
| NRF2-mediated Oxidative Stress Response | PRKCE; EP300; SOD2; PRKCZ; MAPK1; SQSTM1; NQO1; PIK3CA; PRKCI; FOS; PIK3CB; PIK3C3; MAPK8; PRKD1; MAPK3; KRAS; PRKCD; GSTP1; MAPK9; FTL; NFE2L2; PIK3C2A; MAPK14; RAF1; MAP3K7; CREBBP; MAP2K2; AKT1; PIK3R1; MAP2K1; PPIB; JUN; KEAP1; GSK3B; ATF4; PRKCA; EIF2AK3; HSP90AA1 |
| Hepatic Fibrosis/Hepatic Stellate Cell Activation | EDN1; IGF1; KDR; FLT1; SMAD2; FGFR1; MET; PGF; SMAD3; EGFR; FAS; CSF1; NFKB2; BCL2; MYH9; IGF1R; IL6R; RELA; TLR4; PDGFRB; TNF; RELB; IL8; PDGFRA; NFKB1; TGFBR1; SMAD4; VEGFA; BAX; IL1R1; CCL2; HGF; MMP1; STAT1; IL6; CTGF; MMP9 |
| PPAR Signaling | EP300; INS; TRAF6; PPARA; RXRA; MAPK1; IKBKB; NCOR2; FOS; NFKB2; MAP3K14; STAT5B; MAPK3; NRIP1; KRAS; PPARG; RELA; STAT5A; TRAF2; PPARGC1A; PDGFRB; TNF; INSR; RAF1; IKBKG; RELB; MAP3K7; CREBBP; MAP2K2; CHUK; PDGFRA; MAP2K1; NFKB1; JUN; IL1R1; HSP90AA1 |
| Fc Epsilon RI Signaling | PRKCE; RAC1; PRKCZ; LYN; MAPK1; RAC2; PTPN11; |
| | AKT2; PIK3CA; SYK; PRKCI; PIK3CB; PIK3C3; MAPK8; PRKD1; MAPK3; MAPK10; KRAS; MAPK13; PRKCD; MAPK9; PIK3C2A; BTK; MAPK14; TNF; RAF1; FYN; MAP2K2; AKT1; PIK3R1; PDPK1; MAP2K1; AKT3; VAV3; PRKCA |
| G-Protein Coupled Receptor Signaling | PRKCE; RAP1A; RGS16; MAPK1; GNAS; AKT2; IKBKB; PIK3CA; CREB1; GNAQ; NFKB2; CAMK2A; PIK3CB; PIK3C3; MAPK3; KRAS; RELA; SRC; PIK3C2A; RAF1; IKBKG; RELB; FYN; MAP2K2; AKT1; PIK3R1; CHUK; PDPK1; STAT3; MAP2K1; NFKB1; BRAF; ATF4; AKT3; PRKCA |
| Inositol Phosphate Metabolism | PRKCE; IRAK1; PRKAA2; EIF2AK2; PTEN; GRK6; MAPK1; PLK1; AKT2; PIK3CA; CDK8; PIK3CB; PIK3C3; MAPK8; MAPK3; PRKCD; PRKAA1; MAPK9; CDK2; PIM1; PIK3C2A; DYRK1A; MAP2K2; PIP5K1A; PIK3R1; MAP2K1; PAK3; ATM; TTK; CSNK1A1; BRAF; SGK |
| PDGF Signaling | EIF2AK2; ELK1; ABL2; MAPK1; PIK3CA; FOS; PIK3CB; PIK3C3; MAPK8; CAV1; ABL1; MAPK3; KRAS; SRC; PIK3C2A; PDGFRB; RAF1; MAP2K2; JAK1; JAK2; PIK3R1; PDGFRA; STAT3; SPHK1; MAP2K1; MYC; JUN; CRKL; PRKCA; SRF; STAT1; SPHK2 |
| VEGF Signaling | ACTN4; ROCK1; KDR; FLT1; ROCK2; MAPK1; PGF; AKT2; PIK3CA; ARNT; PTK2; BCL2; PIK3CB; PIK3C3; BCL2L1; MAPK3; KRAS; HIF1A; NOS3; PIK3C2A; PXN; RAF1; MAP2K2; ELAVL1; AKT1; PIK3R1; MAP2K1; SFN; VEGFA; AKT3; FOXO1; PRKCA |
| Natural Killer Cell Signaling | PRKCE; RAC1; PRKCZ; MAPK1; RAC2; PTPN11; KIR2DL3; AKT2; PIK3CA; SYK; PRKCI; PIK3CB; PIK3C3; PRKD1; MAPK3; KRAS; PRKCD; PTPN6; PIK3C2A; LCK; RAF1; FYN; MAP2K2; PAK4; AKT1; PIK3R1; MAP2K1; PAK3; AKT3; VAV3; PRKCA |
| Cell Cycle: G1/S Checkpoint Regulation | HDAC4; SMAD3; SUV39H1; HDAC5; CDKN1B; BTRC; ATR; ABL1; E2F1; HDAC2; HDAC7A; RB1; HDAC11; HDAC9; CDK2; E2F2; HDAC3; TP53; CDKN1A; CCND1; E2F4; ATM; RBL2; SMAD4; CDKN2A; MYC; NRG1; GSK3B; RBL1; HDAC6 |
| T Cell Receptor Signaling | RAC1; ELK1; MAPK1; IKBKB; CBL; PIK3CA; FOS; NFKB2; PIK3CB; PIK3C3; MAPK8; MAPK3; KRAS; RELA; PIK3C2A; BTK; LCK; RAF1; IKBKG; RELB; FYN; MAP2K2; PIK3R1; CHUK; MAP2K1; NFKB1; ITK; BCL10; JUN; VAV3 |
| Death Receptor Signaling | CRADD; HSPB1; BID; BIRC4; TBK1; IKBKB; FADD; FAS; NFKB2; BCL2; MAP3K14; MAPK8; RIPK1; CASP8; DAXX; TNFRSF10B; RELA; TRAF2; TNF; IKBKG; RELB; CASP9; CHUK; APAF1; NFKB1; CASP2; BIRC2; CASP3; BIRC3 |
| FGF Signaling | RAC1; FGFR1; MET; MAPKAPK2; MAPK1; PTPN11; AKT2; PIK3CA; CREB1; PIK3CB; PIK3C3; MAPK8; MAPK3; MAPK13; PTPN6; PIK3C2A; MAPK14; RAF1; AKT1; PIK3R1; STAT3; MAP2K1; FGFR4; CRKL; ATF4; AKT3; PRKCA; HGF |
| GM-CSF Signaling | LYN; ELK1; MAPK1; PTPN11; AKT2; PIK3CA; CAMK2A; STAT5B; PIK3CB; PIK3C3; GNB2L1; BCL2L1; MAPK3; ETS1; KRAS; RUNX1; PIM1; PIK3C2A; RAF1; MAP2K2; AKT1; JAK2; PIK3R1; STAT3; MAP2K1; CCND1; AKT3; STAT1 |
| Amyotrophic Lateral Sclerosis Signaling | BID; IGF1; RAC1; BIRC4; PGF; CAPNS1; CAPN2; PIK3CA; BCL2; PIK3CB; PIK3C3; BCL2L1; CAPN1; PIK3C2A; TP53; CASP9; PIK3R1; RAB5A; CASP1; APAF1; VEGFA; BIRC2; BAX; AKT3; CASP3; BIRC3 |
| JAK/Stat Signaling | PTPN1; MAPK1; PTPN11; AKT2; PIK3CA; STAT5B; PIK3CB; PIK3C3; MAPK3; KRAS; SOCS1; STAT5A; PTPN6; PIK3C2A; RAF1; CDKN1A; MAP2K2; JAK1; AKT1; JAK2; PIK3R1; STAT3; MAP2K1; FRAP1; |
| | AKT3; STAT1 |
| Nicotinate and Nicotinamide Metabolism | PRKCE; IRAK1; PRKAA2; EIF2AK2; GRK6; MAPK1; PLK1; AKT2; CDK8; MAPK8; MAPK3; PRKCD; PRKAA1; PBEF1; MAPK9; CDK2; PIM1; DYRK1A; MAP2K2; MAP2K1; PAK3; NT5E; TTK; CSNK1A1; BRAF; SGK |
| Chemokine Signaling | CXCR4; ROCK2; MAPK1; PTK2; FOS; CFL1; GNAQ; CAMK2A; CXCL12; MAPK8; MAPK3; KRAS; MAPK13; RHOA; CCR3; SRC; PPP1CC; MAPK14; NOX1; RAF1; MAP2K2; MAP2K1; JUN; CCL2; PRKCA |
| IL-2 Signaling | ELK1; MAPK1; PTPN11; AKT2; PIK3CA; SYK; FOS; STAT5B; PIK3CB; PIK3C3; MAPK8; MAPK3; KRAS; SOCS1; STAT5A; PIK3C2A; LCK; RAF1; MAP2K2; JAK1; AKT1; PIK3R1; MAP2K1; JUN; AKT3 |
| Synaptic Long Term Depression | PRKCE; IGF1; PRKCZ; PRDX6; LYN; MAPK1; GNAS; PRKCI; GNAQ; PPP2R1A; IGF1R; PRKD1; MAPK3; KRAS; GRN; PRKCD; NOS3; NOS2A; PPP2CA; YWHAZ; RAF1; MAP2K2; PPP2R5C; MAP2K1; PRKCA |
| Estrogen Receptor Signaling | TAF4B; EP300; CARM1; PCAF; MAPK1; NCOR2; SMARCA4; MAPK3; NRIP1; KRAS; SRC; NR3C1; HDAC3; PPARGC1A; RBM9; NCOA3; RAF1; CREBBP; MAP2K2; NCOA2; MAP2K1; PRKDC; ESR1; ESR2 |
| Protein Ubiquitination Pathway | TRAF6; SMURF1; BIRC4; BRCA1; UCHL1; NEDD4; CBL; UBE2I; BTRC; HSPA5; USP7; USP10; FBXW7; USP9X; STUB1; USP22; B2M; BIRC2; PARK2; USP8; USP1; VHL; HSP90AA1; BIRC3 |
| IL-10 Signaling | TRAF6; CCR1; ELK1; IKBKB; SP1; FOS; NFKB2; MAP3K14; MAPK8; MAPK13; RELA; MAPK14; TNF; IKBKG; RELB; MAP3K7; JAK1; CHUK; STAT3; NFKB1; JUN; IL1R1; IL6 |
| VDR/RXR Activation | PRKCE; EP300; PRKCZ; RXRA; GADD45A; HES1; NCOR2; SP1; PRKCI; CDKN1B; PRKD1; PRKCD; RUNX2; KLF4; YY1; NCOA3; CDKN1A; NCOA2; SPP1; |
| | LRP5; CEBPB; FOXO1; PRKCA |
| TGF-beta Signaling | EP300; SMAD2; SMURF1; MAPK1; SMAD3; SMAD1; FOS; MAPK8; MAPK3; KRAS; MAPK9; RUNX2; SERPINE1; RAF1; MAP3K7; CREBBP; MAP2K2; MAP2K1; TGFBR1; SMAD4; JUN; SMADS |
| Toll-like Receptor Signaling | IRAK1; EIF2AK2; MYD88; TRAF6; PPARA; ELK1; IKBKB; FOS; NFKB2; MAP3K14; MAPK8; MAPK13; RELA; TLR4; MAPK14; IKBKG; RELB; MAP3K7; CHUK; NFKB1; TLR2; JUN |
| p38 MAPK Signaling | HSPB1; IRAK1; TRAF6; MAPKAPK2; ELK1; FADD; FAS; CREB1; DDIT3; RPS6KA4; DAXX; MAPK13; TRAF2; MAPK14; TNF; MAP3K7; TGFBR1; MYC; ATF4; IL1R1; SRF; STAT1 |
| Neurotrophin/TRK Signaling | NTRK2; MAPK1; PTPN11; PIK3CA; CREB1; FOS; PIK3CB; PIK3C3; MAPK8; MAPK3; KRAS; PIK3C2A; RAF1; MAP2K2; AKT1; PIK3R1; PDPK1; MAP2K1; CDC42; JUN; ATF4 |
| FXR/RXR Activation | INS; PPARA; FASN; RXRA; AKT2; SDC1; MAPK8; APOB; MAPK10; PPARG; MTTP; MAPK9; PPARGC1A; TNF; CREBBP; AKT1; SREBF1; FGFR4; AKT3; FOXO1 |
| Synaptic Long Term Potentiation | PRKCE; RAP1A; EP300; PRKCZ; MAPK1; CREB1; PRKCI; GNAQ; CAMK2A; PRKD1; MAPK3; KRAS; PRKCD; PPP1CC; RAF1; CREBBP; MAP2K2; MAP2K1; ATF4; PRKCA |
| Calcium Signaling | RAP1A; EP300; HDAC4; MAPK1; HDAC5; CREB1; CAMK2A; MYH9; MAPK3; HDAC2; HDAC7A; HDAC11; HDAC9; HDAC3; CREBBP; CALR; CAMKK2; ATF4; HDAC6 |
| EGF Signaling | ELK1; MAPK1; EGFR; PIK3CA; FOS; PIK3CB; PIK3C3; MAPK8; MAPK3; PIK3C2A; RAF1; JAK1; PIK3R1; STAT3; MAP2K1; JUN; PRKCA; SRF; STAT1 |
| Hypoxia Signaling in the Cardiovascular System | EDN1; PTEN; EP300; NQO1; UBE2I; CREB 1; ARNT; HIF1A; SLC2A4; NOS3; TP53; LDHA; AKT1; ATM; |
| | VEGFA; JUN; ATF4; VHL; HSP90AA1 |
| LPS/IL-1 Mediated Inhibition of RXR Function | IRAK1; MYD88; TRAF6; PPARA; RXRA; ABCA1; MAPK8; ALDH1A1; GSTP1; MAPK9; ABCB1; TRAF2; TLR4; TNF; MAP3K7; NR1H2; SREBF1; JUN; IL1R1 |
| LXR/RXR Activation | FASN; RXRA; NCOR2; ABCA1; NFKB2; IRF3; RELA; NOS2A; TLR4; TNF; RELB; LDLR; NR1H2; NFKB1; SREBF1; IL1R1; CCL2; IL6; MMP9 |
| Amyloid Processing | PRKCE; CSNK1E; MAPK1; CAPNS1; AKT2; CAPN2; CAPN1; MAPK3; MAPK13; MAPT; MAPK14; AKT1; PSEN1; CSNK1A1; GSK3B; AKT3; APP |
| IL-4 Signaling | AKT2; PIK3CA; PIK3CB; PIK3C3; IRS1; KRAS; SOCS1; PTPN6; NR3C1; PIK3C2A; JAK1; AKT1; JAK2; PIK3R1; FRAP1; AKT3; RPS6KB1 |
| Cell Cycle: G2/M DNA Damage Checkpoint Regulation | EP300; PCAF; BRCA1; GADD45A; PLK1; BTRC; CHEK1; ATR; CHEK2; YWHAZ; TP53; CDKN1A; PRKDC; ATM; SFN; CDKN2A |
| Nitric Oxide Signaling in the Cardiovascular System | KDR; FLT1; PGF; AKT2; PIK3CA; PIK3CB; PIK3C3; CAV1; PRKCD; NOS3; PIK3C2A; AKT1; PIK3R1; VEGFA; AKT3; HSP90AA1 |
| Purine Metabolism | NME2; SMARCA4; MYH9; RRM2; ADAR; EIF2AK4; PKM2; ENTPD1; RAD51; RRM2B; TJP2; RAD51C; NT5E; POLD1; NME1 |
| cAMP-mediated Signaling | RAP1A; MAPK1; GNAS; CREB1; CAMK2A; MAPK3; SRC; RAF1; MAP2K2; STAT3; MAP2K1; BRAF; ATF4 |
| Mitochondrial Dysfunction | SOD2; MAPK8; CASP8; MAPK10; MAPK9; CASP9; PARK7; PSEN1; PARK2; APP; CASP3 |
| Notch Signaling | HES1; JAG1; NUMB; NOTCH4; ADAM 17; NOTCH2; PSEN1; NOTCH3; NOTCH 1; DLL4 |
| Endoplasmic Reticulum Stress Pathway | HSPA5; MAPK8; XBP1; TRAF2; ATF6; CASP9; ATF4; EIF2AK3; CASP3 |
| Pyrimidine Metabolism | NME2; AICDA; RRM2; EIF2AK4; ENTPD1; RRM2B; NT5E; POLD1; NME1 |
| Parkinson's Signaling | UCHL1; MAPK8; MAPK13; MAPK14; CASP9; PARK7; |
| | PARK2; CASP3 |
| Cardiac & Beta Adrenergic Signaling | GNAS; GNAQ; PPP2R1A; GNB2L1; PPP2CA; PPP1CC; PPP2R5C |
| Glycolysis/Gluconeogene sis | HK2; GCK; GPI; ALDH1A1; PKM2; LDHA; HK1 |
| Interferon Signaling | IRF1; SOCS1; JAK1; JAK2; IFITM1; STAT1; IFIT3 |
| Sonic Hedgehog Signaling | ARRB2; SMO; GLI2; DYRK1A; GLI1; GSK3B; DYRK1B |
| Glycerophospholipid Metabolism | PLD1; GRN; GPAM; YWHAZ; SPHK1; SPHK2 |
| Phospholipid Degradation | PRDX6; PLD1; GRN; YWHAZ; SPHK1; SPHK2 |
| Tryptophan Metabolism | SIAH2; PRMT5; NEDD4; ALDH1A1; CYP1B1; SIAH1 |
| Lysine Degradation | SUV39H1; EHMT2; NSD1; SETD7; PPP2R5C |
| Nucleotide Excision Repair Pathway | ERCC5; ERCC4; XPA; XPC; ERCC1 |
| Starch and Sucrose Metabolism | UCHL1; HK2; GCK; GPI; HK1 |
| Aminosugars Metabolism | NQO1; HK2; GCK; HK1 |
| Arachidonic Acid Metabolism | PRDX6; GRN; YWHAZ; CYP1B1 |
| Circadian Rhythm Signaling | CSNK1E; CREB1; ATF4; NR1D1 |
| Coagulation System | BDKRB1; F2R; SERPINE1; F3 |
| Dopamine Receptor Signaling | PPP2R1A; PPP2CA; PPP1CC; PPP2R5C |
| Glutathione Metabolism | IDH2; GSTP1; ANPEP; IDH1 |
| Glycerolipid Metabolism | ALDH1A1; GPAM; SPHK1; SPHK2 |
| Linoleic Acid Metabolism | PRDX6; GRN; YWHAZ; CYP1B1 |
| Methionine Metabolism | DNMT1; DNMT3B; AHCY; DNMT3A |
| Pyruvate Metabolism | GLO1; ALDH1A1; PKM2; LDHA |
| Arginine and Proline Metabolism | ALDH1A1; NOS3; NOS2A |
| Eicosanoid Signaling | PRDX6; GRN; YWHAZ |
| Fructose and Mannose Metabolism | HK2; GCK; HK1 |
| Galactose Metabolism | HK2; GCK; HK1 |
| Stilbene, Coumarine and Lignin Biosynthesis | PRDX6; PRDX1; TYR |
| Antigen Presentation Pathway | CALR; B2M |
| Biosynthesis of Steroids | NQO1; DHCR7 |
| Butanoate Metabolism | ALDH1A1; NLGN1 |
| Citrate Cycle | IDH2; IDH1 |
| Fatty Acid Metabolism | ALDH1A1; CYP1B1 |
| Glycerophospholipid Metabolism | PRDX6; CHKA |
| Histidine Metabolism | PRMT5; ALDH1A1 |
| Inositol Metabolism | ERO1L; APEX1 |
| Metabolism of Xenobiotics by Cytochrome p450 | GSTP1; CYP1B1 |
| Methane Metabolism | PRDX6; PRDX1 |
| Phenylalanine Metabolism | PRDX6; PRDX1 |
| Propanoate Metabolism | ALDH1A1; LDHA |
| Selenoamino Acid Metabolism | PRMT5; AHCY |
| Sphingolipid Metabolism | SPHK1; SPHK2 |
| Aminophosphonate Metabolism | PRMT5 |
| Androgen and Estrogen Metabolism | PRMT5 |
| Ascorbate and Aldarate Metabolism | ALDH1A1 |
| Bile Acid Biosynthesis | ALDH1A1 |
| Cysteine Metabolism | LDHA |
| Fatty Acid Biosynthesis | FASN |
| Glutamate Receptor Signaling | GNB2L1 |
| NRF2-mediated Oxidative | PRDX1 |
| Stress Response | |
| Pentose Phosphate Pathway | GPI |
| Pentose and Glucuronate Interconversions | UCHL1 |
| Retinol Metabolism | ALDH1A1 |
| Riboflavin Metabolism | TYR |
| Tyrosine Metabolism | PRMT5, TYR |
| Ubiquinone Biosynthesis | PRMT5 |
| Valine, Leucine and Isoleucine Degradation | ALDH1A1 |
| Glycine, Serine and Threonine Metabolism | CHKA |
| Lysine Degradation | ALDH1A1 |
| Pain/Taste | TRPM5; TRPA1 |
| Pain | TRPM7; TRPC5; TRPC6; TRPC1; Cnr1; cnr2; Grk2; Trpa1; Pomc; Cgrp; Crf; Pka; Era; Nr2b; TRPM5; Prkaca; Prkacb; Prkarla; Prkar2a |
| Mitochondrial Function | AIF; CytC; SMAC (Diablo); Aifm-1; Aifm-2 |
| Developmental Neurology | BMP-4; Chordin (Chrd); Noggin (Nog); WNT (Wnt2; Wnt2b; Wnt3a; Wnt4; Wnt5a; Wnt6; Wnt7b; Wnt8b; Wnt9a; Wnt9b; Wnt10a; Wnt10b; Wnt16); beta-catenin; Dkk-1; Frizzled related proteins; Otx-2; Gbx2; FGF-8; Reelin; Dab1; unc-86 (Pou4f1 or Bm3a); Numb; Reln |

Embodiments of the invention also relate to methods and compositions related to silencing genes, or inducing or increasing expression of genes through altering the loop or domain in which thay are located.

Several further aspects of the invention relate to silencing genes having a defect, and inducing expression of other genomic copies of that same genein the genome that are not defective. Genes associated with a wide range of genetic diseases which are further described on the website of the National Institutes of Health under the topic subsection Genetic Disorders. The genetic brain diseases may include but are not limited to Adrenoleukodystrophy, Agenesis of the Corpus Callosum, Aicardi Syndrome, Alpers' Disease, Alzheimer's Disease, Barth Syndrome, Batten Disease, CADASIL, Cerebellar Degeneration, Fabry's Disease, Gerstmann-Straussler-Scheinker Disease, Huntington's Disease and other Triplet Repeat Disorders, Leigh's Disease, Lesch-Nyhan Syndrome, Menkes Disease, Mitochondrial Myopathies and NINDS Colpocephaly. These diseases are further described on the website of the National Institutes of Health under the subsection Genetic Brain Disorders.

In some embodiments, the condition may be neoplasia. In some embodiments, where the condition is neoplasia, the genes to be targeted (or he locus of the genes that is to be targeted) are any of those listed in Table A. In some embodiments, the condition may be Age-related Macular Degeneration. In some embodiments, the condition may be a Schizophrenic Disorder. In some embodiments, the condition may be a Trinucleotide Repeat Disorder. In some embodiments, the condition may be Fragile X Syndrome. In some embodiments, the condition may be a Secretase Related Disorder. In some embodiments, the condition may be a Prion - related disorder. In some embodiments, the condition may be ALS. In some embodiments, the condition may be a drug addiction. In some embodiments, the condition may be Autism. In some embodiments, the condition may be Alzheimer's Disease. In some embodiments, the condition may be inflammation. In some embodiments, the condition may be Parkinson's Disease.

It is envisaged that the present methods for interfering in chromatin looping is used to change the expression of disease associated proteins, or other proteins in a living cell, through modification of the contacts that the genes encoding these proteins have with other genes in a contact domain . Examples of disease associated proteins proteins associated with Parkinson's disease include but are not limited to α-synuclein, DJ-1, LRRK2, PINK1, Parkin, UCHL1, Synphilin-1, and NURR1.

Examples of addiction-related proteins may include ABAT for example.

Examples of inflammation-related proteins may include the monocyte chemoattractant protein-1 (MCP1) encoded by the Ccr2 gene, the C-C chemokine receptor type 5 (CCR5) encoded by the Ccr5 gene, the IgG receptor IIB (FCGR2b, also termed CD32) encoded by the Fcgr2b gene, or the Fc epsilon R1g (FCER1g) protein encoded by the Fcerlg gene, for example.

Examples of cardiovascular diseases associated proteins may include IL1B (interleukin 1, beta), XDH (xanthine dehydrogenase), TP53 (tumor protein p53), PTGIS (prostaglandin I2 (prostacyclin) synthase), MB (myoglobin), IL4 (interleukin 4), ANGPT1 (angiopoietin 1), ABCG8 (ATP-binding cassette, sub-family G (WHITE), member 8), or CTSK (cathepsin K), for example.

Examples of Alzheimer's disease associated proteins may include the very low density lipoprotein receptor protein (VLDLR) encoded by the VLDLR gene, the ubiquitin-like modifier activating enzyme 1 (UBA1) encoded by the UBA1 gene, or the NEDD8-activating enzyme E1 catalytic subunit protein (UBE1C) encoded by the UBA3 gene, for example.

Examples of proteins associated with Autism Spectrum Disorder may include the benzodiazapine receptor (peripheral) associated protein 1 (BZRAP1) encoded by the BZRAP1 gene, the AF4/FMR2 family member 2 protein (AFF2) encoded by the AFF2 gene (also termed MFR2), the fragile X mental retardation autosomal homolog 1 protein (FXR1) encoded by the FXR1 gene, or the fragile X mental retardation autosomal homolog 2 protein (FXR2) encoded by the FXR2 gene, for example.

Examples of proteins associated with Macular Degeneration may include the ATP-binding cassette, sub-family A (ABC 1) member 4 protein (ABCA4) encoded by the ABCR gene, the apolipoprotein E protein (APOE) encoded by the APOE gene, or the chemokine (C-C motif) Ligand 2 protein (CCL2) encoded by the CCL2 gene, for example.

Examples of proteins associated with Schizophrenia may include NRG1, ErbB4, CPLX1, TPH1, TPH2, NRXN1, GSK3A, BDNF, DISC1, GSK3B, and combinations thereof.

Examples of proteins involved in tumor suppression may include ATM (ataxia telangiectasia mutated), ATR (ataxia telangiectasia and Rad3 related), EGFR (epidermal growth factor receptor), ERBB2 (v-erb-b2 erythroblastic leukemia viral oncogene homolog 2), ERBB3 (v-erb-b2 erythroblastic leukemia viral oncogene homolog 3), ERBB4 (v-erb-b2 erythroblastic leukemia viral oncogene homolog 4), Notch 1, Notch2, Notch 3, or Notch 4, for example.

Examples of proteins associated with a secretase disorder may include PSENEN (presenilin enhancer 2 homolog (C. elegans)), CTSB (cathepsin B), PSEN1 (presenilin 1), APP (amyloid beta (A4) precursor protein), APH1B (anterior pharynx defective 1 homolog B (C. elegans)), PSEN2 (presenilin 2 (Alzheimer disease 4)), or BACE1 (beta-site APP-cleaving enzyme 1), for example.

Examples of proteins associated with Amyotrophic Lateral Sclerosis may include SOD1 (superoxide dismutase 1), ALS2 (amyotrophic lateral sclerosis 2), FUS (fused in sarcoma), TARDBP (TAR DNA binding protein), VAGFA (vascular endothelial growth factor A), VAGFB (vascular endothelial growth factor B), and VAGFC (vascular endothelial growth factor C), and any combination thereof.

Examples of proteins associated with prion diseases may include SOD1 (superoxide dismutase 1), ALS2 (amyotrophic lateral sclerosis 2), FUS (fused in sarcoma), TARDBP (TAR DNA binding protein), VAGFA (vascular endothelial growth factor A), VAGFB (vascular endothelial growth factor B), and VAGFC (vascular endothelial growth factor C), and any combination thereof.

Examples of proteins related to neurodegenerative conditions in prion disorders may include A2M (Alpha-2-Macroglobulin), AATF (Apoptosis antagonizing transcription factor), ACPP (Acid phosphatase prostate), ACTA2 (Actin alpha 2 smooth muscle aorta), ADAM22 (ADAM metallopeptidase domain), ADORA3 (Adenosine A3 receptor), or ADRA1D (Alpha-1D adrenergic receptor for Alpha-1D adrenoreceptor), for example.

Examples of proteins associated with Immunodeficiency may include A2M [alpha-2-macroglobulin]; AANAT [arylalkylamine N-acetyltransferase]; ABCA1 [ATP-binding cassette, sub-family A (ABC1), member 1]; ABCA2 [ATP-binding cassette, sub-family A (ABC1), member 2]; or ABCA3 [ATP-binding cassette, sub-family A (ABC1), member 3]; for example.

Examples of proteins associated with Trinucleotide Repeat Disorders include AR (androgen receptor), FMR1 (fragile X mental retardation 1), HTT (huntingtin), or DMPK (dystrophia myotonica-protein kinase), FXN (frataxin), ATXN2 (ataxin 2), for example.

Examples of proteins associated with Neurotransmission Disorders include SST (somatostatin), NOS1 (nitric oxide synthase 1 (neuronal)), ADRA2A (adrenergic, alpha-2A-, receptor), ADRA2C (adrenergic, alpha-2C-, receptor), TACR1 (tachykinin receptor 1), or HTR2c (5-hydroxytryptamine (serotonin) receptor 2C), for example.

Examples of neurodevelopmental-associated sequences include A2BP1 [ataxin 2-binding protein 1], AADAT [aminoadipate aminotransferase], AANAT [arylalkylamine N-acetyltransferase], ABAT [4-aminobutyrate aminotransferase], ABCA1 [ATP-binding cassette, sub-family A (ABC1), member 1], or ABCA13 [ATP-binding cassette, sub-family A (ABC1), member 13], for example.

Further examples of preferred conditions treatable with the present system include may be selected from: Aicardi-Goutières Syndrome; Alexander Disease; Allan-Herndon-Dudley Syndrome; POLG-Related Disorders; Alpha-Mannosidosis (Type II and III); Alström Syndrome; Angelman; Syndrome; Ataxia-Telangiectasia; Neuronal Ceroid-Lipofuscinoses; Beta-Thalassemia; Bilateral Optic Atrophy and (Infantile) Optic Atrophy Type 1; Retinoblastoma (bilateral); Canavan Disease; Cerebrooculofacioskeletal Syndrome 1 [COFS1]; Cerebrotendinous Xanthomatosis; Cornelia de Lange Syndrome; MAPT-Related Disorders; Genetic Prion Diseases; Dravet Syndrome; Early-Onset Familial Alzheimer Disease; Friedreich Ataxia [FRDA]; Fryns Syndrome; Fucosidosis; Fukuyama Congenital Muscular Dystrophy; Galactosialidosis; Gaucher Disease; Organic Acidemias; Hemophagocytic Lymphohistiocytosis; Hutchinson-Gilford Progeria Syndrome; Mucolipidosis II; Infantile Free Sialic Acid Storage Disease; PLA2G6-Associated Neurodegeneration; Jervell and Lange-Nielsen Syndrome; Junctional Epidermolysis Bullosa; Huntington Disease; Krabbe Disease (Infantile); Mitochondrial DNA-Associated Leigh Syndrome and NARP; Lesch-Nyhan Syndrome; LIS1-Associated Lissencephaly; Lowe Syndrome; Maple Syrup Urine Disease; MECP2 Duplication Syndrome; ATP7A-Related Copper Transport Disorders; LAMA2-Related Muscular Dystrophy; Arylsulfatase A Deficiency; Mucopolysaccharidosis Types I, II or III; Peroxisome Biogenesis Disorders, Zellweger Syndrome Spectrum; Neurodegeneration with Brain Iron Accumulation Disorders; Acid Sphingomyelinase Deficiency; Niemann-Pick Disease Type C; Glycine Encephalopathy; ARX-Related Disorders; Urea Cycle Disorders; COL1A1/2-Related Osteogenesis Imperfecta; Mitochondrial DNA Deletion Syndromes; PLP1-Related Disorders; Perry Syndrome; Phelan-McDermid Syndrome; Glycogen Storage Disease Type II (Pompe Disease) (Infantile); MAPT-Related Disorders; MECP2-Related Disorders; Rhizomelic Chondrodysplasia Punctata Type 1; Roberts Syndrome; Sandhoff Disease; Schindler Disease - Type 1; Adenosine Deaminase Deficiency; Smith-Lemli-Opitz Syndrome; Spinal Muscular Atrophy; Infantile-Onset Spinocerebellar Ataxia; Hexosaminidase A Deficiency; Thanatophoric Dysplasia Type 1; Collagen Type VI-Related Disorders; Usher Syndrome Type I; Congenital Muscular Dystrophy; Wolf-Hirschhom Syndrome; Lysosomal Acid Lipase Deficiency; and Xeroderma Pigmentosum.

Chronic administration of protein therapeutics may elicit unacceptable immune responses to the specific protein. The immunogenicity of protein drugs can be ascribed to a few immunodominant helper T lymphocyte (HTL) epitopes. Reducing the MHC binding affinity of these HTL epitopes contained within these proteins can generate drugs with lower immunogenicity ( Tangri S, et al. ("Rationally engineered therapeutic proteins with reduced immunogenicity" J Immunol. 2005 Mar 15;174(6):3187-96 .) In the present invention, the immunogenicity of the CRISPR enzyme in particular may be reduced following the approach first set out in Tangri et al with respect to erythropoietin and subsequently developed. Accordingly, directed evolution or rational design may be used to reduce the immunogenicity of the CRISPR enzyme (for instance a Cas9) in the host species (human or other species).

In plants, pathogens are often host-specific. For example, Fusarium oxysporum. f sp.lycopersici causes tomato wilt but attacks only tomato, and F. oxysporum f. dianthii Puccinia graminis f. sp.tritici attacks only wheat. Plants have existing and induced defenses to resist most pathogens. Mutations and recombination events across plant generations lead to genetic variability that gives rise to susceptibility, especially as pathogens reproduce with more frequency than plants. In plants there can be non-host resistance, e.g., the host and pathogen are incompatible. There can also be Horizontal Resistance, e.g., partial resistance against all races of a pathogen, typically controlled by many genes and Vertical Resistance, e.g., complete resistance to some races of a pathogen but not to other races, typically controlled by a few genes. In a Gene-for-Gene level, plants and pathogens evolve together, and the genetic changes in one balance changes in other. Accordingly, using Natural Variability, breeders combine most useful genes for Yield, Quality, Uniformity, Hardiness, Resistance. The sources of resistance genes include native or foreign Varieties, Heirloom Varieties, Wild Plant Relatives, and Induced Mutations, e.g., treating plant material with mutagenic agents. Using the present invention, plant breeders are provided with a new tool to alter gene expression in their plants. Accordingly, one skilled in the art can analyze the genome of sources of resistance genes, and in Varieties having desired characteristics or traits employ the present invention to induce the rise of resistance genes, with more precision than previous mutagenic agents and hence accelerate and improve plant breeding programs.

As will be apparent, it is envisaged that the present system can be used to target any polynucleotide sequence of interest. Some examples of conditions or diseases that might be usefully treated using the present system are included in the Tables above and examples of genes currently associated with those conditions are also provided there. However, the genes exemplified are not exhaustive.

### Further Embodiments

Also disclosed is a system wherein information from one or more ligation junctions is used to identify regions of the genome that control or modulate spatial proximity relationships between nucleic acids. In some embodiments, the genomic regions identified establish chromatin loops. In some embodiments, the genomic regions identified demarcate or establish contiguous intervals of chromatin that display elevated proximity between loci within the intervals.

Further disclosed is a system for visualizing, such as system comprising hardware and/or software, the information from one or more ligation junctions. In some examples, the information from one or more ligation junctions is represented in a matrix with entries indicating frequency of interaction. In some examples, a user can dynamically zoom in and out, viewing interactions between smaller or larger pieces of the genome. In some examples, interaction matrices and other 1-D data vectors can be viewed and compared simultaneously. In some examples, the annotations of features can be superimposed on interaction matrices. In some examples, multiple interaction matrices can be simultaneously viewer and compared.

### Target Ligation Junctions and Probes

As part of the in situ Hi-C technioque, described herein, also disclosed are nucleic acids made of two or more end joined nucleic acids, target junctions, produced using the disclosed methods and amplification products thereof, such as RNA, DNA or a combination thereof. An isolated target junction is an end joined nucleic acid, wherein the junction encodes the information about the proximity of the two nucleic acid sequences that make up the target junction in a cell, for example as formed by the methods disclosed herein. The presence of an isolated target junction can be correlated with a disease state or environmental condition. For example, certain disease states may be caused and/or characterized by the differential formation of certain target junctions. Similarly isolated target junction can be correlated to an environmental stress or state, such as but not limited to heat shock, osmolarity, hypoxia, cold, oxidative stress, radiation, starvation, a chemical (for example a therapeutic agent or potential therapeutic agent) and the like.

This disclosure also relates, to isolated nucleic acid probes that specifically bind to target junction, such as a target junction indicative of a disease state or environmental condition. To recognize a target junction, a probe specifically hybridizes to the target junction both 5' and 3' of the site of the junction and spans the site of the target junction, or specifically hybridizes to probe-specific target sequences with the end joined nucleic acid fragments. In some example embodiments, the probe-specific target sequence is at least 50, at least 60, at least 70, at least 80, at least 90, at least 100, at least 110, at least 120, at least 130, at least 140, at least 150, at least 160, at least 170, at least 180, at least 190, or at least 200 base pairs long. In certain example embodiments, the specific nucleic acid sequence is within at least 50, at least 60, at least 70, at least, 80, at least 90, or at least 100 base pairs, in either the 5' or 3' direction, of a restriction site. In certain example embodiments, the specific nucleic sequence comprises less than ten repetitive bases. In certain other example embodiments, the GC content of the specific nucleic acid sequence is between 25% and 80%, between 40% and 70%, or between 50% and 60%.

In some embodiments, the probe is labeled, such as radiolabeled, fluorescently-labeled, biotin-labeled, enzymatically-labeled, or chemically-labeled. Non-limiting examples of the probe is an RNA probe, a DNA probe, a locked nucleic acid (LNA) probe, a peptide nucleic acid (PNA) probe, or a hybrid RNA-DNA probe. Also disclosed are sets of probes for binding to target ligation junction, as well as devices, such as nucleic acid arrays for detecting a target junction.

In embodiments, the total length of the probe, including end linked PCR or other tags, is between about 10 nucleotides and 200 nucleotides, although longer probes are contemplated. In some embodiments, the total length of the probe, including end linked PCR or other tags, is at least about 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190 191, 192, 193, 194, 195, 196, 197, 198, 199 or 200.

In some embodiments the total length of the probe, including end linked PCR or other tags, is less then about 2000 nucleotides in length, such as less than about 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 500, 750, 1000, 1250, 1500, 1750, 2000 nucleotides in length or even greater. In some embodiments, the total length of the probe, including end linked PCR or other tags, is between about 30 nucleotides and about 250 nucleotides, for example about 90 to about 180, about 120 to about 200, about 150 to about 220 or about 120 to about 180 nucleotides in length. In some embodiments, a set of probes is used to target a specific target junction or a set of target junctions.

In some embodiments, the probe is detectably labeled, either with an isotopic or non-isotopic label, alternatively the target junction or amplification product thereof is labeled. Non-isotopic labels can, for instance, comprise a fluorescent or luminescent molecule, biotin, an enzyme or enzyme substrate or a chemical. Such labels are preferentially chosen such that the hybridization of the probe with target junction can be detected. In some examples, the probe is labeled with a fluorophore. Examples of suitable fluorophore labels are given above. In some examples, the fluorophore is a donor fluorophore. In other examples, the fluorophore is an accepter fluorophore, such as a fluorescence quencher. In some examples, the probe includes both a donor fluorophore and an accepter fluorophore. Appropriate donor/acceptor fluorophore pairs can be selected using routine methods. In one example, the donor emission wavelength is one that can significantly excite the acceptor, thereby generating a detectable emission from the acceptor.

An array containing a plurality of heterogeneous probes for the detection of target junctions are disclosed. Such arrays may be used to rapidly detect and/or identify the target junctions present in a sample, for example as part of a diagnosis. Arrays are arrangements of addressable locations on a substrate, with each address containing a nucleic acid, such as a probe. In some embodiments, each address corresponds to a single type or class of nucleic acid, such as a single probe, though a particular nucleic acid may be redundantly contained at multiple addresses. A "microarray" is a miniaturized array requiring microscopic examination for detection of hybridization. Larger "macroarrays" allow each address to be recognizable by the naked human eye and, in some embodiments, a hybridization signal is detectable without additional magnification. The addresses may be labeled, keyed to a separate guide, or otherwise identified by location.

Any sample potentially containing, or even suspected of containing, target joins may be used. A hybridization signal from an individual address on the array indicates that the probe hybridizes to a nucleotide within the sample. This system permits the simultaneous analysis of a sample by plural probes and yields information identifying the target junctions contained within the sample. In alternative embodiments, the array contains target junctions and the array is contacted with a sample containing a probe. In any such embodiment, either the probe or the target junction may be labeled to facilitate detection of hybridization.

Within an array, each arrayed nucleic acid is addressable, such that its location may be reliably and consistently determined within the at least the two dimensions of the array surface. Thus, ordered arrays allow assignment of the location of each nucleic acid at the time it is placed within the array. Usually, an array map or key is provided to correlate each address with the appropriate nucleic acid. Ordered arrays are often arranged in a symmetrical grid pattern, but nucleic acids could be arranged in other patterns (for example, in radially distributed lines, a "spokes and wheel" pattern, or ordered clusters). Addressable arrays can be computer readable; a computer can be programmed to correlate a particular address on the array with information about the sample at that position, such as hybridization or binding data, including signal intensity. In some exemplary computer readable formats, the individual samples or molecules in the array are arranged regularly (for example, in a Cartesian grid pattern), which can be correlated to address information by a computer.

An address within the array may be of any suitable shape and size. In some embodiments, the nucleic acids are suspended in a liquid medium and contained within square or rectangular wells on the array substrate. However, the nucleic acids may be contained in regions that are essentially triangular, oval, circular, or irregular. The overall shape of the array itself also may vary, though in some embodiments it is substantially flat and rectangular or square in shape.

Examples of substrates for the phage arrays disclosed herein include glass (e.g., functionalized glass), Si, Ge, GaAs, GaP, SiO2, SiN4, modified silicon nitrocellulose, polyvinylidene fluoride, polystyrene, polytetrafluoroethylene, polycarbonate, nylon, fiber, or combinations thereof. Array substrates can be stiff and relatively inflexible (for example glass or a supported membrane) or flexible (such as a polymer membrane). One commercially available product line suitable for probe arrays described herein is the Microlite line of MICROTITER^{®} plates available from Dynex Technologies UK (Middlesex, United Kingdom), such as the Microlite 1+ 96-well plate, or the 384 Microlite+ 384-well plate.

Addresses on the array should be discrete, in that hybridization signals from individual addresses can be distinguished from signals of neighboring addresses, either by the naked eye (macroarrays) or by scanning or reading by a piece of equipment or with the assistance of a microscope (microarrays).

### Kits

The agents, and other reagents disclosed herein for use in the disclosed methods can be supplied in the form of a kit. In such a kit, an appropriate amount of one or more of the agent is provided in one or more containers or held on a substrate. Components of the kit include agents for use as a medicament or for use in the treatment of a disorder in a human or animal subject in need thereof, wherein said agent comprises one or more sequence-specific DNA targeting agents selected from the group consisting of a CRISPR-Cas system, a zinc finger protein (ZFP), a zinc finger nuclease (ZFN), a transcription activator-like effector (TALE), a transcription activator-like effector nuclease (TALEN), a catalytically inactive CRISPR-Cas system, and a self-inactivating CRISPR/Cas system, wherein binding of the sequence-specific DNA targeting agents to the one or more genomic loci removes one or more existing chromatin loop or domain structures, introduces one or more new chromatin loop or domain structures, or modifies one or more existing chromatin loop or domain structures in a cell of said subject. Said agents comprise a DNA-targeting element comprising a nucleotide sequence that hybridizes to one or more CTCF or cohesin binding motifs or to a DNA target region in said chromatin DNA proximate to a location where one or more CTCF or cohesin binding motifs are to be introduced into the genome. Alternatively, the agents comprise a DNA-targeting element comprising a zinc finger motif that binds to one or more CTCF or cohesin binding motifs or to a DNA target region in said chromatin DNA proximate to a location where one or more CTCF or cohesin binding motifs are to be introduced into the genome. The agent may be encoded by a vector for delivering said agent to the nucleus of said cell, such as a viral vector. Suitable vectors include a lentiviral, adenoviral, adeno-associated viral, or herpes simplex virus vector.

An agent may be provided suspended in an aqueous solution or as a freeze-dried or lyophilized powder, for instance. The container(s) in which the agent are supplied can be any conventional container that is capable of holding the supplied form, for instance, microfuge tubes, ampoules, or bottles. The amount of agent supplied in the kit can be any appropriate amount, and may depend on the target market to which the product is directed. A kit may contain more than one different agent, such as 2, 3, 4, 5, 6, 7, 8, 9, 10, 11, 12, 13, 14, 15, 20, 25, 50, 100, or more agent. The instructions may include directions for genome editing using the agents, including methods for delivering the agent to the subjects. In certain embodiments, the kit includes nucleic acid probes for in sit Hi-C and/or Hi-C2 that hybridize to target junctions, and instrctions for Hi-C mapping. The components of the kit may be packaged in, or, especially in the probes may me provided as in individual containers (for example, microtubules) or an array substrate (such as, a 96-well or 384-well microtiter plate). In particular embodiments, the kit includes prepackaged probes, such as probes suspended in suitable medium in individual containers (for example, individually sealed EPPENDORF^{®} tubes) or the wells of an array substrate (for example, a 96-well microtiter plate sealed with a protective plastic film). In some embodiments, kits also may include the reagents necessary to carry out methods disclosed herein. In some embodiments, kits may also include the cell lines (e.g., degron cell lines as described herein). In other particular embodiments, the kit includes equipment, reagents, and instructions for the methods disclosed herein.

### Chromatin extrusion explains key features of loop and domain formation in wild-type and engineered genomes

Applicants recently used in situ Hi-C to create kilobase-resolution 3D maps of mammalian genomes. Here, Applicants combine these with new Hi-C, microscopy, and genome-editing experiments in order to study the physical structure of chromatin loops and domains. Applicants find that the observed contact domains are inconsistent with the equilibrium state for an ordinary condensed polymer. Combining Hi-C data and novel mathematical theorems, Applicants show that contact domains are also not consistent with a fractal globule. Instead, Applicants use physical simulations to study two models for genome folding. In the first, inter-monomer attraction during polymer condensation leads to the formation of a "tension globule", a non-equilibrium state in which genome position correlates with spatial position along a linear axis. In the other, CTCF and cohesin act together to extrude loops during interphase. Both models are consistent with the observed contact domains and with the observation that contact domains tend to form inside loops. However, the extrusion model explains a far wider array of observations, such as why loops tend not to overlap and why the CTCF-binding motifs at pairs of loop anchors lie in the convergent orientation. Finally, Applicants perform 13 genome-editing experiments examining the effect of altering CTCF -binding sites on chromatin folding. The convergent rule correctly predicts the affected loops in every case. Moreover, the extrusion model accurately predicts in silico the 3D maps resulting from each experiment using only the location of CTCF-binding sites in the WT. Thus, Applicants show that it is possible to disrupt, restore, and move loops and domains using targeted mutations as small as a single base pair.

Stretched out from end to end, the human genome is over 2 meters long. Yet it must fold up to fit inside a nucleus that is only a few microns wide. At the smallest scale, this folding is well characterized: double-stranded DNA helices wrap around histone proteins, forming a nucleosome every ~200bp (a beads-on-a-string configuration known as the "10nm fiber") (Kornberg (1974) Science 184, 868-871; Komberg and Lorch (1999) Cell 98, 285-294). At larger scales, the physical structure of chromatin is more mysterious.

One common hypothesis is that the 10nm fiber is organized into a higher-order structure known as the "30nm fiber," which has been observed in vitro but not in vivoi (Finch and Klug (1976) Proc Natl Acad Sci USA 73, 1897-1901; Fussner et al. (2011) Trends in Biochem Sci 36, 1-6; Ghirlando and Felsenfeld (2013) Biopolymers 99, 225-232). In the most common model, individual nucleosomes are wound about a central cavity that runs axially along the length of the 30nm fiber. Every six nucleosomes (roughly one kilobase of DNA) correspond to a full turn about this axial cavity, creating a solenoidal structure whose diameter is 30nm. (Several alternative models of the specific positioning of nucleosomes in a 30nm fiber have also been proposed.) Physical models of chromatin packing have implications for the stiffness of chromatin fibers. One way of describing the stiffness of a fiber is its Kuhn length: the minimum length of the fiber such that it is possible for the beginning and the end of the fiber segment to point in the same direction. All published estimates of which Applicants are aware suggest, based on coarse-grained computer simulations, that the Kuhn length of a 30nm fiber under nuclear conditions would range from 30-60kb (Wedemann and Langowski (2002) Biophys J 82; Schiessel (2003) J Phys Condens Matter 15, R699-R774). Reliable estimates of the stiffness of chromatin fibers are essential for modeling higher-order chromatin folding mechanisms.

Another common notion, dating back to the 1970s, is that the human genome is partitioned into domains that are typically several hundred kilobases in length (Goldman (1988) Bioessays 9, 50-55). These studies have relied on many experimental modalities, such as chromatin sedimentation (Cook and Brazell (1975) J Cell Sci 19, 261-279; Hartwig (1982) Biochem Biophys Acta 698, 214-217), fluorescence microscopy (Zehnbauer and Vogelstein (1985) BioEssays 2, 52-54), and - in the last several years - genome-wide DNA proximity ligation data generated using Hi-C (Lieberman-Aiden et al. (2009) Science 326, 289-293; Dixon et al. (2012). Nature 485, 376-380; Sexton et al. (2012) Cell 148, 458-472). Based on Hi-C experiments (Lieberman-Aiden et al. (2009) supra; Sexton et al. (2012) Cell 148, 458-472; Zhang et al. (2012) Cell 148, 908-921) and analytical and computational estimates of equilibration time (Rosa and Everaers (2008) PLoS Comp Bio 4, e1000153), chromatin at the scale of domains is thought to be far from thermodynamic equilibrium. Overall, the internal structure of domains is not well understood (Lieberman-Aiden et al. (2009) supra; Sachs et al. (1995) PNAS 92, 2710-2714; Mateos-Langerak et al. (2009) Proc Natl Acad Sci USA 106, 3812-3817; Bohn and Heermann (2010) PloS One 5, e12218; Barbieri et al. (2012) Proc Natl Acad Sci USA 109, 16173-16178; Naumova et al. (2013) Science 342, 948-953).

A third feature of chromatin folding is the formation of loops, which bring pairs of genomic sites that lie far apart along the linear genome into close spatial proximity (Schleif (1992) Annual Rev biochem 61(1), 199-223.). Many aspects of chromatin looping are poorly understood, including how loops form and whether they assemble into higher-order hubs (Schleif (1992) supra; Bulger and Groudine (1999) Genes & development 13(19), 2465-2477).

Applicants recently reported new, one-kilobase-resolution contact maps of the human genome (Rao et al. (2014) supra). These were created by using in situ Hi-C, which couples DNA-DNA proximity ligation in intact nuclei (nuclear ligation assay) with high-throughput sequencing (Fig 1A). The maps - containing over 15 billion contacts - allowed us to annotate nearly 9,000 contact domains, which are contiguous genomic intervals in which there is an enhanced probability of contact among all loci. Contact domains range in size from tens of kilobases to several megabases, with a median size of 185kb. The maps also allowed us to annotate over 10,000 loops. These loops typically lie between convergent DNA motifs (i.e., motifs pointing toward one another) that bind a complex containing CTCF and cohesin. Notably, Applicants found that many contact domains are also "loop domains" - that is, contact domains whose boundaries are demarcated by the endpoints of a chromatin loop.

Here, Applicants use our new maps to explore the physical structure of chromatin fibers, contact domains, and loops.

First, Applicants demonstrate that chromatin fibers are highly bendable at distances of kilobases, rather than the ~60kb expected for 30nm fibers; this casts doubt on the widespread existence of 30nm fibers in vivo and has significant consequences for the mechanism of loop formation.

Next, Applicants combine Hi-C data, molecular dynamics simulations, and a novel analogue of McKean's dimension-doubling theorem for Brownian motion (McKean (1955) Duke Math J 22, 229-234) to explore how chromatin fibers fold inside contact domains. Consistent with our earlier work based on lower-resolution Hi-C maps, Applicants find that the chromatin packing is inconsistent with an ordinary polymer at equilibrium. Applicants demonstrate that the structure of non-equilibrium globules depends on the strength of the internal forces arising between monomers during the condensation process. When internal forces are weak, the result is isotropic: a fractal globule. When internal forces are sufficiently strong, tension along the polymer chain causes anisotropic condensation, and the resulting "tension globules" contain long intervals in which linear position along the polymer correlates with spatial position along a dominant axis. Notably, the tension globule closely resembles a non-equilibrium polymer model proposed by de Gennes (de Gennes (1985) J de Phys 46, 639-642). Applicants find that the Hi-C data is consistent with a tension globule, but not with a strictly fractal structure. Using physical simulations, Applicants show that loops inside tension globules can give rise to transient contact domains.

Applicants then explore an intriguing suggestion of Nasmyth (Nasmyth (2001) Annu Rev Genet 35:673-745; Alipour and Marko (2012) Nucl Acids Res 1-11) who proposed that loops can form through the extrusion of flexible chromatin fibers by a cohesin-associated complex. Using physical simulations, Applicants probe the chromatin state that would result from such a process, and show that loop extrusion leads to the spontaneous formation of stable contact domains between the loop anchors.

For both the tension globule model and the extrusion model, Applicants show that physical simulations incorporating the locations of CTCF-binding sites seen in chromatin immunoprecipitation (ChIP) with sequencing (ChIP-Seq) data provide reasonable fits to our observed Hi-C data. However, the extrusion model has many appealing properties: it produces better fits to the data, does not require ad hoc assumptions, and explains why loops tend not to overlap and only form between convergent CTCF motifs.

Finally, Applicants use CRISPR-mediated genome editing to delete and invert CTCF motifs at loop anchors. In all cases examined, Applicants find that that the convergent rule correctly predicts which loops will disappear.

### Chromatin is bendable at the kilobase scale, far less stiff than predictions based on a 30nm fiber

At the smallest scale, models of chromatin structure rely on an estimate of the Kuhn length of a chromatin fiber (Rubinstein and Colby (2003) Polymer Physics (Oxford University Press)). Polymer theory predicts that higher order structures can only form at scales an order of magnitude larger than the Kuhn length. Because direct estimates of chromatin flexibility in vivo have not previously been available, inferences about the Kuhn length of chromatin have been based on theoretical, computational, and in vitro models (Ringrose et al. (1999) EMBO J 18, 6630-6641; Dekker et al. (2002) Science 295, 1306-1311; Bystricky et al. (2004) Proc Natl Acad Sci USA 101, 16495-16500).

To experimentally measure the Kuhn length of human chromatin in vivo, Applicants examined the tendency of cross-linked, chromatinized DNA fragments, formed during the Hi-C protocol's initial restriction digestion step, to form single-fragment DNA cycles during the subsequent proximity ligation step. Applicants found that restriction fragments shorter than 200bp (the size of a nucleosome) rarely formed cycles, suggesting that they were too stiff to bend into a DNA circle. The probability of cyclization increased sharply for fragments between 100 and 800bp long, and remained relatively constant for longer fragments (Fig 1B). The results were similar for Hi-C experiments performed using MboI and DpnII (4-cutters, with cutting sites on average every 420bp); HindIII and NcoI (6-cutters, with cutting sites on average every 3.6kb and 4.0kb respectively); and for experiments performed with and without crosslinking. These measurements imply that chromatin is bendable at the scale of individual nucleosomes, and suggest a Kuhn length of roughly 1kb for chromatin fibers.

The estimates derived from our analysis of cyclization were consistent with the results of two other approaches, both of which yield upper bounds on Kuhn length. First, Applicants examined chromatin bendability by measuring the probability, I(s), of contact between two loci as a function of the genomic distance, s, between them. Measuring I(s) can be useful in estimating polymer flexibility because the value of I(s) is maximal at the Kuhn length of a polymer and decreases monotonically as s increases. Using our in situ Hi-C data, Applicants were able to reliably measure I(s) for the human genome at all distances larger than 5kb (i.e., distances much longer than the typical 4-cutter restriction fragment). Applicants found that I(s) exhibits monotonic decline at all distances probed. This implies that the Kuhn length of chromatin is less than 5kb. Second, Applicants note that in our initial report, biologically functional loops as short as 40kb were visually obvious. At least at the specific loci involved in such loops, kilobase-length chromatin fibers must be capable of bending appreciably. Taken together, our findings imply that chromatin is highly flexible at the kilobase scale. They also suggest that contact domains, which range in size from 65kb to 2.7Mb, are large enough to be described using polymer models.

Notably, the Kuhn length observed in our data (≈1 kb) is incompatible with the estimated Kuhn length for the 30nm fiber. This result suggests that 30nm fibers, if they exist, are rare in human nuclear chromatin in vivo. (The flexibility of chromatin may also be relevant to the potential formation of loops by extrusion, as discussed below.)

### Measurements of contact probability using genome-wide averages are inconsistent with an ordinary polymer at equilibrium

In a previous study, Applicants characterized the polymer-like behavior of chromatin regions at the megabase scale by analyzing the contact probability function, I(s), described above, based on Hi-C data, analytical estimates, and in silico studies. In particular, the data for human chromatin showed a power law relationship of the form I(s) ∝ s-y between 500kb and 7Mb, with y = 1.08. Applicants showed that values of y can be used to discriminate between distinct polymer states. Specifically, Applicants noted that y = 1.08 is inconsistent with the classic structure of a globular polymer at equilibrium (known as an "equilibrium globule", which has y=1.5). Interestingly, Applicants found that the observed value of y is consistent with a dense, scale-invariant, isotropic, long-lived polymer state known as the fractal globule (Lieberman-Aiden et al. (2009) supra). Because the fractal globule's unknotted topology makes it easier to physically access individual genomic loci, it furnishes an appealing model for the structure of chromatin.

When Applicants repeated the above analysis on our new, high-resolution maps, Applicants observed a scaling of γ = 1.27 between 300kb and 3Mb. This slightly higher value is consistent with our previous conclusion that chromatin does not fold into an equilibrium globule. Moreover, the value y = 1.27 falls within the range of values that has been predicted for a fractal globule (Lieberman-Aiden E (2010) Evolution and the Emergence of Structure. Ph.D. thesis, Harvard University).

### Genome-wide measurements of chromatin folding inside individual contact domains reveal a polymer state characterized by γ = 0.75

In our original Hi-C study, Applicants could not discern local folding features at scales smaller than ~1 Mb. In our new study with far denser data, Applicants had the opportunity to study folding within contact domains, which are contiguous genomic intervals in which there is an enhanced probability of contact among all loci (Fig 2A). The median size of these contact domains is 185kb. On closer examination, Applicants found that folding measurements differ sharply within contact domains versus across contact domains.

Applicants began by calculating Isame(s) using our genome-wide averaging technique, but only including pairs of loci that were in the same contact domain. Strikingly, the value of y that Applicants obtained, 0.76 (Fig 2E), was markedly lower than the value obtained using the full genome-wide average.

Next, Applicants used our new maps, which contain 200- to 1000-fold more data, to measure the decay in contact probability with distance relative to a fixed DNA locus. So long as the locus was at least 50kb long, Applicants obtained highly reproducible estimates for y at any local position in the human genome. Applicants focused on 1057 distinct 50kb loci, each of which was situated at the midpoint of a high-confidence domain larger than 200kb. The resulting contact probability plots consistently exhibited two distinct regimes. The first regime corresponded to declining contact frequency within a domain (Fig 2B). Values of γ observed in this regime centered on 0.75, with a standard deviation of 0.05. Values of 1 or larger were not seen (Fig 2C). For points outside the domain, however, the contact probability continued to decline, but the power-law regularity disappeared, and was replaced by a more heterogeneous monotonic decline (Fig 2B).

Our findings suggest that, because the frequency of contact between two loci declines markedly when a contact domain boundary is crossed, I(s) - which is calculated predominantly using pairs of loci separated by such a boundary - tends to overestimate y for contact domains.

Applicants wondered whether the distribution of y for contact domains was dependent on the volume of the nucleus that contained them. To check, Applicants compared four human cell types, examining their nuclei using both in situ Hi-C and confocal microscopy. Despite observing nearly three-fold variation in nucleus size (from smallest to largest, GM12878:237±84 µm3; IMR90:381±157 µm3; NHEK: 440±90 µm3; HMEC:728±307 µm3), the intra-domain y measurements were indistinguishable (Fig 2D). The results did not vary significantly in different nuclear compartments (A/B) (12) or subcompartments (A1/A2/B1/B2/B3) (24). When Applicants examined domains in CH12-LX mouse lymphoblasts, the results were also similar. The results were also robust to changes in crosslinking conditions.

Finally, Applicants reasoned that, because the mechanism of site-directed recombination relies on the spatial proximity of pairs of DNA sites, the efficiency of site-directed recombinases might exhibit the same distance dependence observed above. Applicants therefore re-examined published experiments probing the relationship between flippase recombination frequency in human cells and the genomic distance between the two Flippase Recognition Targets (Ringrose et al. (1999) supra). Applicants found that the recombination frequency scaled as a power law with genomic distance, with y = 0.75.

Taken together, the results above suggest that chromatin folding within contact domains is characterized by a value of y that is close to 0.75. Applicants then sought to understand the implications of this exponent - in particular, whether the exponent is consistent with a fractal globule or whether it implies a different polymer state.

### A new mathematical theorem indicates that chromatin folding inside contact domains is not strictly fractal

A difficulty in interpreting experimental measurements of y is the long-standing uncertainty about the values of γ that are consistent with a fractal globule. Approximate methods and physical simulations, including those described above and in our earlier work, have suggested values of y that range from 1 to 1.2. However, no rigorous bounds have been obtained. Applicants therefore sought to derive rigorous bounds on y for a fractal globule.

Specifically, Applicants proved mathematically that the value of γ lies between 1 and 2 for any fractal globule. To do so, Applicants analyzed mathematical functions (denoted f) that continuously map (in other words, fold) the unit segment [0,1] into a higher-dimensional space. Specifically, Applicants focused on fractal curves. These counterintuitive curves are generated by applying a simple folding rule to a simple initial state, and repeating this process ad infinitum. When the folding rule is applied identically at all scales, fractal curves have no characteristic length scale. Because they can continuously transform a 1-dimensional line into a higher dimensional object, such curves have been of interest to mathematicians ever since the first space-filling curves, which map the unit segment onto the unit square, were discovered by Giuseppe Peano (the "Peano curve," in 1890) and David Hilbert (the "Hilbert curve," in 1891). If the repetition process is terminated after only a finite number of steps, the resulting curve is dense, self-similar, and corresponds to a physically realizable polymer chain; for this reason, finite iterations of fractal curves, especially the Hilbert Curve, are often used to model the fractal globule (Schram et al. (2013) J Chem Phys 138, 224901/1-11). By deriving mathematical bounds on the possible values of γ that can be obtained from fractal curves, Applicants can test whether the observed folding pattern of chromatin is consistent with a strict fractal globule.

When characterizing a fractal curve, a commonly used measure is the Minkowski (or "box-counting") dimension, denoted dim(X), which generalizes the common notion of dimension to non-integer values (Falconer (2003) Fractal geometry: mathematical foundations and applications (Wiley)). Just as the number of line segments with width 1/N needed to cover the 1-dimensional unit segment scales as N1, and the number of squares with width 1/N needed to cover the 2-dimensional unit square scales as N2, dim(X) is defined so that the number of boxes with width 1/N needed to cover X scales as Ndim(X). In this way, the Minkowski dimension can be computed for a mathematical set or measured for a physical object. For instance, the Minkowski dimension of a crumpled sheet of paper (≈2.51) provides a measure of its packing density (Gomes (1987) J Phys 20, 283-284). The Minkowski dimension of the boundary of Great Britain (≈1.25) is a measure of the roughness of its coastline (Mandelbrot

(1967) Science 156, 636-638). The Minkowski dimension of a set can also be less than 1: for instance, the set of points in the unit interval whose decimal representation does not contain an odd digit (i.e., .86, .22222) has a Minkowski dimension of 0.699.

Applicants proved mathematically that the process of folding the one-dimensional unit segment [0,1] into a d dimensional fractal curve scales the Minkowski dimension of all subsets of the segment uniformly, i.e., by a constant factor. That is, not only does the curve fold the one-dimensional unit segment into a d dimensional shape, but any k-dimensional subset of the unit segment will fold into a k*d dimensional shape. Our results can be summarized in the following theorem and corollary, whose proofs appear in the Supplemental Information:
Theorem: For any self-similar fractal curve *f([0,1])*, dim *f*(*X*) = *d* · dim *X* for any *X* ⊆ [0,1]. (The proof is in two parts. First, Applicants show that any fractal curve f is a 1/d-Hölder function, which gives an upper bound on dim X. Next, Applicants construct a push-forward measure on f(X), which gives a lower bound on dim X. Both bounds are the same and therefore give the exact value. The full proofs of the theorem and corollary are provided in the Supplemental Information.)
Corollary: The contact probability of a fractal curve satisfies *I(s)* ∝ *s^{-γ}* with γ=2-(*d_{surf}*/*d*), where s is linear distance along the curve, *d_{surf}* is the Minkowski dimension of the curve's surface (that is, the curve's roughness) and *d* is the Minkowski dimension of the curve as a whole.

An illustration of the theorem is the two-dimensional Dragon curve, which doubles the Minkowski dimension of all subsets in its domain (Fig 3A). (This result is notable from the mathematical standpoint insofar as it is a deterministic analog of Henry McKean's well-known "dimension-doubling" theorem, which states that Brownian motion doubles the dimension of subsets (McKean HP (1955) *supra*).)

The corollary may be illustrated by measuring y for classic fractal curves, such as the 2D Hilbert Curve (dsurf=1, d=2, y=3/2; Fig 3B, purple), the 3D Hilbert Curve (dsurf=2, d=3, y=4/3; Fig 3B, blue), and many others. The corollary also implies that, if a curve has an extremely rough surface (dsurf close to d), the value of γ can come arbitrarily close to unity. No such curves are known. As an illustrative example, Applicants generalized the Hilbert Curve, constructing a class of 'inside-out' Hilbert Curves (Fig 3B, teal) whose boundaries are arbitrarily rough and whose y values come arbitrarily close to 1.

Most importantly, because 0 ≤ dsurf/d < 1, the corollary proves that y for a fractal curve must lie between 1 and 2. Thus, our measurements of γ ≈ 0.75 inside contact domains (Fig 3B, red) are inconsistent with the hypothesis that contact domains tend to form fractal globules.

### Physical simulations suggest that γ = 0.75 is consistent with an unknotted, non-equilibrium state that is anisotropic rather than fractal

Another way of exploring the significance of a particular value of y is by computationally modeling chromatin as an extended homopolymer comprising numerous, identical monomers, each of which represents a fixed number of bases. By simulating the dynamics of a condensing polymer chain and the surrounding mixture under various physical assumptions, it is possible to test whether a particular set of conditions leads to a realistic y value.

In our original models, Applicants simulated an extremely simple condensation process in which the collapse of the polymer was driven by external forces, i.e., the crowding of a stretch of chromatin by other components of the nucleoplasm. Through an excluded volume interaction, these components crush the polymer chain into a smaller volume. Such forces can be modeled using a potential function that attracts all monomers equally toward a single point in space (Lieberman-Aiden et al. (2009) supra). Because this potential does not introduce a characteristic length scale into the simulations, the resulting dynamics are scale-invariant, and the polymers collapse isotropically into a fractal globule.

Notably, our earlier models did not examine the effects of internal interactions between the monomers themselves on the polymer condensation process. Attractive forces between individual nucleosomes have been observed in vitro by many groups (Clark and Kimura (1990) J Mol Bio 211, 883896; Cui and Bustamante (2000) Proc Natl Acad Sci USA 97, 127-132; Hansen (2003) Annu Rev Biophys Biomol Struct 31, 361-392; Luger and Hansen (2005) Current Opinion in Structural Biology 15, 188-196), and effective attractions between monomers are seen in all polymer globules, arising naturally when the polymer is immersed in a poor solvent (de Gennes (1985) supra; Halperin and Goldbart (1999) Phys Rev E 61(1): 565; Frisch and Verga (2002) Phys Rev E 66). Therefore, in the present study, Applicants incorporated attractive forces between monomers using the classic Lennard-Jones potential. The Lennard-Jones potential is a model of intermolecular attractions that was originally developed to study van der Waals effects, and is commonly used to describe the attractive forces between nucleosomes in polymer simulations (Wedemann and Langowski (2002) supra; Langowski J, Heermann D (2007) Sem in Cell & Dev Bio 18, 659-667). Applicants examined a class of systems in which both internal attractions between monomers and external crushing forces are present during the condensation process. The ratio of these forces is given by a single parameter, R, which represents the extent to which the system is governed by internal forces between the monomers themselves. In the course of our study, Applicants varied R over roughly eight orders of magnitude.

Applicants probed the condensation process using Langevin dynamics simulations. In this approach, random collisions between the solvent and the polymer are accounted for implicitly, through the use of parameters for both viscosity and temperature. Applicants ran our simulations using the LAMMPS software package (Plimpton S (1995) J Comp Phys 117, 1-19), accelerated using graphical processing units (Brown WM, Wang P, Plimpton SJ, Tharrington AN (2011) Comp Phys Comm 182, 898-911). Each monomer represented 1 kilobase, in order to match the above estimates of Kuhn length; the chain as a whole contained up to 10Mb, or 10,000 monomers. For each condition, Applicants ran at least 100 simulations from randomized starting configurations and calculated y as a function of R for the resulting globular states.

Our simulations revealed a family of non-equilibrium states (Fig 4A). When internal forces are weak (R≪1), the polymer collapse closely resembles the isotropic dynamics observed with pure external forces (R=0), and results in a fractal globule. However, because inter-monomeric attractions decay as the monomers move apart, internal forces introduce a length scale into the system. When they are sufficiently strong (R≫1), Applicants find that the polymer condensation process transitions into an anisotropic regime: first, tiny globules form along an extended chain; then tension along the chain causes the globules to concatenate in a linear fashion (Fig 4B). This model of polymer condensation was first postulated by the theorist de Gennes (de Gennes (1985) supra). The resulting state - which Applicants dub a "tension globule" - is not scale-invariant. Instead, it contains long intervals in which genomic position is correlated with spatial position along a linear axis.

Importantly, the values of γ obtained for the condensed state differ depending on the regime. When R is small, y(R) is slightly larger than unity, consistent with our earlier fractal globule simulations and with those of other groups. When R is large, y(R) is roughly 0.72, consistent with our observations for contact domains (Fig 4C). These two possibilities are connected by a region in parameter space where y(R) transitions from one regime to the other. Interestingly, all of the non-equilibrium states in this family are dense and largely unknotted, features that had previously been associated only with fractal globules.

Our findings were robust to variations in numerous simulation parameters. In particular, Applicants performed over 3000 simulations to confirm that the internal structure of tension globules, and the value of γ they display, was independent of the length of the polymer chain, the initial configuration of the chain, the solvent temperature, the viscosity, and the total simulation time. The results were also robust to the mechanism underlying the internal forces. They did not change significantly when Applicants replaced the Lennard-Jones potential with a Yukawa potential, a model of screened electrostatic forces, in which the attractions decay much more rapidly with distance (Chodaparambil et al. (2007) Nat struc mol bio 14(11):1105-1107).

As with our mathematical analyses, our physical simulations again suggest that the structure of nuclear chromatin inside contact domains is not consistent with a fractal globule. However, our simulations show that the structure is consistent with a tension globule resulting from a condensation regime dominated by internal attractions between the monomers themselves.

### Contact domains form spontaneously between the anchors of a loop during condensation of a tension globule

As noted in the introduction, one of the most surprising features of our in situ Hi-C maps is that contact domains often correspond to loops - that is, the two boundaries of the domain lie at the loop's two anchor loci, which are spatially proximate. Applicants dubbed this common configuration a "loop domain." Typically, each anchor site contains a motif that binds a complex containing CTCF and cohesin. These motifs almost always occur in the convergent orientation, i.e., pointing toward one another. The ubiquity of this configuration suggests that the binding of CTCF and cohesin may be responsible for the formation of loops and domains at precise genomic coordinates.

Applicants used our physical simulations to explore whether bringing together two anchor points followed by condensation into a tension globule might be sufficient to cause the intervening points to become a contact domain. Indeed, Applicants found that the formation of a loop led to enhanced contact frequency between all pairs of loci in the interval demarcated by the two loop anchors, i.e., to the formation of a contact domain (Fig 4D). These contact domains exhibited values of γ that match our experimental observations (y = 0.77 ± 0.077 for simulated domains, versus y = 0.75 ± 0.05 for domains observed in Hi-C maps).

Thus, contact domains could arise through the formation of a tension globule in which the anchor points of a loop come into contact through diffusion in three dimensions, and this contact is stabilized by a protein complex containing CTCF and cohesin.

However, this model does not account for the fact that loops typically involve consecutive anchor points. Simple three-dimensional diffusion would tend to produce a tangle of overlapping loops (that is, a point in the interior of one loop anchored to a point outside the loop). It is also difficult to understand how such a model could lead to the strong tendency of CTCF/cohesin binding motifs at pairs of loop anchors to lie in a convergent orientation. Applicants therefore considered alternative models for loop formation.

### The data are consistent with loop formation by extrusion complexes

Nasmyth (Nasmyth K (2001) Annu Rev Genet 35:673-745; Alipour E, Marko JF (2012) Nucl Acids Res 1-11) proposed a model based on an "extrusion complex" (composed of proteins and possibly other biomolecules) containing two DNA binding subunits that are physically tethered together. The extrusion complex is loaded onto chromatin at a single locus; initially, its subunits are bound to nearby DNA elements, forming a tiny chromatin loop between them. Next, DNA is extruded through the subunits such that the two subunits move in opposite directions with respect to the genome: one forward, one reverse. (Of course, the subunits are not moving with respect to one another in 3D space, since they are part of a single complex.) As a result, a growing loop is extruded until the extrusion complex - whose processivity is assumed to be finite - eventually dissociates from DNA (Fig 5A, i - iii).

Applicants explored the behavior of extrusion complexes in our simulations as follows. The extrusion complexes are bound to the polymer at a density that depends on their concentration, and they dissociate at a rate that depends on their processivity. Extrusion complexes cannot pass through one another. If the loops produced by neighboring extrusion complexes are immediately adjacent at any point (i.e., the DNA element in the forward subunit of one extrusion complex is too close to the DNA element in the reverse subunit of the next extrusion complex), then one of the two colliding complexes falls off.

Applicants added one novel feature to Nasmyth's model, based on our observations about the role and orientation of CTCF/cohesin motifs. Applicants designated certain monomers as anchors, and assigned each anchor a forward or reverse orientation. Applicants assume that the DNA binding subunits of the extrusion complex preferentially recognize specific DNA sequences and have orientations, with one recognizing forward anchors and one recognizing reverse anchors. Under these assumptions, the extrusion process proceeds in the following way: the progress of the forward subunit of an extrusion complex may be halted by a forward anchor, but not by a reverse anchor; and conversely the progress of the reverse subunit of an extrusion complex may be halted by a reverse anchor, but not by a forward anchor. In short, the two subunits recognize the presence of a particular motif on a particular DNA strand - such as an appropriately oriented CTCF/cohesin motif - by more tightly binding the target DNA element, and halting the extrusion process through the subunit.

Applicants began by simulating a polymer containing pairs of convergent anchors 1Mb apart. When an extrusion complex landed between the anchors, it began extruding a loop until its subunits eventually arrived at the two anchor monomers. At this point, the extrusion came to a halt, yielding a "persistent loop" between the anchors: i.e., a loop that was present for a protracted period (Fig 5B). Eventually, the extrusion complex dissociated from the polymer. When Applicants examined the contact maps for the polymers in our simulation, Applicants made three observations. First, a prominent peak was present between the two anchors, reflecting the formation of a persistent loop. Second, extrusion of the chromatin fiber led to enhanced contact frequency between all pairs of loci in the interval between the two anchors, i.e., to the formation of a contact domain. Finally, Applicants found that these contact domains exhibit extremely linear contact probability scalings with values of y that match our experimental observations (y = 0.72 ± 0.06 for simulated domains, versus y = 0.75 ± 0.05 for domains observed in Hi-C maps). These findings reflect the equilibrium state of a long polymer immersed in a solvent containing extrusion complexes. They were extremely robust, and did not depend on the inter-monomeric potential (external or internal) and the initial condition (fractal globule, tension globule, or extended filament). When Applicants modeled more complex arrangements of loop anchors, Applicants found that pairs of convergent anchors led to both persistent loops and contact domains with realistic y values.

In contrast to the model in which tension globules form by condensation with loop anchors finding each other by diffusion, the extrusion model with oriented DNA-binding/recognition subunits has many attractive features.

### Intra-domain distances measured by 3D-FISH match simulation results for both tension globules and the extrusion model

Applicants examined whether the tension globule model and extrusion model recapitulate spatial distances observed experimentally. Applicants examined 4 pairs of loci using 3D-FISH. Each pair lay in a single domain; the genomic distance between the loci ranged from 320kb to nearly a megabase. Applicants measured at least 50 3D distances for each locus pair. Applicants compared the resulting distributions to distributions for monomers at a comparable distance from one another obtained using our simulations of both tension globules and extrusion models. In both cases, Applicants found that the simulated distributions matched the experimental distributions almost as closely (Kolmogorov-Smirnov statistic with tension globule: 0.15; K-S statistic with extrusion model: 0.19) as experimental replicates matched one another (K-S statistic: 0.18). Thus, both models not only accurately recapitulate the observed contact probabilities, but they also recapitulate the observed distribution of 3D distance measurements.

### The network of loops contains hundreds of isolated cliques, consistent with chromatin rosettes formed by consecutive extrusion complexes

Finally, Applicants explored higher-order relationships among the location of loops. To probe these relationships, Applicants constructed a "loop network" for GM12878 lymphoblastoid cells. The nodes of this network are genomic loci containing at least 1 loop anchor, and its edges indicate the presence of a loop connecting the incident loci. Applicants then sought to find network motifs - patterns of nodes and edges whose frequency was higher than expected.

Applicants were particularly interested in 'isolated cliques' in the loop network. An isolated clique consists of a set of N ≥ 3 loci such that any pair in the set is connected by a loop (i.e., the set is a 'clique') but none of the loci are connected by loops with loci outside the set (i.e., the set is 'isolated') (Fig 6).

To identify isolated cliques, Applicants allowed for errors in loop calling. Because an isolated clique of size N has N(N-1)/2 loops, even a small false negative rate will prevent the recognition of large cliques. To account for false negatives, Applicants allowed cliques in which up to N-2 of the N(N-1)/2 loops satisfy a less stringent threshold than used in our standard loop annotation procedure (Rao et al. (2014) supra).

Applicants found that isolated cliques were dramatically enriched in the loop network. For instance, in GM12878 Applicants observed 206 isolated cliques with 3 nodes (9-fold enrichment), 16 cliques with 4 nodes (28-fold), and 1 clique with 5 nodes (161-fold). (The enrichments are relative to an ensemble of randomized control networks analyzed under the same procedures.)

Applicants also re-analyzed the data after allowing for a small number of loops (≤N-2) between loci inside the clique and loci outside the clique. The number of isolated cliques identified rises substantially: in GM12878, Applicants observed 567 isolated cliques with 3 nodes (6.2-fold enrichment relative to an ensemble of randomized control networks analyzed under the same criteria), 86 cliques with 4 nodes (12-fold), 5 cliques with 5 nodes (14-fold), and one clique with 6 nodes (41-fold).

These cliques had several notable features. First, they typically (in 63% of cases) involved a series of consecutive loops, i.e., the clique loci were positioned one-after-another in the human genome, with no other loop anchors intervening. This feature is consistent with the fact that loops tend not to overlap. Second, the clique loci exhibited a highly characteristic pattern of CTCF binding. The first clique locus (the locus closest to the p-terminus of the chromosome) typically contained a bound CTCF motif in the forward orientation (97%, an 4.3-fold enrichment). The last clique locus contained a bound CTCF motif in the reverse orientation (97%, an 4.1-fold enrichment). The middle clique loci typically contained a pair of nearby CTCF motifs pointing away from one another (in 52% of cases, a 6.1-fold enrichment; median distance: 4.6kb), such that the first motif pointed toward the preceding clique locus and the second motif pointed toward the subsequent clique locus. This divergent configuration at clique loci is thus consistent with the convergent rule for CTCF looping that Applicants recently described. It is also consistent with the requirement that loops cannot overlap, even if the overlap is small.

One possible interpretation of these isolated cliques is the formation of a "chromatin rosette" comprising a set of consecutive loops whose anchors are all simultaneously co-located at a single spatial hub. As such, our data suggest hundreds of possible chromatin rosettes located in an interphase human genome. The existence of rosettes has been proposed by several groups (Sachs R, Engh G, Trask B, Yokota H, Hearst J (1995) PNAS 92, 2710-2714; León P, Macaya G (1983) Chromosoma 88(4): 307-314), and the presence of other types of hubs has been carefully documented at individual loci such as beta-globin (Splinter E et al. (2006) Genes Dev 20(17):2349-54).

Interestingly, the extrusion model predicts that the genomic intervals inside chromatin loops can be nearly adjacent in the genomic sequence, but cannot overlap. This is precisely what is seen in a chromatin rosette. In contrast, the model of anchor points being brought together by diffusion is less likely to produce rosettes.

It is important to emphasize one major limitation of our analysis. While the pattern of higher-order relationships among loops is consistent with the possibility of chromatin rosettes occurring in individual nuclei, our data are based on pairwise contacts across an ensemble of cells. From these data, Applicants cannot tell whether the various loops in an isolated clique occur simultaneously in individual nuclei. Of course, it is possible that some of our cliques reflect simultaneous loops, whereas others do not.

### Both models can be used to recapitulate Hi-C experimental results given the locations of CTCF binding

Next, Applicants sought to explore whether our models could be used to recapitulate Hi-C experimental results in silico using CTCF ChIP-Seq data alone.

Applicants began by using the extrusion model to simulate the folding of a 2.3Mb target region on chromosome 4 (20.3-22.6Mb). Our algorithm created an in silico representation of the region as a uniform polymer and then added forward and reverse anchors placed at the binding sites of CTCF observed in experimental ChIP-Seq data for the region. The strength of each anchor (i.e., the likelihood that a subunit on the appropriate strand would halt when sliding across the anchor) reflected the amplitude of the CTCF peak. The orientation of each anchor was assigned based on the strand of the CTCF motif associated with the peak. The algorithm did not use Hi-C data as an input. Applicants then simulated the results of exposing this model polymer to a solvent containing extrusion complexes, which functioned as described in our extrusion model. Applicants found that the contact matrix resulting from these simulations closely resembled the contact matrix obtained using Hi-C experiments. In particular, the position of peaks and contact domains in the simulated matrix corresponded to what was observed in our kilobase resolution Hi-C experiment, and appropriate y values were obtained inside contact domains. When Applicants repeated this procedure for other target regions, the results were similar.

Next, Applicants sought to simulate the same target region using the tension globule model. As before, Applicants identified peaks in CTCF ChIP-Seq data, and assigned each peak an orientation based on the strand of the CTCF motif associated with the peak. In order to achieve a reasonable correspondence with experimental results, Applicants had to impose a number of rules and fit various parameters: loops were only allowed between pairs of convergent peaks, and the likelihood of such a loop depended on: (i) the strength of the peaks; (ii) the distance between the peaks; and (iii) the number and strength of intervening CTCF peaks. Applicants fit the parameters so that the frequencies of the loops matched the frequencies estimated from ChIP-Seq data. The results of the simulation were similar to the results of Hi-C experiments (Pearson's r = 0.922).

Importantly, the tension globule model differs from the extrusion model in that achieving a good fit requiring imposing various ad hoc penalties, which do not correspond to any natural processes in three-dimensional diffusion. Even so, the fit was not as accurate as the fit produced by simulations based on loop extrusion. By contrast, the extrusion model involved much more natural assumptions.

### Genome editing of CTCF/cohesin motifs disrupts corresponding loops and contact domains

In our Hi-C data, the formation of loops is strongly associated with the presence of a pair of CTCF motifs in the convergent orientation. Both of the physical models described above suggest that these motifs play a causal role in loop formation.

To study the formation of loops experimentally, Applicants used CRISPR/Cas9-based genome editing to modify CTCF motifs in a targeted fashion and then explored the resulting changes in loop structure.

Applicants focused on HAP1, a human, haploid, fibroblast-like cell line, because the use of a haploid cell line avoids the issues raised by allelic heterogeneity for both Hi-C and CRISPR experiments. Applicants generated an in situ Hi-C map of wild-type HAP1 cells, with 1.1B reads. Applicants annotated 8,334 loops and 4,332 contact domains in the map.

Based on this map, Applicants chose to study a target region on chromosome 8 containing three loci: A (133.9 Mb), B (134.2 Mb), and C (134.5 Mb). Each pair of these three loci form loops with one another, consistent with the presence of a hub. CTCF sites are present at each locus in accordance with the convergent rule: locus A has a forward-oriented CTCF motif (dubbed A/Forward); locus B has a reverse-oriented CTCF motif (B/Reverse) followed by a forward-oriented motif (B/Forward) (the two motifs do not overlap); and locus C has a reverse-oriented motif (C/Reverse). All three loops are associated with contact domains.

Under the convergent rule, Applicants would predict that deleting the B/Forward site would disrupt the loop between B and C but have no effect on the other two loops. In particular, disruption would not affect the loop between A and B, which, according to the convergent CTCF rule, would be anchored at B/Reverse rather than B/Forward. To test this hypothesis, Applicants performed genome editing to create a deletion in the B/Forward motif and grew a clonal population of the resulting cells. Applicants then repeated the in situ Hi-C experiment on the disrupted cells, and mapped loops genome-wide. As predicted by the convergent rule, the loop from B to C was disrupted. The A/B and A/C loops were not affected. More generally, Applicants did not observe significant alteration of any loop, genome-wide, in the mutant cells, with the exception of the B/C loop.

Applicants then used genome-editing to test additional predictions of the convergent rule. To reduce sequencing costs, Applicants developed an inexpensive way to monitor the results only in the target region by performing HYbrid Capture on the in situ Hi-C library, a method Applicants dubbed "Hi-C2". (Applicants validated the Hi-C2 method by applying it to wild-type HAP1 and our B/Forward deletion mutant, and confirmed that the results were equivalent to those obtained using ordinary in situ Hi-C.)

Applicants tested two further predictions of the convergent rule in the target region: (i) inversion of the B/Forward site should have the same effect as deletion of the site - namely, the B/C loop should disappear; and (ii) deletion of B/Reverse should lead to the disappearance of the A/B loop. In both cases, the experimental Hi-C2 data matched these predictions.

Next, Applicants probed a second target region, on chromosome 1, containing three loci: D (@180.5Mb), E(@180.8Mb), and F(@181.1Mb) whose contact map was again consistent with the presence of a hub: all three were connected to one another by loop domains; each loop is associated with CTCF motifs in the convergent orientation. As before, deletion of E/Forward led to the disappearance of the E/F loop. When Applicants took the E/Forward mutant and further deleted E/Reverse, the D/E loop disappeared. The D/F loop remained, as predicted by the convergent rule.

Finally, Applicants targeted a third region, on chromosome 5, containing three loci: G (@180.5Mb), H (@180.8Mb), and I (@181.1Mb) whose contact map was again consistent with the presence of a hub. Applicants inserted a single base pair into the G/Forward site, thereby disrupting the CTCF binding site. Both the G/H loop and the G/I loop disappeared.

In every single case above, the convergent rule predicted exactly which loops would be affected by a genome editing experiment. These results confirm that convergent CTCF sites play a causal role in the formation of loops, and show that it is possible to re-engineer chromatin loops in a targeted fashion.

It is noteworthy that the experiments described above targeted isolated cliques in HAP1, similar to those revealed in our network analysis of GM12878. If the loops in these cliques had been simultaneous, disruption of only one loop would be impossible. (If loops A/B and A/C are present simultaneously, then B and C must also be in close proximity.) Our ability to disrupt B/C alone, without eliminating either A/B or A/C (and the similar findings when Applicants disrupted loops A/B and E/F) suggests that, in the case of the two cliques in question, the loops are not simultaneous.

These experiments also shed light on the mechanisms of contact domain formation. In all but two cases, the disruption of loops led to the attenuation, but not the disappearance, of the contact domain spanned by the loop. This behavior is not expected under the tension globule model, which would predict the complete disappearance of the contact domain. However, it may be associated with a behavior seen in our extrusion simulations. In our extrusion simulations, a genomic interval bounded by two loop anchors that do not loop to one another still forms a domain, since the sliding of extrusion complexes in the interval can be impeded by other extrusion complexes whose subunits occupy the loop anchors. In fact, the cases where contact domains remained after editing of a loop anchor locus are examples of this scenario: they were all cases in which two loop anchor motifs were present at the locus (forward and reverse), but only one motif - and only one loop - was disrupted. In order to completely eliminate a domain in our extrusion simulations, it is necessary to disrupt all loop anchor motifs located at the domain's boundary, so that the boundary locus ceases to be a loop anchor. Strikingly, this behavior is seen in our editing experiments. The disruption of the forward motif at G, a locus which contains no other loop anchor motif, led to the disappearance of the contact domain bounded at G. Similarly, the simultaneous disruption of both E/Forward and E/Reverse, leaving no other loop anchor motifs at locus E, led to the disappearance of both the D/E and E/F domains. As such, there is a strong correspondence between the contact maps obtained experimentally in our genome editing experiments and the contact maps predicted by our loop extrusion simulations. Our results suggest that it may be possible to re-engineer contact domains in a targeted fashion.

### Discussion

With the dramatic improvements in resolution that can be achieved using *in situ* Hi-C, it is now possible to probe the physical and mechanical properties of chromatin genome-wide. Our results illuminate the structure of chromatin at multiple scales: chromatin fibers, contact domains, and loops.

At the smallest scale, the winding of DNA around histones has long been known to form the flexible 10nm fiber. This fiber is widely believed to coil into the larger, stiffer 30nm fiber, although recent studies using microscopy, electron spectroscopy, and X-ray scattering have failed to find evidence for 30nm fibers in vivo (Fussner E et al. (2012) EMBO Rep 13, 992-996; Joti Y et al. (2012) Nucleus 3, 404-410; Nishino Y et al. (2012) EMBO J 31, 1644-1653; Ricci MA, Manzo C, Garcia-Parajo M, Lakadamyali M, Cosma MP (2015) Cell 160, 1145-1158). Our Hi-C data allows us to measure the Kuhn length, or bendability, of chromatin fibers, and to thereby compare the mechanical properties of fibers in vivo to the values predicted under various models. Strikingly, Applicants find that chromatin fibers are highly bendable, with a Kuhn length of roughly 1kb. This value is far smaller than what would be expected for a 30nm fiber (30-60kb) (Wedemann and Langowski (2002) supra; Schiessel (2003) supra), suggesting that 30nm fibers, if they exist, are rare in intact chromatin. Interestingly, our findings suggest that, at the scale of the typical gene (~15kb), chromatin is highly flexible. This observation is broadly relevant to physical models of loop formation, transcription, and replication. Of particular relevance for the present study, the flexibility of chromatin fibers inferred from our experimental Hi-C data is compatible with (and essential for) loop and domain formation through extrusion.

In our original Hi-C study (Lieberman-Aiden et al. (2009) supra), Applicants probed the physical structure of chromatin at the megabase scale by calculating the relationship between the 1D distance separating two loci, s, and the probability of physical contact between them, I(s). Because the size of our dataset was limited, Applicants performed this calculation using a genome-wide average. For values of s between 500kb and 7Mb, Applicants found power-law behavior: specifically, I(s) ∝ s-y with y=1.08. This value of γ was inconsistent with an ordinary condensed polymer at equilibrium (for which y = 1.5) but is consistent with a fractal globule. Fractal globules are an appealing model for chromatin because they are dense and unknotted, suggesting how chromatin can be tightly packed while remaining physically accessible. The value of the genome-wide average has been reproduced in many subsequent studies, including this one, with similar results.

In our recent Hi-C experiments at kilobase resolution (Rao et al. (2014) supra), Applicants observed a large number of contact domains (median length, 200kb) that together partition the genome. In the present study, Applicants explore the structure of chromatin inside individual domains by exploiting the vastly higher resolution of our new maps to calculate I(s), in a locus-specific fashion, genome-wide. The contact probability exhibits a power-law behavior at fine scale, but with a different exponent, y = 0.75, than that observed from our low-resolution genome-wide average. Applicants show that this value is robust across domains, cell types, and species, and is independent of nuclear volume. It is also robust to changes in experimental conditions, such as the use or the specific conditions of crosslinking. Notably, Applicants find that measurements of y obtained from averages over larger scales overestimate the value of y within domains, because the contact frequency declines sharply when a domain boundary is crossed. When only pairs of loci that lie in the same domain are included, Applicants show that genome-wide averages also yield a value of γ = 0.75.

The value y = 0.75 is inconsistent with an ordinary polymer at equilibrium. To rigorously determine whether such a value could be consistent with a fractal globule architecture for individual domains, Applicants proved a novel mathematical theorem describing how the Minkowski (fractal) dimension of a set changes when the set is mapped using a fractal curve. As a corollary, Applicants find that values of y inside a fractal globule must lie between 1 and 2, implying that chromatin inside domains is inconsistent with the fractal globule model. Interestingly, our theoretical observations highlight the growing potential of genomic datasets to suggest increasingly sophisticated mathematical results in areas seemingly unrelated to biology. In the present case, our theorem provides a deterministic analog of a well-known result by McKean for Brownian motion (McKean HP (1955) Duke Math J 22, 229-234). Moreover, Applicants illustrate our corollary by constructing a novel variant of the famous Hilbert curve, first described by David Hilbert in 1891. In Hilbert's original construction, a 1D curve snakes continuously through a smooth-bounded 2D square, filling all points as it passes. Our "Inside-Out" Hilbert curve snakes through a 2D shape with arbitrarily rough fractal boundaries. Our findings suggest that the study of genome folding as a whole may - perhaps unexpectedly - serve as a fruitful catalyst for discoveries in mathematics.

Another way of interpreting values of y is by using physical simulations to identify polymer states with similar y values. In our original Hi-C study, Applicants showed that a polymer that was crushed by external forces naturally folds into a fractal globule with a value of γ = 1. However, there is also evidence that attractive forces exist between nucleosomes and other chromatin components. Therefore, in the present work, Applicants considered the possibility that internal forces - attractions between pairs of monomers - may also play a role. Specifically, Applicants used molecular dynamics simulations to probe the condensation of a polymer in response to a combination of external and internal forces. Applicants found that varying the ratio of internal and external forces results in a family of possible structures, all of which are dense and unknotted. Within this family, two dominant regimes are observed. At one extreme - when external forces dominate - the result of the condensation process is symmetric, yielding a classic fractal globule with y = 1. At the other extreme - when internal forces dominate - tension arises along the polymer chain, leading to anisotropic condensation with y = 0.72. Thus, the value of y observed in these "tension globules" closely matches the value of γ observed in Hi-C contact domains. Tension globules contain long stretches in which position along the polymer in 1D correlates with position along a linear axis in 3D, and closely resemble a non-equilibrium polymer state first postulated by de Gennes. When Applicants explored the consequences of loop formation in a tension globule, Applicants found that it leads to the formation of a contact domain and does not affect the value of y. Taken together, the kilobase-resolution maps published in our recent report are consistent with the existence of tension globules in which loops are associated with the formation of contact domains.

Nonetheless, the tension globule model has important drawbacks. These drawbacks emerge from the putative mechanism of loop formation in a tension globule, which is the classic model of loop formation through diffusion. In this model, looping proteins (such as CTCF) initially bind to DNA anchor motifs. When diffusion brings two anchors into close spatial proximity, the proteins dimerize, forming a chromatin loop between the anchor motifs. This diffusive process may take a long time, and would tend to lead to a tangle of overlapping loops. It is also hard to understand, in a diffusive model, why the CTCF/cohesin motifs at pairs of loop anchors must lie in the convergent orientation.

To overcome these limitations, Applicants explore a different model of loop formation based on a proposal by Nasmyth (Nasmyth K (2001) Annu Rev Genet 35:673-745; Alipour E, Marko JF (2012) Nucl Acids Res 1-11), who hypothesized that loops form during metaphase chromosome condensation through the action of an extrusion complex comprising two tethered DNA binding subunits, each of which extrude DNA as they slide - relative to the genome - in opposite directions. He specifically suggested that such a process might involve cohesin proteins, which form a tripartite ring that can slide along DNA and chromatin. To date, little direct evidence has been observed in support of this model.

Applicants show, by means of physical simulations, that the extrusion of a loop leads to the formation of a contact domain between the loop's two anchors, whose y value closely matches the value seen in our Hi-C maps. The kilobase-resolution Hi-C maps are thus consistent with both models (and may be consistent with other models as well).

In fact, Applicants show that simulations with both models can be used to recapitulate the results of Hi-C experiments, using only data about CTCF-binding sites from ChIP-Seq. The contact matrices resulting from such simulations correlate strongly with the results of kilobase-resolution Hi-C experiments at short range (<2Mb), and there is a strong correspondence between the position of peaks and contact domains in our simulations and their position in actual Hi-C experiments. Notably, the tension globule simulations required ad hoc penalties for loops between non-consecutive CTCF motifs, and yielded less accurate results.

Several possibilities exist for the structure of the extrusion complex. One possibility is that the extrusion complex comprises two cohesin rings and two CTCF proteins (Fig 8). The complex is loaded onto DNA via loading of the cohesin rings at adjacent DNA sites and the simultaneous binding of the CTCF proteins nearby. Each CTCF/cohesin pair serves as a single DNA binding subunit. These subunits extrude DNA in opposing directions: one is a forward subunit, and the other is a reverse subunit.

Because DNA strands are intrinsically oriented (5'→3'), the forward and reverse subunits must engage the two DNA anchor sites in antisymmetric fashion in order for them to slide in opposite directions. Although additional structural studies would be needed to confirm this hypothesis, Applicants can use ChIP-Seq to look for antisymmetric behavior in the relative positioning of CTCF and Cohesin with respect to chromatin loop anchors. Despite the fact that both proteins are associated with the same DNA motif, Applicants find that they exhibit antisymmetric behavior: CTCF tends to be positioned near the motif, towards the outside of a loop, whereas RAD21 and SMC3 are positioned approximately 20bp away, towards the loop interior. In other words, the CTCF/cohesin motif at a loop anchor points away from the centroid of the CTCF peak and toward the centroid of the RAD21 and SMC3 peaks. This supports the notion that the forward and reverse subunits engage DNA in antisymmetric fashion, and suggests that the cohesin ring trails behind the CTCF protein as they slide along DNA, with the CTCF protein serving as a "brake" that is capable of stalling the extrusion process.

If the extrusion complex subunits engage DNA antisymmetrically, then, as chromatin is extruded, the zinc fingers of the two CTCF proteins track along opposite strands of the DNA double helix. Sliding continues in either direction until it is either slowed or stopped by the presence of a CTCF motif on the appropriate strand.

Of course, there are other possible models. For instance, the extrusion complex may include CTCF, but not cohesin. In such a model, cohesin binding would occur only after an extrusion complex containing CTCF has formed a long-range loop. Alternatively, the extrusion complex may include cohesin, but not CTCF. In such a model, CTCF proteins bind independently to their target motif, and either permit or prevent the sliding of cohesin rings depending on their orientation.

Although both the tension globule and the extrusion model are consistent with our kilobase-resolution maps, the latter model both (i) leads to better simulation results, and (ii) more importantly, naturally explains two key features not explained by the tension globule. First, extruded loops cannot overlap, whereas simple diffusion is likely to result in substantial overlap and entanglement. In fact, Applicants rarely observe overlapping loops in our data (4-fold depletion). Instead, the putative rosettes suggest that consecutive loops span adjacent but nonoverlapping genomic intervals. Second, the extrusion model can explain why loops must lie between convergent CTCF motifs.

Our physical simulations fail to explain one important feature of Hi-C data: the observation that contact domains fall into at least two compartments and six subcompartments, each consisting of loci that tend to show similar patterns of long-range interactions. Compartmentalization, seen in humans and many other species, manifests as a plaid arrangement in Hi-C maps. The fact that these compartments are not recapitulated by our simulations indicates that, although CTCF and cohesin help establish loop anchors and contact domain boundaries, other mechanisms are responsible for positioning each contact domain in the appropriate nuclear neighborhood. In particular, Applicants have previously reported that compartments and subcompartments tend to be associated with distinctive chromatin modifications.

Our work also demonstrates that it is possible to re-engineer loops and domains in a targeted fashion by modifying a small number of bases in the CTCF/cohesin motifs that lie at loop anchors. The effect on the loops depends on the orientation of the CTCF/cohesin motif, consistent with the convergent rule for looping described in our previous report. Applicants show that inserting a single base pair is sufficient to eliminate multiple loops and domains, thereby affecting genome folding at the megabase scale. Moreover, Applicants show that our extrusion model simulations can predict the Hi-C contact map of an engineered locus using only binding sites for CTCF in wild-type cells as input.

The ability to read out the 3D structure of a genome has improved rapidly in recent years. As shown by our genome-editing experiments, it may now be possible to not only "read" 3D-folding patterns, but to write them. Going forward, the ability to interpret what Applicants read and write in terms of physical mechanisms remains a central challenge, and it will be important to devise experimental tests that can directly distinguish between the possible models of chromatin structure. These tests will likely include efforts to interfere with the function of CTCF and cohesin. In some examples, the methods described herein can be used for engineering genomes in 3D, 3D prediction (how will it fold); de novo genome assembly for whole genomes; reading and writing genomes in 3D and predicting structures as well as many other applications.

### RNA-seq

In certain embodiments, the invention involves single cell RNA sequencing (see, e.g., Kalisky, T., Blainey, P. & Quake, S. R. Genomic Analysis at the Single-Cell Level. Annual review of genetics 45, 431-445, (2011); Kalisky, T. & Quake, S. R. Single-cell genomics. Nature Methods 8, 311-314 (2011); Islam, S. et al. Characterization of the single-cell transcriptional landscape by highly multiplex RNA-seq. Genome Research, (2011); Tang, F. et al. RNA-Seq analysis to capture the transcriptome landscape of a single cell. Nature Protocols 5, 516-535, (2010); Tang, F. et al. mRNA-Seq whole-transcriptome analysis of a single cell. Nature Methods 6, 377-382, (2009); Ramskold, D. et al. Full-length mRNA-Seq from single-cell levels of RNA and individual circulating tumor cells. Nature Biotechnology 30, 777-782, (2012); and Hashimshony, T., Wagner, F., Sher, N. & Yanai, I. CEL-Seq: Single-Cell RNA-Seq by Multiplexed Linear Amplification. Cell Reports, Cell Reports, Volume 2, Issue 3, p666-673, 2012).

In certain embodiments, the invention involves plate based single cell RNA sequencing (see, e.g., Picelli, S. et al., 2014, "Full-length RNA-seq from single cells using Smart-seq2" Nature protocols 9, 171-181, doi:10.1038/nprot.2014.006).

In certain embodiments, the invention involves high-throughput single-cell RNA-seq. In this regard reference is made to Macosko et al., 2015, "Highly Parallel Genome-wide Expression Profiling of Individual Cells Using Nanoliter Droplets" Cell 161, 1202-1214; International patent application number PCT/US2015/049178, published as WO2016/040476 on March 17, 2016; Klein et al., 2015, "Droplet Barcoding for Single-Cell Transcriptomics Applied to Embryonic Stem Cells" Cell 161, 1187-1201; International patent application number PCT/US2016/027734, published as WO2016168584A1 on October 20, 2016; Zheng, et al., 2016, "Haplotyping germline and cancer genomes with high-throughput linked-read sequencing" Nature Biotechnology 34, 303-311; Zheng, et al., 2017, "Massively parallel digital transcriptional profiling of single cells" Nat. Commun. 8, 14049 doi: 10.1038/ncomms14049; International patent publication number WO2014210353A2; Zilionis, et al., 2017, "Single-cell barcoding and sequencing using droplet microfluidics" Nat Protoc. Jan;12(1):44-73; Cao et al., 2017, "Comprehensive single cell transcriptional profiling of a multicellular organism by combinatorial indexing" bioRxiv preprint first posted online Feb. 2, 2017, doi: dx.doi.org/10.1101/104844; Rosenberg et al., 2017, "Scaling single cell transcriptomics through split pool barcoding" bioRxiv preprint first posted online Feb. 2, 2017, doi: dx.doi.org/10.1101/105163; Vitak, et al., "Sequencing thousands of single-cell genomes with combinatorial indexing" Nature Methods, 14(3):302-308, 2017; Cao, et al., Comprehensive single-cell transcriptional profiling of a multicellular organism. Science, 357(6352):661-667, 2017; and Gierahn et al., "Seq-Well: portable, low-cost RNA sequencing of single cells at high throughput" Nature Methods 14, 395-398 (2017).

In certain embodiments, the invention involves single nucleus RNA sequencing. In this regard reference is made to Swiech et al., 2014, "In vivo interrogation of gene function in the mammalian brain using CRISPR-Cas9" Nature Biotechnology Vol. 33, pp. 102-106; Habib et al., 2016, "Div-Seq: Single-nucleus RNA-Seq reveals dynamics of rare adult newborn neurons" Science, Vol. 353, Issue 6302, pp. 925-928; Habib et al., 2017, "Massively parallel single-nucleus RNA-seq with DroNc-seq" Nat Methods. 2017 Oct;14(10):955-958; and International patent application number PCT/US2016/059239, published as WO2017164936 on September 28, 2017.

In certain embodiments, accessible chromatin is assayed by tagmentation after modulation of loops (e.g., by eliminating cohesin). The term "tagmentation" refers to a step in the Assay for Transposase Accessible Chromatin using sequencing (ATAC-seq) as described. (See, Buenrostro, J. D., Giresi, P. G., Zaba, L. C., Chang, H. Y., Greenleaf, W. J., Transposition of native chromatin for fast and sensitive epigenomic profiling of open chromatin, DNA-binding proteins and nucleosome position. Nature methods 2013; 10 (12): 1213-1218). Specifically, a hyperactive Tn5 transposase loaded in vitro with adapters for high-throughput DNA sequencing, can simultaneously fragment and tag a genome with sequencing adapters. In certain embodiments, tagmentation is used to introduce adaptor sequences to genomic DNA in regions of accessible chromatin (e.g., between individual nucleosomes) (see, e.g., US20160208323A1; US20160060691A1; WO2017156336A1; J. D. Buenrostro et al., Single-cell chromatin accessibility reveals principles of regulatory variation. Nature 523, 486-490 (2015); and Cusanovich, D. A., Daza, R., Adey, A., Pliner, H., Christiansen, L., Gunderson, K. L., Steemers, F. J., Trapnell, C. & Shendure, J. Multiplex single-cell profiling of chromatin accessibility by combinatorial cellular indexing. Science. 2015 May 22;348(6237):910-4. doi: 10.1126/science.aab1601. Epub 2015 May 7).

In certain embodiments, genes that may be associated with chromatin looping are screened by perturbation of target genes. Methods and tools for genome-scale screening of perturbations in single cells using CRISPR-Cas9 have been described, herein referred to as perturb-seq (see e.g., Dixit et al., "Perturb-Seq: Dissecting Molecular Circuits with Scalable Single-Cell RNA Profiling of Pooled Genetic Screens" 2016, Cell 167, 1853-1866; Adamson et al., "A Multiplexed Single-Cell CRISPR Screening Platform Enables Systematic Dissection of the Unfolded Protein Response" 2016, Cell 167, 1867-1882; and International publication serial number WO/2017/075294).

All documents cited or referenced in herein cited documents, together with any manufacturer's instructions, descriptions, product specifications, and product sheets for any products mentioned herein may be employed in the practice of the invention.

For the purpose of clarity and a concise description, features are described herein as part of the same or separate embodiments. However, it will be appreciated that the scope of the invention may include embodiments having combinations of all or some of the features described.

The following examples are provided to illustrate certain particular features and/or embodiments. These examples should not be construed to limit the invention to the particular features or embodiments described.

### EXAMPLES

### Example 1 - Rapid degradation of RAD21 using an auxin-inducible degron system

Applicants employed an auxin-inducible degron (AID) (Natsume et al., 2016) to destroy RAD21, a core component of the cohesin complex. In this system, constitutive expression of the auxin-activated ubiquitin ligase TIR1 leads, in the presence of auxin, to rapid degradation of proteins tagged with an AID domain. Applicants used this system in HCT-116, a human colorectal carcinoma cell line. This cell line had been previously modified by (Natsume et al., 2016) so that both RAD21 alleles were tagged with an AID domain and a fluorescent mClover ("RAD21-mAC") (**Fig. 1A**). Applicants confirmed that RAD21-mAC was efficiently degraded after 6 hours of auxin treatment using fluorescence microscopy and ChIP (**Fig. 1B****,** **8****, see Methods),** and that cohesin could no longer associate with DNA using ChIP-Seq with antibodies for SMC1, a different cohesin subunit (**Fig. 1C****,D**).

### Example 2 - Histone modification patterns are unaffected by cohesin loss

Applicants first examined the effects of cohesin degradation on key epigenomic features associated with genome folding, using ChIP-Seq. Applicants examined the distribution of CTCF (associated with loop anchors) and the histone modifications H3K27me3, H3K36me3, H3K27Ac, H3K4me1, H3K4me3, H3K9me3, H4K16Ac, H4K20me3, H3K79me2, and H2.AZ (associated with compartment intervals). Cohesin loss had little effect on these features (**Fig. 1C****,D;** **Fig. 8C**-**E, see Methods).**

### Example 3 - Loop domains are rapidly lost after degradation of cohesin

Applicants then turned to study genome folding itself, beginning with loop domains. Loops arise when two loci on the same chromosome are tethered together. (For clarity, the loci will be referred to as "loop anchors", the tethered pair as a "link", and the interval between them as a "loop".) Loop anchors are typically a pair of DNA motifs in the convergent orientation (i.e., the motifs face each other) that bind CTCF and cohesin (Rao et al., 2014). Loops frequently form a contact domain-that is, an interval in which all loci exhibit higher contact frequency with one another (than random loci at similar distance along the genome sequence); this structure is called a "loop domain" (Rao et al., 2014).

To examine loop domains, Applicants used *in situ* Hi-C (Rao et al., 2014), which combines DNA-DNA proximity ligation and high-throughput sequencing to create maps showing the frequency of physical contact between all pairs of loci across the genome. Loop domains are manifest in Hi-C maps as a bright "peak" pixel (indicating the link between the two loop anchors) at the corner of a bright square (indicating the presence of a contact domain).

Applicants generated roughly 6 billion Hi-C contacts from HCT-116 cells before (3.0B) and immediately after (2.9B) auxin treatment. In the untreated cells, our algorithms annotated 3,170 loops, of which 2,140 were loop domains. Strikingly, the loop domains disappeared upon cohesin loss. The result was evident by visual examination (**Fig. 2A****,** **Fig. 9C****,** **Fig. 15****;** (Durand et al., 2016a)). Moreover, the algorithms found only 9 loop domains after auxin treatment. Upon close inspection, all were found to be false positives (see Methods). (Applicants return below to examine loops *not* associated with contact domains.)

To see if these changes were reversible, Applicants performed a time-course analysis in which untreated cells were exposed to auxin for six hours, after which auxin was withdrawn (**Fig. 2B****,** **Fig. 9B**). Low resolution Hi-C was performed immediately before treatment, as well as at a series of time points during treatment (at 20, 40, 60, 240, and 360 minutes) and after withdrawal (at 20, 40, 60, 180, 360, 1080, and 1440 minutes). To assess whether the anchors of the loop domains seen in the pre-treatment data continued to be linked (that is, co-located in space) subsequently, Applicants used a method called Aggregate Peak Analysis (APA) (Durand et al., 2016b), which superimposes the signals from a set of peak pixels, thus allowing Applicants to observe an aggregate signal even in sparse datasets where individual signals cannot be resolved (**Fig. 2B**). The APA signal was initially strong, but was gone by 40 minutes after treatment, and remained absent throughout the rest of the treatment period (**Fig. 2B****,** **9B**). The disappearance of the loop-domain links closely mirrored the depletion of cohesin levels in the samples during the treatment period, as ascertained by measuring mClover fluorescence (**Fig**. **8**). After auxin was withdrawn, the APA signal quickly increased, largely recovering by 1 hour (**Fig. 2B****,** **9B**).

These results indicate that the formation of loop domains requires cohesin; that loop domains rapidly disappear after the cohesin tethering the link has been degraded; and that the restoration of cohesin rescues the loop domains.

### Example 4 - Loop domains spanning NIPBL and superenhancers recover more rapidly

To explore the formation process for individual loop domains, Applicants generated 2.6 billion additional contacts from the Hi-C experiments after 20, 40, 60 and 180 minutes of auxin withdrawal (678M, 665M, 618M, and 675M contacts, respectively). Using these improved maps, Applicants were able to calculate individual recovery curves for 2038 of the 2140 loop domains observed in the untreated cells (**Fig. 2C****, see Methods**). (The time-resolved maps were not sufficiently deep to assess the 102 loop domains shorter than 100kb.) Recovery rates for individual loop domains varied dramatically. Faster recovery was strongly associated with high levels of NIPBL binding between the loop anchors, as well as enrichment of promoter and enhancer elements, and of activating histone marks like H3K36me3 and H4K16Ac. By contrast, loop domains that recovered slowly were typically depleted for these features, but enriched for repressive marks like H3K27me3 and H3K9me3 (**Fig. 2D-E**, **Fig. 9D-F**, **Fig. 16**). The most extreme difference Applicants found was in the presence of superenhancers (also called stretch enhancers, or SEs), which are regions of the mammalian genome containing a very high density of enhancer elements, and which are marked by extremely high levels of H3K27 acetylation (Hnisz et al., 2013; Parker et al., 2013). Fast loop domains (recovery rate > 95^{th} percentile) were 159-fold more likely than slow loop domains (<5^{th} percentile) to span an SE (2.94 SEs/Mb vs. 0.02 SEs/Mb), and 26-fold more likely to contain strong NIPBL binding sites (4.23/Mb vs. 0.17/Mb) (Fig. S2E-F).

Taken together, these results indicate that the rate of loop domain re-formation varies greatly across the genome, and is associated with factors ranging from cohesin loading to local regulatory activity.

### Example 5 - Loss of cohesin is associated with stronger genome compartmentalization

Next, Applicants examined the effects of cohesin loss on compartmentalization. Compartmentalization refers to the fact that the genome is partitioned into intervals (which can range from 14 kb to more than 5 Mb) belonging to a small number of types, such that intervals of the same type exhibit an enhanced contact frequency with one another (Lieberman-Aiden et al., 2009; Rao et al., 2014). Intervals are thereby assigned to two compartments (A or B, closely associated with open and closed chromatin, respectively) and, more finely, into six subcompartments (A1, A2, B1, B2, B3, B4). The "compartment intervals" that lie in a particular compartment are associated with distinctive patterns of chromatin marks (Rao et al., 2014). Because loci within a compartment interval are of the same type, they exhibit an increased contact frequency with one another and frequently form contact domains. In this case, Applicants call the contact domain a "compartment domain." The enhanced contact frequency between compartment intervals in the same subcompartment also gives rise to a plaid pattern in Hi-C maps (Lieberman-Aiden et al., 2009).

Whereas loop domains disappear entirely after cohesin loss, compartmentalization is preserved (**Fig. 3A**). Following auxin treatment, there is no significant change in either the compartment domains, as defined by the presence of the corresponding squares along the diagonal in the Hi-C contact map (**Fig. 3B****; see Methods**), or in the plaid pattern, as defined by the eigenvectors of the Hi-C correlation map (**Fig. 3A**; mean Pearson's r = 0.968 across all chromosomes). The data is consistent with a previous report that genome compartmentalization is preserved after depletion of cohesin (Seitan et al., 2013) or CTCF (Nora et al., 2017).

Applicants then examined the interaction between compartments and loop domains. Specifically, Applicants examined the compartment boundaries (transition points between compartment intervals) that either (i) lay in the interior of a loop domain in untreated cells or (ii) coincided with a loop-domain anchor in untreated cells (**Fig. 3C-E**). In the former case, the correlation in the genome-wide contact pattern on opposite sides of compartment boundaries showed a much greater decrease in treated vs. untreated cells-that is, the plaid pattern across the genome became much stronger in the absence of cohesin (**Fig. 3C-E****,** **Fig. 17**). The results were similar when Applicants examined boundaries between intervals that were enriched vs. depleted for H3K27Ac (which marks intervals in the "A" compartment (Rao et al., 2014)) or intervals that were enriched vs. depleted for H3K27me3 (which marks intervals in the "B1" subcompartment (Rao et al., 2014)) (**Fig. 3F****,** **10A-D****, see Methods**). These data indicate that the compartmentalization process that brings together loci with similar histone marks does not rely on cohesin. On the contrary, the strengthening of the plaid pattern after cohesin loss suggests that the formation of cohesin-dependent loop domains interferes with compartmentalization by promoting the co-localization of locus pairs with different histone modification patterns. The data is consistent with the observation that genome compartmentalization is weakened in cells where the cohesin-antagonist *WAPL* is knocked out, leading to larger loop domains (Haarhuis et al., 2017).

### Example 6 - Links between superenhancers strengthened after loss of cohesin

Next, Applicants examined loops not associated with contact domains. Whereas 1,030 such loops were annotated in untreated cells, only 72 were annotated after cohesin loss. Upon close examination, 57 were false positives (**see Methods**). (The loop-detection algorithms have a higher false-discovery rate after cohesin loss, since true positives are so rare.) The remaining 15 loops were much larger than those seen in untreated cells (median: 1.75 Mb, vs. 0.275 Mb). Given their large size, Applicants found that loops could be more reliably identified in treated cells by running our peak detection algorithm at coarser resolution (50-100 kb vs. 5-10 kb) (**see Methods**). This identified an additional 46 loops that were confirmed by manual inspection (**Fig. 4A****,** **11A****, see Methods**). Afterwards, the size difference between the 61 "cohesin-independent loops" and the cohesin-associated loops was even more dramatic (**Fig. 4B**, median size: 23.15 Mb).

Applicants sought to understand the basis of these "cohesin-independent loops." Applicants found that they do not demarcate the boundaries of contact domains (0 of 61 [0%]; vs. 2140 of 3170 [68%] for cohesin-associated loops). Remarkably, many cohesin-independent loop anchors form links with each another - manifest as focal peaks in the Hi-C heatmap - even when the anchors reside on different chromosomes (**Fig. 4A****,** **Fig. 18**)**.** In total, Applicants identified 203 such interchromosomal links. Interchromosomal links are not seen between the anchors of cohesin-associated loops.

The anchors of cohesin-independent loops also exhibit very different patterns of protein binding. The proportion that binds CTCF is much lower (20% vs. 90% for cohesin-associated loops; **Fig. 4C****,** **Fig. 11A****).** Moreover, there is no tendency for the CTCF motifs at loop anchors to point into the loop (5 of 9 (56%) point inward vs. 2770 of 2919 (95%) for cohesin-associated loops.

Notably, the cohesin-independent loop anchors are highly enriched for superenhancers. Applicants found that 41 of the 64 cohesin-independent loop anchors overlapped with the 387 superenhancers in HCT116 cells-a 37.5-fold enrichment (p<10⁻¹⁵). For the 100 strongest superenhancers, the enrichment was 76-fold (30 of 64, p<10⁻¹⁵; **Fig. 4D****,** **11B**). Interestingly, cohesin-independent loops and the associated loops and links between superenhancers could be seen in the untreated cells as well, but were much weaker (**Fig. 4A****,E,** **Fig. 5A****,** **Fig. 11C-E****,** **Fig. 18****, see Methods).**

Strikingly, Applicants observed large cliques forming between the anchors of the cohesin-independent loops (**Fig. 5A****,** **Fig. 19**)**.** Large cliques are not seen for cohesin-associated loops (**Fig. 9A**).

In many respects, the cohesin-independent loops resemble the superloops Applicants previously observed on the inactive X chromosome (Darrow et al., 2016; Rao et al., 2014): they are very large (up to 77Mb), the intervals they span do not form contact domains, and their anchors tend to form cliques and are marked by H3K27 acetylation (**Fig. 11F**)**.** Applicants also found that the superloops tend to occur simultaneously, forming hubs containing 3 or more loci. Therefore, Applicants sought to test whether cohesin-independent loop anchors would exhibit the same behavior.

To probe this question, Applicants examined concatemers-that is, Hi-C reads which bring together 3 or more loci, and indicate that the loci in question were simultaneously co-located in a particular cell during the Hi-C experiment. Applicants identified 57 million unique "triples," which bring together 3 loci; 32 million unique quadruples, and 130 thousand quintuples (**Fig. 5B****, see Methods**). Instead of a 2-dimensional heatmap, concatemers are naturally represented as an n-dimensional matrix, or tensor, showing the collision frequency (i.e. the frequency of simultaneous physical contact) between any set of n loci in the genome (**Fig. 5C**). Hubs involving n loci manifest in the n-dimensional contact tensor as peaks in collision frequency with respect to the local n-dimensional neighborhood.

Because the number of entries in an n-dimensional contact tensor scales as the genome size to the nth power, contact tensors can be exceedingly sparse. Applicants therefore did not expect to see triples corresponding to any particular set of three cohesin-independent loop anchors. Instead, Applicants developed a variant of APA for contact tensors, superimposing the signal from all possible sets of three cohesin-independent loop anchors that lie on a single chromosome (**see Methods**). This analysis revealed 11 collisions involving three cohesin-independent loop anchors in the auxin-treated data, as compared to an expected value of 0.41 collisions based on the density of collisions in the local neighborhood (**Fig. 5D-F****,** **Fig 12A-B**). These findings indicate that, like superloops, cohesin-independent loop anchors tend to form hubs involving three or more loci. By contrast, no collisions were found in the untreated data. This is consistent with our finding that cohesin-independent loops are much weaker in the presence of cohesin.

### Example 7 - Molecular dynamics simulations integrating extrusion and compartmentalization can recapitulate Hi-C experimental results

To test the hypothesis that the Hi-C contact maps observed are consistent with the presence of two distinct folding mechanisms, Applicants modeled a 2.1 Mb region on chromosome 3 (Sanborn et al., 2015). The model treated the region as a block copolymer consisting of two types of chromatin, A or B, determined by classifying loci based on ChIP-Seq binding data; and containing CTCF binding sites, whose position and strength were derived from CTCF and SMC1 ChIP-Seq tracks, and whose orientation was determined by examining the human genome reference (**Fig. 6A****, see Methods**). Applicants used molecular dynamics simulations to examine the behavior of this polymer in a solvent containing extrusion complexes (thus modeling loop extrusion (Fudenberg et al., 2016; Sanborn et al., 2015)), and in the presence of attractive forces between like monomers (thus modeling compartmentalization (Di Pierro et al., 2016)). The resulting ensemble was used to create an *in silico* contact map for the region.

Applicants found that the resulting contact maps accurately recapitulated the experimental results in both untreated and treated cells (**Figure 6A****,B**), and illustrate the change in long-range contact pattern that is seen when a loop spans a compartment boundary (**Fig. 20**). These findings suggest that the most prominent features observed in Hi-C data sets (loops, domains, and compartments) can be recapitulated by simulations that use only ChIP-Seq data as input, in the context of a model combining extrusion and compartmentalization.

The above simulations assume that phase separation leads to the compartmentalization of chromatin intervals bearing similar patterns of histone marks. There are multiple models that could account for such a tendency. In one, phase separation is facilitated by protein chaperones that recognize histone tails. Alternatively, similar nucleosomes might directly attract one another through histone tail interactions. To see whether tail interactions can guide folding at the oligonucleosome scale given the mechanical, electrostatic, and entropic constraints on chromatin fibers Applicants simulated short fibers using a mesoscale approach (Bascom and Schlick, 2017; Bascom et al., 2017; Grigoryev et al., 2016). These simulations have three components: linker DNA beads (each representing 9 bp), nucleosome core particles (rigid bodies with charged surfaces), and histone tail beads (each representing 5 aa) (**Fig. 13A-****B**)**.** Applicants found that histone tail interactions overcame constraints on the chromatin chain, leading either to focal contacts between short chromatin intervals or to global condensation, depending on the initial configuration and epigenetic state of the fiber (**Fig 13C-E**)**.** Of course, these findings do not rule out a central role for protein chaperones *in vivo.*

Cohesin loss results in strong down-regulation of genes near superenhancers, but does not bring about widespread ectopic activation

Finally, Applicants sought to investigate the role of cohesin in regulating gene expression. Cohesin has been proposed to facilitate interactions between enhancers and promoters (Kagey et al., 2010; Merkenschlager and Nora, 2016). Loop domains are thought to regulate this process by preventing enhancers from forming ectopic interactions with targets that lie in a different loop domain (Lupiáñez et al., 2015; Flavahan et al., 2016). Applicants therefore characterized the effects of cohesin loss on nascent transcription by performing precision nuclear run-on sequencing (PRO-Seq) in treated and untreated HCT116 cells (Engreitz et al., 2016; Jonkers and Lis, 2015) (**Fig. 7A**). Applicants chose an early timepoint - 6 hours after auxin treatment - with the aim of examining direct consequences, rather than indirect effects due to changes in cell state.

To look for signs of ectopic activation, Applicants examined the 14,853 genes that were not expressed (RPKM<0.5) in untreated cells. Of these genes, 1% (216) were activated after treatment (p<0.05, >30% change in RPKM, RPKM>0.5 in treated cells). Thus, while cohesin plays a role in preventing ectopic activation, most genes remain inactivated even in the absence of cohesin and loop domains.

Applicants next looked for changes in the 12,222 genes that were expressed (RPKM>0.5) in untreated cells (**Fig. 5B**). Here again, most genes (87%, 10,615) exhibited similar levels of transcription after cohesin degradation (RPKM changed by less than 30%). Strong effects were infrequent: 64 genes (0.5%) showed a 2-fold change, and 2 genes showed a 5-fold change (**Fig. 7B**). While the quantitative impact may seem modest, such changes can have important biological impacts (Flavahan et al., 2016).

Of genes that exhibited a strong change in transcription, more were downregulated than upregulated (61% vs. 39%)-suggesting that cohesin-associated loops may both facilitate activation of promoters by their distal enhancer elements and block activation by inappropriate enhancers, with the former being somewhat more common.

Applicants wondered how cohesin facilitated these promoter-enhancer contacts. Applicants noticed that many of the genes that were downregulated (by >1.75-fold) were located within 500kb of superenhancers (23 of 49, 4.8-fold enrichment, **Fig. 7C****,D; 14A-B**). Of these genes, 29% were located with 500kb of one of the top 100 superenhancers (8.5-fold enrichment). Strikingly, these superenhancers were often located at the anchors of the cohesin-independent links seen in treated cells (8 of 19, a 13.7-fold enrichment).

The above results are interesting from the standpoint of transcriptional regulation. In the absence of cohesin, superenhancers associated with the downregulated genes exhibit a strong tendency to form links with one another. By contrast, in the presence of cohesin, the majority of these superenhancers were located in the interior of cohesin-associated loop (in 13 of 19 cases) and the long-distance cohesin-independent links were much weaker.

### Example 8 - DISCUSSION

Here, Applicants explore the 4D Nucleome of a human colon cancer cell line during cohesin loss and recovery, achieving Hi-C map resolutions of 5kb with a time resolution of 20 minutes. Applicants find that cohesin is required for the establishment and maintenance of loop domains. After cohesin loss, Applicants also find that: (i) histone marks are unchanged; (ii) compartment structure is strengthened in the absence of cohesin, as loop domains spanning multiple compartment intervals lead to mixing among loci in different compartments; (iii) only a small subset of genes exhibit large changes in transcription level. As auxin is withdrawn, cohesin levels recover, and Applicants are able to measure the rate of formation for nearly every loop domain, genome-wide. Loop domains that recover quickly are much more likely to span superenhancers and binding sites of the cohesin loading factor NIPBL. Finally, Applicants identify a class of cohesin-independent loops, links and hubs connecting superenhancer loci on many chromosomes.

### Comparison with other studies

It is important to set the results in the context of other studies related to loop formation. While there is clear agreement that deletion of individual CTCF binding sites can result in a loss of cohesin binding and can abolish loops and contact domains (Guo et al., 2015; Sanborn et al., 2015; de Wit et al., 2015), there have been conflicting reports about the effects of depleting cohesin or CTCF.

Early Hi-C studies of cohesin and CTCF depletion, using both gene knockouts and proteolytic cleavage, reported that contact domains remained (Seitan et al., 2013; et al., 2013; Zuin et al., 2014). The discrepancy may be due to (i) the fact that low resolution Hi-C analysis cannot not distinguish between loop domains, which are sensitive to cohesin depletion, and compartment domains, which are not; and (ii) the possibility that the cohesin depletion was incomplete (**Fig. 9C**). More recently, CTCF depletion followed by higher-resolution Hi-C (Nora et al., 2017) revealed the disappearance of a subset of contact domains. The data disclosed herein for cohesin depletion is consistent with this study.

Two recent studies have also sought to examine the effects of depletion of *NIPBL,* which encodes a cohesin loader protein. They report opposite conclusions. The (Schwarzer et al., 2016) preprint reported, on the basis of new experiments, that the genome-wide aggregate signal from loop domains is absent following *NIPBL* deletion. By contrast, a recent publication reported the continued presence of individual loop domains after the near-complete depletion of *NIPBL,* although these loop domains were abnormally small (Haarhuis et al., 2017). In any case, it is unclear what effect NIBPL depletion would be expected to have on loop domain formation because, although *NIPBL* facilitates cohesin loading, it may not be essential for cohesin loading. Indeed, cohesin loading independent of NIPBL has been observed *in vitro,* albeit at low efficiency (Davidson et al., 2016; Stigler et al., 2016).

Finally, an exciting recent experiment demonstrated that deletion of WAPL, a cohesin antagonist that removes cohesin from chromatin, results in the formation of thousands of new loops and loop domains, which are larger than those found when WAPL is intact (Haarhuis et al., 2017). The results provided herein are consistent with these findings.

### Two mechanisms that guide genome folding

The results highlight two distinct mechanisms that guide genome folding. The first is the cohesin-dependent formation of loop domains. The data presented above are consistent with several models of this process. Applicants (Sanborn et al., 2015) and others (Alipour and Marko, 2012; Fudenberg et al., 2016; Nasmyth, 2001) have hypothesized that the underlying physical process is the formation of loops by extrusion. In this model, loop domains form when a hypothetical cohesin-based extrusion complex ("Xcom"), which comprises two physically tethered subunits, binds chromatin at a particular location; subsequently, the subunits slide in opposite directions until they arrive at a bound CTCF protein. Thus, the disappearance of cohesin can eliminate all loop domains without influencing CTCF binding. Other models include the initial formation of loops via 3D diffusion of anchor loci, followed by cohesin-mediated stabilization. See (Sanborn et al., 2015) for a fuller discussion.

The second mechanism is the cohesin-independent compartmentalization of chromatin intervals with similar histone marks (Lieberman-Aiden et al., 2009; Rao et al., 2014). This observation is also compatible with several models: histone modifications might drive the formation of compartments (i.e., "phase separation" (Hnisz et al., 2017; Jost et al., 2014; Di Pierro et al., 2016)); compartmentalization might lead to histone remodeling; or both processes might be caused by a third mechanism.

The data presented above - specifically, the fact that cohesin loss does not affect histone modifications, but does cause long-range contact patterns to better match patterns of histone marks - is more consistent with histone patterns governing genome compartmentalization, rather than the reverse. This phase separation process could involve histone-tail interactions, or the binding of reader proteins that target modified histones to specific locations in the nucleus (Wijchers et al., 2016). Interestingly, two recent studies have provided evidence that the protein HP1a, which binds the heterochromatic H3K9me mark, forms liquid droplets *in vivo* via phase separation, such that H3K9me heterochromatin is contained within the droplets (Larson et al., 2017; Strom et al., 2017). Of course, it remains possible that independent mechanisms may shape both histone mark patterns and genome compartmentalization.

### The speed of loop extrusion

Applicants show that loop domains disappear shortly after auxin-induced cohesin loss, and reappear shortly after auxin withdrawal, implying that (i) they require cohesin both for formation and maintenance; and (ii) they do not represent stable states of chromosome condensation.

In the loop extrusion model, the two physically tethered subunits of the Xcom bind chromatin at a single site, and then slide in opposite directions along chromatin. Measurements of loop re-formation enable estimates of extrusion speed. For instance, in figure 2E, Applicants show a ~900kb loop that is restored within 40 minutes of auxin withdrawal. Thus, this loop is extruded at an average rate of no less than 375 bp/s, with each Xcom subunit sliding at no less than 188 bp/s. Our estimates are lower bounds, as they ignore the time needed for auxin to disappear and for cohesin to re-form and be loaded on chromatin. (Given the correlation between loop reformation speed and Nipbl, loading time may be a significant consideration.) These estimates are similar to ones obtained studying the SMC complex in *B. subtilis* [500-1000 bp/s] (Wang et al., 2017).

The rate estimates bear on the protein motors involved when Xcom subunits slide. For instance, it is possible that cohesin itself serves as a motor during the extrusion process. However, single molecule studies of cohesin translocation *in vitro,* have yielded an estimated sliding rate of only 1-2bp per second on chromatin (Davidson et al., 2016; Stigler et al., 2016). These could indicate that cohesin alone - under the conditions probed in those experiments - is unlikely to be the principal source of translocase activity. Similarly, RNA polymerase II can push cohesin along DNA (Davidson et al., 2016) but elongation rate estimates (9-90 bp/s; (Jonkers and Lis, 2015)) are slower than what Applicants observe. This suggests that other translocases may be involved in loop extrusion.

### Two classes of loops

Applicants observe a population of loops that are frequently anchored at superenhancers and do not depend on cohesin. One explanation is that these loops, too, form by extrusion, but using alternative protein complexes, such as condensin, instead of cohesin. However, the superenhancer anchors also form links with one another when they lie on different chromosomes, whereas loop extrusion cannot form interchromosomal links. The data is therefore less consistent with a model where where superenhancer loops form by extrusion, and more consistent with the presence of an alternative mechanism, perhaps based on some form of facilitated diffusion or phase separation (Sanborn et al., 2015).

In particular, superenhancer links may represent compartmental co-segregation of small, H3K27-acetylated intervals (**Fig. 5G**), which accounts for why these links can join loci on different chromosomes, why they are weaker in the presence of cohesin, and why their anchors form large cliques and higher-order hubs.

Notably, loops and links between superenhancers increase in strength rapidly following cohesin loss, reaching a plateau within hours. This implies that compartmentalization is capable of inducing intrachromosomal loops and interchromosomal links at rapid rates, comparable to those of loop-domain formation. (The findings may be related to those of other studies, which have noted enhanced interactions between higher-order intrachromosomal interactions between domains containing superenhancers (Beagrie et al., 2017).)

### The interplay between loop extrusion and compartmentalization

Using the high-resolution contact maps, Applicants are also able to examine the ways in which loop domain formation and compartmentalization interact. It is commonly thought that compartment intervals are typically megabases in length, and are subdivided into smaller domains in a hierarchical fashion (Dixon et al., 2012; Nora et al., 2012). Here, Applicants demonstrate that compartment intervals can be as short as tens of kilobases, and can overlap loop domains in complex ways. For instance, Applicants observe numerous examples of loop domains spanning multiple compartment intervals.

In such cases, Applicants find that loop extrusion, by facilitating contacts between all loci in the loop domain, can enhance the contact frequency of loci that would ordinarily lie in different subcompartments. Thus, the long-range contact pattern seen for each locus is a mixture of the pattern that would ordinarily be seen for loci in the corresponding subcompartment, and the pattern seen for other loci in the loop. This mixing disappears upon cohesin depletion (**Fig. 7E**). Similarly, deletion of WAPL appears to increase the processivity of the Xcom, and thus increases the size of loops (Haarhuis et al., 2017). Consistent with the observations, these larger loops are associated with extensive mixing, which obscures long-range compartment patterns.

### The interplay between cohesin and gene regulation

Many studies have proposed that cohesin facilitates interactions between enhancers and promoters, thereby upregulating the transcription of many genes (Kagey et al., 2010; Merkenschlager and Nora, 2016). Moreover, studies have also suggested that loop domains formed between CTCF and cohesin binding sites create insulated regulatory neighborhoodspartially protecting genes with a loop domain from the influence of enhancers outside the domains (Flavahan et al., 2016; Lupiáñez et al., 2015). This study, combining rapid depletion of cohesin and measurement of nascent transcription using PRO-Seq, allows Applicants to more clearly dissect the direct effects of cohesin loss on transcription.

Applicants find that a very small set of genes, often lying near superenhancers, becomes strongly downregulated after cohesin loss. However, most genes are not strongly affected. This suggests that cohesin-dependent loop domains themselves play at most a modest role in facilitating or disrupting interactions between promoters and enhancers. Of course, Applicants cannot dismiss modest effects on overall level of transcription as unimportant. For example, modest increases in the expression of receptor tyrosine kinase genes can have meaningful effects on cell proliferation (Flavahan et al., 2016).

Nevertheless, it is particularly interesting to compare the above findings with earlier studies, using similar methods, that showed a strong correlation between the presence of a loop domain and many-fold increases in the expression of genes at the loop domain anchor (Kagey et al., 2010; Rao et al., 2014). Taken in isolation, these earlier results are consistent with a model where the formation of loop domains routinely causes many-fold changes in gene expression. By contrast, the data presented here are more consistent with a model where the formation of loop domains influences gene transcription, but rarely causes many-fold changes.

One possibility is that both processes might be independent consequences of upstream regulatory events. For example, changes in accessibility of a gene promoter might facilitate both transcription factor binding, activating the gene, and cohesin arrest, activating the loop domain. Alternatively, large increases in gene expression may alter the accessibility of CTCF motifs at the promoter and cause loop domain formation.

This study suggests a model where cohesin-associated looping, by increasing the frequency of contact between loci within loop domains and by disturbing patterns of compartmentalization, facilitates mixing between elements (such as genes and superenhancers) that would otherwise be segregated. Thus, compartmentalization and extrusion - through independent and complementary mechanisms - interact to shape transcription.

**Table 1: Hi-C Experiments, Related to Fig. 2**

| Library | **Cell type** | **Biolog ical Replic ate number** | **Auxin Treatm ent Time (min)** | **Auxin Withdra wal Time (min)** | **Total Sequenced Reads** | **Total Contacts** | **Used in which main figure s?** | **Used in which supp figures?** |
|---|---|---|---|---|---|---|---|---|
| **HCT-116 RAD21-mAC no auxin treatment main experiment (unsynchronized)** | | | | | | | | |
| Rao-2017-HIC001 | HCT-116-RAD21-mAC | 1 | N | N | 591,780,1 63 | 445,535,1 72 | | |
| Rao-2017-HIC002 | HCT-116-RAD21-mAC | 1 | N | N | 659,448,8 71 | 489,029,7 47 | | |
| Rao-2017-HIC003 | HCT-116-RAD21-mAC | 1 | N | N | 709,541,9 51 | 523,419,7 77 | | |
| Rao-2017-HIC004 | HCT-116-RAD21-mAC | 2 | N | N | 335,561,6 77 | 254,485,6 56 | | |
| Rao-2017-HIC005 | HCT-116-RAD21-mAC | 2 | N | N | 414,047,5 32 | 311,178,4 49 | | |
| Rao-2017-HIC006 | HCT-116-RAD21-mAC | 2 | N | N | 432,223,0 16 | 325,482,9 84 | | |
| Rao-2017-HIC007 | HCT-116-RAD21-mAC | 2 | N | N | 333,231,5 54 | 255,826,7 30 | | |
| **TOTAL** | | | | | **3,475,834 ,764** | **2,604,958 ,515** | Fig 2A,2C- E,3A-F,4A-D,5A-F,6A,7 C | Fig S2C-F,S3A-D,S4A-D,S5A,S7A-B; Data S1, I-VI |
| | | | | | | | | |

| **HCT-116 RAD21-mAC 6hr auxin treatment main experiment (unsynchronized)** | | | | | | | | |
|---|---|---|---|---|---|---|---|---|
| Rao-2017-HIC008 | HCT-116-RAD21-mAC | 1 | 360 | N | 402,253,3 86 | 307,015,2 29 | | |
| Rao-2017-HIC009 | HCT-116-RAD21-mAC | 1 | 360 | N | 453,604,4 01 | 343,067,6 14 | | |
| Rao-2017-HIC010 | HCT-116-RAD21-mAC | 1 | 360 | N | 664,778,5 35 | 492,184,8 95 | | |
| Rao-2017-HIC011 | HCT-116-RAD21-mAC | 2 | 360 | N | 467,405,2 68 | 348,568,9 06 | | |
| Rao-2017-HIC012 | HCT-116-RAD21-mAC | 2 | 360 | N | 377,985,3 55 | 283,405,3 58 | | |
| Rao-2017-HIC013 | HCT-116-RAD21-mAC | 2 | 360 | N | 436,936,8 66 | 329,083,1 76 | | |
| Rao-2017-HIC014 | HCT-116-RAD21-mAC | 2 | 360 | N | 562,189,5 38 | 420,465,6 86 | | |
| **TOTAL** | | | | | **3,365,153 ,349** | **2,523,790 ,864** | Fig 2A,2C- E,3A- F,4A- D,5A- F,6A,7 C | Fig S2C-F,S3A-D,S4A-D,S5A,S7A-B; Data S1, I-VI |
| | | | | | | | | |

| **HCT-116 RAD21-mAC no auxin treatment (G1 synchronized/arrested)** | | | | | | | | |
|---|---|---|---|---|---|---|---|---|
| Rao-2017-HIC015 | HCT-116-RAD21-mAC | 3 | N | N | 49,752,30 7 | 38,041,18 0 | | |
| Rao-2017-HIC016 | HCT-116-RAD21-mAC | 3 | N | N | 58,795,02 8 | 44,757,18 4 | | |
| Rao-2017-HIC017 | HCT-116-RAD21-mAC | 3 | N | N | 58,487,65 1 | 43,802,15 8 | | |
| Rao-2017-HIC018 | HCT-116-RAD21-mAC | 3 | N | N | 53,254,48 3 | 40,462,76 4 | | |
| **TOTAL** | | | | | **220,289,4 69** | **167,063,2 86** | Fig 5B- D | Fig S5A; Data S1, I.H-L |
| | | | | | | | | |

| **HCT-116 RAD21-mAC 6hr auxin treatment (G1 synchronized/arrested)** | | | | | | | | |
|---|---|---|---|---|---|---|---|---|
| Rao-2017-HIC019 | HCT-116-RAD21-mAC | 3 | 360 | N | 46,269,45 6 | 34,284,66 1 | | |
| Rao-2017-HIC020 | HCT-116-RAD21-mAC | 3 | 360 | N | 61,992,55 1 | 46,256,48 1 | | |
| Rao-2017-HIC021 | HCT-116-RAD21-mAC | 3 | 360 | N | 69,383,45 7 | 51,719,47 4 | | |
| Rao-2017-HIC022 | HCT-116-RAD21-mAC | 3 | 360 | N | 64,347,36 2 | 48,497,14 2 | | |
| TOTAL | | | | | **241,992,8 26** | **180,757,7 58** | Fig 5B,E | Fig S5B; Data S1, I.H-L |
| | | | | | | | | |

| **HCT-116 RAD21-mAC no auxin treatment and 6hr auxin treatment additional data (unsynchronized)** | | | | | | | | |
|---|---|---|---|---|---|---|---|---|
| Rao-2017-HIC023 | HCT-116-RAD21-mAC | 4 | N | N | 42,084,62 7 | 29,532,57 5 | Fig 5B- D | Fig S5A |
| Rao-2017-HIC024 | HCT-116-RAD21-mAC | 4 | N | N | 40,125,31 7 | 27,316,93 7 | Fig 5B- D | Fig S5A |
| Rao-2017-HIC025 | HCT-116-RAD21-mAC | 5 | N | N | 81,006,48 9 | 57,706,32 8 | Fig 5B- D | Fig S5A |
| Rao-2017-HIC026 | HCT-116-RAD21-mAC | 5 | N | N | 79,169,37 4 | 50,650,94 0 | Fig 5B- D | Fig S5A |
| Rao-2017-HIC027 | HCT-116-RAD21-mAC | 5 | N | N | 72,935,30 2 | 52,129,53 6 | Fig 2A,2C- E,3A- F,4A- D,5A- F,6A,7 CFig 5B- D | Fig S5A |
| Rao-2017-HIC028 | HCT-116-RAD21-mAC | 4 | 360 | N | 59,572,70 6 | 42,956,06 7 | Fig 5B,E | Fig S5B |
| Rao-2017-HIC029 | HCT-116-RAD21-mAC | 5 | 360 | N | 71,492,57 8 | 50,421,74 9 | Fig 5B,E | Fig S5B |
| Rao-2017-HIC030 | HCT-116-RAD21-mAC | 5 | 360 | N | 68,838,34 8 | 49,482,94 8 | Fig 5B,E | Fig S5B |
| Rao-2017-HIC031 | HCT-116-RAD21-mAC | 5 | 360 | N | 63,328,67 6 | 45,789,78 7 | Fig 5B,E | Fig S5B |
| | | | | | | | | |

| **HCT-116 RAD21-mAC 6hr auxin treatment and 20min withdrawal** | | | | | | | | |
|---|---|---|---|---|---|---|---|---|
| Rao-2017-HIC032 | HCT-116-RAD21-mAC | 6 | 360 | 20 | 312,085,2 04 | 226,595,7 27 | | |
| Rao-2017-HIC033 | HCT-116-RAD21-mAC | 6 | 360 | 20 | 185,231,4 12 | 134,274,4 64 | | |
| Rao-2017-HIC034 | HCT-116-RAD21-mAC | 7 | 360 | 20 | 233,634,1 82 | 162,365,8 15 | | |
| Rao-2017-HIC035 | HCT-116-RAD21-mAC | 7 | 360 | 20 | 219,854,7 12 | 155,180,4 15 | | |
| **TOTAL** | | | | | **950,805,5 10** | **678,416,4 21** | Fig 2C- E | Fig S2D-F; Data S1, II; Data S1, V |
| | | | | | | | | |

| **HCT-116 RAD21-mAC 6hr auxin treatment and 40min withdrawal** | | | | | | | | |
|---|---|---|---|---|---|---|---|---|
| | | | | | | | | |
| Rao-2017-HIC036 | HCT-116-RAD21-mAC | 6 | 360 | 40 | 226,917,7 13 | 158,778,6 31 | | |
| Rao-2017-HIC037 | HCT-116-RAD21-mAC | 6 | 360 | 40 | 219,970,8 13 | 156,463,0 52 | | |
| Rao-2017-HIC038 | HCT-116-RAD21-mAC | 7 | 360 | 40 | 272,502,0 10 | 186,640,6 25 | | |
| Rao-2017-HIC039 | HCT-116-RAD21-mAC | 7 | 360 | 40 | 232,963,6 34 | 163,011,4 32 | | |
| **TOTAL** | | | | | **952,354,1 70** | **664,893,7 40** | Fig 2C- E | Fig S2D-F; Data S1, II; Data S1, V |
| | | | | | | | | |

| **HCT-116 RAD21-mAC 6hr auxin treatment and 60min withdrawal** | | | | | | | | |
|---|---|---|---|---|---|---|---|---|
| Rao-2017-HIC040 | HCT-116-RAD21-mAC | 6 | 360 | 60 | 214,655,2 86 | 154,010,2 28 | | |
| Rao-2017-HIC041 | HCT-116-RAD21-mAC | 6 | 360 | 60 | 222,693,4 01 | 159,874,8 81 | | |
| Rao-2017-HIC042 | HCT-116-RAD21-mAC | 7 | 360 | 60 | 256,653,6 36 | 163,055,2 00 | | |
| Rao-2017-HIC043 | HCT-116-RAD21-mAC | 7 | 360 | 60 | 214,645,7 60 | 140,901,1 08 | | |
| **TOTAL** | | | | | **908,648,0 83** | **617,841,4 17** | Fig 2C- E | Fig S2D-F; Data S1, II; Data S1, V |
| | | | | | | | | |

| **HCT-116 RAD21-mAC 6hr auxin treatment and 180min withdrawal** | | | | | | | | |
|---|---|---|---|---|---|---|---|---|
| Rao-2017-HIC044 | HCT-116-RAD21-mAC | 6 | 360 | 180 | 214,088,1 73 | 153,791,0 44 | | |
| Rao-2017-HIC045 | HCT-116-RAD21-mAC | 6 | 360 | 180 | 232,098,2 78 | 168,576,8 77 | | |
| Rao-2017-HIC046 | HCT-116-RAD21-mAC | 7 | 360 | 180 | 242,979,4 51 | 170,974,1 81 | | |
| Rao-2017-HIC047 | HCT-116-RAD21-mAC | 7 | 360 | 180 | 260,183,2 10 | 182,058,0 88 | | |
| **TOTAL** | | | | | **949,349,1 12** | **675,400,1 90** | Fig 2C- E | Fig S2D-F; Data S1, II; Data S1, V |
| | | | | | | | | |

| **HCT-116 RAD21-mAC cohesin degradation time course** | | | | | | | | |
|---|---|---|---|---|---|---|---|---|
| Rao-2017-HIC048 | HCT-116-RAD21-mAC | 8 | N | N | 4,299,762 | 3,110,577 | Fig 2B, 4E | Fig S2B, S5E |
| Rao-2017-HIC049 | HCT-116-RAD21-mAC | 8 | N | N | 6,801,495 | 5,208,595 | Fig 2B, 4E | Fig S2B, S5E |
| Rao-2017-HIC050 | HCT-116-RAD21-mAC | 8 | 20 | N | 8,400,921 | 6,510,391 | Fig 2B, 4E | Fig S2B, S5E |
| Rao-2017-HIC051 | HCT-116-RAD21-mAC | 8 | 20 | N | 6,313,737 | 4,785,644 | Fig 2B, 4E | Fig S2B, S5E |
| Rao-2017-HIC052 | HCT-116-RAD21-mAC | 8 | 40 | N | 6,013,738 | 4,593,726 | Fig 2B, 4E | Fig S2B, S5E |
| Rao-2017-HIC053 | HCT-116-RAD21-mAC | 8 | 40 | N | 5,728,146 | 4,426,420 | Fig 2B, 4E | Fig S2B, S5E |
| Rao-2017-HIC054 | HCT-116-RAD21-mAC | 8 | 60 | N | 6,386,115 | 4,769,235 | Fig 2B, 4E | Fig S2B, S5E |
| Rao-2017-HIC055 | HCT-116-RAD21-mAC | 8 | 60 | N | 6,457,390 | 4,837,457 | Fig 2B, 4E | Fig S2B, S5E |
| Rao-2017-HIC056 | HCT-116-RAD21-mAC | 8 | 240 | N | 11,149,80 2 | 8,506,966 | Fig 2B, 4E | Fig S2B, S5E |
| Rao-2017-HIC057 | HCT-116-RAD21-mAC | 8 | 240 | N | 14,937,26 6 | 11,407,35 5 | Fig 2B, 4E | Fig S2B, S5E |
| Rao-2017-HIC058 | HCT-116-RAD21-mAC | 8 | 360 | N | 7,211,621 | 5,424,025 | Fig 2B, 4E | Fig S2B, SSE |
| Rao-2017-HIC059 | HCT-116-RAD21-mAC | 8 | 360 | N | 8,704,938 | 6,657,305 | Fig 2B, 4E | Fig S2B, S5E |
| | | | | | | | | |

| **HCT-116 RAD21-mAC auxin withdrawal time course** | | | | | | | | |
|---|---|---|---|---|---|---|---|---|
| Rao-2017-HIC060 | HCT-116-RAD21-mAC | 6 | N | N | 6,442,623 | 4,770,627 | N/A | N/A |
| Rao-2017-HIC061 | HCT-116-RAD21-mAC | 6 | N | N | 5,541,151 | 4,170,183 | N/A | N/A |
| Rao-2017-HIC062 | HCT-116-RAD21-mAC | 6 | 360 | N | 5,169,106 | 3,810,912 | N/A | N/A |
| Rao-2017-HIC063 | HCT-116-RAD21-mAC | 6 | 360 | N | 4,611,705 | 3,547,685 | N/A | N/A |
| Rao-2017-HIC032S | HCT-116-RAD21-mAC | 6 | 360 | 20 | 5,879,904 | 4,556,668 | Fig 2B, 4E | Fig S2B, S5E |
| Rao-2017-HIC033S | HCT-116-RAD21-mAC | 6 | 360 | 20 | 5,310,307 | 4,116,520 | Fig 2B, 4E | Fig S2B, S5E |
| Rao-2017-HIC036S | HCT-116-RAD21-mAC | 6 | 360 | 40 | 5,293,483 | 4,014,759 | Fig 2B, 4E | Fig S2B, S5E |
| Rao-2017-HIC037S | HCT-116-RAD21-mAC | 6 | 360 | 40 | 4,630,753 | 3,537,543 | Fig 2B, 4E | Fig S2B, S5E |
| Rao-2017-HIC040S | HCT-116-RAD21-mAC | 6 | 360 | 60 | 3,586,476 | 2,751,617 | Fig 2B, 4E | Fig S2B, S5E |
| Rao-2017-HIC041S | HCT-116-RAD21-mAC | 6 | 360 | 60 | 3,957,367 | 3,041,419 | Fig 2B, 4E | Fig S2B, S5E |
| Rao-2017-HIC044S | HCT-116-RAD21-mAC | 6 | 360 | 180 | 23,567,32 6 | 18,199,91 5 | Fig 2B, 4E | Fig S2B, S5E |
| Rao-2017-HIC045S | HCT-116-RAD21-mAC | 6 | 360 | 180 | 25,368,32 9 | 19,974,41 9 | Fig 2B, 4E | Fig S2B, S5E |
| Rao-2017-HIC064 | HCT-116-RAD21-mAC | 6 | 360 | 360 | 20,497,67 3 | 15,624,03 0 | Fig 2B, 4E | Fig S2B, S5E |
| Rao-2017-HIC065 | HCT-116-RAD21-mAC | 6 | 360 | 360 | 21,457,17 5 | 16,592,35 3 | Fig 2B, 4E | Fig S2B, S5E |
| Rao-2017-HIC066 | HCT-116-RAD21-mAC | 6 | 360 | 1080 | 24,247,69 1 | 18,706,57 2 | Fig 2B, 4E | Fig S2B, S5E |
| Rao-2017-HIC067 | HCT-116-RAD21-mAC | 6 | 360 | 1080 | 22,791,82 2 | 17,334,83 9 | Fig 2B, 4E | Fig S2B, S5E |
| Rao-2017-HIC068 | HCT-116-RAD21-mAC | 6 | 360 | 1440 | 30,218,44 8 | 23,617,74 6 | Fig 2B, 4E | Fig S2B, S5E |
| Rao-2017-HIC069 | HCT-116-RAD21-mAC | 6 | 360 | 1440 | 25,037,09 4 | 19,239,18 2 | Fig 2B, 4E | Fig S2B, S5E |

### Example 9 - Degron Complementation Assay

Some of the best assays for loop extrusion currently available are in vivo assays. In particular, one can assay loading and translocation via ChIP-Seq (Vian, et al., 2018), loop formation and translocation speed via Hi-C (Rao, et al., 2017), and CTCF barrier activity via both Hi-C and ChIP-Seq. As such, it would be convenient to utilize these in vivo genomic assays to test the biochemical function of protein mutants, both to elucidate the function of specific residues/domains of proteins involved in loop extrusion, as well as to identify the pathologic mechanism of mutations seen in these proteins in patients. Using the degron system, where the endogenous protein of interest can be degraded to completion, Applicants can quickly assay the effects of mutants in their native context. In order to clarify the molecular mechanisms governing the interactions of these proteins. Applicants can perform Hi-C and ChIP-Seq after depletion of endogenous proteins and expression of defined mutants that abrogate particular functions or binding interfaces of the target proteins.

Using the degron system, Applicants can directly measure the influence of cellular processes on loop formation (Vian et al, 2018). By degrading and then restoring cohesin and monitoring loop reformation while simultaneously either (i) inhibiting transcription with flavopiridol, (ii) inhibiting replication with thymidine, (iii) or depleting ATP with oligomycin, Applicants can rule out the possibility that RNA or DNA polymerases are the extrusion motor, as loop reformation occurs without issue in the absence of transcription or replication (**Fig. 21**). We can also observe that loop formation requires ATP (although we cannot distinguish between the possibilities that cohesin requires ATP for loading, or for translocation, or for both). Applicants also tested interchromasomal cohesin-independent links requirement for ATP after cohesin degradation and observed that depletion of ATP strengthens the interactions between superenhancers (**Fig. 23**).

We have recently developed, in collaboration with the Masato Kanemaki laboratory, NIPBL-AID degron HCT-116 cell line. By performing similar genomic analyses as above, we will continue to measure *in vivo* parameters of the loop extrusion process. For instance, by performing Hi-C over an auxin treatment time course in NIPBL-AID cells, we can measure the rate of cohesin unloading by factors like WAPL, since depletion of NIPBL will quickly stop cohesin loading (Murayama and Uhlmann, 2014), after which degradation of looping will occur as a function of the active unloading rate.

Applicants have developed a number of HCT-116 cell lines (CTCF-AID, CTCF-AID-RAD21-AID double degron, TOP2A-AID, SNF2H-AID) to better study the properties of CTCF/cohesin loop anchors in mammalian genomes. For instance, many studies over the past two decades have implicated CTCF in diverse roles as a transcription factor, insulator, boundary element for cohesin extrusion, and pausing factor for RNA polymerase II to promote alternative splicing, but how all of these various roles are accomplished and their relationship to each other is unknown. By comparing chromosome folding, histone modification and gene expression patterns after CTCF degradation alone (CTCF-AID), cohesin degradation alone (RAD21-AID), and degradation of both simultaneously (CTCF-AID-RAD21-AID double degron), Applicants can systematically identify any cohesin-independent roles of CTCF. Additionally, there are a number of proteins that are recurrent at loop anchors, such as a TOP2A/B and SNF2H (the ATPase subunit of a number of ISWI chromatin remodeling complexes that is responsible for the creation of a well positioned 20 nucleosome array around CTCF sites), whose function at loop anchors is largely unknown. By performing *in situ* Hi-C, ChIP-Seq and PRO-Seq in these cell lines, Applicants can systematically dissect the role of these proteins in loop extrusion and cellular function.

Finally, Applicants have developed a number of HCT-116 cell lines with degrons for other SMC-family proteins (SMC2-AID, SMC5-AID, SMC5-AID-SMC6-AID double degron). By performing Hi-C along auxin treatment and withdrawal timecourses at various points during the cell cycle in these cell lines, Applicants can search for evidence for loop extrusion by these cohesin-related proteins *in vivo* and measure their dynamics.

***Determining the molecular basis for the cohesin extrusion cycle and CTCF barrier activity:*** The protein CTCF is thought to act as an oriented brake to halt cohesin-mediated loop extrusion in one direction along the genome but not the other, but how this braking activity is mediated mechanistically is unknown. An important question regarding CTCF barrier activity is whether there is a protein domain mediating the ability to halt extrusion or whether CTCF halts cohesin via steric occlusion. As a proof of principle, Applicants introduced either (i) WT CTCF, (ii) CTCF lacking its N terminal domain, or (iii) CTCF lacking its C terminal domain by transfection into CTCF-AID cells. By performing Hi-C before and after auxin treatment (endogenous CTCF degradation) Applicants demonstrate that the N terminal domain of CTCF is required for extrusion barrier activity and thus, loop formation (Fig. 22). Notably, while this result is consistent with the orientation of CTCF on DNA (the N termini face the interior of the loop and thus it would be the side of the protein first encountered by extruding cohesin), it contradicts previous *in vitro* biochemical work (Xiao et al, 2011) suggesting an interaction between the CTCF C terminus and SA2 (a cohesin cofactor), highlighting the power of the degron system and our *in vivo* assays. Applicants can systematically identify the key residues in the N terminus of CTCF that are necessary for barrier activity by transfecting plasmids expressing mutant CTCF proteins with tiled mutations on the N terminus and assaying via Hi-C after degradation of endogenous CTCF. Similarly, Applicants can develop HCT-116 degron lines for SA1, SA2, and SA1+SA2 double degron (the cohesin ring co-factors that are thought to mediate the interaction between cohesin and CTCF). By depleting endogenous SA protein, introducing SA proteins with tiled mutations across various domains, and assaying looping via Hi-C, Applicants can similarly identify the residues on the cohesin complex required for interaction with CTCF. By combining this biochemical information with structural information available for how various cohesin co-factors like SA, RAD21, and WAPL interact with one another, Applicants can elucidate the basis for CTCF-mediated braking of loop extrusion by cohesin.

Another important unresolved question regarding loop extrusion by SMC-family proteins like cohesin, is the specific mechanism by which DNA is passed through the complex as it extrudes. Applicants can breakdown the biochemical basis of the cohesin extrusion cycle by depleting endogenous cohesin proteins via auxin-inducible degrons and introducing mutated version of the cohesin proteins. For instance, while it is known that WAPL-mediated unloading of cohesin from DNA involves opening the interface between RAD21 and SMC3, it is unknown whether the RAD21-SMC3 exit gate must open during each cohesin extrusion cycle in order to processively grow a larger loop. By introducing a plasmid encoding a covalently fused RAD21-SMC3 protein (or a covalently fused RAD21-SMC1 protein) into the RAD21-AID cells after degradation of endogenous RAD21, Applicants can probe the effect of closing kleisin-SMC interfaces on cohesin loading, translocation and loop formation via ChIP-Seq and Hi-C. Applicants can also develop HCT-116 degron cell lines for SMC1 and SMC3 (and both together), in order to test the effects of mutations in the ATPase domains, the hinge domains and the coiled coil domains of these proteins. For instance, by introducing mutants that either abrogate the SMC1 or the SMC3 ATPase domains respectively, Applicants can isolate the role of each domain individually. In addition, Applicants can assess the role simply of nucleotide binding versus ATP hydrolysis in cohesin conformational change and the extrusion cycle.

An advantage of *in vivo* assays of biochemical function is the ease with which they can be parallelized to assay many different mutants simultaneously. While Applicants can start by comprehensively analyzing a handful of targeted mutations in CTCF and cohesin subunits as described above, Applicants can then progress to a more systematic, high-throughput mutagenesis screen of CTCF and cohesin. By using individual cells as test tubes and performing single cell Hi-C or ChIP-Seq via split-pool barcoding such that each cell receives a single protein mutant, and both the protein mutant DNA and the Hi-C contacts or protein-DNA interactions can be uniquely barcoded via combinatorial synthesis, Applicants can assess hundreds of CTCF and cohesin mutants in their respective degron cell lines and comprehensive characterize binding motifs and functional residues. Taken together, these experiments will greatly increase our mechanistic understanding of loop extrusion.

***Determining the molecular basis for pathology for CTCF and cohesin mutations seen in patients:*** Many diseases are caused by mutations in CTCF or the subunits of the cohesin complex. For instance, mutations in CTCF cause a syndrome that includes intellectual disability; mutations in *NIPBL, SMC1A, SMC3,* or *RAD21* cause Cornelia de Lange syndrome (CdLS); and recurrent mutations in *SA2* or *RAD21* are seen in acute myeloid leukemia (AML). In many of mutations seen in patients, a mechanistic understanding of how the mutation causes the pathology is unknown. Moreover, as the number of exomes/genomes from patients continues to increase in the coming years, it is inevitable that variants of uncertain significance (VUS) will be observed in these genes, and a strategy for identifying whether a particular VUS is benign or pathogenic will be essential.

First, Applicants can analyze a catalog of causal mutations seen in patients with CTCF mutations and intellectual disability or CdLS. Applicants can identify the molecular mechanisms that lead these mutations to be pathologic by performing ChIP-Seq, Hi-C and PRO-Seq in the respective HCT-116 degron cell lines after transfecting with plasmids expressing the mutant proteins and degrading the endogenous WT protein. By associating differences in protein binding and loop formation assayed by ChIP-Seq and Hi-C with gene expression changes seen in PRO-Seq, we will identify principles that govern genes affected by pathologic mutations that will allow us to identify downstream genes that may be important for the symptoms seen in the diseases by examining tissue-specific binding patterns of CTCF and cohesin from existing datasets.

In addition, given that CTCF, RAD21, NIPBL and the SA proteins (together) are essential proteins, by utilizing the degron lines, Applicants can test the pathogenicity of thousands of mutants in parallel simply by performing a rescue screen. For each gene, Applicants can create high throughput libraries of plasmids encoding mutant proteins along with a reporter fluorescent protein each with a unique barcode. Since the degron lines are not viable for long periods of time after auxin treatment since the proteins being degraded are essential, Applicants can transfect the mutant libraries into the respective degron line, degrade the endogenous protein for one week, and then sort fluorescent cells. By then sequencing the barcodes that are strongly depleted in the surviving fluorescent cells compared to the representation in the initial mutant library, Applicants can identify mutants that are not capable of rescuing endogenous protein loss and are thus likely pathologic.

Taken together, these rescue experiments will elucidate the landscape of pathologic mutations in CTCF and cohesin proteins and provide a great deal of insight into the basis of the diseases involving these proteins.

### Example 10 - Targeted rengineering of chromatin loop and domain structures

Applicants determined that tiling dCas9 at a CTCF site capable of forming a loop with another convergent CTCF site can block loop formation. **Figure 24A** shows that blocking of the A forward CTCF motif on chromosome 8 with a single dCas9/gRNA combo results in weak loop attenuation, but does not completely abolish either A-B or A-C loops. **Figure 24A** also shows that block of the B reverse CTCF motif with a single dCas9/gRNA combo results in weak loop attenuation, but does not completely abolish the A-B loop. **Figure 24A** **and** **Figure 25** show that block of the A loop anchor (including the CTCF motif itself) with 7 dCas9/gRNAs eliminates both the A-B and A-C loops. **Figure 24B** shows that tiling of 7 dCas9/gRNAs from chromosome 6 (>100kb from either loop anchor) leads to attenuation of loop formation. Thus, Applicants show for the first time that more than one dCas9 is required to be targeted to the loop anchor to eliminate loop formation.

### Example 11 - Experimental Procedures

**HCT-116 cells.** Applicants obtained HCT-116-CMV-OsTir1 and HCT-116-RAD21-mAID-mClover cells (HCT-116 RAD21-mAC) from (Natsume et al., 2016). The cells were cultured in McCoy's 5A medium supplemented with 10% FBS, 2 mM L-glutamine, 100 U/ml penicillin, and 100ug/ml streptomycin at 37C with 5% CO2. Degradation of the AID-tagged RAD21 was induced by the addition of 500uM indole-3-acetic acid (IAA; Sigma Aldrich). For our standard *in situ* Hi-C, ChIP-Seq, and PRO-Seq experiments on untreated cells and cells treated for 6 hours, medium was aspirated at t=0, and either replaced with fresh medium (untreated) or medium containing 500uM IAA. The cells were then washed, trypsinized and processed for downstream experiments at t=6hrs.

For our time course experiments, cells were treated with 500uM IAA and crosslinked with 1% formaldehyde directly in wells of a 6-well plate at various time points after treatment (20, 40, 60, 240, 360 minutes). For the auxin withdrawal experiments, after 6 hours treatment with 500um IAA, the cells were trypsinized, washed twice in fresh media and replated in 6-well plates in fresh media. They were then crosslinked with 1% formaldehyde directly in the 6-well plates at various time points after auxin withdrawal (20, 40, 60, 180, 360, 1080, 1440 minutes).

In order to ensure that the results were not due to the HCT-116 cells stalling in mitosis, Applicants also repeated our Hi-C experiments after cell synchronization and arrest of the cells at the G1/S boundary. First, Applicants added 2mM thymidine to arrest the cells in S-phase and incubated the cells for 12 hours. Applicants then trypsinized and spun down the cells and replated in fresh media, allowing the cells to grow for 12 hours to exit from S-phase. Applicants then added mimosine to a final concentration of 400uM and incubated the cells for 12 hours to arrest the cells at the G1/S boundary. Applicants then replaced media with either complete media + 500uM IAA + 400uM mimosine (treated cells) or complete media + 400uM mimosine (untreated cells) and incubated the cells for 6 hours before processing for downstream experiments.

**Microscopy.** Live HCT116 RAD21-mAC cells in growth medium without phenol red were added to a chambered coverglass (Lab-Tek #155409) 24 hours prior to imaging and incubated at 37°C, 5%CO2, allowing them to attach to the coverglass. One hour before imaging, the growth medium was replaced with 2 µg/ml of Hoechst 33342 in phosphate-buffered saline (PBS) to visualize nuclei. Time-lapse widefield fluorescence microscopy was performed on a DeltaVision OMX microscope (GE Healthcare) equipped with a 37°C incubation chamber, using a 60x oil immersion objective. Cells were treated with 500µM of IAA immediately before imaging. Images were collected every 10 minutes from 0 to 60 minutes following treatment (DAPI filter at 5%T, 100ms for Hoechst; FITC filter at 100%T, 100ms for mClover), and deconvolved using the built-in SoftWoRx software. The Hoechst images were adjusted in Photoshop by increasing brightness by 92 and contrast by 92 in legacy mode (applied equally to the entire image for all timepoints). The mClover images were adjusted in Photoshop by increasing brightness by 164 and contrast by 123 in legacy mode (applied equally to the entire image for all timepoints). The images were merged in Photoshop using the "screen" function.

**In situ Hi-C.** Applicants generated 69 *in situ* Hi-C libraries using the MboI restriction enzyme following the protocol described in(Rao et al., 2014) without modifications. In brief, the *in situ* Hi-C protocol involves crosslinking cells with formaldehyde, permeabilizing them with nuclei intact, digesting DNA with a suitable 4-cutter restriction enzyme, filling the 5'-overhangs while incorporating a biotinylated nucleotide, ligating the resulting blunt-end fragments, shearing the DNA, capturing the biotinylated ligation junctions with streptavidin beads, and analyzing the resulting fragments with paired-end sequencing.

Applicants generated 7 libraries each for the main maps (untreated HCT-116 RAD21-mAC cells and HCT-116 RAD21-mAC cells treated for 6 hours with IAA) comprised of two sets of biological replicates each (three and four technical replicate libraries per biological replicate). In addition, Applicants generated four technical replicate libraries each for untreated and treated HCT-116 RAD21-mAC cells after cell synchronization and arrest. Applicants also generated an additional 5 libraries from untreated HCT-116 RAD21-mAC cells and 4 libraries from HCT-116 RAD21-mAC cells treated for 6 hours with IAA (two additional biological replicates each) that were not included in our main maps, but were used for analysis of higher-order contacts (see below). Applicants generated four libraries (two biological replicates) for each of our loop-resolution maps along the auxin withdrawal time course (20, 40, 60 and 180 mins after auxin withdrawal). Finally, Applicants generated two technical replicate libraries per time point of our auxin treatment and withdrawal time course. Similar results were obtained with Hi-C libraries from synchronized and arrested cells (**Fig. 15**), so for all analyses presented in the main text and figures of the manuscript (other than the time course analyses), Applicants utilized the high resolution maps from the unsynchronized cells. Further details about the Hi-C libraries and details about which experiments were used in which figures are provided in Table 1.

**ChIP-Seq.** ChIP-Seq for H3K27Ac, H3K4me1, H3K4me3, H3K36me3, H3K27me3, H3K9me3, H4K16Ac, H4K20me3, H3K79me2, and H2.AZ was performed using a native ChIP-Seq protocol. Chromatin from untreated HCT-116 RAD21-mAC cells or cells treated for 6 hours with 500uM IAA was digested with Mnase (Sigma) in digestion buffer (50 mM Tris-HCl, pH7.6, 1 mM CaCl₂, 0.2% Triton X-100, butyrate 5 mM) for 5' at 37°C and dialyzed against RIPA buffer for 2hrs at 4°C. Five microgram of respective antibody was incubated with 40 µl of Dynabeads Protein A (or G) for 40 min at room temperature. Antibody-bound beads were added to 500 µl of sonicated or Mnase-digested chromatin, incubated at 4°C overnight, and washed twice with RIPA buffer, twice with RIPA buffer containing 0.3M NaCl, twice with LiCl buffer (0.25 M LiCl, 0.5% Igepal-630, 0.5% sodium deoxycholate), once with TE (pH 8.0) plus 0.2% Triton X-100, and once with TE (pH 8.0). ChIP DNA was purified by phenol-chloroform extraction followed by ethanol precipitation. Libraries were prepped for Illumina sequencing and 50bp single-end reads were sequenced on a HiSeq2000 or 2500 (Illumina). Applicants also performed ChIP-Seq for RAD21 and CTCF following the same protocol as above, except that cells were fixed with 1% formaldehyde for 10 minutes at 37°C and fixation was quenched by the addition of glycine to a final concentration of 125mM for 10 minutes. The fixed cells were sonicated using a Branson sonifier at amplitude 35%, 12 cycles of 20 seconds of sonication and 30 seconds of pause. Antibody was added to the sonicated chromatin as above and the samples were further processed as above.

Applicants also performed ChIP-Seq for SMC1 and an additional replicate for CTCF following the protocol outlined by the ENCODE consortium (2012).

All ChIP-Seq experiments were processed in parallel with whole cell extract input controls.

**PRO-Seq.** To measure changes in transcription resulting from cohesin loss, Applicants performed precision run-on sequencing (PRO-Seq) (Jonkers and Lis, 2015), a variant of global run-on sequencing (GRO-Seq), using a single biotinylated nucleotide (biotin-11-CTP) as previously described (Engreitz et al., 2016). Applicants made one modifications to the protocol: at the end of each biotin enrichment, Applicants eluted biotinylated RNAs from the streptavidin-coated magnetic beads by heating beads in 25 µl of 20 mM Tris-HCl pH 7.5, 10 mM EDTA, 2% N-lauroylsarcosine at 95°C for 5 minutes, followed by a magnetic-bead nucleic acid purification with 20 µl of MyONE SILANE beads. During the nuclei preparation step, Applicants processed pairs of RAD21-mAC cells with and without auxin treatment in parallel. In addition, Applicants performed PRO-Seq on HCT-116 CMV-OsTIR1 cells, the parental cell line of RAD21-mAC containing the *OsTIR1* gene integrated at the AAVS1 locus and no mAID tags integrated on any protein. By performing PRO-Seq on CMV-OsTIR1 cells with and without auxin treatment, Applicants could control for transcriptional effects of the auxin treatment itself on HCT-116 cells, as well as any consequences of tagging the RAD21 protein.

### QUANTIFICATION AND STATISTICAL ANALYSIS

**Hi-C Data Processing.** All Hi-C libraries were sequenced either on an Illumina NextSeq500 (either 80 or 85bp paired-end reads) or a HiSeqX (150bp paired-end reads). All resulting data was processed using Juicer (Durand et al., 2016b; Rao et al., 2014). The data was aligned against the hg19 reference genome. All contact matrices used for further analysis were KR-normalized with Juicer.

Loops were annotated in both untreated and treated maps using HiCCUPS (Durand et al., 2016b; Rao et al., 2014). Loops were called at 5kb, 10kb, and 25kb resolutions and merged as described in (Rao et al., 2014). Default parameters as described in (Durand et al., 2016b; Rao et al., 2014) were used with the exception that an additional enrichment filter was added. Applicants noted that due to karyotypic abnormalities in the HCT-116 cell line, many rearrangements were annotated in both the untreated and treated maps. Since rearrangements appear as very intense pixels off-diagonal, Applicants removed any peak calls that displayed an observed/expected enrichment of >4.5. Empirically, this max threshold removed peak annotations due to rearrangements; notably, nearly the same number of annotated peaks were removed from the untreated and the treated annotations, 277 and 269 respectively. In the end, Applicants annotated 3,170 loops in our untreated maps and 81 loops in our treated maps.

Domains were annotated in both untreated and treated maps using Arrowhead (Durand et al., 2016b; Rao et al., 2014). Domains were called at 5kb and 10kb resolutions using default parameters and merged (retaining the 5kb domain annotation for any pair of domains annotated in both the 5kb and 10kb annotations). Applicants annotated 9,845 domains in our untreated maps and 2,090 domains in our treated maps.

**ChIP-Seq Data Processing.** All ChIP-Seq data was aligned to hg19 with BWA (Li and Durbin, 2010) (Li and Durbin, 2010) (Li and Durbin, 2010), deduplicated using PicardTools, and analyzed with MACS 2.0 (Liu, 2014). All data was normalized against the corresponding input control using the '-c' option of MACS 2.0. ChIP-Seq peaks were called using the 'callpeak' function of MACS 2.0 with default parameters. For H3K4me3 and H3K4me1, Applicants additionally used the '-broad' option of MACS 2.0 and merged close by peaks to identify broad peaks. Finally, for H3K4me1 and H3K27Ac, Applicants filtered peaks called by MACS by requiring an adjusted -log10 p-value of at least 7 and 5 respectively in order to ensure that Applicants had a high quality peak annotation. Finally, in order to annotate broad-source domains, Applicants utilized RSEG (Song and Smith, 2011) using the following flags: '-b 25000', '-i 20', '-mode 2'.

Signal tracks were calculated by using the 'bdgcmp' option of MACS 2.0 with the 'FE' (fold-enrichment) method. All data for downstream analysis was averaged and extracted from these tracks.

**PRO-Seq Data Processing.** For analysis of PRO-Seq data, Applicants aligned 30-bp paired-end reads to the hg19 reference (bowtie2 v2.1.0, (Langmead and Salzberg, 2012)), removed duplicate reads (Picard picard.sourceforge.net), and discarded reads with MAPQ < 30. Applicants counted reads overlapping RefSeq genes (collapsed by gene symbol to the longest isoform) - this quantification procedure includes signal both at the paused position (near the TSS) as well as in the gene body. Applicants identified genes showing significant differences in transcription with DESeq2 (Love et al., 2014), excluding genes with zero coverage in all samples and calling significance at Benjamini-Hochberg corrected *p*-value < 0.05.

To determine whether there were global changes in the total amount of transcription (up or down) that would affect the normalization and analysis of these experiments, Applicants included a spike-in control in three of the four PRO-Seq replicates for each of untreated and treated RAD21-mAC and CMV-OsTIR1 cells. Specifically, Applicants added ~500,000 Drosophila S2 cells at the beginning of the protocol. Upon sequencing of these libraries, Applicants counted the number of spike-in reads by aligning to the Drosophila genome (dmel3) with bowtie2 v2.1.0. Applicants observed similar fractions of reads mapping to the Drosophila spike-in in the matched pairs of degron-expressing and control replicate experiments, indicating that there are not significant global changes in the total amount of transcription upon cohesin loss.

**Random Shuffle Annotations.** When performing quantitative analyses on our feature annotations, it was frequently desirable to have a "random control" for the feature annotation in question. Applicants generated such annotations through a random permutation procedure. For one-dimensional features, such as peak loci, Applicants randomly placed the one-dimensional features throughout the genome such that (1) the number of features on any one chromosome stayed the same; (2) the random features did not overlap any gaps in the assembly (i.e. centromeres, telomeres, etc.). Similarly, for two-dimensional features (domains, peaks), Applicants randomly placed the two ends of the features across the genome such that (1) the size distribution of the two-dimensional features stayed the same; (2) the number of features on any one chromosome stayed the same; (3) the interval between the ends of the randomized two-dimensional features did not overlap any gaps in the assembly.

**Analysis of CTCF and cohesin binding.** In order to confirm that degradation of RAD21 resulted in abrogation of full cohesin complex binding to chromatin, Applicants performed ChIP-Seq for RAD21 and SMC1 (see above for experimental details). Applicants visually confirmed that cohesin binding was significantly diminished (**see** **Fig. 1C**). Applicants also analyzed the RAD21 and SMC1 signal at all ChIP-Seq peaks called in our data from untreated cells using MACS 2.0. The heatmaps clearly showed that cohesin binding was eliminated upon degradation of RAD21. Applicants saw an average 81% reduction in binding strength of RAD21 (mean enrichment = 12.58 in untreated HCT-116 RAD21-mAC cells; mean enrichment = 2.39 in treated cells) and 75% reduction of SMC1 (mean enrichment = 16.23 in untreated HCT-116 RAD21-mAC cells; mean enrichment = 4.00 in treated cells). Moreover, 97% of peaks called in untreated cells (from a merged list of peaks binding both RAD21 and SMC1) were not called after auxin treatment (**Fig. 8C**). These results demonstrate that Applicants were able to quickly abrogate cohesin binding to chromatin to near completion using our auxin-inducible degron system.

Applicants also performed ChIP-Seq for CTCF to establish whether CTCF binding was dependent on cohesin binding or not. Visual inspection and analysis of signal at all peaks called in data from untreated cells using MACS 2.0 clearly demonstrated that CTCF remained bound after RAD21 degradation (**Fig. 1C****,D**). The average enrichment at all CTCF binding sites called by ENCODE was 10.89 in untreated cells and 8.93 in treated cells (The difference in enrichment was likely due to differential quality of the immunoprecipitation rather than true biological differences; one of our replicate experiments showed an average increase of CTCF binding after auxin treatment and the other showed an average decrease.) This data supports a model in which cohesin is a member of an extrusion complex that slides across DNA, whereas CTCF finds it binding sites independently of the extrusion complex and acts as an oriented brake to halt extrusion complex translocation.

**Analysis of histone modification patterns.** Applicants calculated average signal over 5kb bins across the entire genome and correlated signal between ChIP-Seq experiments before and after auxin treatment for each of CTCF, H3K27me3, H3K9me3, H3K4me1, H3K4me3 and H3K27Ac. All modifications showed high correlations before and after auxin treatment (Spearman's r = 0.80 [CTCF]; 0.95 [H3K27me3]; 0.95 [H3K9me3]; 0.94 [H3K4me1], 0.70 [H3K4me3]; 0.90 [H3K27Ac]; 0.96 [H2.AZ]; 0.94 [H3K36me3]; 0.96 [H3K79me2]; 0.89 [H4K20me3]; 0.95 [H4K16Ac]).

Additionally, Applicants examined overlaps of our ChIP-Seq peak annotations before and after auxin treatment for H3K4me3, H3K4me1 and H3K27Ac. High overlap (90%, 85% and 81% respectively) was seen for all three marks, indicating that the positions of active promoters and enhancers are largely unchanged after cohesin loss (**Fig. 8D**).

Finally, Applicants examined the positions and enrichments of broad source marks (H3K27me3, H3K9me3, H2AZ, H3K36me3, H4K17Ac, H3K79me2). Applicants called broad domains in our data from untreated cells using RSEG (see above), and identified a high-quality list of boundaries by retaining only the broad domains that were >=100kb in size and restricting to boundaries of these domains where at least a 50% change in signal across the boundary was seen (when averaging over the 50kb upstream and downstream of the boundary). Applicants identified 2907 such boundaries for H3K27me3, 2647 boundaries for H3K9me3, 6356 boundaries for H2.AZ, 4829 boundaries for H3K36me3, 2515 boundaries for H4K16Ac, and 3634 boundaries for H3K79me2. Applicants then extracted the data +/- 200kb from the boundary in both untreated and auxin-treated cells for each mark. The heatmaps and average profiles across the boundaries called in untreated cells indicated that the positions of the boundaries were unchanged after auxin treatment (i.e. there was no spreading of histone modifications) and the average enrichments of histone modifications over the broad enriched domains were unchanged (**Fig. 8E**).

Taken together, these results suggest that histone modification patterns are largely unaffected by loss of cohesin.

**Evaluation of loops and loop domains.** Applicants used HiCCUPS to calculate local enrichments on treated maps for the 3,170 loops Applicants annotated with HiCCUPS in untreated maps. No loop showed at least 1.3-fold enrichment over local backgrounds and <30% FDR q-value. This clearly demonstrates that the vast majority of looping is lost after cohesin degradation.

Applicants identified loop domains as in (Rao et al., 2014), by searching for loop-domain pairs where the peak pixel was within the smaller of 50kb or 0.2 of the length of the domain at the corner of the domain. Using this procedure, Applicants identified 2,140 loop domains in untreated cells and only 9 in treated cells. Of the 9, 8 were false positives due to rearrangements in HCT-116 cells and one was a false positive due to extensive compartmentalization that was mistakenly annotated as a loop by HiCCUPS. Notably, the high false discovery rate after auxin treatment is due to the very small number of true positives (in this case, no detectable true positives). The false discovery rates of HiCCUPS and Arrowhead before auxin treatment were comparable to the FDRs documented in (Rao et al., 2014). This clearly demonstrates that loop domains are lost after cohesin degradation.

Applicants also assessed the loss of loop domains and loops via aggregate peak analysis (APA). Applicants used default parameters at 10kb resolution, excluding loop domains and loops within 300kb of the diagonal to avoid distance decay effects and extracting a 200kb by 200kb submatrix around every loop domain or loop. In aggregate, the signal from loop domains and loops was clearly and completely lost after auxin treatment: the APA score (fold-enrichment of the peak pixel over the mean value of the 36 pixels in the 6x6 box in the lower left of the aggregate matrix) went from 2.10 to 0.78 for loop domains and 2.09 to 0.78 for all loops. (The APA scores <1 after treatment are expected since random pixels would show an APA score <1 because of the contact probability distance decay.) All visual signs of looping and domain formation were also lost in the aggregate matrices (**Fig. 9C**). In addition, Applicants confirmed that the loss of loops/loop domains was not a result of any cell cycle effects; Applicants visually observed loss of loop domains after auxin treatment in G1/S arrested cells **(****Fig. 15H-L****)** and observed similar results to above via APA (**Fig. 9C**).

In order to assess the dynamics of loop and domain formation Applicants used APA and aggregate domain analysis (ADA) to assess loop, loop domain, and domain strength across a time course of auxin treatment and withdrawal.

ADA uses the same principle of aggregating submatrices across a feature list, but instead of calculating a score representing the focal enrichment of a peak pixel against pixels to its lower left (the APA score), Applicants calculate a score representing the enrichment of contacts just inside the domain boundaries over the contacts just outside the boundary, i.e. a gradient across the boundary. More specifically, Applicants compare the average contacts in the pixels [i+3, j-13:j-3], [i+4,j-12:j-2], [i+5,j-11:j-1], [i+1:i+11,j-5], [i+2:i+12,j-4], [i+3:i+13,j-3] (the inside domain pixels) to the pixels [i-5,j-21:j-11], [i-4,j-20:j-10], [i-3,j-19:j-9], [i+11:i+21,j+5], [i+10:i+20,j+4], [i+9:i+19,j+3] (the outside domain pixels) where (i,j) is the center of the aggregate matrix (i.e. the corner of the domains). Here, Applicants extracted a 200kb by 200kb matrix at 5kb resolution around every domain corner.

For APA on the time course experiments, as with the APA on our deep maps, Applicants used default parameters at 10kb resolution.

In **Fig. 2B****,** Applicants show the APA scores for all loop domains greater than 300kb in size. The APA scores demonstrate that after cohesin is degraded, loop domains are completely lost between 40-60 minutes after treatment. From our own imaging (**Fig. 8A****,B**) and imaging performed in Natsume et al. (2016), Applicants know that the half life of cohesin after auxin treatment is about 20 minutes. Thus, loop domains are lost within minutes of cohesin degradation, indicating that cohesin is required for active maintenance of loop domain structures, not just establishment. After withdrawal of auxin, loop domains form similarly quickly, with strong loop domain signal by 60 minutes after withdrawal. This time includes the time for cohesin levels to recover and thus likely represents a very conservative upper bound on the time required for loop domain formation. Additionally, it indicates that loop domain structures are dynamically maintained during interphase.

**In** **Fig. 9B****,** Applicants show similar results for all loops greater than 300kb in size called in untreated cells. Additionally, Applicants show ADA scores for all contact domains larger than 300kb in size called in untreated cells. While the ADA scores for all domains show a sharp decline in the first 60 minutes after auxin treatment, they plateau above 1, indicating some residual domain signal from al domains. Since, as Applicants noted in (Rao et al., 2014), not all domains are loop domains, this suggest residual retention of non-loop domains. As Applicants noted in (Rao et al., 2014), non-loop domains are usually created by compartment intervals. The retention of compartment domains is discussed below.

**Analysis of previous cohesin-depletion Hi-C data** sets. Previous Hi-C studies after cohesin or CTCF depletion showed limited effects, with both contact domains and compartments present after depletion (Seitan et al., 2013; Sofueva et al., 2013; Zuin et al., 2014). However, in these studies, the authors performed low resolution Hi-C experiments, raising the possibility that either (i) the authors could not resolve the difference between loop domains (which disappear after cohesin loss) and compartment domains (which remain) due to resolution issues, or (ii) incomplete depletion of cohesin or CTCF led to modest phenotypes. The authors in all three studies acknowledge the possibility that the limited effects they see may have been due to incomplete depletions.

To test this hypothesis, Applicants re-analyzed data from these three studies. Applicants downloaded the raw fastqs for all Hi-C experiments performed in the studies and processed them with Juicer (in exactly the same way that Applicants processed all the Hi-C data generated for this study. Although the experiments did not have sufficient resolution to visualize individual loops, Applicants looked for the statistical signal of loop enrichment in aggregate using APA (Durand et al., 2016b; Rao et al., 2014). For the mouse data sets generated in Seitan et al. and Sofueva et al., Applicants used a loop list Applicants had previously generated in CH12-LX mouse lymphoblast cells (Rao et al., 2014) with the added filter that Applicants removed loops with >4.5 enrichment over local background in order to stay consistent with the methods used in this study (see above). For the human data sets generated in Zuin et al., Applicants used the loop list of 3,170 loops in untreated HCT-116 RAD21-mAC cells described above. Applicants observed positive APA scores (>1) and visible focal enrichment in all experiments generated in previous studies, before and after cohesin or CTCF depletion (**Fig. 9C**). By contrast, our maps after auxin treatment show complete loss of APA signal and no visible focal enrichment, even when APA is performed on low resolution data sets (**Fig. 2B**, **9C**). In previous studies, the APA score was weaker after cohesin or CTCF depletion but still clearly visible and notably, positive APA signal was seen in every replicate experiment performed in previous studies. Taken together, this suggests that a major confound of previous studies was the incomplete depletion of cohesin or CTCF, and along with the low resolution of the Hi-C experiments, likely explains the limited effects seen.

**Evaluation of the dynamics of loop domain formation.** In order to better understand the process of loop domain reformation, Applicants utilized loop-resolution Hi-C datasets generated after 6 hours of auxin treatment and 20, 40, 60 or 180 minutes of auxin withdrawal. Applicants sequenced 951M, 952M, 909M, and 949M reads from each of the timepoints respectively. Applicants then converted our list of loop domains identified in untreated RAD21-mAC cells (generated at 5kb, 10kb and 25kb resolutions) into a standard 25kb resolution, by identifying the 25kb pixel containing the peak pixel as well as the 8 25kb pixels around it, and assigning the new 25kb peak pixel as the one of nine containing the most contacts. (Applicants analyzed the 9 25kb pixels rather than just the 25kb pixel containing the original peak pixel in order to account for small amounts of noise in the peak localization.)

Using this list of 1,988 loop domains at 25kb resolution (leaving out loop domains <100kb in size which are more difficult to analyze at 25kb resolution), Applicants used HiCCUPS to identify the observed and local expected values for all peak pixels in our untreated, auxin treated, 20 min withdrawal, 40 min withdrawal, 60 min withdrawal, and 180 min withdrawal data sets. In order to compare between datasets of different sizes, Applicants scaled the observed and expected values for each map down by a scalar factor equal to the (# of contacts in the map/# of contacts in the 20 min withdrawal map). (The 20 min withdrawal map was our lowest sequencing depth map.) For each loop domain, Applicants constructed a recovery curve, setting the number of observed contacts in the untreated map as 1, the number of observed contacts in the auxin treated map as 0, and (observed - [6hr treat observed])/([no treat observed] - [6hr treat observed]) as the value at every other time point (**Fig. 2C**). (Similar results were observed if Applicants used an observed/local expected metric, or a z-score metric [(observed-local expected)/sqrt(local expected)]; data not shown.)

To rank loop domains by speed of recovery, Applicants utilized a metric where Applicants calculated the difference between the loop domain's recovery at a particular time point and the recovery of the median loop domain at that time point. Applicants calculated this value for all loop domains at the 40 minute and 60 minute time points and summed the two to get a recovery score for each loop domain. To identify features associated with variation in loop domain recovery time, Applicants stratified loop domains by the above recovery score, and compared loop domains from different quantiles to a number of features (NIPBL binding, promoters, enhancers, histone modifications). For punctate features (such as NIPBL binding sites, promoters and enhancers), Applicants calculated the density of peaks across each domain (i.e. peaks/Mb). For broad source features (such as H3K36me3 and H3K27me3), Applicants calculated the average enrichment across each domain. For each quantile, Applicants calculated the enrichment of a feature by comparing the average peak density or signal enrichment of the quantile to the average peak density or signal enrichment across all loop domains (**Fig. 2D**). Additionally, Applicants included superenhancers (as annotated by Hnisz, et al Cell 2013) and strong NIPBL binding sites (the top 10% of binding sites called in a merged list of peaks called in both untreated and auxin treated data sets) in our feature list. Applicants found that activating marks, and in particular superenhancers and strong NIPBL binding sites, were enriched in loop domains that recovered quickly (fast loop domains) vs loop domains that recovered slowly (slow loop domains).

Applicants also stratified loop domains by their density of NIPBL ChIP-Seq peaks and H3K27Ac ChIP-Seq peaks. Applicants found that the top 10% of loop domains by density of NIPBL peaks (>=24 peaks/Mb) showed 38% recovery by 40 minutes, while the bottom 10% of loop domains by density of NIPBL peaks (0 peaks/Mb) showed only 11% recovery by 40 minutes. Similarly, the top 10% of loop domains by density of H3K27Ac peaks (>=54.4 peaks/Mb) showed 38% recovery by 40 minutes, while the bottom 10% of loop domains by density of H3K27Ac (<=2.76 peaks/Mb) showed only 8% recovery by 40 minutes (**Fig. 9D**).

Applicants also repeated the above analyses after restricting to loop domains that exhibited no NIPBL or H3K27Ac binding within 50kb of either loop anchor, in order to test whether features in the interior of a loop domain could affect the formation of loops. Applicants observed similar enrichments of activating factors, and especially strong NIPBL sites and superenhancers at the loop domains that recovered more quickly vs those that recovered more slowly. The top 10% of loop domains by NIPBL density (>=12 peaks/Mb) showed 27% recovery by 40 minutes vs. 9% recovery by 40 minutes for the bottom 10% of loop domains (0 peaks/Mb). The top 10% of loop domains by H3K27Ac density (28 peaks/Mb) showed 29% recovery by 40 minutes vs. 7% recovery by 40 minutes for the bottom 10% of loop domains (0 peaks/Mb). The association between NIPBL and enhancers and faster loop domain recovery, even when restricting to domains that only exhibit these features in the interior of the domain far from the anchors, is highly suggestive of an extrusive process; the ability of a feature far from the anchors of a loop to modulate loop domain formation time is more consistent with an extrusion model rather than a 3D diffusion model. Of course, these features may be simply correlated with other features at the anchors, or may modify the flexibility of the chromatin fiber in a way that affects the rate of 3D diffusion between the anchors; further experiments are needed to elucidate the relationship between NIPBL, superenhancers and loop domain formation speed.

**Evaluation of genome compartmentalization.** The most common method used for classifying Hi-C patterns is the principal component (PC) approach, which Applicants introduced in (Lieberman-Aiden et al., 2009). In this approach, each intrachromosomal contact matrix is converted to an observed/expected matrix, and the first principal component of this matrix is used to bifurcate the data into two clusters. Applicants showed in (Rao et al., 2014) that this method does not capture compartment structure accurately at high resolutions; however it is useful for comparing gross compartmentalization patterns.

Applicants first calculated the first three principal components of the 25-kb resolution observed/expected matrix for each chromosome (constructed using Juicer) using scikit learn's RandomizedPCA function. Applicants chose the principal component most correlated with GC content and assigned sign such that the vector was positively correlated with the GC content vector. Applicants then calculated the correlation of the eigenvector for each chromosome between untreated and treated maps. The mean correlation was 0.968.

To identify transitions in compartment state at higher resolution, Applicants used a combination of techniques. First, Applicants calculated an edge score using an algorithm similar to Canny edge detection. For every 25kb locus in the genome, Applicants looked at the corresponding column of the 25kb log2(observed/expected) matrix. For every pixel (i,j) in column j, Applicants calculated a gradient = [i,j:j+3] - [i,j-4,j-1]. Applicants then searched for stretches of at least 7 pixels in the column with a gradient x such that abs(x) was greater than 0.5. Applicants then extended the edges by including pixels adjacent to an edge that had a gradient of at least 0.3. Finally, Applicants summed the number of pixels in a column belonging to an edge to calculate the edge score for a locus. Applicants then called local peaks in this track which could correspond to compartment state transitions since a compartment state transition at locus I will create an edge between locus i-1 and i.

Since loci in the same compartment will exhibit the same rises and falls in contact probability as one slides along the genome, Applicants reasoned that adjacent pixels should exhibit high correlations of the derivative of their contact patterns and low correlations could indicate a compartment state transition. As described in Section V.a.3 of Rao et al., 2014, this is akin to measures in finance that correlate returns of prices to identify similarities between stocks. To calculate this sliding derivative correlation score, Applicants calculated the gradient in the log2(observed/expected) matrix over every boundary called in our edge score track. More specifically, for every locus i, and all boundaries j in boundary set J that were within 15Mb of i, Applicants calculated the difference of mean([j:j+5,i]) and mean([j-6:j-1,i]). Applicants then calculated the Spearman correlation coefficient of these two vectors (one vector for the gradients at all boundaries j in J for the pixels upstream of i, and one vector for the pixels downstream of i). Applicants excluded the derivative signal at pixels not located at compartment state transitions as defined by the edge score to reduce noise, reasoning that pixels inside compartment intervals were unlikely to contribute meaningful rises/falls in contact probability. Similarly, Applicants only included pixels within 15mb of i to reduce the noise arising from sparsity far off the diagonal.

Applicants then identified compartment boundaries by calling local peaks in the edge score track and local valleys in the sliding derivative correlation score track and merging the two peak call lists.

Applicants identified 4,325 boundaries in untreated cells for a median compartment size of 425 kb and 4,424 boundaries in treated cells for a median compartment size of 475 kb. These are very likely conservative upper bounds on the true median compartment size, since Applicants utilized stringent peak calling and compartment structure can be difficult to detect in maps that are not extremely high resolution (Rao et al., 2014).

To assess the presence and strength of contact domains after auxin treatment, Applicants used the Arrowhead algorithm (Durand et al., 2016b; Rao et al., 2014). The Arrowhead algorithm calculates a corner score for every pixel, where higher corner score values represent a higher likelihood that a pixel is at the corner of a domain (see Section IV.a.3 of the Extended Experimental Procedures of Rao et al., 2014). For the list of 9,845 contact domains identified by the Arrowhead algorithm in untreated RAD21-mAC cells, Applicants compared the corner scores of the contact domains to the corner scores of random pixels with an identical chromosome and length distribution. The median corner score in untreated cells for all domains called in untreated cells was the 97th percentile of random corner scores. Applicants then calculated the corner scores in treated cells for the list of contact domains annotated in untreated cells, as well as the corner scores for the random control. Here, the median corner score for annotated contact domains was only the 86th percentile of random corner scores. (Notably, the distributions of scores for random pixels did not change, see **Fig. 3B****.**) This indicates that contact domains were significantly weakened after auxin treatment. However, there was still some residual signal.

Since Applicants knew that loop domains were completely eliminated from our previous analyses and that compartment structure remained after treatment, Applicants reasoned that the residual signal was arising from retained compartment domains (contact domains whose boundaries overlap compartment interval boundaries). To test this, Applicants identified 974 contact domains whose boundaries overlapped a compartment interval boundary (within 25 kb), i.e. compartment domains. Additionally, Applicants identified 410 contact domains whose boundaries were not within 100 kb of a compartment boundary even after using a relaxed threshold for identifying compartment boundaries, i.e. a high confidence set of non-compartment domains. Applicants then analyzed the corner scores for each of these sets of domains separately in treated cells and found that while the median score for compartment domains was 89^{th} percentile of the random corner scores, the median score for non-compartment domains was only 72nd percentile of the random corner scores. This indicates that the residual signal stems from retained compartment domains. Thus, while loop domains are completely eliminated, contact domain structure arising from genome compartmentalization is still present after auxin treatment, although the domains are weaker than those found in untreated cells.

It is commonly thought in the literature that contact domains and compartment intervals form a hierarchy, with compartment intervals often being subdivided into multiple contact domains, but each contact domain belonging to only one compartment interval. Having determined that loop domains and compartmentalization formed via independent mechanisms, Applicants wondered whether loop domains and compartment intervals shared characteristic hierarchical relationships or whether they truly formed independently in the genome.

To assess whether compartment boundaries could be spanned by loop domains, Applicants intersected our loop domain annotation and our compartment boundary annotation. Specifically, Applicants identified compartment boundaries in our treated maps that were contained within a loop domain called in untreated cells and >100kb away from either loop anchor (obviously this excludes loop domains smaller than 200kb from the analysis). Applicants identified 349 such boundaries. Visual examination also confirmed that these boundaries were true compartment state transitions lying inside loop domains (**Fig. 3C****,D**). Note that this is a lower bound on the number of compartment boundaries spanned by loop domains, as Applicants used stringent distances from loop anchors to reduce false positives and our compartment boundary annotation has false negatives as well. This demonstrates that there is no true hierarchy between compartmentalization and loop domain formation, contrary to what has been suggested in the literature.

Applicants wondered what happen to compartment strength at these boundaries when loop domains were eliminated. To analyze this, Applicants calculated the average sliding derivative correlation score (see above) for the 1Mb intervals centered on the 349 compartment boundaries contained within loop domains before and after auxin treatment. Applicants observed that the boundaries contained within loop domains showed a strong increased in compartment strength (larger dip in the sliding correlation score) after the elimination of loop domains: 0.10 decrease in the sliding correlation score in untreated cells vs. 0.31 in treated cells. In contrast, when Applicants identified 389 compartment boundaries in treated cells that were positioned at loop domain anchors annotated in untreated cells (within 25kb), Applicants found that there a much more modest increase in compartment strength after treatment: 0.35 decrease in the sliding correlation score in untreated cells vs. 0.53 in treated cells (**Fig. 3E**). This indicates that cohesin facilitates mixing of distinct compartment states and causes decreases in compartmentalization unless it is halted at the compartment boundary.

The results were similar when Applicants examined compartment boundaries inside all loops: Applicants identified 593 compartment boundaries in treated cells that were spanned by loops and at least 100kb away from either loop anchor, and Applicants identified 503 compartment boundaries in treated cells that were positioned at loop anchors. Applicants saw an 0.11 decrease in the sliding correlation score in untreated cells vs. 0.37 decrease in treated cells for compartment boundaries spanned by loops, and an 0.38 decrease in the sliding correlation score in untreated cells versus an 0.54 decrease in treated cells for compartment boundaries at loop anchors (**Fig 10D**).

To assess whether the changes in compartmentalization seen after treatment corresponded to epigenetic activity, Applicants performed a similar analysis except instead of calling compartment boundaries, Applicants identified transitions in broad histone modification state for H3K27Ac and H3K27me3. Since histone modifications have been shown to very closely correlate with compartmentalization (Lieberman-Aiden et al., 2009; Rao et al., 2014; Sexton et al., 2012), Applicants reasoned that changes in histone modification within loop domains and loops should show greater changes in compartmentalization to better match the histone modification pattern compared to changes in histone modification status at loop anchors. Applicants identified changes in H3K27Ac status by creating a 25kb binary track that was either 0 if the enrichment was less than 0.35 or 1 if the enrichment was greater than 0.35. Applicants then calculated the absolute value of a smoothed gradient (using the kernel [1 1 1 -1 -1 -1]) and called local peaks to identify changes in histone modification status. Applicants identified 264 H3K27Ac transitions spanned by loop domains (same definition as above) and 307 H3K27Ac transitions positioned at loop domain anchors. The H3K27Ac signal in the 1Mb intervals around these transitions did not change after auxin treatment (**Fig. 3F**). However, while there was very little change in the compartmentalization strength at transitions at loop domain boundaries (0.41 dip in sliding correlation in untreated vs. 0.49 in treated), there was a dramatic increase in compartmentalization strength at transitions spanned by loop domains (0.02 dip in sliding correlation in untreated vs. 0.19 in treated). This indicates that removal of loop domains by cohesin loss leads to genome compartmentalization that more closely matches histone modification patterns.

Similar results were seen for H3K27Ac transitions spanned by all loops: Applicants identified 426 H3K27Ac transitions in untreated cells that were spanned by loops and at least 100kb away from either loop anchor, and Applicants identified 381 H3K27Ac transitions in untreated cells that were positioned at loop anchors. The H3K27Ac signal in the 1Mb intervals around these transitions did not change after auxin treatment (**Fig. 3F**). Applicants saw an 0.41 decrease in the sliding correlation score in untreated cells vs. 0.50 decrease in treated cells for H3K27Ac transitions spanned by loops, and an 0.10 decrease in the sliding correlation score in untreated cells versus an 0.26 decrease in treated cells for H3K27Ac transitions at loop anchors **(****Fig 10B**).

Applicants also performed this analysis for H3K27me3. Applicants calculated the gradient at every 25kb locus i the genome by taking the absolute value of the difference between the summed log2 fold-enrichment for pixels i-8 to i-1 and the summed log2 fold-enrichment for pixels i+1 to i+8. Applicants called local peaks on this gradient track to identify loci where the broad H3K27me3 modification status changed. Applicants identified 209 H3K27me3 transitions spanned by loop domains (same definition as above) and 384 H3K27me3 transitions positioned at loop domain anchors. The H3K27me3 signal in the 1Mb intervals around these transitions did not change after auxin treatment (**Fig. 10A**). However, while there was very little change in the compartmentalization strength at transitions at loop domain boundaries (0.29 dip in sliding correlation in untreated vs. 0.33 in treated), there was a stronger increase in compartmentalization strength at transitions spanned by loop domains (0.01 increase in sliding correlation in untreated vs. 0.03 dip in treated).

Similar results were seen for H3K27me3 transitions spanned by all loops: Applicants identified 391 H3K27me3 transitions in untreated cells that were spanned by loops and at least 100kb away from either loop anchor, and Applicants identified 469 H3K27me3 transitions in untreated cells that were positioned at loop anchors. The H3K27me3 signal in the 1Mb intervals around these transitions did not change after auxin treatment (**Fig**. **10C**). Applicants saw an 0.27 decrease in the sliding correlation score in untreated cells vs. 0.31 decrease in treated cells for H3K27me3 transitions spanned by loops, and an 0.03 decrease in the sliding correlation score in untreated cells versus an 0.12 decrease in treated cells for H3K27me3 transitions at loop anchors **(****Fig 10C**).

Taken together, these results suggest that cohesin facilitates mixing of chromatin with different histone modification states and loss of cohesin leads to better correspondence of genome compartmentalization with histone modification patterns and gene activity.

**Annotation and analysis of cohesin-independent links.** Applicants first annotated loops in our maps for auxin-treated RAD21-mAC cells using default HiCCUPS parameters for 5,10, and 25kb resolutions (Durand et al., 2016b; Rao et al., 2014) with the additional requirement that the peak pixel show less than 4.5-fold enrichment over local expecteds (in order to remove as many false positives as possible due to rearrangements and assembly issues, see above). Using this procedure, Applicants annotated 81 loops in treated RAD21-mAC cells. When Applicants visually examined these loops, Applicants found that 66 were false positives, with 55 of the false positives due to assembly issues, issues with repetitive elements or structural rearrangements. The false discovery rate for HiCCUPS is much higher in treated cells because the number of true positives is dramatically lower. As mentioned above, the false discovery rate in untreated cells was comparable to the rates described previously in (Rao et al., 2014); in fact, as one might expect false positives to arise from artifacts in the data that are independent of cohesin-mediated looping, the reduction by nearly 98% of numbers of loops called by HiCCUPS after auxin treatment is a powerful proof of its accuracy. When Applicants examined the 15 true positive loops annotated by HiCCUPS, Applicants found that they had a dramatically different distance distribution than cohesin-associated loops: where the median size of a cohesin-associated loop was 275kb, the median size of these 15 loops was 1.75Mb. Applicants also noticed that the anchors involved in these 15 loops were often forming long-range loops at distances of tens of megabases and hundreds of megabases. Applicants reasoned that HiCCUPS using default parameters for loop detection was missing many of these extremely long-range loops because of the extra stringency of the HiCCUPS lambda chunking procedure for multiple hypothesis testing for pixels with low counts (i.e. pixels far off the diagonal). To call more of these long-range loops, Applicants decided to modify the HiCCUPS parameters similar to make the parameters more similar to those used to identify the extremely long-range "superloops" on the inactive X chromosome (Rao et al., 2014; Darrow et al., 2016).

Applicants decided to annotate loops in auxin-treated RAD21-mAC cells with the parameters used in to annotate superloops on the inactive X chromosome (which also form between loci tens to hundreds of megabases apart). More specifically, Applicants annotated loops by running HiCCUPS at 50 and 100kb resolutions with the following parameters: p = 2,1; w = 4,2; fdr = 10%, 10%. Applicants additionally filtered loops that were within 5 Mb of the diagonal, had less than a 2-fold observed/expected for any of the local expected, and had fewer than 3 pixels clustered into the peak pixels (see section VI.a.5 of Rao et al., 2014). This annotation yielded 88 loops. After visual examination, Applicants found that 46 of these loops corresponded to true positives while the other 42 were false positives (22 were due to issues with repetitive regions and 15 were due to other forms of structure in the contact map, for instance interactions between broad compartment intervals). Combining these 46 loops with the 15 loops annotated with high resolution HiCCUPS, Applicants obtained a final curated list of 61 intrachromosomal cohesin-independent loops.

Applicants first identified the loop anchors contributing to the cohesin-independent loops. Applicants merged all adjacent loci involved in one of the 61 loops annotated above. Applicants then expanded all loop anchor loci to be 100kb in size, yielding a list of 64 loop anchor loci.

To assess the presence and orientation of CTCF at loop anchor loci for both cohesin-associated and cohesin-independent loop anchors, Applicants followed the procedure exactly from section VI.e.7 of (Rao et al., 2014). In order to use comparable loop anchor sizes, Applicants collapsed each 100kb cohesin-independent loop anchor to the 15kb interval in the center of the 100kb interval. Applicants found that while 90% of cohesin-associated loop anchors were associated with CTCF binding, only 20% of cohesin-independent loop anchors were associated with CTCF binding. More over, while 95% of unique CTCF motifs in cohesin-associated loop anchors pointed towards the interior of the loop (consistent with the convergent rule), the unique CTCF motifs in cohesin-independent loops did not exhibit any such bias (56% pointing towards the interior of the loop) (**Fig. 4C**). This strongly suggests that cohesin-independent loops form via a mechanism other than extrusion.

To analyze enrichment of proteins bound at cohesin-independent loop anchors, Applicants reproduced the analysis from section VI.e.7 of (Rao et al., 2014), using the 100kb loop anchors and comparing to the average of 100 randomly shuffled loop anchor lists (see the section on Random Shuffle controls above). Applicants downloaded peak calls for 36 DNA-binding proteins or histone modifications in HCT-116 cells from ENCODE (ENCODE Consortium, 2012). Applicants also utilized an annotation of stitched and ranked (by H3K27Ac enrichment) superenhancers and enhancers from (Hnisz et al., 2013). For each of the proteins or histone modifications, Applicants calculated the percentage of loop anchors that overlap the feature as well as the enrichment over the percentage of random anchors overlapping the feature. Applicants found that strong H3K27Ac sites and superenhancers (especially the strongest 100 superenhancers) were very strongly enriched at cohesin-independent loop anchors (**Fig. 4D**). Applicants also wondered whether broad H3K4me3 peaks were enriched at cohesin-independent loop anchors. In order to assess this, Applicants first created an annotation of broad H3K4me3 peaks. In brief, Applicants called peaks using MACS 2.0 with the '-broad' option enabled, and then merged peaks that were within 5kb of each other. Applicants then retained peaks from this merged list that were >10kb long to yield a final list of 549 broad H3K4me3 peaks. Applicants analyzed enrichment of broad H3K4me3 peaks at cohesin-independent loop anchors as Applicants did for all other protein peak calls (see above). Notably, Applicants observed that 36% (23/64) of cohesin-independent loop anchors overlap a broad H3K4me3 peak, a 21-fold enrichment over random chance.

Applicants also performed the analyses listed above on automated lists of cohesin-independent loops without any manual curation. Applicants found that the results showing a lack of CTCF binding at cohesin-independent loop anchors and a lack of CTCF orientation preference were similar (**Fig. 11A**). Applicants also found that superenhancers were strongly enriched at loop anchors generated from the 88 loop list automatedly called with low resolution HiCCUPS; the top 100 superenhancers were 47-fold enriched (present at 30/115 loop anchors). **See** **figure 11B****.** This indicates that the results were not biased by our use of a manually curated loop list.

Applicants noticed that our 64 cohesin-independent loop anchors determined from the 61 loop intrachromosomal list often formed focal interchromosomal links between pairs of loop anchors and that there were large cliques of interactions between anchors (**Fig. 4A****,E, 5A**). This is in stark contrast to cohesin-associated loop anchors, which show no such enrichment for extremely long Intrachromosomal interactions or interchromosomal interactions, either when examined individually or in aggregate via APA (**Fig 9A**). This strongly suggests that cohesin-independent loops and links form via a mechanism other than extrusion, since extrusion cannot occur on two topologically distinct molecules..

To annotate these interchromosomal links between pairs of cohesin-independent loop anchors, Applicants used HiCCUPS to calculate local enrichments at 100kb resolution for all possible interchromosomal pairs of cohesin-independent loop anchors. Applicants then identified enriched focal interchromosomal interactions by filtering for links that were enriched at least 5.5-fold over local background (empirically chosen to ensure a <10% false discovery rate). Using this procedure, Applicants identified 203 interchromosomal cohesin-independent links. This likely underestimates the true number of interchromosomal cohesin-independent links, as evidenced by **Fig. 5A****.**

Applicants analyzed the change in strength of cohesin-independent links after auxin treatment by using APA at 100kb resolution. APA analysis clearly demonstrated that while cohesin-independent links (both intra and interchromosomal) were weakly present before auxin treatment, they were ~2-fold strengthened after auxin-treatment (**Fig 11C****,D**). This result was robust to using either our manually curated lists (of 61 intra and 203 inter chromosomal links) or automatedly generated lists (all intrachromosomal pairs of the 47 superenhancers overlapping anchors in the 88-loop automated list from above and all interchromosomal pairs of the 47 superenhancers overlapping anchors in the 88-loop automated list from above) (**Fig 11C****,D**).

Applicants also analyzed induction of cohesin-independent links across an auxin treatment and withdrawal time course. This analysis was performed as above with the cohesin-associated loops and loop domains, but at 100kb resolution instead of 10kb resolution and for both our 61 intrachromosomal links and our 203 interchromosomal links. The opposite pattern of cohesin-associate loop formation was seen; APA scores for cohesin-independent links rapidly increased upon auxin treatment and rapidly dropped upon auxin withdrawal (**Fig. 4E**). Similar results were seen upon performing the time course APA at 100kb resolution using all intrachromosomal pairs of the 47 superenhancer overlapping anchors in the 88-loop automated list from above (**Fig. 11E**).

**Analysis of higher order contacts.** In order to assess whether the large cliques between superenhancers that Applicants observed after cohesin loss corresponded to higher order hubs, Applicants utilized higher order contacts present at low frequencies in *in situ* Hi-C data. The Juicer pipeline (Durand, et al, 2016b) separately outputs abnormal chimeric read pairs (i.e. read pairs that map to more than 2 loci). Applicants combined all of the data from untreated RAD21-mAC cells and all our data from treated RAD21-mAC cells (unsynchronized and synchronized/arrested), parsed the chimeric abnormal reads and deduped them (using the same deduping procedure used in Juicer, only applied to three, four or five positions, rather than just two), retaining unique reads that mapped to three or more positions with MAPQ>=10. With this procedure, Applicants obtained 32M triples, 18M quadruples, and 75K quintuples in our untreated data set, and 25M triples, 14M quadruples and 55K quintuples in our treated data set.

Given the sparsity of our higher-order data set, Applicants were unable to call individual hubs. However, Applicants reasoned that just as APA enabled us to examine the aggregate enrichment of loops in low-resolution pairwise Hi-C data sets, Applicants could perform 3D-APA to examine the aggregate enrichment of trio hubs in a low-resolution triple tensor. In order to perform 3D-APA, Applicants identified 131 intrachromosomal trios of cohesin-independent loop anchors, where each pair of loop anchors in the trio were at least 10Mb apart (Applicants merged loop anchors that were within 1 Mb of each other, in order to avoid double counting in the 3D-APA aggregate tensor). Applicants then extracted a 3.9Mb x 3.9Mb x 3.9Mb sub-tensor at 300kb resolution, centered on the 300kb x 300kb x 300kb voxel containing each trio, and summed these cubes to get an aggregate 3D-APA sub-tensor. When summing the 131 cubes, the cubes were always oriented so that the upstream locus was on the z-axis, the middle locus was on the x-axis and the downstream locus was on the y-axis.

While Applicants did not observe many quintuples, Applicants did observe high rates of quadruples, so Applicants devised several strategies to project quadruples onto triple space in order to use them along with our triples in searching for hubs. A naive strategy would be to extract all 4 choose 3 triples from each quadruple and count each one separately. However, using this method does not allow us to utilize Poisson statistics to calculate expected models, as the four triples extracted from a given quadruple are not independent. The most stringent way to handle this issue is by randomly discarding one of the four loci. However, this tends to underutilize the information contained in quadruples. For instance, if one has a quadruple ABCD, where the triple ABC falls within my 3D-APA sub-tensor but D is outside of all of the sub-tensors, using the random projection method, there is a 75% chance of completely discarding the ABCD quadruple, despite the fact that it contains some information relevant to the hypothesis being tested in 3D-APA. In order to maximize utilization of the information contained quadruples but simultaneously ensure that Poisson statistics were still applicable, Applicants developed a "Poisson-projection" method. Namely, for a quadruple ABCD, Applicants would examine all four contained triples (ABC, ABD, ACD, BCD). If one and only one fell inside our 3D-APA sub-tensor (say ABC), then Applicants would include that as a triple for further analysis. If more than one of the four fell within our 3D-APA sub-tensor, Applicants would randomly choose one of the triples that fell inside our 3D-APA sub-tensor to include in the analysis. By randomly choosing among the contained triples that fell within the bounds of our 3D-APA sub-tensor, Applicants can avoid double counting and maintain the independence of events necessary for Poisson statistics to apply.

Using the triple dataset as well as the Poisson projection of our quadruples, Applicants identified 11 contacts that fell within the center voxel of the 3D-APA sub-tensor in our auxin-treated dataset, where as Applicants saw none in our untreated dataset. No other voxel in the sub-tensor for either dataset (out of 4394 voxels) contained more than 5 reads (Fig. 5D,E). Applicants also extracted the aggregate 3D-APA sub-tensors corresponding to shifting one or more the loci in each trio by 3.9Mb. No other voxel in the sub-tensor for either dataset (out of 118,638 voxels) contained more than 8 reads (**Fig. 5F**). In order to assess the statistical significance of seeing 11 contacts in the center voxel of our 3D-APA sub-tensor after auxin treatment, Applicants also calculated a number of local expecteds (Darrow, et al 2016). The center voxel of our 3D-APA tensor after auxin treatment was strongly enriched relative to all expected models. Applicants also tested statistical significance against a local expected model that accounts for 2D bias, that is, the fact that three loci that show pairwise enrichments in the 2D matrix (i.e. they form loops) will show enrichments in the 3D tensor that corresponds simply to the product of their 2D enrichments and not to any higher order simultaneity; the 11 contacts Applicants observe in the center voxel after auxin treatment is still significantly enriched (**Fig**. **12B**, bottom model).

Taken together, these results highlight that superenhancers at cohesin-independent loop anchors interact simultaneously in higher order hubs after cohesin loss (**Fig**. **5G**).

**Simulations of extrusion and compartmentalization.** Simulations were run for 200,000 timesteps with only Lennard-Jones intermonomeric forces and then for 800,000 timesteps with 8 extrusion complexes. In the HOOMD-blue molecular dynamics package (Glaser et al., 2015) (Anderson et al., 2008; Glaser et al., 2015) (Anderson et al., 2008; Glaser et al., 2015), temperature is set to 2.0 and gamma (viscosity) is set to 0.02. Contact maps and globules are shown from the final frame of simulation. In simulations of the auxin-treated condition, the final 800,000 timesteps were simulated without extrusion. All other parameters are as described in (Sanborn et al., 2015).

CTCF and cohesin binding strengths were determined by integrating a Gaussian fit to ChIP-Seq data around every CTCF motif. Simulated extrusion binding strengths were determined by taking the geometric mean of the CTCF and cohesin binding strengths and renormalizing to a binding probability, as described in (Sanborn et al., 2015).

Each monomer was assigned to either an "A" or a "B" type. Lennard-Jones forces between different-type monomers was set to 98% the strength of LJ forces between same-type monomers. Because compartment transitions can only be defined in Hi-C maps at coarse resolutions (25kb and above), the compartment transition of each simulation replicate was varied randomly within 30kb (30 monomers) of defined transition points.

Compartment transitions were determined in one of two ways: (1) A/B compartment states were annotated by hand for the regions that were simulated using the treated Hi-C maps or (2) 9 histone modifications (H3K27me3, H3K9me3, H3K36me3, H2.AZ, H3K79me2, H4K17Ac, H3K4me1, H3K27Ac, H4K20me3) were clustered into 6 clusters using k-means clustering, the clusters were further collapsed into two clusters (A or B) based on whether each cluster had a positive enrichment for H3K36me3 (A) or not (B). This two cluster track was then used as input for simulation. For the k-means clustering, the histone modification data was first converted to a z-score value for each mark in order to account for differences in the dynamic range between marks. The latter input was used for the simulation shown in **Fig. 6A****,B,** demonstrating our ability to recapitulate all the major features of Hi-C datasets (loops, domains, and compartments) using only ChIP-Seq data as input. Both hand compartment annotation and automated compartment annotation simulations are shown in **Fig. 20** for comparison.

**Oligonucleosome Resolution Simulations of Chromatin Fibers.** Segregated interactions between short chromatin intervals, such as the cohesin-independent loop anchors Applicants observed, have not previously been reported, and require the strength of the interaction between similarly-decorated nucleosomes to overcome the stiffness of the local chromatin fiber.

To explore whether such aggregation or segregating mechanisms are physically feasible on the oligonucleosome level, Applicants simulated a coarse-grained mesoscale chromatin fiber **(****Fig. 13A****,B)** of 100 nucleosomes (~20kb) with NRL = 200bp without linker histone, consisting of either wild-type nucleosomes, nucleosomes in which the histone tails are rigid, reflecting the known effect of acetylation, as in the H4K16Ac mark, or 4 intervals of equal length, with the intervals alternating between wild-type nucleosomes and nucleosomes in which the histone tails are rigid. (The biophysical consequences of the H4K16Ac mark have been studied in detail, and are well understood in terms of more rigid tails, which in tum inhibit tail/tail intemucleosome interactions and thus disrupt crucial stabilization of condensed chromatin fiber). In brief, the mesoscale chromatin model (Bascom and Schlick, 2017) represents the DNA as coarse-grained beads (each ~9bp) using a worm-like chain model; the nucleosome core particle with the wrapped DNA but without the histone tails is treated as a rigid body with ~300 pseudocharges that mimick the electrostatic environment of the nucleosome; and the histone tails are coarse grained to beads of about 5 amino acids using united-atom polymer chain models to mimick atomistic behavior. (See full details in Bascom, Kim, and Schlick 2017, and Grigoryev et al, 2016). Monte Carlo sampling of 40 million or more steps are performed for three ensembles of 100-nucleosome systems: wildtype fiber, all-folded control, and alternating construct where the pattern of 25-wt, 25-folded tails was repeated twice. From ensembles of up to 35 trajectories, contact maps were produced, and resulting chromatin configurations analyzed.

As seen in the contact maps and corresponding images in **Fig. 13E****,** demarcated zones emerge in the ensemble of the altemating constructs: the wt nucleosomes segregate from the other nucloeosmes as well tend to associate with one another, while the folded-tail nucleosomes segregate separately. The alternating construct tends to adopt an overall figure-8 shaped fold or hierarchical loop (Grigoryev et al., 2016). The contact maps of these alternating constructs reveal these checkerboard patterns in marked contrast to the control wt and all-folded tail systems (Fig. **13C**-E). The most dense regions in the contact maps of the alternating constructs come from wt/wt local and nonlocal interactions. These are followed by the local folded/folded and wt/folded interactions.

That such a striking segregation effect arises from simple alternating fiber constructs suggests that short chromatin intervals separate in sequence can spontaneously separate in space in specific patterns due to charge effects that alter the intrinsic histone-tail flexibility of specfiic nucleosomes. Already, Applicants have shown that such domain segregation naturally emerges from nucleosome-free or depleted regions (Bascom, Kim, and Schlick 2017), but here the nucleosome spacing is uniform. Such alterations in tail flexibility can be caused by chemical modifications of the histone tails, DNA, or linker histones, as well as by protein anchoring, which can restrict the range of interactions to specific domains. It is likely that protein binding could amplify intrinsic segregation as well as induce domain aggregation of the fiber. Results with only H4 tails folded (mimicking H4K16Ac) are very similar to the case of all-folded tails (data not shown). In vivo and in vitro experiments on this length scale are also needed to probe these effects further.

**Assessment of changes in transcription after cohesin loss.** To look for signs of ectopic activation, Applicants examined the 14,853 genes that were not expressed (RPKM<0.5) in untreated cells. Applicants identified 2,145 genes that were significantly (adjusted p<0.05) changed by DESeq2. Of these genes, 1% (216) were ectopically activated after treatment (p<0.05, >30% change in RPKM, RPKM>0.5 in treated cells). In addition, 7% of these genes (1063) exhibited "leaky" transcription in treated cells: a larger PRO-Seq signal (p<0.05, >1.3 fold change difference) that fell short of the threshold for an expressed gene (i.e., RPKM was still below 0.5). 1.4% of these genes were significantly downregulated (>1.3-fold change), but it is unclear what reductions in expression at such low levels of expression mean biologically.

Applicants next looked for changes in the 12,222 genes that were expressed (RPKM>0.5) in untreated cells (**Fig**. **6B**). Applicants identified 4,196 genes that were significantly changed (adjusted p<0.05) changed by DESeq2. Here again, most genes (87%, 10,615) exhibited similar levels of transcription after cohesin degradation (RPKM changed by less than 30%). The remaining genes (13%, 1607) showed a larger transcriptional effect (p<0.05, >30% change in RPKM). Stronger effects were seen, but less frequently: 64 genes (0.5%) showed a 2-fold change, and 2 genes showed a 5-fold change (**Fig. 7B**).

Applicants identified 49 genes that were 1.75-fold downregulated with p<0.05 after auxin treatment. Applicants noticed that many of the genes that were downregulated (by >1.75-fold) were located within 500kb of superenhancers (23 of 49, 4.8-fold enrichment compared to randomly shuffling the positions of the TSS of the 49 genes across the genome, **Fig. 7C****,D).** Of these genes, 29% (14 of 49) were located with 500kb of one of the top 100 superenhancers (8.5-fold enrichment compared to randomly shuffling the positions of the TSS of the 49 genes across the genome). The overall distribution of distance to the nearest superenhancer was shifted significantly closer compared to randomly selected genes (**Fig. 7D**). Strikingly, these superenhancers were often located at the anchors of the cohesin-independent links seen in treated cells (8 of 19, a 13.7-fold enrichment).

To rule out the possibility that changes in gene expression were due to the auxin hormone itself, Applicants performed PRO-Seq on HCT-116-CMV-OsTIR1 cells (HCT-116 cells with OsTIR1 at the *AAVS1* locus but no mAID tag on any protein) before and after auxin treatment. Only 105 genes were detected as significantly different, and only 56 genes were detected as significantly different with at least a 1.3-fold change. This indicates that our results are not confounded by the auxin hormone itself.

To rule out the possibility that tagging RAD21 itself led to significant transcriptional consequences, Applicants compared our auxin-treated PRO-Seq data to a control of untreated HCT-116-CMV-OsTIR1 cells. The following paragraphs are the analyses from above except with the numbers from the CMV-OsTIR1 control. Analogous plots to those shown in **Fig. 7B** **and** **7D** for the CMV-OsTIR1 control are shown in **Fig. 14C-D**.

To look for signs of ectopic activation, Applicants examined the 14,884 genes that were not expressed (RPKM<0.5) in untreated cells. Applicants identified 2,284 genes that were significantly (adjusted p<0.05) changed by DESeq2. Of these genes, 1% (255) were ectopically activated after treatment (p<0.05, >30% change in RPKM, RPKM>0.5 in treated cells). In addition, 7% of these genes (1179) exhibited "leaky" transcription in treated cells: a larger PRO-Seq signal (p<0.05, >1.3 fold change difference) that fell short of the threshold for an expressed gene (i.e., RPKM was still below 0.5). 1.8% of these genes were strongly downregulated (>1.3-fold change), but it is unclear what reductions in expression at such low levels of expression mean biologically.

Applicants next looked for changes in the 12,191 genes that were expressed (RPKM>0.5) in untreated cells (**Fig. 5B**). Applicants identified 4,251 genes that were significantly changed (adjusted p<0.05) changed by DESeq2. Here again, most genes (85%, 10,330) exhibited similar levels of transcription after cohesin degradation (RPKM changed by less than 30%). The remaining genes (15%, 1861) showed a larger transcriptional effect (p<0.05, >30% change in RPKM). Stronger effects were seen, but less frequently: 86 genes (1%) showed a 2-fold change, and 3 genes showed a 5-fold change (**Fig. 14C**).

Applicants identified 43 genes that were 2-fold downregulated with p<0.05 after auxin treatment. Applicants noticed that many of the genes that were downregulated (by >2-fold) were located within 500kb of superenhancers (28 of 43). Of these genes, 49% (21 of 43) were located with 500kb of one of the top 100 superenhancers. The overall distribution of distance to the nearest superenhancer was shifted significantly closer compared to randomly selected genes **(****Fig. 14D****).**

Applicants previous analyses (Rao et al 2014) have suggested that a subset of cell-type specific loops is associated with very strong gene activation (>10-fold upregulation of gene expression in the cell type where the loop is present). Applicants repeated the same analysis from Rao et al (2014) with our HCT-116 untreated map from this study and our GM12878 map from our previous study in order to identify cases where cell-type specific loops appeared in HCT-116 and genes were simultaneously upregulated to then assess the effects of loop loss. To our surprise, Applicants only identified 68 cell type specific loops (compared to ~600 each per pair of cell types in Rao et al 2014). In order to increase statistical power, Applicants identified 518 loops that had been called in one of our cell types in Rao et al 2014 (HMEC, IMR90, K562, HUVEC, HeLa, NHEK) but not in GM12878, or vice versa and had been associated with 10-fold upregulation of a gene whose promoter lay at the loop anchor. Applicants sought to then identify cases where these loop-gene pairs were present in HCT-116 to then examine the results of loop loss. However, only 15 of the 518 loops were conserved in HCT-116. Of those 15 loops and the 12 genes associated with them, 9 were not expressed in HCT-116 (of the other three, two were downregulated, and one was upregulated). These data are consistent with a number of possibilities: First, it is possible that HCT-116 is somehow distinct from all of our previously examined cell types and exhibits different distal regulatory principles. Alternatively, it is possible that the tagging of cohesin and resulting potential loss of stability results in preferential loss of cell type specific loops. However, even if this were the case, the fact that Applicants do not see large-scale expression changes between our untagged HCT-116 cells (above) and the auxin-treated RAD21-mAC cells (only 3 genes with a greater than 5-fold change in expression), suggests that the association between loop appearance and strong (>10-fold) gene activation may not be such that loop formation causes gene activation. In fact, it may be the case that some other regulatory event catalyzes both loop formation and gene activation, or that gene activation itself enables loop formation.

### References

Alipour, E., and Marko, J.F. (2012). Self-organization of domain structures by DNA-loop-extruding enzymes. Nucleic Acids Res. 40, 11202-11212.

Anderson, J.A., Lorenz, C.D., and Travesset, A. (2008). General purpose molecular dynamics simulations fully implemented on graphics processing units. Journal of Computational Physics 227, 5342-5359.

Bascom, G, and Schlick, T (2017). Linking chromatin fibers to gene folding by hierarchical looping. Biophysical Journal 112: 434--445.

Bascom, G.D., Kim, T., and Schlick, T. (2017). Kilobase Pair Chromatin Fiber Contacts Promoted by Living-System-Like DNA Linker Length Distributions and Nucleosome Depletion. J Phys Chem B 121, 3882-3894.

Beagrie, R.A., Scialdone, A., Schueler, M., Kraemer, D.C., Chotalia, M., Xie, S.Q., Barbieri, M., de Santiago, I., Lavitas, L.-M.M., Branco, M.R., et al. (2017). Complex multi-enhancer contacts captured by genome architecture mapping. Nature 543, 519-524.

Darrow, E.M., Huntley, M.H., Dudchenko, O., Stamenova, E.K., Durand, N.C., Sun, Z., Huang, S.-C.C., Sanbom, A.L., Machol, I., Shamim, M., et al. (2016). Deletion of DXZ4 on the human inactive X chromosome alters higher-order genome architecture. Proc. Natl. Acad. Sci. U.S.A. 113, E4504-12.

Davidson, I.F., Goetz, D., Zaczek, M.P., Molodtsov, M.I., Huis In 't Veld, P.J., Weissmann, F., Litos, G., Cisneros, D.A., Ocampo-Hafalla, M., Ladumer, R., et al. (2016). Rapid movement and transcriptional re-localization of human cohesin on DNA. EMBO J. 35, 2671-2685.

Dixon, J.R., Selvaraj, S., Yue, F., Kim, A., Li, Y., Shen, Y., Hu, M., Liu, J.S., and Ren, B. (2012). Topological domains in mammalian genomes identified by analysis of chromatin interactions. Nature 485, 376-380.

Durand, N.C., Robinson, J.T., Shamim, M.S., Machol, I., Mesirov, J.P., Lander, E.S., and Aiden, E.L. (2016a). Juicebox Provides a Visualization System for Hi-C Contact Maps with Unlimited Zoom. Cell Syst 3, 99-101.

Durand, N.C., Shamim, M.S., Machol, I., Rao, S.S., Huntley, M.H., Lander, E.S., and Aiden, E.L. (2016b). Juicer Provides a One-Click System for Analyzing Loop-Resolution Hi-C Experiments. Cell Syst 3, 95-98.

Engreitz, J.M., Haines, J.E., Perez, E.M., Munson, G., Chen, J., Kane, M., McDonel, P.E., Guttman, M., and Lander, E.S. (2016). Local regulation of gene expression by IncRNA promoters, transcription and splicing. Nature 539, 452-455.

Flavahan, W.A., Drier, Y., Liau, B.B., Gillespie, S.M., Venteicher, A.S., Stemmer-Rachamimov, A.O., Suvà, M.L., and Bernstein, B.E. (2016). Insulator dysfunction and oncogene activation in IDH mutant gliomas. Nature 529, 110-114.

Fudenberg, G., Imakaev, M., Lu, C., Goloborodko, A., Abdennur, N., and Mimy, L.A. (2016). Formation of Chromosomal Domains by Loop Extrusion. Cell Rep 15, 2038-2049.

Glaser, J, Nguyen, TD, Anderson, JA, and Lui, P (2015). Strong scaling of general-purpose molecular dynamics simulations on GPUs. Computer Physics ....

Grigoryev, S.A., Bascom, G., Buckwalter, J.M., Schubert, M.B., Woodcock, C.L., and Schlick, T. (2016). Hierarchical looping of zigzag nucleosome chains in metaphase chromosomes. Proc. Natl. Acad. Sci. U.S.A. 113, 1238-1243.

Guo, Y., Xu, Q., Canzio, D., Shou, J., Li, J., Gorkin, D.U., Jung, I., Wu, H., Zhai, Y., Tang, Y., et al. (2015). CRISPR Inversion of CTCF Sites Alters Genome Topology and Enhancer/Promoter Function. Cell 162, 900-910.

Haarhuis, J.H.I.H., van der Weide, R.H., Blomen, V.A., Yáñez-Cuna, J.O., Amendola, M., van Ruiten, M.S., Krijger, P.H.L.H., Teunissen, H., Medema, R.H.H., van Steensel, B., et al. (2017). The Cohesin Release Factor WAPL Restricts Chromatin Loop Extension. Cell 169, 693-707.e14.

Hnisz, D., Abraham, B.J., Lee, T.I., Lau, A., Saint-André, V., Sigova, A.A., Hoke, H.A., and Young, R.A. (2013). Super-enhancers in the control of cell identity and disease. Cell 155, 934-947.

Hnisz, D., Shrinivas, K., Young, R.A., Chakraborty, A.K., and Sharp, P.A. (2017). A Phase Separation Model for Transcriptional Control. Cell 169, 13-23.

Jonkers, I., and Lis, J.T. (2015). Getting up to speed with transcription elongation by RNA polymerase II. Nat. Rev. Mol. Cell Biol. 16, 167-177.

Jost, D., Carrivain, P., Cavalli, G., and Vaillant, C. (2014). Modeling epigenome folding: formation and dynamics of topologically associated chromatin domains. Nucleic Acids Res. 42, 9553-9561.

Kagey, M.H., Newman, J.J., Bilodeau, S., Zhan, Y., Orlando, D.A., van Berkum, N.L., Ebmeier, C.C., Goossens, J., Rahl, P.B., Levine, S.S., et al. (2010). Mediator and cohesin connect gene expression and chromatin architecture. Nature 467, 430-435.

Langmead, B., and Salzberg, S.L. (2012). Fast gapped-read alignment with Bowtie 2. Nat. Methods 9, 357-359.

Larson, A.G., Elnatan, D., Keenen, M.M., Trnka, M.J., Johnston, J.B., Burlingame, A.L., Agard, D.A., Redding, S., and Narlikar, G.J. (2017). Liquid droplet formation by HP1α suggests a role for phase separation in heterochromatin. Nature 547, 236-240.

Li, H., and Durbin, R. (2010). Fast and accurate long-read alignment with Burrows-Wheeler transform. Bioinformatics 26, 589-595.

Lieberman-Aiden, E., van Berkum, N.L., Williams, L., Imakaev, M., Ragoczy, T., Telling, A., Amit, I., Lajoie, B.R., Sabo, P.J., Dorschner, M.O., et al. (2009). Comprehensive mapping of long-range interactions reveals folding principles of the human genome. Science 326, 289-293.

Liu, T. (2014). Use model-based Analysis of ChIP-Seq (MACS) to analyze short reads generated by sequencing protein-DNA interactions in embryonic stem cells. Methods Mol. Biol. 1150, 81-95.

Love, M.I., Huber, W., and Anders, S. (2014). Moderated estimation of fold change and dispersion for RNA-seq data with DESeq2. Genome Biol. 15, 550*.*

Lupiáñez, D.G.G., Kraft, K., Heinrich, V., Krawitz, P., Brancati, F., Klopocki, E., Horn, D., Kayserili, H., Opitz, J.M., Laxova, R., et al. (2015). Disruptions of topological chromatin domains cause pathogenic rewiring of gene-enhancer interactions. Cell 161, 1012-1025.

Merkenschlager, M., and Nora, E.P.P. (2016). CTCF and Cohesin in Genome Folding and Transcriptional Gene Regulation. Annu Rev Genomics Hum Genet 17, 17-43.

Nasmyth, K. (2001). Disseminating the genome: joining, resolving, and separating sister chromatids during mitosis and meiosis. Annu. Rev. Genet. 35, 673-745.

Natsume, T., Kiyomitsu, T., Saga, Y., and Kanemaki, M.T. (2016). Rapid Protein Depletion in Human Cells by Auxin-Inducible Degron Tagging with Short Homology Donors. Cell Rep 15, 210-218.

Nora, E.P.P., Lajoie, B.R., Schulz, E.G., Giorgetti, L., Okamoto, I., Servant, N., Piolot, T., van Berkum, N.L., Meisig, J., Sedat, J., et al. (2012). Spatial partitioning of the regulatory landscape of the X-inactivation centre. Nature 485, 381-385.

Nora, E.P.P., Goloborodko, A., Valton, A.-L.L., Gibcus, J.H., Uebersohn, A., Abdennur, N., Dekker, J., Mirny, L.A., and Bruneau, B.G. (2017). Targeted Degradation of CTCF Decouples Local Insulation of Chromosome Domains from Genomic Compartmentalization. Cell 169, 930-944.e22.

Parker, S.C., Stitzel, M.L., Taylor, D.L., Orozco, J.M., Erdos, M.R., Akiyama, J.A., van Bueren, K.L., Chines, P.S., Narisu, N., Black, B.L., et al. (2013). Chromatin stretch enhancer states drive cell-specific gene regulation and harbor human disease risk variants. Proc. Natl. Acad. Sci. U.S.A. 110, 17921-17926.

Di Pierro, M., Zhang, B., Aiden, E.L., Wolynes, P.G., and Onuchic, J.N.N. (2016). Transferable model for chromosome architecture. Proc. Natl. Acad. Sci. U.S.A. 113, 12168-12173.

Rao, S.S., Huntley, M.H., Durand, N.C., Stamenova, E.K., Bochkov, I.D., Robinson, J.T., Sanbom, A.L., Machol, I., Omer, A.D., Lander, E.S., et al. (2014). A 3D map of the human genome at kilobase resolution reveals principles of chromatin looping. Cell 159, 1665-1680.

Sanbom, A.L., Rao, S.S., Huang, S.-C.C., Durand, N.C., Huntley, M.H., Jewett, A.I., Bochkov, I.D., Chinnappan, D., Cutkosky, A., Li, J., et al. (2015). Chromatin extrusion explains key features of loop and domain formation in wild-type and engineered genomes. Proc. Natl. Acad. Sci. U.S.A. 112, E6456-65.

Schwarzer, W, Abdennur, N, and Goloborodko, A (2016). Two independent modes of chromosome organization are revealed by cohesin removal. bioRxiv.

Seitan, V.C., Faure, A.J., Zhan, Y., McCord, R.P., Lajoie, B.R., Ing-Simmons, E., Lenhard, B., Giorgetti, L., Heard, E., Fisher, A.G., et al. (2013). Cohesin-based chromatin interactions enable regulated gene expression within preexisting architectural compartments. Genome Res. 23, 2066-2077.

Sofueva, S., Yaffe, E., Chan, W.-C.C., Georgopoulou, D., Vietri Rudan, M., Mira-Bontenbal, H., Pollard, S.M., Schroth, G.P., Tanay, A., and Hadjur, S. (2013). Cohesin-mediated interactions organize chromosomal domain architecture. EMBO J. 32, 3119-3129.

Song, Q., and Smith, A.D. (2011). Identifying dispersed epigenomic domains from ChIP-Seq data. Bioinformatics 27, 870-871.

Splinter, E., Heath, H., Kooren, J., Palstra, R.-J., Klous, P., Grosveld, F., Galjart, N., and de Laat, W. (2006). CTCF mediates long-range chromatin looping and local histone modification in the beta-globin locus. Genes & Development 20, 2349-2354.

Stigler, J., Çamdere, G.Ö., Koshland, D.E., and Greene, E.C. (2016). Single-Molecule Imaging Reveals a Collapsed Conformational State for DNA-Bound Cohesin. Cell Rep 15, 988-998.

Strom, A.R., Emelyanov, A.V., Mir, M., Fyodorov, D.V., Darzacq, X., and Karpen, G.H. (2017). Phase separation drives heterochromatin domain formation. Nature 547, 241-245.

Wang, X, Brandão, HB, Le, T., and Laub, MT (2017). Bacillus subtilis SMC complexes juxtapose chromosome arms as they travel from origin to terminus. Science.

Wendt, K.S., Yoshida, K., Itoh, T., Bando, M., Koch, B., Schirghuber, E., Tsutsumi, S., Nagae, G., Ishihara, K., Mishiro, T., et al. (2008). Cohesin mediates transcriptional insulation by CCCTC-binding factor. Nature 451, 796-801.

Wijchers, P.J., Krijger, P.H., Geeven, G., Zhu, Y., Denker, A., Verstegen, M.J., Valdes-Quezada, C., Vermeulen, C., Janssen, M., Teunissen, H., et al. (2016). Cause and Consequence of Tethering a SubTAD to Different Nuclear Compartments. Mol. Cell 61, 461-473.

De Wit, E., Vos, E.S., Holwerda, S.J., Valdes-Quezada, C., Verstegen, M.J., Teunissen, H., Splinter, E., Wijchers, P.J., Krijger, P.H., and de Laat, W. (2015). CTCF Binding Polarity Determines Chromatin Looping. Mol. Cell 60, 676-684.

Zuin, J., Dixon, J.R., van der Reijden, M.I., Ye, Z., Kolovos, P., Brouwer, R.W.W., van de Corput, M.P.P., van de Werken, H.J., Knoch, T.A., van IJcken, W.F., et al. (2014). Cohesin and CTCF differentially affect chromatin architecture and gene expression in human cells. Proc. Natl. Acad. Sci. U.S.A. 111, 996-1001.

(2012). An integrated encyclopedia of DNA elements in the human genome. Nature 489, 57-74.

Various modifications and variations of the described methods, pharmaceutical compositions, and kits of the invention will be apparent to those skilled in the art. Although the invention has been described in connection with specific embodiments, it will be understood that it is capable of further modifications and that the invention as claimed should not be unduly limited to such specific embodiments. Indeed, various modifications of the described modes for carrying out the invention that are obvious to those skilled in the art are intended to be within the scope of the invention. This application is intended to cover any variations, uses, or adaptations of the invention following, in general, the principles of the invention and including such departures from the present disclosure come within known customary practice within the art to which the invention pertains and may be applied to the essential features herein before set forth.

## Claims

1. An *in vitro* or *ex vivo* method of reversibly eliminating chromatin loops in a cell comprising:
contacting the cell with an agent capable of reducing expression, function or activity of one or more members of the cohesin complex,
wherein the cells comprise an inducible degradation system,
further wherein the one or more members of the cohesin complex proteins are tagged with an inducible degradation molecule and the agent induces reversible degradation of the tagged protein.

2. The method of claim 1, wherein the one or more members of the cohesin complex are selected from the group consisting of Rad21, SA1/2, Smc3 and Smc1.

3. The method of claim 1 or 2, wherein the inducible degradation system is an inducible degron system, wherein the target protein is fused to an auxin-inducible degron, and wherein the agent is auxin.

4. The method of claim 1 or 2, wherein the agent is a small molecule, optionally wherein the agent comprises a degrader molecule and further optionally wherein the degrader molecule is a PROTAC molecule, or wherein the agent is a genetic modifying agent, optionally wherein the genetic modifying agent comprises a Cas13 system or RNAi.

5. The method of any of the preceding claims, further comprising measuring the rate of cohesin independent loop formation after contacting or treating with an agent capable of reducing expression, function or activity of one or more members of the cohesin complex; and/or further comprising:
withdrawing cohesin-reducing or degrading agent or ceasing causing cohesin-dependent loop domains to diminish or be eliminated, and
measuring rate of loop reforming after withdrawal.

6. The method of claim 5, further comprising performing gene expression analysis and a process that combines DNA-DNA proximity ligation and high throughput screening or *in situ* Hi-C, thereby reforming loops and observing gene expression change.

## Patentansprüche

1. In-vitro- oder Ex-vivo-Verfahren zum reversiblen Eliminieren von Chromatinschleifen in einer Zelle, umfassend:
In Kontakt bringen der Zelle mit einem Mittel, das die Expression, Funktion oder Aktivität eines oder mehrerer Mitglieder des Kohäsinkomplexes reduzieren kann,
wobei die Zellen ein induzierbares Abbau-System umfassen,
wobei ferner das eine oder die mehreren Mitglieder der Kohäsinkomplexproteine mit einem induzierbaren Abbaumolekül markiert sind und das Mittel einen reversiblen Abbau des markierten Proteins induziert.

2. Verfahren nach Anspruch 1, wobei das eine oder die mehreren Mitglieder des Kohäsinkomplexes aus der Gruppe ausgewählt sind, die aus Rad21, SA 1 /2, Smc3 und Smc1 besteht.

3. Verfahren nach Anspruch 1 oder 2, wobei das induzierbare Abbau-System ein induzierbares Degron-System ist, wobei das Zielprotein mit einem Auxin-induzierbaren Degron fusioniert ist und wobei das Mittel Auxin ist.

4. Verfahren nach Anspruch 1 oder 2, wobei der Wirkstoff ein kleines Molekül ist, wobei der Wirkstoff optional ein Degradationsmolekül umfasst und wobei das Degradationsmolekül optional ein PROTAC-Molekül ist, oder wobei der Wirkstoff ein genetischer Modifikationswirkstoff ist, wobei der genetische Modifikationswirkstoff optional ein Cas13-System oder RNAi umfasst.

5. Verfahren nach einem der vorstehenden Ansprüche, das ferner umfasst:
Messen der Geschwindigkeit der Cohesin-unabhängigen Schleifenbildung nach Kontakt oder Behandlung mit einem Mittel, das die Expression, Funktion oder Aktivität eines oder
mehrerer Mitglieder des Cohesin-Komplexes reduzieren kann; und/oder ferner umfassend: Entfernen des Cohesin-reduzierenden oder -abbauenden Mittels oder Beenden der Verringerung oder Eliminierung der Cohesin-abhängigen Schleifendomänen und Messen der Geschwindigkeit der Schleifenbildung nach dem Entfernen.

6. Verfahren nach Anspruch 5, das ferner umfasst:
Durchführen einer Genexpressionsanalyse und eines Verfahrens, das DNA-DNA-Proximity-Ligation und Hochdurchsatz-Screening oder In-situ-Hi-C kombiniert, wodurch Schleifen neu gebildet werden und Veränderungen der Genexpression beobachtet werden.

## Revendications

1. Procédé *in vitro* ou ex *vivo* d'élimination réversible de boucles de chromatine dans une cellule comprenant :
la mise en contact de la cellule avec un agent apte à réduire l'expression, la fonction ou l'activité d'un ou de plusieurs membres du complexe cohésine,
dans lequel les cellules comprennent un système de dégradation inductible,
dans lequel, en outre, les un ou plusieurs membres des protéines de complexe cohésine sont marqués par une molécule de dégradation inductible et l'agent induit une dégradation réversible de la protéine marquée.

2. Procédé selon la revendication 1, dans lequel les un ou plusieurs membres du complexe cohésine sont sélectionnés dans le groupe consistant en Rad21, SA1/2, Smc3 et Smc1.

3. Procédé selon la revendication 1 ou 2, dans lequel le système de dégradation inductible est un système de dégron inductible, dans lequel la protéine cible est fusionnée à un dégron inductible par auxine, et dans lequel l'agent est une auxine.

4. Procédé selon la revendication 1 ou 2, dans lequel l'agent est une petite molécule, éventuellement dans lequel l'agent comprend une molécule dégradante et en outre, éventuellement, dans lequel la molécule dégradante est une molécule PROTAC, ou dans lequel l'agent est un agent modificateur génétique, éventuellement dans lequel l'agent modificateur génétique comprend un système Cas13 ou un ARNi.

5. Procédé selon l'une quelconque des revendications précédentes, comprenant en outre la mesure de la vitesse de formation de boucles indépendantes de la cohésine après mise en contact ou traitement avec un agent apte à réduire l'expression, la fonction ou l'activité d'un ou de plusieurs membres du complexe cohésine ; et/ou comprenant en outre :
le retrait de l'agent réducteur de ou dégradant la cohésine ou l'interruption de la diminution ou de l'élimination provoquée des domaines de boucles dépendantes de la cohésine, et
la mesure de la vitesse de reformation des boucles après retrait.

6. Procédé selon la revendication 5, comprenant en outre la réalisation d'une analyse d'expression génique et d'un processus qui combine une ligature de proximité ADN-ADN et un criblage à haut débit ou un Hi-C *in situ,* ce qui permet de reformer les boucles et d'observer le changement d'expression génique.
